# FASCICULE DE BREVET EUROPEEN

(11) **EP 2 521 726 B1**
(45) Date de publication et mention de la délivrance du brevet: **18.12.2013**
(21) Numéro de dépôt: 11704258.0
(22) Date de dépôt: 06.01.2011
(51) Int. Cl.: C07D 471/04, A61K 31/519, A61P 9/00

(54) **DÉRIVÉS DE 5-OXO-5,8-DIHYDROPYRIDO[2,3-D]PYRIMIDINE COMME INHIBITEURS DE KINASES CAMKII POUR LE TRAITEMENT DE MALADIES CARDIOVASCULAIRES**
5-OXO-5,8-DIHYDROPYRIDO[2,3-D]PYRIMIDIN-DERIVATE ALS CAMKII-KINASEN-HEMMER ZUR BEHANDLUNG VON KARDIOVASKULÄREN ERKRANKUNGEN
5-OXO-5,8-DIHYDROPYRIDO[2,3-D]PYRIMIDINE DERIVATIVES AS CAMKII KINASES INHIBITORS FOR THE TREATMENT OF CARDIOVASCULAR DISEASES

(30) Priorité: 08.01.2010 FR 1050103
(43) Date de publication de la demande: 14.11.2012
(73) Titulaire: SANOFI, 75008 Paris (FR)
(72) Inventeur: BEAUVERGER, Philippe, F-75013 Paris (FR); BEGIS, Guillaume, F-75013 Paris (FR); BISCARRAT, Sandrine, F-75013 Paris (FR); DUCLOS, Olivier, F-75013 Paris (FR); MCCORT, Gary, F-75013 Paris (FR)
(74) Mandataire: Catillon, Marie
(86) Numéro de dépôt international: PCT/FR2011/050019
(87) Numéro de publication internationale: WO 2011/086306

(56) Documents cités:
- HUI HUANG ET AL: "Design and synthesis of a pyrido[2,3-d]pyrimidin-5-one class of anti-inflammatory FMS inhibitors", BIOORGANIC & MEDICINAL CHEMISTRY LETTERS, LNKD- DOI:10.1016/J.BMCL.2008.02.070, vol. 18, no. 7, 2008, pages 2355-2361, XP022574962, ISSN: 0960-894X
- BABU MAVUNKEL ET AL: "Pyrimidine-based inhibitors of CaMKIIdelta", BIOORGANIC & MEDICINAL CHEMISTRY LETTERS, LNKD- DOI:10.1016/J.BMCL.2008.02.056, vol. 18, no. 7, 2008, pages 2404-2408, XP022574971, ISSN: 0960-894X cité dans la demande
- RONG ZHANG ET AL: "Calmoduline kinase II inhibition protects against structural heart disease", NATURE MEDICINE, vol. 11, no. 4, 2005, pages 409-417, XP002588339, cité dans la demande

## Description

La présente invention se rapporte à des dérivés de 5-oxo-5,8-dihydro-pyrido[2, 3-d]pyrimidine, à leur préparation et à leur application en thérapeutique.

Le calcium est un élément clé de la signalisation intracellulaire conduisant à des réponses variées par exemple au niveau cardiaque et cérébral. Le calcium a aussi un rôle essentiel de second messager dans différents processus intracellulaires comme l'apoptose, la régulation du cycle cellulaire, l'expression de gènes, la signalisation hormonale et la réponse cellulaire au stress oxydatif. Pour induire ces réponses biologiques, le calcium utilise un récepteur intracellulaire ubiquitaire, la calmoduline. Le complexe calcium-calmoduline peut ensuite se lier et activer notamment la famille des « Ca2+/calmodulin-dependant protein kinases » (CaMKs) qui sont des sérine/thréonine protéines kinases.

CaMKII est un membre de la famille des CaMKs dont 4 isoformes connues (α, β, γ et δ) sont distribuées dans différents types tissulaires. Ainsi CaMKIIα et CaMKIIβ sont principalement localisées au niveau du cerveau et des muscles squelettiques alors que CaMKIIδ et CaMKIIδ sont exprimées dans de nombreux tissus et organes incluant le coeur, les poumons et les reins.

Récemment, les 4 isoformes de CaMKII (α, β, y et δ) ont été cristalisées avec des inhibiteurs compétitifs de l'ATP relativement peu sélectifs (Rellos et al. Plosbiology 2010, 8, e1000426). Comme attendu, les 4 structures sont très similaires en raison de la très grande homologie de séquence des 4 isoformes de CaMKII en particulier au niveau de la poche de liaison de l'ATP. Sur la base de ces données, on peut considérer qu'un inhibiteur se fixant au niveau de la poche de liaison de l'ATP de l'isoforme δ est également inhibiteur des isoformes α, β et γ.

De nombreux éléments supportant le rôle délétère de CaMKII dans le développement des pathologies cardiaques ont été rapportés dans la littérature :
- l'augmentation de l'expression et de l'activité de CaMKIIδ a été démontrée dans des modèles expérimentaux d'hypertrophie cardiaque et dans l'insuffisance cardiaque chez l'homme (Hagemann et al., Mol Cell Biochem 2001, 220:69-76; Boknik et al., Cardiovasc Res 2001, 51:717-728 ; Hempel et al., Basic Res Cardiol 2002;97 Suppl 1:I96-101 ; Colomer et al., Mol Endocrinol 2003, 17:183-192 ; Zhang et al., Circ Res 2003, 92:912-919 ; Currie et al., FEBS Lett 1999, 459:244-248 ; Hoch et al., Circ Res 1999, 84:713-721; Kirchhefer et al., Cardiovasc Res 1999, 42:254-261) ;
- le développement de l'hypertrophie cardiaque et de l'insuffisance cardiaque a été établi chez des souris transgéniques surexprimant CaMKIIδ au niveau cardiaque (Zhang et al., Circ Res 2003, 92:912-919 ; Zhang et al., J Biol Chem 2002, 277:1261-1267 ; Maier et al., Circ Res 2003, 92:904-911) ;
- la protection de souris contre l'infarctus du myocarde, l'arythmie cardiaque, l'hypertrophie cardiaque et l'insuffisance cardiaque par inhibition de CaMKII a été démontrée au moyen d'un inhibiteur de l'activation de CaMKII de type petite molécule (inhibiteur KN-93), d'un inhibiteur peptidique du pseudo-substrat de CaMKII (AC3-1) ou au moyen de souris présentant une délétion du gène CaMKIIδ (Zhang et al., Nat Med 2005, 11:379-380 ; Yang et al., Am J Physiol Heart Circ Physiol 2006, 291:H3065-H3075 ; Vila-Petroff et al., Cardiovasc Res. 2007, 73:689-98 ; Wu et al., Circulation 2002, 106:1288-1293 ; Khoo et al., Circulation 2006, 114:1352-1359 ; Backs et al., Proc Natl Acad Sci 2009, 106:2342-2347, Ling et al., J Clin Invest. 2009, 119:1230-40). Ces éléments confirment l'utilisation potentielle des inhibiteurs de CaMKII pour la prévention et/ou le traitement de l'infarctus du myocarde, de l'arythmie cardiaque, de l'hypertrophie cardiaque et de l'insuffisance cardiaque.

Il a par ailleurs été démontré que l'extinction de CaMKIIδ conduit à 80% de réduction de la formation de néo-intima dans un modèle de lésion de la carotide par ballonnet chez le rat (House et al., Arterioscler Thromb Vasc Biol. 2008, 28:441-7), indiquant que les inhibiteurs de CaMKII peuvent également être utilisés pour le traitement de la resténose.

CaMKII contribue de surcroit à la prolifération des cellules stellaires hépatiques (Soliman et al., Cell Calcium 2009, 45, 284-292) ainsi qu'à la prolifération des fibroblastes cardiaques et à la production de matrice extracellulaire par ces cellules (Zhang et al., J Cardiovasc Pharmacol. 2010, 55 :96-105).

Ces éléments font de CaMKII une nouvelle cible thérapeutique dans le traitement des maladies fibrotiques incluant la fibrose hépatique, la fibrose cardiaque, la fibrose pancréatique, la fibrose rénale, la fibrose pulmonaire, la fibrose cutanée, la fibrose intestinale et la fibrose oculaire.

Il a été également proposé recemment que CaMKII était impliquée dans l'apoptose de cellules endothéliales induite par un stress du réticulum endoplasmique ou de cellules neuronales induite par la 6-hydroxydopamine qui peuvent être des évenements importants respectivement dans l'athérosclérose et la maladie de Parkinson (Timmins et al., J Clin Invest. 2009, 119: 2925-2941). D'autre part, dans un modèle d'insuffisance rénale aiguë induite par un stress du réticulum endoplasmique par voie systémique chez la souris, une diminution de l'apoptose des cellules épithéliales tubulaires ainsi qu'une préservation de la fonction rénale ont été observées dans le cas des souris CaMKIIγ KO (Timmins et al., J Clin Invest. 2009, 119 :2925-2941) suggérant un application possible d'un inhibiteur de CaMKII dans le traitement de pathologies rénales en particulier l'insuffisance rénale aiguë.
Par ailleurs un effet neuroprotecteur par un inhibiteur peptidique de l'activité « autonome » de CaMKII, en l'occurrence le CN21, a été récemment rapporté (Vest et al. J Biol Chem 2010, 285 : 20675-20682). Ce type d'inhibition de CaMKII peut offrir une nouvelle approche thérapeutique pour la neuroprotection après un accident vasculaire cérébral.

De plus dans un modèle murin de douleur d'origine inflammatoire, Il a été montré que l'inhibition de l'activité de CaMKII par la trifluoperazine permettait la réversion dose-dépendante d'une allodynie d'origine mécanique et d'une hyperalgésie d'origine thermique (Luo et al. J Pharm Exp Ther 2008 325 : 267-275). Ces résultats suggèrent que des inhibiteurs de CaMKII pourraient permettre de traiter la douleur chronique.

Il a été également rapporté que des inhibiteurs de CaMKII en particulier le SMP-114 permettaient l'inhibition de l'expression de HIF-1α et l'inhibition significative de la production de VEGF dans des macrophages Westra et al. BMC Musculoskeletal Disorders 2010, 11 :61-72). Dans ce cas l'activité inhibitrice de CaMKII pourrait être en partie responsable de l'effet anti-athritique du SMP-114 et plus généralement on peut penser qu'un inhibiteur de CaMKII pourrait permettre de traiter l'arthrite rhumatoïde.

Un certain nombre d'inhibiteurs compétitifs de CaMKII sont bien connus dans la littérature comme le KN-93 et le peptide AIP (autocamtide-2-related inhibitory peptide). Des dérivés d'isoxazoles ont été également décrits comme inhibiteurs de CaMKII (EP1815867) et plus récemment des peptides comme le CN21 ont été divulgués comme inhibiteurs de l'activité autonome de CaMKII (WO2009/042906).

D'autres inhibiteurs compétitifs de type aryl-indolyl maléimide (Levy et al. Bioorg Med Chem Letters 2008, 18 :2390-2394, 2395-2398 et 2399-2403) ont été décrits dans la littérature. Des inhibiteurs non compétitifs possédant un motif pyrimidine sont également connus (Mavunkel et al. Bioorg Med Chem Letters 2008, 18 :2404-2408).

La présente invention a pour objet de nouveaux composés inhibiteurs de CaMKII répondant à la formule (I) : 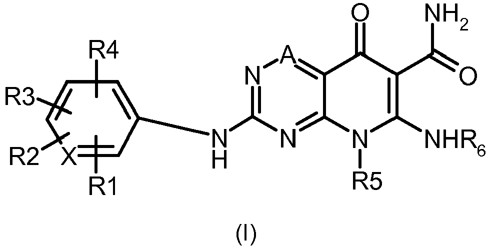 dans laquelle :
➢ **A** représente CH ou C(alkyle);
➢ **X** représente CH, C(alkyle) ou N ;
➢ **R1, R2**, **R3** et **R4**, identiques ou différents, représentent chacun indépendamment les uns des autres :
   - un atome d'hydrogène ;
   - un alkyle linéaire, ramifié ou cyclique, éventuellement substitué par un ou plusieurs :
      ○ atomes d'halogène;
      ○ -OR₉ ;
      ○ -NR₉R'₉;
      ○ -CN ;
      ○ -C(O)OR₉;
      ○ -C(O)NR₉R_{9'};
      ○ -S(O)ₚR₁₀;
      ○ -S(O)₂NR₉R'₉ ;
      où **R₉, R'₉, R₁₀ et p** sont tels que définis ci-dessous
   - un groupe -S(O)ₚR₁₀ où p et R₁₀ sont tels que definis ci-dessous;
   - un groupe -OR₁₀ où R₁₀ est tel que defini ci-dessous;
   - un atome d'halogène ;
   - un groupe -N(R₁₁)C(O)R₁₂, où
      (i) **R₁₁ et R₁₂** représentent, indépendamment l'un de l'autre, un atome d'hydrogène ou un alkyle, linéaire, ramifié ou cyclique, éventuellement substitué par un ou plusieurs substituants choisis parmi les atomes d'halogène, les groupes -OR₉ et les groupes -NR₉R'₉, ou
      (ii) **R₁₁ et R₁₂** forment ensemble avec les atomes auxquels ils sont rattachés un hétérocycloalkyle, de manière à former un lactame;
   - un groupe -N(R₁₄)-CH₂-C(O)NR₁₅R₉, où **R₁₄ et R₁₅** forment ensemble avec les atomes auxquels ils sont rattachés un hétérocycloalkyle, de manière à former une pipérazinone et où **R₉** est tel que défini ci-dessous ;
   - un groupe -C(O)NR₁₆R₁₇ avec **R₁₆ et R₁₇** forment ensemble avec l'atome d'azote auquel ils sont rattachés un hétérocycloalkyle
   - un groupement **-T-U,** où :
      **■ T** représente :
         ○ une liaison simple,
         ○ un groupe alkylène, linéaire ou ramifié ;
         ○ un groupe -C(O)-,
         ○ un groupe -S(O)p- où p est tel que défini ci-dessous, ou
         ○ un groupe -O-(CH₂)ₙ - où n est tel que defini ci-dessous,
            avec **U** représentant un hétérocycle comprenant un ou plusieurs hétéroatomes choisi parmi N, O et S(O)p, où p est tel que défini ci-dessous, ledit hétérocycle étant saturé, insaturé ou aromatique, éventuellement mono- ou di- ou poly-substitué par un, deux ou plusieurs substituant(s) choisi(s) parmi :
            ❖ les groupes -OR₇, où R₇ est tel que défini ci-dessous,
            ❖ les atomes d'halogène,
            ❖ les groupes -C(O)R₇ où R₇ est tel que défini ci-dessous,
            ❖ les alkyles linéaires, ramifiés ou cycliques, éventuellement substitués par un ou plusieurs substituants choisis parmi les atomes d'halogène, les groupes -OR₁₀, les groupes -NR₉R'₉ et le groupe -CN, où R₉, R'₉ et R₁₀ sont tels que définis ci-dessous; et
            ❖ les hétérocycles saturés, insaturés ou aromatiques, éventuellement substitué par un ou plusieurs substituants choisis parmi les atomes d'halogène, les groupes -OR₉, les groupes -NR₉R'₉ et les groupes alkyles, lesdits groupes alkyles étant éventuellement substitués par un ou plusieurs atomes d'halogène ;
               étant entendu que lorsque **U** est un groupe hétérocycloalkyle comprenant au moins un atome d'azote, ledit substituant est avantageusement choisi parmi :
            ❖ les groupes -C(O)R₇ où R₇ est tel que défini ci-dessous ; et
            ❖ les alkyles linéaires, ramifiés ou cycliques, éventuellement substitués par un ou plusieurs substituants choisis parmi les atomes d'halogène, les groupes -OR₁₀, les groupes -NR₉R'₉ et le groupe -CN, où R₉, R'₉ et R₁₀ sont tels que définis ci-dessous;
            et ledit substituant étant avantageusement porté par ledit atome d'azote, ou
      ■ **T** représente :
         ○ un groupe -C(O)- ;
         ○ un groupe -S(O)₂- ; ou
         ○ un groupe -O-(C2-C3)alkylène- ;
         avec **U** représentant un groupe -NR₉R'₉ où R₉ et R'₉ sont tels que définis ci-dessous ; ou
      ■ **T** représente :
         ○ un groupe -C(O)- ; ou
         ○ un groupe -O-(C2-C3)alkylène-;
         lorsque **U** représente un groupe -OR₉, où R₉ est tel que défini ci-dessous ; ou
      ■ **T** représente :
         ○ un groupe alkylène, linéaire ou ramifié; ou
         ○ un groupe -O-(C2-C3)alkylène;
   avec **U** représentant un groupe -NR₈R₉ où R₉ et R₈ sont tels que définis ci-dessous;
ou bien deux groupements adjacents choisis parmi **R1, R2, R3 et R4** sont liés et forment avec les deux carbones qui les portent un hétérocycle saturé, insaturé ou aromatique, éventuellement substitué par un ou plusieurs groupe(s) alkyle(s), linéaire(s), ramifié(s) ou cyclique(s), le(s)dits groupes alkyle(s) étant éventuellement substitué(s) par un ou plusieurs substituants choisis parmi les atomes d'halogène, les groupes -OR₁₀, et les groupes -NR₉R'₉, où R₉, R'₉ et R₁₀ sont tels que définis ci-dessous, ledit hétérocycle étant fusionné avec le cycle aromatique,
➢ **R₅** représente :
   - un alkyle, linéaire ou ramifié, éventuellement substitué par un ou plusieurs substituants choisis parmi les atomes d'halogène, les groupes -OR₉, les groupes -NR₉R'₉, le groupe -CN, les groupes -C(O)NR₉R_{9'}, les groupes - S(O)ₚR₁₀ et les groupes cycloalkyle éventuellement substitués par un groupe - NR₉R'₉, où R₉, R'₉, R₁₀ et p sont tels que définis ci-dessous,
   - un groupe cycloalkyle, éventuellement substitué par un groupe -NR₉R'₉, où R₉ et R'₉ sont tels que définis ci-dessous,
   - un groupe alcoxy -OR₉, où R₉ est tel que défini ci-dessous,
   - un aryle éventuellement substitué par un ou plusieurs substituants choisis parmi les groupes alkyle (C1-C3), les atomes d'halogène et les groupes -O-(C1-C3) alkyle, ou
   - un groupe -(CH₂)ₜ-R₁₃, où **R₁₃** et **t** sont tels que définis ci-dessous,
➢ **R₆** représente un atome d'hydrogène ou un alkyle linéaire, ramifié ou cyclique, et où :
➢ **R₇** représente un atome d'hydrogène ou un alkyle linéaire, ramifié ou cyclique, éventuellement substitué par un ou plusieurs substituants choisis parmi les atomes d'halogène, les groupes -OR₉ et les groupes -NR₉R'₉ avec R₉ et R'₉ tels que définis ci-dessus;
➢ **R₈** représente un groupe hétéroaryle, avantageusement une pyridine ;
➢ **R₉ et R'₉** représentent, indépendamment l'un de l'autre, un atome d'hydrogène ou un alkyle linéaire, ramifié ou cyclique ;
➢ **R₁₀** représente un atome d'hydrogène ou un alkyle linéaire, ramifié ou cyclique éventuellement substitué par un ou plusieurs atomes d'halogène,
➢ **R₁₃** représente un hétéroaryle ou un hétérocycloalkyle éventuellement substitué par un ou plusieur(s) substituant(s) choisi(s) parmi les alkyles linéaires, ramifiés ou cycliques, étant entendu que lorsque ledit hétérocycloalkyle comprend au moins un atome d'azote, ce dernier peut éventuellement porter ledit substituant,
➢ **t** représente 1 ou 2,
➢ **n** représente 0, 1, 2 ou 3, et
➢ **p** représente 0, 1 ou 2,
sous forme d'acide, de base ou de sel d'addition à un acide ou à une base, ainsi que sous forme d'hydrate ou de solvat.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, et sauf mention différente dans le texte, on entend par :
➢ un atome d'halogène : un atome de fluor, de chlore, de brome ou d'iode ;
➢ un groupe alkyle : un groupe aliphatique saturé, linéaire ou ramifié, pouvant comporter 1, 2, 3, 4, 5 ou 6 atomes de carbone (abrégé par -(C1-C6)alkyle). A titre d'exemples, on peut citer comme (i) groupe -C1alkyle, le groupe méthyle, comme (ii) groupe -C2alkyle, le groupe éthyle, comme (iii) groupe -C3alkyle, le groupe propyle, isopropyle, comme (iv) groupe -C4alkyle, le groupe butyle, isobutyle, tertbutyle, comme (v) groupe -C5alkyle le groupe pentyle, isopentyle;
➢ un groupe alkylène : un groupe alkyle tel que précédemment défini, divalent saturé, linéaire ou ramifié, pouvant comporter 1, 2, 3, 4 ou 5 atomes de carbone (abrégé -(C1-C5)alkylène-). A titre d'exemple, on peut citer les radicaux méthylène (ou -CH2-), éthylène (ou -CH2-CH2-), propylène (-CH2-CH2-CH2- ou -C(CH3)₂-) ;
➢ un groupe cycloalkyle : un groupe alkyle cyclique pouvant comporter 3, 4, 5 ou 6 atomes de carbone, abrégé également -(C3-C6)cycloalkyle et dans lequel tous les atomes de carbone sont engagés dans le cycle. A titre d'exemples, on peut citer les groupes cyclopropyle, cyclobutyle, cyclopentyle, cyclohexyle ;
➢ un groupe alcoxy : un radical -O-alkyle où le groupe alkyle est tel que précédemment défini. A titre d'exemples, on peut citer les groupes -O-(C1-C5)alkyle ou -(C1-C5)alcoxy, et en particulier comme (i) groupe -O-C1alkyle, le groupe -Ométhyle, comme (ii) groupe -O-C2alkyle, le groupe -Oéthyle, comme (iii) groupe -O-C3alkyle, le groupe -Opropyle, -Oisopropyle, comme (iv) groupe -O-C4alkyle, le groupe -Obutyle, - Oisobutyle, -Otertbutyle, comme (v) groupe -O-C5alky le groupe -Opentyle, - Oisopentyle, -Oneopentyle;
➢ un groupe aryle : un groupe aromatique mono ou bicyclique comprenant entre 5 et 10 atomes de carbone, avantageusement entre 5 et 6 atomes de carbone. A titre d'exemples de groupes aryles, on peut citer le groupe phényle et le groupe naphtyle ;
➢ un groupe hétérocycle : un groupe mono ou bicyclique comprenant de 3 à 10 chaînons, comprenant un ou plusieurs hétéroatomes choisis parmi O, N ou S.

Ledit hétérocycle peut être saturé ou partiellement insaturé et comprendre une ou plusieurs doubles liaisons. On parle alors de groupe hétérocycloalkyle. A titre d'hétérocycle non aromatique ou hétérocycloalkyle, on peut citer les lactames, pipérazinone, l'époxyéthyle, l'oxiranyle, l'aziridinyle, le tétrahydrofuranyle, le dioxolanyle, le pyrrolidinyle, le pipéridinyle, le pyrazolidinyle, l'imidazolidinyle, le tétrahydrothiophényle, le dithiolanyle, le thiazolidinyle, le tétrahydropyranyle, le dioxanyle, le morpholinyle, le pipéridyle, le pipérazinyle, le tétrahydrothiopyranyle, le dithianyle, le thiomorpholinyle, le dihydrofuranyle, le 2-imidazolinyle, le 2,-3-pyrrolinyle, le pyrazolinyle, le dihydrothiophényle, le dihydropyranyle, le pyranyle, le tétrahydropyridyle, le dihydropyridyle, le tétrahydropyrinidinyle, le dihydrothiopyranyle, et les groupes correspondants issus de la fusion avec un noyau phényle, et plus particulièrement les cycles morpholinyle, dioxalanyle, benzothiazolidinyle, pyrrolidinyle, benzopyrrolidinyle.

Avantageusement, un hétérocycloalkyle est un groupe alkyle cyclique éventuellement ponté comprenant 4, 5, 6 ou 7 atomes de carbone et comprenant 1, 2 ou 3 hétéroatomes choisi(s) parmi l'oxygène, l'azote et le soufre, tels que par exemple les groupes pipéridinyle, pipérazinyle, pyrrolidinyle, hexaméthylèneimino, morpholinyle, 1,1-dioxydotetrahydrothiényle;

Ledit hétérocycle peut également être aromatique, comprenant de 5 à 10 chaînons et représenter alors un groupe hétéroaryle.

A titre d'hétéroaryle, on peut notamment citer les groupes représentatifs suivants benzimidazolyle, benzothiazolyle, furyle, imidazolyle, indolyle, indolizinyle, isoxazolyle, isoquinolinyle, isothiazolyle, oxadiazolyle, pyrazinyle, pyridazinyle, pyrazolyle, pyridynile, pyrimidinyle, pyrrolyle, quinazolinyle, quinolinyle, 1,3, 4-thiadiazolyle, thiazolyle, thienyle et triazolyle.

Avantageusement, un groupe hétéroaryle est un groupe aromatique cyclique comprenant 2, 3, 4 ou 5 atomes de carbone et comprenant 1, 2 ou 3 hétéroatomes, qui peu(ven)t être choisi(s) parmi l'atome d'azote, l'atome d'oxygène et l'atome de soufre, indépendemment l'un de l'autre, de manière à être identiques ou différents, lorsqu'ils sont au nombre de 2 ou indépendemment les uns des autres, de manière à être identiques ou différents, lorsqu'ils sont au nombre de 3. On peut notamment citer par exemple les groupes pyridynile, pyrrole, furanyle.

Lorsque l'hétérocycle est substitué, le ou les substituant(s) peuvent se trouver sur un ou des atome(s) de carbone compris dans ledit hétérocyle et/ou, le cas échéant sur le ou les hétéroatome(s) dudit hétérocycle. Lorsque l'hétérocycle comporte plusieurs substituants, ceux-ci peuvent être portés des atomes différents ou le cas échéant, par un même atome,
➢ un groupe protecteur Pg : un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York),
➢ un groupe partant : un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, p. 310-316.

Parmi les composés de formule (I) objets de l'invention, un premier groupe de composés est constitué par les composés pour lesquels :
**A** représente CH ou C(CH₃), avantageusement **A** représente CH ; et/ou
**X** représente CH ou N, avantageusement X représente CH ; et/ou
**R₁, R₂, R₃ et R₄,** identiques ou différents, représentent chacun indépendamment les uns des autres :
   - un atome d'hydrogène ;
   - un alkyle linéaire, ramifié ou cyclique, éventuellement substitué par un ou plusieurs :
      ○ atomes d'halogène ;
      ○ -OR₉ ;
      ○ -NR₉R'₉;
      ○ -CN ;
      ○ -C(O)OR_{9;}
      ○ -C(O)NR₉R_{9' ;}
      ○ -S(O)ₚR_{10;}
      ○ -S(O)₂NR₉R'₉ ;
   - un groupe -S(O)ₚR₁₀;
   - un groupe -OR₁₀;
   - un atome d'halogène ;
   - un groupe -N(R₁₁)C(O)R_{12,}
   - un groupe -N(R₁₄)-CH₂-C(O)NR₁₅R₉, où **R₁₄ et R₁₅** forment ensemble avec les atomes auxquels ils sont rattachés un hétérocycloalkyle, de manière à former une pipérazinone et où **R₉** est tel que défini ci-dessous ;
   - un groupe -C(O)NR₁₆R₁₇ avec **R₁₆** et **R₁₇** forment ensemble avec l'atome d'azote auquel ils sont rattachés un hétérocycloalkyle, de manière à former avantageusement une pipéridinyle ou une pyrrolidinyle,
   - un groupement **-T-U,** où :
      **■ T** représente :
         ○ une liaison simple,
         ○ un groupe alkylène, linéaire ou ramifié ;
         ○ un groupe -C(O)-,
         ○ un groupe -S(O)p- où p est tel que défini ci-dessous, ou
         ○ un groupe -O-(CH₂)ₙ - où n est tel que defini ci-dessous,
            avec **U** représentant un hétérocycle comprenant un ou plusieurs hétéroatomes choisi parmi N, O et S(O)p, où p est tel que défini ci-dessous, ledit hétérocycle étant saturé, insaturé ou aromatique, éventuellement mono- ou di- ou poly-substitué par un, deux ou plusieurs substituant(s) choisi(s) parmi :
            ❖ les groupes -OR₇,
            ❖ les atomes d'halogène,
            ❖ les groupes -C(O)R₇,
            ❖ les alkyles linéaires, ramifiés ou cycliques, éventuellement substitués par un ou plusieurs substituants choisis parmi les atomes d'halogène, les groupes -OR₁₀, les groupes -NR₉R'₉ et le groupe -CN;
            ❖ les hétérocycles saturés, insaturés ou aromatiques, éventuellement substitué par un ou plusieurs substituants choisis parmi les atomes d'halogène, les groupes -OR₉ , les groupes -NR₉R'₉ et les groupes alkyles, lesdits groupes alkyles étant éventuellement substitués par un ou plusieurs atomes d'halogène ;
               étant entendu que lorsque **U** est un groupe hétérocycloalkyle comprenant au moins un atome d'azote, ledit substituant est avantageusement choisi parmi :
            ❖ un groupe -C(O)R₇; et
            ❖ les alkyle linéaires, ramifiés ou cycliques, éventuellement substitués par un ou plusieurs substituant(s) choisi(s) parmi les atomes d'halogène, les groupes -OR₁₀, les groupes - NR₉R'₉ et le groupe -CN;
            et ledit substituant étant avantageusement porté par ledit atome d'azote, ou
      ■ **T** représente :
         ○ un groupe -C(O)- ;
         ○ un groupe -S(O)₂- ; ou
         ○ un groupe -O-(C2-C3)alkylène-;
         avec **U** représentant un groupe -NR₉R'₉ ;
         ou
      ■ **T** représente :
         ○ un groupe -C(O)- ; ou
         ○ un groupe -O-(C2-C3)alkylène-;
         avec **U** représentant un groupe -OR₉;
         ou
      ■ **T** représente :
         ○ un groupe alkylène, linéaire ou ramifié; ou
         ○ un groupe -O-(C2-C3)alkylène-;
      avec **U** représentant un groupe -NR₈R₉; ou bien deux groupements adjacents choisis parmi **R1, R2, R3 et R4** sont liés et forment avec les deux carbones qui les portent un hétérocycle saturé, insaturé ou aromatique, éventuellement substitué par un ou plusieurs groupes alkyles, linéaires, ramifiés ou cycliques, le(s)dits groupes alkyles étant éventuellement substitué(s) par un ou plusieurs substituants choisis parmi les atomes d'halogène, les groupes -OR₁₀, et les groupes -NR₉R'₉,
   et/ou
**R₅** représente :
   - un alkyle, linéaire ou ramifié, éventuellement substitué par un ou plusieurs substituants choisis parmi les atomes d'halogène, les groupes -OR₉ , les groupes -NR₉R'₉, le groupe -CN, les groupes -C(O)NR₉R_{9'}, les groupes - S(O)ₚR₁₀ et les groupes cycloalkyle éventuellement substitués par un groupe - NR₉R'₉,
   - un groupe cycloalkyle, éventuellement substitué par un groupe -NR₉R'₉,
   - un groupe alcoxy -OR₉,
   - un aryle éventuellement substitué par un ou plusieurs substituants choisis parmi les groupes alkyle (C1-C3), les atomes d'halogène et les groupes -O-(C1-C3) alkyle, ou
   - un groupe -(CH₂)ₜ-R₁₃,
   avantageusement **R₅** représente (i) un alkyle, linéaire ou ramifié, éventuellement substitué par un ou plusieurs substituants choisis parmi les atomes d'halogène, les groupes -OR₉, les groupes -NR₉R'₉ et les groupes cycloalkyles, (ii) un groupe cycloalkyle (iii), un aryle éventuellement substitué par un ou plusieurs substituants choisis parmi les groupes alkyle (C1-C3), les atomes d'halogène et les groupes - O-(C1-C3) alkyle,ou (iv) un groupe un groupe -(CH₂)ₜ-R₁₃,
   et/ou
**R₆** représente un atome d'hydrogène ou un alkyle linéaire, ramifié ou cyclique, avantageusement **R₆** représente un atome d'hydrogène,
   et/ou
**R₇** représente un atome d'hydrogène ou un alkyle linéaire, ramifié ou cyclique, éventuellement substitué par un ou plusieur(s) substituant(s) choisi(s) parmi les atomes d'halogène, les groupes -OR₉ et les groupes -NR₉R'₉;
   et/ou
**R₈** représente un groupe hétéroaryle, avantageusement une pyridine ;
   et/ou
**R₉ et R'₉** représentent, indépendamment l'un de l'autre, un atome d'hydrogène ou un alkyle linéaire, ramifié ou cyclique ;
   et/ou
**R₁₀** représente un atome d'hydrogène ou un alkyle linéaire, ramifié ou cyclique éventuellement substitué par un ou plusieurs atomes d'halogène,
   et/ou
**R₁₁ et R₁₂** représentent, indépendamment l'un de l'autre, un atome d'hydrogène ou un alkyle, linéaire, ramifié ou cyclique, éventuellement substitué par un ou plusieurs substituant(s) choisi(s) parmi les atomes d'halogène, les groupes -OR₉ et les groupes-NR₉R'₉, ou **R₁₁ et R₁₂** forment ensemble avec les atomes auxquels ils sont rattachés un hétérocycloalkyle, de manière à former un lactame;
   et/ou
**R₁₃** représente un hétéroaryle ou un hétérocycloalkyle éventuellement substitué par un ou plusieurs substituants choisis parmi les alkyles linéaires, ramifiés ou cycliques, étant entendu que lorsque ledit hétérocycloalkyle comprend au moins un atome d'azote, ce dernier peut éventuellement porter ledit substituant, avantageusement **R₁₃** est un tétrahydrofuranyle,
   et/ou
**t** représente 1 ou 2,
   et/ou
**n** représente 0, 1, 2 ou 3,
   et/ou
**p** représente 0, 1 ou 2
   et/ou
sous forme d'acide, de base ou de sel d'addition à un acide ou à une base, ainsi que sous forme d'hydrate ou de solvat.

Parmi les composés de formule (I) objets de l'invention, un second groupe de composés est constitué par les composés pour lesquels :
➢ **A** représente CH,
➢ **X** représente CH, C(alkyle) ou N, avantageusement **X** représente CH,
➢ **R₁, R₂, R₃ et R₄,** identiques ou différents, représentent chacun indépendamment les uns des autres :
   ○ un atome d'hydrogène,
   ○ un alkyle linéaire, ramifié ou cyclique, éventuellement substitué par un ou plusieurs atomes d'halogène, un groupe -OR₉ ou -NR₉R'₉, où R₉ et R'₉ représentent indépendamment l'un de l'autre un atome d'hydrogène ou un alkyle linéaire, ramifié ou cyclique,
   ○ un groupe -S(O)ₚR₁₀ ou un groupe -OR₁₀, où R₁₀ représente un atome d'hydrogène ou un alkyle linéaire, ramifié ou cyclique éventuellement substitué par un ou plusieurs atomes d'halogène, et p représente 0, 1 ou 2,
   ○ un atome d'halogène,
   ○ un groupe -N(R₁₁)C(O)R₁₂, où R₁₁ et R₁₂ représentent, indépendamment l'un de l'autre, un atome d'hydrogène ou un alkyle, linéaire, ramifié ou cyclique ou R₁₁ et R₁₂ forment ensemble avec les atomes auxquels ils sont rattachés un hétérocycloalkyle, de façon à former un lactame ;
   ○ un groupement -T-U, où
      ■ T représente :
         - une liaison simple,
         - un groupe alkylène, linéaire ou ramifié,
         - un groupe -C(O)-,
         - un groupe -S(O)p, où p représente 0, 1 ou 2,
         - Un groupe -O-(CH₂)ₙ- où n représente 0, 1, 2 ou 3,
            et **U** représente un hétérocycle comprenant un ou plusieurs hétéroatomes choisi parmi N, O et S(O)p où p représente 0, 1 ou 2, ledit hétérocycle étant de formule : 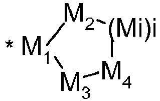
         Où
         - * représente la position d'attachement de U à T ;
         - **M₁** représente un atome de C ou N ;
         - **M₂** et **M₃** identiques ou différents représentent un atome de C, N, O ou S(O)p où p=0, 1 ou 2 ;
         - **M₄** représente un atome de C, C(=O), N, O ou S(O)p où p=0, 1 ou 2;
            Chacun des **Mᵢ,** identiques ou différents, représentent un atome de C, C(=O), N, O ou S(O)p où p=0, 1 ou 2 ;
         - i=0, 1, 2 ou 3 ;
            étant entendu que chacun des M₁, M₂, M₃, M₄ ou Mᵢ peut être éventuellement substitué si une valence est disponible et/ou les M₁, M₂, M₃, M₄ ou Mᵢ adjacents peuvent être liés par une double liaison le cas échéant ;
         ledit hétérocycle **U** étant saturé, insaturé ou aromatique éventuellement mono- ou di- ou poly-substitué par un, deux ou plusieurs substituant(s) choisi(s) parmi :

   ○ les groupes -OR₇, où R₇ représente un atome d'hydrogène ou un alkyle linéaire, ramifié ou cyclique,
   ○ les atomes d'halogène,
   ○ les groupes -COR₇ où R₇ représente un atome d'hydrogène ou un alkyle linéaire, ramifié ou cyclique,
   ○ les alkyles linéaires, ramifiés ou cycliques, avantageusement un méthyle, un éthyle ou un cyclopropyle, éventuellement substitués par un ou plusieurs atome(s) d'halogène,
   ○ les hétérocycles saturés, insaturés ou aromatiques, notamment un hétérocycle comprenant un N, avantageusement un morpholinyle, un pyrrolidinyle ou un pipéridinyle, éventuellement substitués par un ou plusieurs groupements choisis parmi les atomes d'halogène et les groupes alkyles éventuellement substitués par un ou plusieurs atomes d'halogène;
   ou bien deux groupements adjacents parmi R₁, R₂, R₃ et R₄ sont liés et forment avec les deux carbones qui les portent un hétérocycle saturé, insaturé ou aromatique, éventuellement substitué par un ou plusieurs groupes alkyle, linéaire, ramifié ou cyclique, lesdits groupes alkyle étant éventuellement substitués par au moins un groupe choisi parmi les groupes -NR₉R'₉, où R₉ et R'₉ représentent indépendamment l'un de l'autre un atome d'hydrogène ou un groupe alkyle, ledit hétérocycle étant fusionné avec le cycle aromatique,
➢ R₅ représente (i) un alkyle linéaire ou ramifié, éventuellement substitué par un ou plusieurs substituants choisis parmi les atomes d'halogène, les groupes cycloalkyles, les groupes -NR₉R'₉, où R₉ et R'₉ représentent indépendamment l'un de l'autre un atome d'hydrogène ou un groupe alkyle et les groupes -OR₉, où R₉ représente un atome d'hydrogène ou un groupe alkyle, (ii) un aryle éventuellement substitué par un ou plusieurs substituants choisis parmi les groupes alkyle (C1-C3), les atomes d'halogène et les groupes -O-(C1-C3) alkyle, et
➢ **R₆** représente un atome d'hydrogène ou un alkyle linéaire, ramifié ou cyclique, sous forme d'acide, de base ou de sel d'addition à un acide ou à une base, ainsi que sous forme d'hydrate ou de solvat.

Parmi les composés de formule (I) objets de l'invention, un autre groupe de composés est constitué par les composés répondant à la formule (I') suivante : 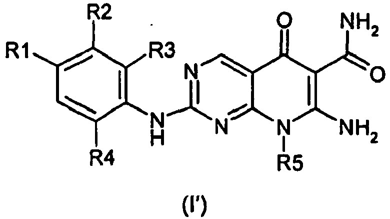 dans laquelle :
➢ R₁ représente:
   - un atome d'hydrogène,
   - un alkyle linéaire, ramifié ou cyclique, éventuellement substitué par un ou plusieurs substituants choisis parmi les atomes d'halogène, les groupes -NR₉R'₉, où R₉ et R'₉ représentent indépendamment l'un de l'autre un atome d'hydrogène ou un groupe alkyle, avantageusement un méthyle,
   - -OR₁₀, où R₁₀ représente un atome d'hydrogène ou un alkyle linéaire, ramifié ou cyclique éventuellement substitué par un ou plusieurs atomes d'halogène,
   - un atome d'halogène,
   - un groupement -T-U, où
      T représente :
      ○ une liaison simple,
      ○ un groupe groupe alkylène, avantageusement un groupe - (CH₂)₁₋₃-,
      ○ un groupe- C(O)-,
      ○ un groupe -O-(CH₂)ₙ où n représente 0, 1, 2 ou 3,
         et **U** représente un hétérocycle saturé, insaturé ou aromatique, éventuellement mono- ou di-substitué par un substituant, avantageusement choisi parmi :
      ○ les atomes d'halogène,
      ○ les groupes -COR₇ où R₇ représente atome d'hydrogène ou un alkyle linéaire, ramifié ou cyclique, avantageusement un méthyle,
      ○ les alkyles linéaires, ramifiés ou cycliques, avantageusement un méthyle, un éthyle ou un cyclopropyle, ledit alkyle étant éventuellement substitué par un ou plusieurs halogène,
      ○ les hétérocycles, avantageusement saturés comprenant un atome d'azote, avantageusement un morpholinyle, un pyrrolidinyle ou un pipéridinyle, ledit hétérocycle étant éventuellement substitué par un ou plusieurs groupement choisis parmi les atomes d'halogène ;
**➢ R2, R3 et R4** identiques ou différents représentent indépendamment :
   ○ un atome d'hydrogène,
   ○ un alkyle linéaire, ramifié ou cyclique, éventuellement substitué par un ou plusieurs substituants choisis parmi les atomes d'halogène et les groupes -NR₉R'₉, où R₉ et R'₉ représentent indépendamment l'un de l'autre un atome d'hydrogène ou un alkyle, avantageusement un méthyle,
   ○ un groupe -OR₁₀, où R₁₀ représente un atome d'hydrogène ou un alkyle éventuellement substitué par un ou plusieurs atomes d'halogène,
   ○ un halogène,
   ○ un groupe -T-U avec T représentant une liaison et U un morpholinyle,
      ou
**➢ R₁ et R₂** sont liés et forment avec les deux carbones qui les portent un hétérocycle choisi parmi une pipéridine, un thiazole, un tétrahydrofurane et un dioxane, ledit hétérocycle étant éventuellement substitué par un alkyle linéaire, ramifié ou cyclique éventuellement substitué par -NR₉R'₉, où R₉ et R'₉ représentent' indépendamment l'un de l'autre un atome d'hydrogène ou un méthyle,
   ledit hétérocycle étant fusionné avec le cycle aromatique,
➢ R5 représente (i) un alkyle linéaire ou ramifié comprenant de 1 à 5 atomes de carbone, avantageusement un éthyle, éventuellement substitué par un ou plusieurs substituants choisis parmi les atomes d'halogène avantageusement un atome de fluor ou un groupe -OH, (ii) un aryle éventuellement substitué par un ou plusieurs substituants choisis parmi les groupes alkyle (C1-C3) avantageusement un groupe méthyle, les atomes d'halogène avantageusement un atome de fluor et les groupes -O-(C1-C3) alkyle avantageusement un groupe méthoxy,
sous forme d'acide, de base ou de sel d'addition à un acide ou à une base, ainsi que sous forme d'hydrate ou de solvat.

Parmi les composés de formule (I) ou (I') ci-dessus, on peut mentionner les composés pour lesquels :
➢ **A** représente CH ; et/ou
➢ **X** représente CH ; et/ou
➢ **R₁, R₂, R₃ et R₄** ne représentent pas tous un atome d'hydrogène; et/ou
➢ **R₁** est différent d'un atome d'hydrogène ; et/ou
➢ **R₁** est choisi parmi : 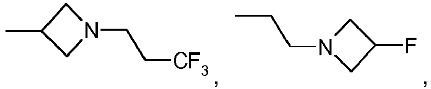 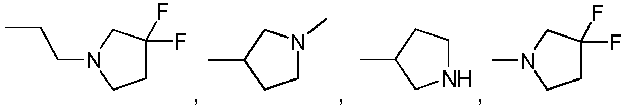 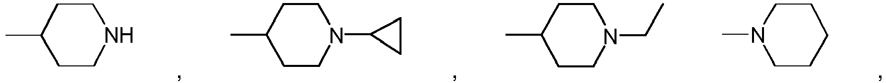 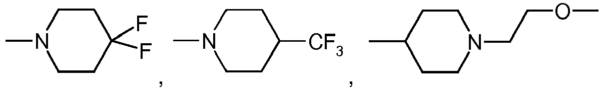 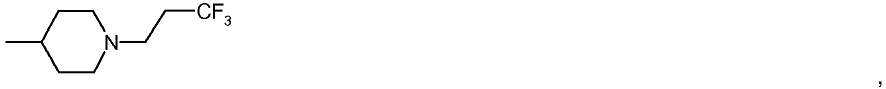 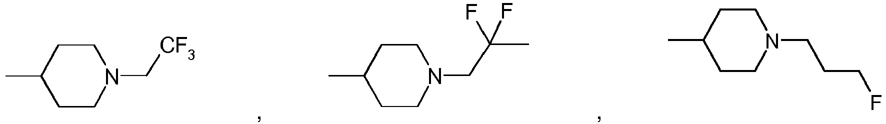 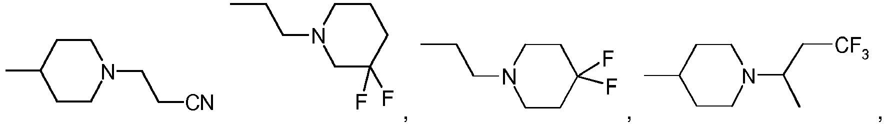 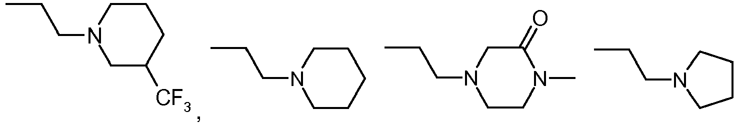 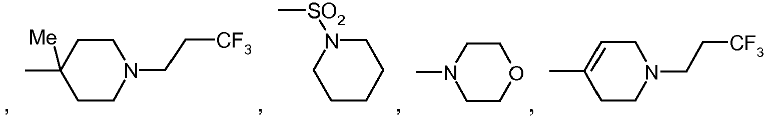 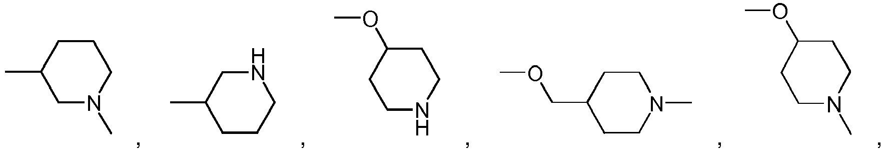 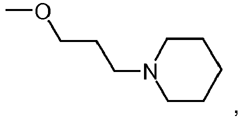 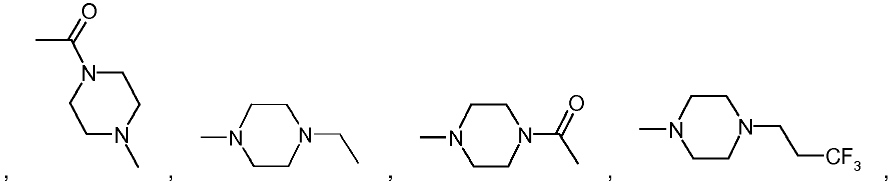 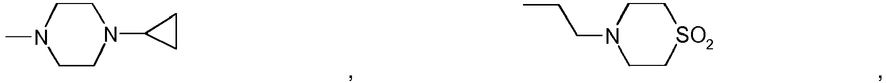 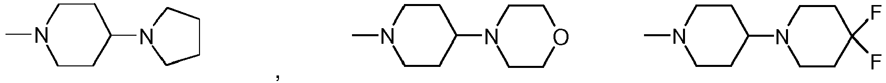 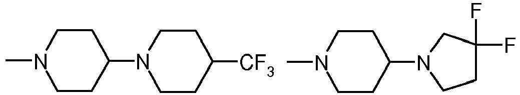 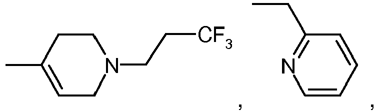 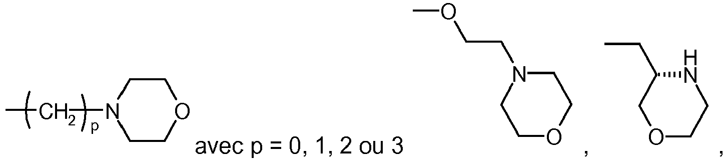 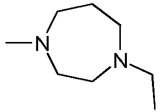 et/ou
➢ **R5** représente (i) un alkyle linéaire ou ramifié avantageuseusement un éthyle (ii) un aryle éventuellement substitué par un ou plusieurs substituants choisis parmi les groupes alkyle (C1-C3) avantageusement un groupe méthyle, les atomes d'halogène avantageusement un atome de fluor; et/ou
➢ **U** représente un hétérocycle saturé, insaturé ou aromatique, éventuellement mono- ou di-substitué comprenant au moins un atome d'azote et/ou un atome d'oxygène, et/ou
➢ **U** représente un hétérocycle saturé comprenant au moins un atome d'azote ; et/ou
➢ **U** représente un hétérocycle choisi parmi :
   ○ un azétidinyle, avantageusement un azétidin-1-yle ou un azétidin-3-yle ;
   ○ un pyrrolidinyle, avantageusement un pyrrolidin-1-yle ou un pyrrolidin-3-yle ;
   ○ un oxopyrrolidinyle, avantageusement un 2-oxo-pyrrolidin-1-yle ;
   ○ un pipéridinyle, avantageusement pipéridin-1-yle, un pipéridin-2-yle ou un pipéridin-4-yle, de manière particulièrement avantageuse un pipéridin-1-yle ou un pipéridin-4-yle ;
   ○ un 1,2,3,6-tétrahydro-pyridinyle, avantageusement un 1,2,3,6-tétrahydro-pyridin-4-yle ;
   ○ un pyridinyle, avantageusement un pyridin-2-yle ;
   ○ un pipérazinyle, avantageusement un pipérazin-1-yle ;
   ○ un diazépanyle, avantageusement un diazépan-1-yle ;
   ○ un morpholinyle, avantageusement un morpholin-4-yle ou un morpholin-3-yle, notamment un 4-(R)-1-morpholin-3-yle, de manière particulièrement avantageuse un morpholin-4-yle ;
   ○ un 1,1-dioxo-1lambda6-thiomorpholin-4-yle ;

Parmi les composés de formule (I) objets de l'invention, on peut notamment citer les composés suivants dans l'ordre des composés du tableau ci-après:
7-Amino-8-éthyl-2-(4-hydroxy-phénylamino)-5-oxo-5,8-dihydro-pyrido[2,3-d]pyrimidine-6-carboxamide;
7-Amino-2-(benzothiazol-6-ylamino)-8-éthyl-5-oxo-5,8-dihydro-pyrido[2,3-d]pyrimidine-6-carboxamide ;
7-Amino-2-[4-(cyclopropanecarbonyl-méthyl-amino)-phénylamino]-8-éthyl-5-oxo-5,8-dihydro-pyrido[2,3-d]pyrimidine-6-carboxamide ;
7-Amino-8-éthyl-2-[4-(4-méthyl-pipérazine-1-carbonyl)-phénylamino]-5-oxo-5,8-dihydro-pyrido[2,3-d]pyrimidine-6-carboxamide ;
7-Amino-2-(4-cyclopentyloxy-phénylamino)-8-éthyl-5-oxo-5,8-dihydro-pyrido[2,3-d]pyrimidine-6-carboxamide ;
7-Amino-8-éthyl-2-[4-(4-éthyl-pipérazine-1-yl)-phénylamino]-5-oxo-5,8-dihydro-pyrido[2,3-d]pyrimidine-6-carboxamide ;
7-Amino-8-éthyl-5-oxo-2-[4-(4-pyrrolidin-1-yl-pipéridin-1-yl)-phénylamino]-5, 8-dihydro-pyrido[2,3-d]pyrimidine-6-carboxamide ;
7-Amino-8-cyclopentyl-2-(4-morpholin-4-yl-phénylamino)-5-oxo-5,8-dihydro-pyrido[2,3-d]pyrimidine-6-carboxamide ;
7-Amino-8-éthyl-5-oxo-2-[4-(pipéridine-1-sulfonyl)-phénylamino]-5,8-dihydro-pyrido[2,3-d]pyrimidine-6-carboxamide ;
2-[4-(4-Acétyl-pipérazin-1-yl)-phénylamino]-7-amino-8-éthyl-5-oxo-5,8-dihydro-pyrido[2,3-d]pyrimidine-6-carboxamide ;
7-Amino-8-éthyl-2-(4-morpholin-4-yl-benzylamino)-5-oxo-5,8-dihydro-pyrido[2,3-d]pyrimidine-6-carboxamide ;
7-Amino-8-éthyl-5-oxo-2-(quinolin-3-ylamino)-5,8-dihydro-pyrido[2,3-d]pyrimidine-6-carboxylamide ;
7-Amino-8-éthyl-5-oxo-2-[4-(3-pipéridin-1-yl-propoxy)-phénylamino]-5,8-dihydro-pyrido[2,3-d]pyrimidine-6-carboxamide ;
7-Amino-8-éthyl-5-oxo-2-(3,4,5,6-tétrahydro-2H-[1,2']bipyridinyl-5'-ylamino)-5,8-dihydro-pyrido[2,3-d]pyrimidine-6-carboxamide ;
7-Amino-8-éthyl-5-oxo-2-[4-(2-oxo-pyrrolidin-1-yl)-phénylamino]-5,8-dihydro-pyrido[2,3-d]pyrimidine-6-carboxamide ;
7-Amino-8-éthyl-5-oxo-2-(quinolin-6-ylamino)-5,8-dihydro-pyrido[2,3-d]pyrimidine-6-carboxamide ;
7-Amino-8-éthyl-2-(3-morpholin-4-yl-phénylamino)-5-oxo-5,8-dihydro-pyrido[2,3-d]pyrimidine-6-carboxamide ;
7-Amino-2-(2,3-dihydro-benzo[1,4]dioxin-6-ylamino)-8-éthyl-5-oxo-5,8-dihydro-pyrido[2,3-d]pyrimidine-6-carboxamide ;
7-Amino-8-éthyl-2-(3-fluoro-4-hydroxy-phénylamino)-5-oxo-5,8-dihydro-pyrido[2,3-d]pyrimidine-6-carboxamide ;
7-Amino-8-éthyl-2-(3-méthylsulfanyl-phénylamino)-5-oxo-5,8-dihydro-pyrido[2,3-d]pyrimidine-6-carboxamide ;
7-Amino-8-éthyl-2-(4-morpholin-4-yl-phénylamino)-5-oxo-5,8-dihydro-pyrido[2,3-d]pyrimidine-6-carboxamide ;
7-Amino-8-éthyl-2-(2-fluoro-phénylamino)-5-oxo-5,8-dihydro-pyrido[2,3-d]pyrimidine-6-carboxamide ;
7-Amino-2-[4-(4,4-difluoro-[1,4']bipipéridinyl-1'-yl)-2-méthoxy-phénylamino]-8-éthyl-5-oxo-5,8-dihydro-pyrido[2,3-d]pyrimidine-6-carboxamide
7-Amino-8-éthyl-2-[2-méthoxy-4-(4-trifluorométhyl-[1,4']bipipéridinyl-1'-yl)-phénylamino]-5-oxo-5,8-dihydro-pyrido[2,3-d]pyrimidine-6-carboxamide
7-Amino-2-{4-[4-(3,3-difluoro-pyrrolidin-1-yl)-pipéridin-1-yl]-2-méthoxy-phénylamino}-8-éthyl-5-oxo-5,8-dihydro-pyrido[2,3-d]pyrimidine-6-carboxamide
7-Amino-8-éthyl-2-[4-(4-éthyl-pipérazin-1-yl)-2-fluoro-6-méthoxy-phénylamino]-5-oxo-5,8-dihydro-pyrido[2,3-d]pyrimidine-6-carboxamide ;
7-Amino-2-[4-(1-cyclopropyl-pipéridin-4-yl)-2-méthoxy-phénylamino]-8-éthyl-5-oxo-5,8-dihydro-pyrido[2,3-d]pyrimidine-6-carboxamide ;
7-Amino-2-(2-diméthylaminométhyl-chroman-6-ylamino)-8-éthyl-5-oxo-5,8-dihydropyrido[2,3-d]pyrimidine-6-carboxamide ;
7-Amino-2-[5-chloro-4-(4-cyclopropyl-pipérazin-1-yl)-2-méthoxy-phénylamino]-8-éthyl-5-oxo-5,8-dihydro-pyrido[2,3-d]pyrimidine-6-carboxamide ;
7-Amino-8-éthyl-2-[2-méthoxy-4-(4-morpholin-4-yl-pipéridin-1-yl)-phénylamino]-5-oxo-5,8-dihydro-pyrido[2,3-d]pyrimidine-6-carboxyamide ;
7-Amino-2-[4-(4-cyclopropyl-pipérazin-1-yl)-2-difluorométhoxy-phénylamino]-8-éthyl-5-oxo-5,8-dihydro-pyrido[2,3-d]pyrimidine-6-carboxamide ;
7-Amino-2-[4-(4-cyclopropyl-pipérazin-1-yl)-2-méthoxy-phénylamino]-8-éthyl-5-oxo-5,8-dihydro-pyrido[2,3-d]pyrimidine-6-carboxamide ;
7-Amino-8-éthyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide ;
7-Amino-2-[2-chloro-4-(4-éthyl-pipérazin-1-yl)-phénylamino]-8-éthyl-5-oxo-5,8-dihydro-pyrido[2,3-d]pyrimidine-6-carboxamide ;
7-Amino-8-éthyl-2-[4-(4-éthyl-pipérazin-1-yl)-3-trifluorométhyl-phénylamino]-5-oxo-5,8-dihydro-pyrido[2,3-d]pyrimidine-6-carboxamide ;
7-Amino-8-éthyl-2-(4-morpholin-4-ylméthyl-phénylamino)-5-oxo-5,8-dihydro-pyrido[2,3-d]pyrimidine-6-carboxamide ;
7-Amino-8-éthyl-5-oxo-2-{4-[4-(3,3,3-trifluoro-propyl)-pipérazin-1-yl]-phénylamino}-5,8-dihydro-pyrido[2,3-d]pyrimidine-6-carboxamide ;
7-Amino-8-éthyl-2-[4-(4-éthyl-pipérazin-1-yl)-3-fluoro-phénylamino]-5-oxo-5,8-dihydro-pyrido[2,3-d]pyrimidine-6-carboxamide ;
6-(4-Morpholin-4-yl-phénylamino)-9-oxo-1,3,4,9-tétrahydro-2H-1,4a,5,7-tétraaza-phénanthrene-10-carboxamide ;
7-Amino-8-éthyl-2-[4-(4-éthyl-pipérazin-1-yl)-2-méthoxy-phénylamino]-5-oxo-5,8-dihydro-pyrido[2,3-d]pyrimidine-6-carboxamide ;
7-Amino-8-éthyl-2-[4-(1-méthyl-pipéridin-4-ylméthoxy)-phénylamino]-5-oxo-5,8-dihydro-pyrido[2,3-d]pyrimidine-6-carboxamide ;
7-Amino-2-[4-(4-cyclopropyl-pipérazin-1-yl)-phénylamino]-8-éthyl-5-oxo-5,8-dihydro-pyrido[2,3-d]pyrimidine-6-carboxamide ;
7-Amino-8-éthyl-2-[4-(4-éthyl-pipérazin-1-yl)-2-méthyl-phénylamino]-5-oxo-5,8-dihydro-pyrido[2,3-d]pyrimidine-6-carboxamide ;
7-Amino-8-éthyl-2-[4-(4-éthyl-[1,4]diazépan-1-yl)-phénylamino]-5-oxo-5,8-dihydro-pyrido[2,3-d]pyrimidine-6-carboxamide ;
8-(4-Morpholin-4-yl-phénylamino)-5-oxo-1,2,3,5-tétrahydro-3,7,9,9b-tétraaza-cyclopenta[a]naphthalène-4-carboxamide;
7-Amino-8-éthyl-5-oxo-2-{4-[2-(3-trifluorométhyl-pipéridin-1-yl)-éthyl]-phénylamino}-5,8-dihydro-pyrido[2,3-d]pyrimidine-6-carboxamide;
7-Amino-8-éthyl-2-[4-(3-morpholin-4-yl-propyl)-phénylamino]-5-oxo-5,8-dihydro-pyrido[2,3-d]pyrimidine-6-carboxamide;
7-Amino-8-éthyl-5-oxo-2-{4-[1-(2,2,2-trifluoro-éthyl)-pipéridin-4-yl]-phénylamino}-5,8-dihydro-pyrido[2,3-d]pyrimidine-6-carboxamide;
7-Amino-8-éthyl-2-(4-(S)-1-morpholin-3-ylméthyl-phénylamino)-5-oxo-5,8-dihydro-pyrido[2,3-d]pyrimidine-6-carboxamide;
7-Amino-8-isobutyl-2-[4-(2-morpholin-4-yl-éthyl)-phénylamino]-5-oxo-5,8-dihydro-pyrido[2,3-d]pyrimidine-6-carboxamide;
7-Amino-8-éthyl-2-{4-[2-(3-fluoro-azétidin-1-yl)-éthyl]-phénylamino}-5-oxo-5,8-dihydro-pyrido[2,3-d]pyrimidine-6-carboxamide;
7-Amino-2-[4-(2-morpholin-4-yl-éthyl)-phénylamino]-5-oxo-8-propyl-5,8-dihydro-pyrido[2,3-d]pyrimidine-6-carboxamide;
7-Amino-8-éthyl-2-[4-(2-hydroxy-éthyl)-phénylamino]-5-oxo-5,8-dihydro-pyrido[2,3-d]pyrimidine-6-carboxamide;
7-amino-8-éthyl-2-(4-hydroéthyl-phénylamino)-5-oxo-5,8-dihydropyrido[2,3-d]pyrimidine-6-carboxamide ;
7-Amino-8-éthyl-2-(2-méthyl-1,2,3,4-tétrahydro-isoquinolin-6-ylamino)-5-oxo-5,8-dihydro-pyrido[2,3-d]pyrimidine-6-carboxamide;
7-Amino-2-{4-[2-(1,1-dioxo-1lambda6-thiomorpholin-4-yl)-éthyl]-phénylamino}-8-éthyl-5-oxo-5,8-dihydro-pyrido[2,3-d]pyrimidine-6-carboxamide;
7-Amino-8-éthyl-2-{4-[2-(4-méthyl-3-oxo-pipérazin-1-yl)-éthyl]-phénylamino}-5-oxo-5,8-dihydro-pyrido[2,3-d]pyrimidine-6-carboxamide;
7-Amino-8-éthyl-5-oxo-2-{4-[1-(3,3,3-trifluoro-propyl)-pipéridin-4-yl]-phénylamino}-5,8-dihydro-pyrido[2,3-d]pyrimidine-6-carboxamide;
7-Amino-2-(4-diméthylaminométhyl-phénylamino)-5-oxo-8-(2,2,2-trifluoro-éthyl)-5,8-dihydro-pyrido[2,3-d]pyrimidine-6-carboxamide;
7-Amino-2-[3-chloro-4-(4-pyrrolidin-1-yl-pipéridin-1-yl)-phénylamino]-8-éthyl-5-oxo-5,8-dihydro-pyrido[2,3-d]pyrimidine-6-carboxamide;
7-Amino-8-éthyl-5-oxo-2-(4-pyridin-2-ylméthyl-phénylamino)-5,8-dihydro-pyrido[2,3-d]pyrimidine-6-carboxamide;
7-Amino-2-{4-[2-(3,3-difluoro-pyrrolidin-1-yl)-éthyl]-phénylamino}-8-éthyl-5-oxo-5,8-dihydro-pyrido[2,3-d]pyrimidine-6-carboxamide;
7-Amino-8-éthyl-5-oxo-2-(4-pyrrolidin-3-yl-phénylamino)-5,8-dihydro-pyrido[2,3-d]pyrimidine-6-carboxamide;
7-Amino-8-éthyl-5-oxo-2-[4-(2-pipéridin-1-yl-éthyl)-phénylamino]-5,8-dihydro-pyrido[2,3-d]pyrimidine-6-carboxamide;
7-Amino-2-[4-(2-morpholin-4-yl-éthyl)-phénylamino]-5-oxo-8-(2,2,2-trifluoro-ethyl)-5,8-dihydro-pyrido[2,3-d]pyrimidine-6-carboxamide
7-Amino-2-{4-[2-(4,4-difluoro-pipéridin-1-yl)-éthyl]-phénylamino}-8-éthyl-5-oxo-5,8-dihydro-pyrido[2,3-d]pyrimidine-6-carboxamide;
7-Amino-8-éthyl-2-[4-(1-méthyl-pyrrolidin-3-yl)-phénylamino]-5-oxo-5,8-dihydro-pyrido[2,3-d]pyrimidine-6-carboxamide;
7-Amino-2-{4-[2-(3,3-difluoro-pipéridin-1-yl)-éthyl]-phénylamino}-8-éthyl-5-oxo-5,8-dihydro-pyrido[2,3-d]pyrimidine-6-carboxamide;
7-Amino-8-(3-méthoxy-propyl)-2-[4-(2-morpholin-4-yl-éthyl)-phénylamino]-5-oxo-5,8-dihydro-pyrido[2,3-d]pyrimidine-6-carboxamide;
7-Amino-8-éthyl-2-[4-(1-méthyl-pipéridin-3-yl)-phénylamino]-5-oxo-5,8-dihydro-pyrido[2,3-d]pyrimidine-6-carboxamide;
7-Amino-2-(4-diméthylaminométhyl-phénylamino)-8-isopropyl-5-oxo-5,8-dihydro-pyrido[2,3-d]pyrimidine-6-carboxamide;
7-Amino-8-éthyl-5-oxo-2-(4-pipéridin-3-yl-phénylamino)-5,8-dihydro-pyrido[2,3-d]pyrimidine-6-carboxamide;
7-Amino-8-éthyl-5-oxo-2-(1,2,3,4-tétrahydro-isoquinolin-7-ylamino)-5,8-dihydro-pyrido[2,3-d]pyrimidine-6-carboxamide;
7-Amino-8-éthyl-5-oxo-2-(1,2,3,4-tétrahydro-isoquinolin-6-ylamino)-5,8-dihydro-pyrido[2,3-d]pyrimidine-6-carboxamide;
7-Amino-8-éthyl-2-[4-(1-méthyl-pipéridin-4-yloxy)-phénylamino]-5-oxo-5,8-dihydro-pyrido[2,3-d]pyrimidine-6-carboxamide;
7-Amino-8-éthyl-2-[4-(2-morpholin-4-yl-éthoxy)-phénylamino]-5-oxo-5,8-dihydro-pyrido[2,3-d]pyrimidine-6-carboxamide;
7-Amino-2-(3-diméthylaminométhyl-phénylamino)-8-éthyl-5-oxo-5,8-dihydro-pyrido[2,3-d]pyrimidine-6-carboxamide;
7-Amino-8-éthyl-5-oxo-2-[4-(4-pyrrolidin-1-yl-pipéridin-1-yl)-phénylamino]-5,8-dihydro-pyrido[2,3-d]pyrimidine-6-carboxamide;
7-Amino-8-éthyl-5-oxo-2-[4-(pipéridin-4-yloxy)-phénylamino]-5,8-dihydro-pyrido[2,3-d]pyrimidine-6-carboxamide;
7-Amino-8-(3-amino-propyl)-2-(4-morpholin-4-yl-phénylamino)-5-oxo-5,8-dihydro-pyrido[2,3-d]pyrimidine-6-carboxamide;
7-Amino-8-éthyl-2-[4-(2-morpholin-4-yl-éthyl)-phénylamino]-5-oxo-5,8-dihydro-pyrido[2,3-d]pyrimidine-6-carboxamide;
7-Amino-2-(4-diméthylaminométhyl-phénylamino)-8-éthyl-5-oxo-5,8-dihydro-pyrido[2,3-d]pyrimidine-6-carboxamide;
7-Amino-8-éthyl-2-[4-(1-éthyl-pipéridin-4-yl)-phénylamino]-5-oxo-5,8-dihydro-pyrido[2,3-d]pyrimidine-6-carboxamide;
7-Amino-8-éthyl-5-oxo-2-(4-pipéridin-4-yl-phénylamino)-5,8-dihydro-pyrido[2,3-d]pyrimidine-6-carboxamide;
7-Amino-2-[3-chloro-4-(4-éthyl-pipérazin-1-yl)-phénylamino]-8-éthyl-5-oxo-5,8-dihydro-pyrido[2,3-d]pyrimidine-6-carboxamide
7-Amino-8-éthyl-2-[3-méthoxy-4-(3-pipéridin-1-yl-propoxy)-phénylamino]-5-oxo-5,8-dihydro-pyrido[2,3-d]pyrimidine-6-carboxamide
7-Amino-8-éthyl-5-oxo-2-{4-[2-(4-trifluorométhyl-pipéridin-1-yl)-éthyl]-phénylamino}-5,8-dihydro-pyrido[2,3-d]pyrimidine-6-carboxamide
7-Amino-2-{4-[2-(cis-2,6-diméthyl-morpholin-4-yl)-éthyl]-phénylamino}-8-éthyl-5-oxo-5,8-dihydro-pyrido[2,3-d]pyrimidine-6-carboxamide
7-Amino-2-(4-diéthylaminométhyl-phénylamino)-8-éthyl-5-oxo-5,8-dihydro-pyrido[2,3-d]pyrimidine-6-carboxamide
7-Amino-2-(4-diméthylaminométhyl-phénylamino)-8-(3-méthoxy-propyl)-5-oxo-5,8-dihydro-pyrido[2,3-d]pyrimidine-6-carboxamide
7-Amino-8-éthyl-2-[4-(4-éthyl-pipérazin-1-yl)-2-fluoro-phénylamino]-5-oxo-5,8-dihydro-pyrido[2,3-d]pyrimidine-6-carboxamide
7-Amino-2-{4-[1-(2-cyano-éthyl)-pipéridin-4-yl]-phénylamino}-8-éthyl-5-oxo-5,8-dihydro-pyrido[2,3-d]pyrimidine-6-carboxamide
7-Amino-8-éthyl-2-{4-[1-(3-fluoro-propyl)-pipéridin-4-yl]-phénylamino}-5-oxo-5,8-dihydro-pyrido[2,3-d]pyrimidine-6-carboxylic acid amide
7-Amino-8-éthyl-2-[4-(4-éthyl-pipérazin-1-yl)-3-méthyl-phénylamino]-5-oxo-5,8-dihydro-pyrido[2,3-d]pyrimidine-6-carboxamide
7-Amino-2-[4-(4-cyclopropyl-pipérazin-1-yl)-2-éthyl-phénylamino]-8-éthyl-5-oxo-5,8-dihydro-pyrido[2,3-d]pyrimidine-6-carboxamide
(±)-7-Amino-2-trans-[4-(2-diméthylamino-cyclopropyl)-phénylamino]-8-éthyl-5-oxo-5,8-dihydro-pyrido[2,3-d]pyrimidine-6-carboxamide
7-Amino-2-[4-(4-cyclopropyl-pipérazin-1-yl)-5-fluoro-2-méthoxy-phénylamino]-8-éthyl-5-oxo-5,8-dihydro-pyrido[2,3-d]pyrimidine-6-carboxamide
7-Amino-2-[4-(4-cyclopropyl-pipérazin-1-yl)-3-fluoro-2-méthoxy-phénylamino]-8-éthyl-5-oxo-5,8-dihydro-pyrido[2,3-d]pyrimidine-6-carboxamide
7-Amino-2-[4-(4-cyclopropyl-pipérazin-1-yl)-2-ethoxy-phénylamino]-8-éthyl-5-oxo-5, 8-dihydro-pyrido[2,3-d]pyrimidine-6-carboxamide
7-Amino-8-éthyl-5-oxo-2-(4-propyl-phénylamino)-5,8-dihydro-pyrido[2,3-d]pyrimidine-6-carboxamide
7-Amino-8-éthyl-5-oxo-2-(4-propoxy-phénylamino)-5,8-dihydro-pyrido[2,3-d]pyrimidine-6-carboxamide
7-Amino-8-éthyl-2-(6-méthoxy-pyridin-3-ylamino)-5-oxo-5,8-dihydro-pyrido[2,3-d]pyrimidine-6-carboxamide
7-Amino-8-éthyl-2-(4-fluoro-phénylamino)-5-oxo-5,8-dihydro-pyrido[2,3-d]pyrimidine-6-carboxamide
7-Amino-8-éthyl-2-(4-méthoxy-phénylamino)-5-oxo-5,8-dihydro-pyrido[2,3-d]pyrimidine-6-carboxamide
7-Amino-2-(benzo[1,3]dioxol-5-ylamino)-8-éthyl-5-oxo-5,8-dihydro-pyrido[2,3-d]pyrimidine-6-carboxamide
7-Amino-8-éthyl-5-oxo-2-(4-pipéridin-1-yl-phénylamino)-5,8-dihydro-pyrido[2,3-d]pyrimidine-6-carboxamide
8-Éthyl-2-{2-méthoxy-4-[1-(3,3,3-trifluoro-propyl)-pipéridin-4-yl]-phénylamino}-7-méthylamino-5-oxo-5,8-dihydro-pyrido[2,3-d]pyrimidine-6-carboxamide
7-Amino-8-isobutyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-cyclopropylméthyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-(2-méthoxy-éthyl)-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-(tétrahydro-furan-2-ylméthyl)-5,8-dihydro-pyrido[2,3-d]pyrimidine-6-carboxamide
7-Amino-8-éthyl-5-oxo-2-{4-[1-(3,3,3-trifluoro-propyl)-azetidin-3-yl]-phénylamino}-5,8-dihydro-pyrido[2,3-d]pyrimidine-6-carboxamide
7-Amino-2-{5-fluoro-2-méthoxy-4-[1-(3,3,3-trifluoro-propyl)-pipéridin-4-yl]-phénylamino}-8-isobutyl-5-oxo-5,8-dihydro-pyrido[2,3-d]pyrimidine-6-carboxamide
7-Amino-8-éthyl-5-oxo-2-[4-(1,2,3,6-tétrahydro-pyridin-4-yl)-phénylamino]-5,8-dihydro-pyrido[2,3-d]pyrimidine-6-carboxamide
7-Amino-2-(2-méthoxy-4-pipéridin-4-yl-phénylamino)-5-oxo-8-phényl-5,8-dihydro-pyrido[2,3-d]pyrimidine-6-carboxamide
7-Amino-8-(2-hydroxy-éthyl)-2-(2-méthoxy-4-pipéridin-4-yl-phénylamino)-5-oxo-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-thiazol-2-ylméthyl-5,8-dihydro-pyrido[2,3-d]pyrimidine-6-carboxamide
7-Am ino-2-{2-méthoxy-4-[1-(3,3,3-trifluoro-propyl)-pipéridin-4-yl]-phénylamino}-5-oxo-8-thiazol-5-ylméthyl-5,8-dihydro-pyrido[2,3-d]pyrimidine-6-carboxamide
7-Amino-8-(2-hydroxy-2-méthyl-propyl)-2-(2-méthoxy-4-pipéridin-4-yl-phénylamino)-5-oxo-5,8-dihydro-pyrido[2,3-d]pyrimidine-6-carboxamide
8-(2-Acétylamino-éthyl)-7-amino-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-(2-amino-éthyl)-2-(4-morpholin-4-yl-phénylamino)-5-oxo-5,8-dihydro-pyrido[2,3-d]pyrimidine-6-carboxamide
7-Amino-2-(4-morpholin-4-yl-phénylamino)-5-oxo-8-pyrrolidin-3-ylméthyl-5,8-dihydro-pyrido[2,3-d]pyrimidine-6-carboxamide
7-Amino-8-éthyl-2-[4-(4-éthyl-pipérazin-1-yl)-2-hydroxy-phénylamino]-5-oxo-5,8-dihydro-pyrido[2,3-d]pyrimidine-6-carboxamide
7-Amino-2-(4-morpholin-4-yl-phénylamino)-5-oxo-8-pipéridin-4-ylméthyl-5,8-dihydro-pyrido[2,3-d]pyrimidine-6-carboxamide
7-Amino-2-(2-méthoxy-4-morpholin-4-yl-phénylamino)-5-oxo-8-pyrrolidin-3-ylméthyl-5,8-dihydro-pyrido[2,3-d]pyrimidine-6-carboxamide
7-Amino-8-éthyl-2-(2-méthoxy-4-pipéridin-4-yl-phénylamino)-4-méthyl-5-oxo-5,8-dihydro-pyrido[2,3-d]pyrimidine-6-carboxamide
7-Am ino-2-{2-méthoxy-4-[1-(3,3,3-trifluoro-propyl)-pipéridin-4-yl]-phénylamino}-5-oxo-8-thiophèn-2-ylméthyl-5,8-dihydro-pyrido[2,3-d]pyrimidine-6-carboxamide
7-Amino-8-isobutyl-2-[2-méthoxy-4-(4-morpholin-4-yl-pipéridin-1-yl)-phénylamino]-5-oxo-5,8-dihydro-pyrido[2,3-d]pyrimidine-6-carboxamide
7-Amino-2-(4-morpholin-4-yl-phénylamino)-5-oxo-8-pyrrolidin-2-ylméthyl-5,8-dihydro-pyrido[2,3-d]pyrimidine-6-carboxamide
7-Amino-8-éthyl-2-{2-méthoxy-4-[1-(3,3,3-trifluoro-propyl)-1,2,3,6-tétrahydro-pyridin-4-yl]-phénylamino}-5-oxo-5,8-dihydro-pyrido[2,3-d]pyrimidine-6-carboxamide
7-Amino-2-[4-(4-éthyl-pipérazin-1-yl)-2-méthoxy-phénylamino]-8-(2-hydroxy-2-méthyl-propyl)-5-oxo-5,8-dihydro-pyrido[2,3-d]pyrimidine-6-carboxamide
7-Amino-8-(2-hydroxy-2-méthyl-propyl)-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-(2-hydroxy-2-méthyl-propyl)-2-[4-(4-éthyl-pipéridin-1-yl)-2-méthoxy-phénylamino]-5-oxo-5,8-dihydro-pyrido[2,3-d]pyrimidine-6-carboxamide
7-Amino-2-[4-(1-cyclopropyl-pipéridin-4-yl)-2-méthoxy-phénylamino]-8-(2-hydroxy-2-méthyl-propyl)-5-oxo-5,8-dihydro-pyrido[2,3-d]pyrimidine-6-carboxamide
7-Amino-8-(2-hydroxy-2-méthyl-propyl)-2-{2-méthoxy-4-[1-(2-méthoxy-éthyl)-pipéridin-4-yl]-phénylamino}-5-oxo-5,8-dihydro-pyrido[2,3-d]pyrimidine-6-carboxamide
7-Amino-2-[2-méthoxy-4-(1-méthyl-pipéridin-4-yl)-phénylamino]-5-oxo-8-phényl-5,8-dihydro-pyrido[2,3-d]pyrimidine-6-carboxamide
7-Amino-8-(3-fluoro-phényl)-2-[2-méthoxy-4-pipéridin-4-yl-phénylamino]-5-oxo-5,8-dihydro-pyrido[2,3-d]pyrimidine-6-carboxamide
7-Amino-8-(4-fluoro-phényl)-2-[2-méthoxy-4-pipéridin-4-yl-phénylamino]-5-oxo-5,8-dihydro-pyrido[2,3-d]pyrimidine-6-carboxamide
7-Amino-2-[2-méthoxy-4-pipéridin-4-yl-phénylamino]-5-oxo-8-m-tolyl-5,8-dihydro-pyrido[2,3-d]pyrimidine-6-carboxamide
7-Amino-2-[2-méthoxy-4-pipéridin-4-yl-phénylamino]-5-oxo-8-p-tolyl-5,8-dihydro-pyrido[2,3-d]pyrimidine-6-carboxamide
7-Amino-8-(3-méthoxy-phényl)-2-(2-méthoxy-4-pipéridin-4-yl-phénylamino)-5-oxo-5,8-dihydro-pyrido[2,3-d]pyrimidine-6-carboxamide
7-Amino-2-[2-méthoxy-4-(2-pyrrolidin-1-yl-éthyl)-phénylamino]-5-oxo-8-phényl-5,8-dihydro-pyrido[2,3-d]pyrimidine-6-carboxamide
7-Amino-8-(4-fluoro-phényl)-2-[2-méthoxy-4-(1-méthyl-pipéridin-4-yl)-phénylamino]-5-oxo-5,8-dihydro-pyrido[2,3-d]pyrimidine-6-carboxamide
7-Amino-2-[2-méthoxy-4-(1-méthyl-pipéridin-4-yl)-phénylamino]-5-oxo-8-m-tolyl-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-phényl-5,8-dihydro-pyrido[2,3-d]pyrimidine-6-carboxamide
7-Amino-2-[4-(4-éthyl-pipérazin-1-yl)-2-méthoxy-phénylamino]-5-oxo-8-phényl-5,8-dihydro-pyrido[2,3-d]pyrimidine-6-carboxamide
à l'état de base ou de sel d'addition à un acide, ainsi qu'à l'état d'hydrate ou de solvat.

Les combinaisons des groupes de composés selon l'invention font également partie de l'invention à titre de mode de réalisation.

Conformément à l'invention, on peut préparer les composés de formule générale (I) selon le procédé qui suit.

Selon un premier mode de réalisation, le procédé selon l'invention comprend l'étape de substitution nucléophile d'un composé de formule (IX) 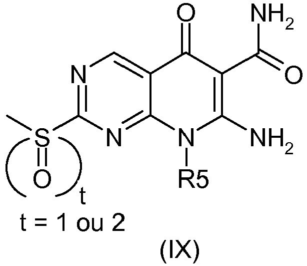 au moyen d'une amine primaire de formule (A) : 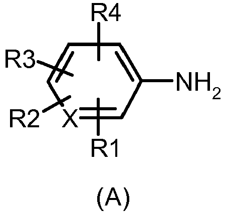 dans lesquelles R₁, R₂, R₃, R₄, R₅ sont définis comme en formule générale (I). Ce procédé conduit à la formation de composé de formule (I), en particulier de formule (I").

Conformément au schéma 1 ci-dessous, on fait réagir le 4-chloro-2-méthylthiopyridine-5-carboxylate d'éthyle (II avec A = CH) (préparée selon Todd et coll., J. Amer. Chem. Soc. (1943), 65, 350-354 ; Pesson, M., Eur. J. Med. Chem. (1974), 9(6), 585-590 ; Shadbolt, R., J. Chem. Soc. (1967), 13, 1172-1178) ou bien le 4-chloro-6-méthyl-2-méthylthiopyridine-5-carboxylate d'éthyle (II avec A = C-Me) (préparée selon la méthode décrite dans WO2005/105801) avec une amine de formule R₅-NH₂ (dans laquelle R₅ est tel que défini précédemment en rapport avec la formule (I) selon l'invention) dans un solvant organique tel que le THF ou dioxane, et en présence d'une base organique, par exemple la triéthylamine, à température ambiante selon la méthode décrite par D. Boschelli et coll. dans J. Med. Chem. (1998), 41, 4365-4377, pour fournir un dérivé 4-amino-2-méthylthiopyridine-5-carboxylate d'éthyle de formule (III).

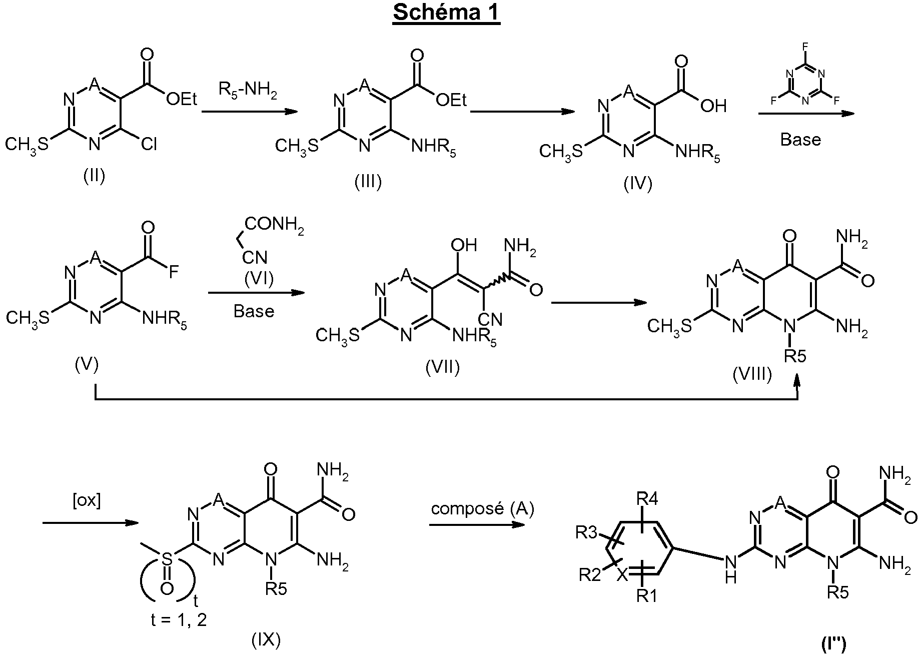

Le groupement ester du dérivé 4-aminopyrimidine de formule (III) est ensuite hydrolysé à l'aide d'hydroxyde de sodium ou de potassium ou de lithium, à une température comprise entre 20 et 100°C, pour conduire à l'acide carboxylique correspondant de formule (IV). Le groupement acide carboxylique est ensuite activé par des méthodes connues par l'Homme de l'art, telle que sa transformation en chlorure d'acide à l'aide du chlorure de thionyle, ou avantageusement par sa transformation en fluorure d'acide de formule (V) en le faisant réagir avec un excès de fluorure de cyanuryle (C₃N₃F₃) dans un solvant inerte tel que le dichlorométhane, en présence d'une base organique, telle que la pyridine ou avantageusement la triéthylamine. Pour plus de détails sur cette réaction on pourra consulter Synthesis 1973, 487.

Le fluorure d'acide de formule (V) ainsi obtenu est condensé avec du cyanoacétamide de formule (VI), en présence d'une base forte telle que l'hydrure de sodium (NaH) dans un solvant polaire, avantageusement le DMF, à température ordinaire. Si l'on ajoute 2 équivalents de NaH, on peut obtenir un β-céto-cyanoacétamide de formule (VII), que l'on cyclise en dérivé pyrido[2,3-d]pyrimidine de formule (VIII) par chauffage dans un solvant polaire tel que le DMF, le DMSO ou le n-butanol, à une température supérieure à 100°C. Par contre, si on contacte le fluorure d'acide de formule (V) avec du cyanoacétamide de formule (VI) en présence de 2 équivalents d'hydrure de sodium dans un premier temps, puis on ajoute un troisième équivalent après la formation *in situ* du β-céto-cyanoacétamide, alors on cyclise directement à température ordinaire le composé de formule (V) en dérivé pyrido[2,3-d]pyrimidine de formule (VIII). Le groupement méthyl-sulfanyle des composés de formule (VIII) est ensuite oxydé (symbole [Ox] dans le schéma 1) en dérivés sulfoxyde (n = 1) et sulfone (n = 2) correspondants par réaction avec des agents d'oxydation tels que l'acide *m*-perchlorobenzoïque, l'eau oxygénée, le perborate de sodium et l'hydrogénosulfate de potassium ou le trans-2-phénylsulfonyl-3-phenyloxaziridine, comme décrit par exemple par D. Boschelli et coll. dans J. Med. Chem. (1998), 41, 4365-4377 et dans la demande internationale PCT publiée sous le numéro WO 96/34867. Le mélange de sulfone et de sulfoxide de formule (IX) réagit avec une amine primaire par substitution nucléophile aromatique dans un solvant polaire tel que le DMF, le DMSO ou la NMP, à une température comprise entre 75 et 150°C, pour fournir les composés de formule (I") sous groupe des composés de formule (I) avec A représentant -CH= et R₆ représentant un atome d'hydrogène, objets de la présente invention. Il est entendu que dans le procédé décrit dans le schéma 1, les groupes R₁, R₂, R₃, R₄ ou R₅ peuvent comporter un ou plusieurs groupes protecteurs temporaires.

Selon un autre mode de réalisation, le procédé selon l'invention comprend l'étape de réaction d'un composé de formule générale (XIV) ou (XV): 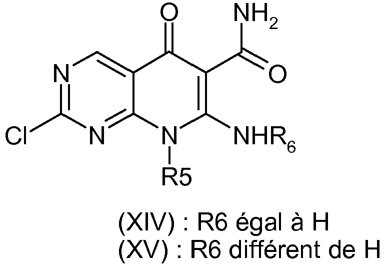 et d'un composé de formule générale (A) : 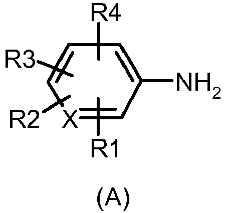 dans lesquelles R₁, R₂, R₃, R₄, R₅ et R₆ sont définis comme dans la formule générale (I), par une réaction de substitution nucléophile ou par une réaction de couplage de type Büchwald/Hartwig catalysée par un complexe du palladium. Ce procédé conduit à la formation de composé de formule (I), en particulier de formule (I")et (I"').

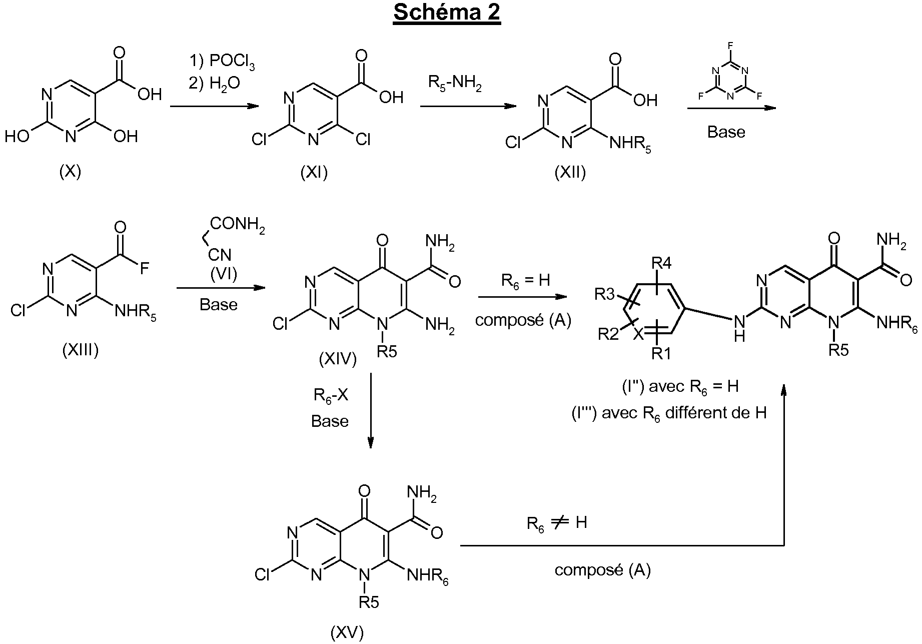

Conformément au schéma 2, on fait réagir l'acide uracil-5-carboxylique (X) avec l'oxychlorure de phospore en présence de *N*,*N*-diéthylaniline selon la méthode de V.H. Smith et B.E. Christensen (J. Am. Chem. Soc. 1955, 20, 829) pour donner l'acide 2,4-dichloro-5-pyrimidinecarboxylique (XI). On peut avantageusement utiliser directement le chlorure de l'acide 2,4-dichloro-5-pyrimidinecarboxylique commercial que l'on soumet à une hydrolyse ménagée. L'atome de chlore en position 4 de l'intermédiaire (XI) est sélectivement substitué par une amine de formule R₅-NH₂ (dans laquelle R₅ est tel que défini précédemment en rapport avec la formule (I) selon l'invention dans un solvant organique tel que le THF ou dioxane, et en présence d'une base organique, par exemple la triéthylamine, à température ambiante pour fournir un dérivé d'acide 4-amino-2-chloropyrimidine-5-carboxylique de formule (XII). Le groupement acide carboxylique est ensuite activé par des méthodes connues par l'Homme de l'art, telle que sa transformation en chlorure d'acide à l'aide du chlorure de thionyle, ou avantageusement par sa transformation en fluorure d'acide de formule (XIII) en le faisant réagir avec un excès de fluorure de cyanuryle dans un solvant inerte tel que le dichlorométhane, en présence d'une base organique, telle que la triéthylamine.
Le fluorure d'acide de formule (XIII) ainsi obtenu est condensé avec du cyanoacétamide de formule (VI), en présence d'un excès d'une base forte telle que l'hydrure de sodium (NaH) dans un solvant polaire, avantageusement le DMF, à température ordinaire. En présence de 2 équivalents d'hydrure de sodium puis ajout d'un troisième équivalent après formation *in situ* du β-céto-cyanoacétamide, on transforme directement à température ordinaire le composé de formule (XIII) en dérivé pyrido[2,3-d]pyrimidine de formule (XIV). L'intermédiaire chloré (XIV) peut réagir directement avec une amine primaire ou secondaire par substitution nucléophile aromatique ou par couplage de Buchwald/Hartwig catalysé par un métal tel que le palladium à une température comprise entre 75 et 150°C éventuellement dans un appareil à micro-ondes, pour fournir les composés de formule (I") sous groupe des composés de formule (I) objets de la présente invention où R6 représentant un atome d'hydrogène et A représente -CH=. L'intermédiaire chloré (XIV) peut être préalablement alkylé au niveau du groupe amino exocyclique pour donner (XV) en présence d'une base telle que l'hydrure de sodium ou le tert-butylate de potassium et d'un agent alkylant avantageusement un iodure d'alkyle dans un solvant polaire tel que la DMF pour conduire finalement aux composés de formule (I"'), sous groupe des composés de formule (I) objets de la présente invention avec R6 ≠ H et tel que défini ci-dessus et A représentant -CH=. Il est entendu que dans le procédé décrit dans le schéma 2, les groupes R₁, R₂, R₃, R₄, R₅ ou R₆ peuvent comporter un ou plusieurs groupes protecteurs temporaires.

Dans les schémas 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme de l'art.

L'invention, selon un autre de ses aspects, a également pour objet les composés de formules (VII), (VIII) (IX), et (XV) avec R6 n'étant pas un atome d'hydrogène, lesdits composés étant définis dans les schémas de synthèse 1 et 2. Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les abréviations et formules brutes suivantes sont utilisées :
- CH₂Cl₂: dichlorométhane
- CLHP: chromatographié liquide haute performance
- CL/SM: chromatographie liquide/ spectrométrie de masse
- d: doublet
- DMF: diméthylformamide
- DMSO: diméthyl sulfoxide
- dppf: diphenylphosphinoferrocène
- Et₃N: triéthylamine
- h: heure(s)
- HCl: acide chlorhydrique
- MHz: MegaHertz
- m: massif ou multiplet
- mn: minute(s)
- MeOH: méthanol
- MgSO₄: sulfate de magnésium
- NaCl: chlorure de sodium
- NaHCO₃: hydrogénocarbonate de sodium
- NaOH: hydroxyde de sodium
- Na₂SO₄: sulfate de sodium
- NH₄Cl: chlorure d'ammonium

- NH₄OH: hydroxyde d'ammonium
- NMP: *N*-méthylpyrolidinone
- ppm: partie par million
- s: singulet
- t: triplet
- THF: tétrahydrofurane

### Exemple 1 : 7-Amino-8-éthyl-2-[(4-hydroxyphényl)amino]-5-oxo-5,8-dihydropyrido[2,3-d]pyrimidine-6-carboxamide

### 1.1 : 4-Ethylamino-2-méthylthio-5-pyrimidine carboxylate d'éthyle

A une solution de 15,0 g (64,0 mmoles) de 4-chloro 2-méthylthio-5-pyrimidine carboxylate d'éthyle et 195 mL (1,39 moles) de triéthylamine dans 226 mL de THF, on ajoute 14,6 mL d'une solution aqueuse d'éthylamine à 70 %. Le mélange est agité à température ambiante durant 2 heures. Après dilution dans l'eau, on extrait à l'acétate d'éthyle et on sèche la phase organique sur MgSO₄. On purifie le produit par chromatographie sur gel de silice en éluant avec un mélange cyclohexane : AcOEt (8:15). On obtient 13,7 g de produit attendu sous forme d'huile incolore. Rendement = 89%.

### 1.2 : Acide 4-éthylamino-2-méthylthio 5-pyrimidine carboxylique

Un mélange contenant 6,33 g (26,0 mmoles) de 4-éthylamino-2-méthylthio-5-pyrimidine carboxylate d'éthyle, 65 mL (65,0 mmoles) de NaOH 1 N et 60 mL d'éthanol est chauffé à reflux pendant 1 heure. On évapore l'éthanol sous pression réduite et on dilue le résidu dans 100 mL d'eau. On additionne 65 mL d'une solution de HCl aqueux 1 N et on essore le précipité blanc formé. On rince le solide avec de l'eau et on le sèche sous vide. On obtient 5,1 g de produit attendu sous forme d'un solide blanc. Point de fusion = 188°C. Rendement = 92%.

### 1.3 : Fluorure d'acide 4-éthylamino-2-méthylthio-5-pyrimidine carboxylique

A une solution contenant 3,0 g (14,0 mmoles) d'acide 4-éthylamino-2-méthylthio-5-pyrimidine carboxylique et 2,1 mL (15,0 mmoles) de triéthylamine dans 50 mL de CH₂Cl₂, on ajoute 2,3 mL (28,0 mmoles) de fluorure de cyanuryle. Le mélange est agité durant 3 heures et la solution est diluée avec 250 mL de CH₂Cl₂ et 75 mL d'eau glacée. Après décantation la phase organique est lavée avec 75 mL d'eau glacée et séchée sur MgSO₄. Le solvant est ensuite évaporé sous pression réduite. On obtient 3,0 g de produit attendu sous forme d'une gomme verdâtre. Le rendement est quantitatif.

### 1. 4 : 7-Amino-8-éthyl-2-(méthylthio)-5-oxo-5,8-dihydropyrido[2,3-d]pyrimidine carboxamide

A une solution de 1,18 g (14,0 mmoles) de cyanoacétamide dans 20 mL de DMF anhydre refroidie à 0-5°C, on ajoute 1,12 g (28 mmoles) de NaH à 60 %. Après l'addition, on agite 10 minutes à température ambiante puis on refroidit à nouveau avec un bain eau-glace et on ajoute la solution du fluorure d'acide (14,0 mmoles) préparé en 1.3 dans 30 mL de DMF anhydre. Le mélange réactionnel est agité à température ambiante pendant 30 minutes, puis on ajoute 0,56 g (14,0 mmoles) de NaH à 60%. On agite 1h à température ambiante, puis on ajoute 100 mL d'eau. Le précipité est séparé par filtration, rincé à l'eau, essoré puis séché à l'étuve. On obtient 2,85 g du produit attendu sous forme d'un solide jaune. Rendement = 73%.

### 1. 5 : 7-Amino-8-éthyl-2-(méthylsulfonyl)-5-oxo-5,8-dihydropyrido[2,3-d]pyrimidine-6-carboxamide

A une solution de 0,60 g (2,15 mmoles) de composé obtenu en 1.4 dans 20 mL de N-méthylpyrrolidinone, on ajoute 1,08 g (4,83 mmoles) d'acide *méta*-chloroperbenzoïque. On agite à température ambiante pendant 24 heures, puis on évapore à sec. Le résidu solide est repris dans une solution de NaHCO₃ aq. puis filtré. Le solide est essoré puis séché à l'étuve. On obtient 0,4 g du produit attendu sous forme d'un solide jaune pâle. Rendement = 60%.

### 1.6 : 7-Amino-8-éthyl-2-[(4-hydroxyphényl)amino]-5-oxo-5,8-dihydropyrido[2,3-d]pyrimidine-6-carboxamide

A une solution de 0,40 g (1,29 mmoles) du produit obtenu en 1.5 dans 4 mL de DMSO anhydre, on ajoute 0,160 g (1,47 mmoles) de 4-aminophénol. Le mélange réactionnel est chauffé à 110°C pendant une nuit, puis évaporé à sec. Le résidu solide est purifié par chromatographie sur gel de silice en éluant avec un gradient dichlorométhane : méthanol (100 : 0 à 90 : 10). On obtient 0,090 g du produit attendu sous forme d'un solide blanc cassé. Rendement = 20%. PF > 260°C. M+H⁺ = 341.
RMN ¹H (DMSO-d6 ; 400 MHz) : δ 11,70 (s large, 1 H); 9,85 (s, 1 H) ; 8,89 (s, 1 H) ; 7,95 (s large, 1 H); 7,48 (d, 2H); 7,72 (d, 2H); 4,29 (q, 2H); 1,20 (t, 3H).

### Exemple 2 : 7-Amino-8-éthyl-2-[4-(4-méthyl-pipérazine-1-carbonyl)-phénylamino]-5-oxo-5,8-dihydro-pyrido[2,3-d]pyrimidine-6-carboxamide

### 2.1 (4-Méthyl-pipérazin-1-yl)-(4-nitro-phényl)-méthanone

Un mélange de 2.0 g d'acide 4-nitrobenzoïque (11,97 mmoles), 10,42 mL de diisopropyléthylamine (59,84 mmoles) et 1,46 mL de 1-méthylpipérazine (13,16 mmoles) dans 40 mL de DMF est refroidi avec un bain de glace à 2-5°C. On ajoute ensuite 11,53 g (35,9 mmoles) de tétrafluoroborate de O-benzotriazolyl tétraméthylisouronium (TBTU, n°CAS = 125700-67-6) par portions et on agite à température pendant une nuit. Le mélange réactionnel est dilué avec du dicholorométhane et une solution de NaClaq saturée. Après décantation, la phase aqueuse est extraite au dichlorométhane. Les phases organiques sont regroupées, séchées sur Na₂SO₄, filtrées et concentrées sous vide. Le résidu huileux contenant un insoluble est essoré puis rincé avec du dichlorométhane et un peu de méthanol. On obtient 1,67 g du produit attendu sous forme d'un solide rose pâle que l'on utilise tel quel sans purification ultérieure. Rendement = 56%.

### 2.2: (4-Amino-phényl)-(4-méthyl-pipérazin-1-yl)-méthanone

A 1,67 g (6,70 mmoles) de produit préparé en 2.1 partiellement solubilisé dans 50 mL de méthanol, on ajoute sous atmosphère inerte, 71 mg de palladium sur charbon à 10%. Le mélange réactionnel est agité à température ambiante sous 3 bars d'hydrogène pendant 2h30 puis filtré sur célite. Après évaporation à sec, on obtient 1,5 g du produit attendu sous forme d'une huile orange que l'on utilise telle quelle dans l'étape suivante. Rendement quantitatif.

### 2.3 : Acide 2,4-dichloro-5-pyrimidinecarboxylique

### - Méthode A :

A 10,0 g (64,06 mmoles) d'acide uracil-5-carboxylique (Aldrich Chemical Company) partiellement solubilisés dans 20 mL de DMF, on ajoute à froid 59,7 mL (0,64 mole) d'oxychlorure de phospore et 10,3 mL (64,7 mmoles) de *N*,*N*-diéthylaniline puis on chauffe le mélange à 90°C pendant 2h40. Après retour à l'ambiante et évaporation de la moitié de l'excès de POCl₃, on verse le milieu sur de la glace puis on extrait à l'éther. Les phases éthérées sont réunies, séchées sur sulfate de sodium, filtrées et concentrées sous vide. On obtient 8,1 g (41,97 mmoles) d'acide 2,4-dichloro-5-pyrimidinecarboxylique avec un rendement de 65%.

### - Méthode B :

A une solution de 9 g (42,8 mmoles) de chlorure de l'acide 2,4-dichloro-5-pyrimidinecarboxylique (Manchester Organics Limited) dans 60 mL d'éther, on ajoute 10 mL d'eau et le mélange réactionnel est agité vigoureusement à 35°C pendant 1 heure. Après addition d'éther et décantation, la phase organique est séchée sur Na₂SO₄, filtrée et concentrée sous vide. On obtient 7,7 g d'une huile incolore qui solidifie rapidement à l'air et que l'on engage immédiatement dans l'étape suivante. Rendement = 93%.

### 2.4 : Acide 2-chloro-4-(éthylamino)pyrimidinecarboxylique

On dissout 8,1 g (41,97 mmoles) d'acide 2,4-dichloro-5-pyrimidinecarboxylique dans 84 mL de THF en présence de 81,3 mL (0,923 mole) de triéthylamine. On ajoute 3.4 mL d'éthylamine aqueuse à 70% (41,97 mmoles) et on agite à température ambiante pendant 1 h45. Après évaporation des solvants, on reprend avec de l'eau et on ajoute lentement une solution aqueuse de HCl 1 N jusqu'à pH 2. On isole le précipité par filtration, on rince à l'eau puis avec un mélange éther/pentane et on sèche sous vide. On obtient 7,2 g d'un solide contenant très majoritairement l'acide 2-chloro-4-(éthylamino)pyrimidinecarboxylique et utilisé tel quel dans l'étape suivante.

### 2.5 : Fluorure d'acide 2-chloro-4-(éthylamino)pyrimidinecarboxylique

A une solution contenant 7,0 g (34,72 mmoles) d'acide préparé en 2.4 et 5,32 mL (38,19 mmoles) de triéthylamine dans 98 mL de dichlorométhane, on ajoute 5,89 mL (69,79 mmoles) de fluorure de cyanuryle. Le mélange est agité durant 3 heures et la solution est diluée avec 590 mL de CH₂Cl₂ et 190 mL de NaHCO₃aq glacée. La phase organique est lavée deux fois avec 190 mL de NaHCO₃aq glacée et séchée sur MgSO₄. Le solvant est ensuite évaporé sous pression réduite. On obtient 7,0 g de produit attendu sous forme d'une huile rouge qui solidifie lentement et que l'on engage telle quelle dans l'étape suivante. Rendement quantitatif.

### 2.6: 7-Amino-2-chloro-8-éthyl-5-oxo-5,8-dihydropyrido[2,3-d]pyrimidine-6-carboxamide

A une solution refroidie à 2-5°C avec un bain de glace de 6,29 g (74,78 mmoles) de cyanoacétamide dans 140 mL de DMF anhydre, on ajoute 5,98 g (149,55 mmoles) d'hydrure de sodium à 60 % par portions. On agite 15 minutes à 2-5°C puis on additionne rapidement cette suspension à la solution de 14,5 g (71,22 mmoles) du fluorure d'acide préparé en 2.5 dans 155 mL de DMF anhydre et préalablement refroidie à 2-5°C avec un bain de glace. Le mélange est agité une nuit à température ambiante puis on refroidit à 2-5°C et on ajoute 2,99 g (74,78 mmoles) d'hydrure de sodium à 60% par portions. On agite le milieu 4 heures à température ambiante puis on ajoute lentement de la glace pour détruire l'excès d'hydrure et on verse le mileu réactionnel sur un mélange eau-glace. On acidifie en versant une solution aqueuse d'acide chlorhydrique HCl 0,1 N. Le précipité formé est isolé par filtration, rincé à l'eau puis séché à l'étuve avant d'être rincé au pentane. On obtient finalement 14,0 g de produit attendu sous forme d'un solide orange pâle. Rendement = 73,4%.

### 2.7 : 7-Amino-8-éthyl-2-[4-(4-méthyl-pipérazine-1-carbonyl)-phénylamino]-5-oxo-5,8-dihydro-pyrido[2,3-d]pyrimidine-6-carboxamide

Dans un tube pour micro-ondes de 10 mL, on place un mélange de 0,3 g (1,12 mmole) de produit préparé en 2.6 et 491 mg (2,24 mmoles) de produit préparé en 2.2 dans 6 mL de NMP. On place le tube scellé dans le four à micro-ondes (appareil CEM, modèle Discover) et le mélange est chauffé sous pression à 120°C pendant 60 minutes à une puissance de 75 W puis refroidi à température ambiante. On ajoute 15 mL d'eau puis 5 mL de NaHCO₃aq saturée et le précipité formé est essoré puis séché à l'étuve. Le solide brut est purifié par chromatographie sur colonne de silice en éluant avec un gradient dichlorométhane : méthanol (100 : 0 à 90 : 10). On obtient 0,055 g du produit attendu sous forme d'une poudre orange. PF = 281 °C. %. M+H⁺ = 451. Rendement = 11 %.
RMN ¹H (DMSO-d₆, 400 MHz) : δ 11,8 (s large, 1H);10,3 (s, 1H);10,2 (d, 1H); 9,0 (s, 1 H); 8,0 (s large, 1 H); 7,8 (d, 2H); 7,40(d, 2H); 7,2 (d, 1 H); 4,4 (q, 2H); 3,5 (m, 4H); 3,30 (s, 3H); 2,30 (m, 4H); 1,3 (t, 3H).

### Exemple 3: 7-Amino-8-cyclopentyl-2-(4-morpholin-4-yl-phénylamino)-5-oxo-5,8-dihydro-pyrido[2,3-d]pyrimidine-6-carboxamide

### 3.1 : Acide 2-chloro-4-(cyclopentylamino)pyrimidinecarboxylique

Une suspension de 10 g (51,82 mmoles) d'acide 2,4-dichloro-5-pyrimidinecarboxylique dans 100 mL de THF est refroidie avec un bain de glace à 2-5°C et on ajoute goutte à goutte une solution de 5,12 mL (51,82 mmoles) de cyclopentylamine et de 21,67 mL (155,45 mmoles) de triéthylamine dans 40 mL de THF. On agite à température ambiante pendant 2h00. On évapore le THF puis on dilue à l'eau. On acidifie avec une solution aqueuse de HCl 1 N jusqu'à pH 2 puis on extrait deux fois avec du dichlorométhane. Les phases organiques sont regroupées, séchées sur Na₂SO₄, filtrées et concentrées sous vide. Le solide récupéré est trituré dans l'éther, essoré et séché à l'étuve. On obtient 5,28 g du produit attendu sous forme d'un solide beige que l'on utilise tel quel dans l'étape suivante. Rendement = 42%.

### 3.2 : Fluorure d'acide 2-chloro-4-(cyclopentylamino)pyrimidinecarboxylique

A une suspension de 5,24 (21,68 mmoles) de produit préparé en 3.1 dans 140 mL de dichlorométhane, on ajoute 3,02 mL (21,68 mmoles) de triéthylamine puis 2,75 mL (32,52 mmoles) de fluorure de cyanuryle. Le mélange est agité à température ambiante pendant 4 heures et la solution est diluée avec 150 mL de dichlorométhane. La phase organique est lavée trois fois avec 200 mL de NaHCO₃aq glacée, séchée sur MgSO₄, filtrée et concentrée sous vide. On obtient 5,2 g du produit attendu sous forme d'un solide orange. Rendement = 98%.

### 3.3 : 7-Amino-2-chloro-8-cyclopentyl-5-oxo-5,8-dihydropyrido[2,3-d]pyrimidine-6-carboxamide

A une solution refroidie à 2-5°C avec un bain de glace de 1,88 g (22,41 mmoles) de cyanoacétamide dans 20 mL de DMF anhydre, on ajoute 1.8 g (44,82 mmoles) d'hydrure de sodium à 60 % par portions. On agite 15 minutes à 2-5°C puis on ajoute rapidement cette suspension à une solution de 5,2 g (21,34 mmoles) du fluorure d'acide préparé en 3.2 dans 20 mL de DMF anhydre et préalablement refroidie à 2-5°C. Le mélange est agité une nuit à température ambiante puis on refroidit à 2-5°C avec un bain de glace et on ajoute 0,90 g (22,41 mmoles) d'hydrure de sodium à 60% par portions. On agite le mélange réactionnel pendant 3 heures à température ambiante puis on verse sur un mélange de glace, de 100 mL d'eau et de 150 mL de HClaq 1 N. Le précipité jaune formé est isolé par filtration, rincé à l'eau puis séché à l'étuve avant d'être rincé au pentane. On obtient finalement 5,4 g d'un mélange contenant le produit cyclisé attendu et le produit non cyclisé sous forme d'un poudre orange. Le mélange est purifié par chromatographie sur colonne de silice en éluant avec un gradient dichlorométhane : méthanol (99 : 1 à 96 : 4). On obtient finalement 0,6 g du produit attendu sous forme d'un solide jaune pâle. Rendement = 9%.

### 3.4 : 7-Amino-8-cyclopentyl-2-(4-morpholin-4-yl-phénylamino)-5-oxo-5,8-dihydro-pyrido[2,3-d]pyrimidine-6-carboxamide

Dans un tube pour micro-ondes de 10 mL, on place un mélange de 184 mg (0,60 mmole) de produit préparé en 3.3 et 0,266 g (1,49 mmole) de *N*-(4-aminophényl)morpholine (Lancaster) dans 6 mL de NMP. Le tube scellé est placé dans le four à micro-ondes (appareil CEM, modèle Discover) et le mélange est chauffé sous pression à 90°C pendant 1 heure à une puissance de 100 W puis refroidi à température ambiante. On ajoute 20 mL d'eau puis 5 mL de NaHCO₃aq saturée et le précipité formé est essoré puis séché à l'étuve. Le solide brut est purifié par chromatographie sur colonne de silice en éluant avec un gradient dichlorométhane : méthanol (98 : 2 à 95 : 5) : On obtient 0,065 g du produit attendu sous forme d'une poudre beige. Rendement = 24%. PF = 265°C. M+H⁺ = 450.
RMN ¹H (DMSO-d₆, 400 MHz) : δ 11,5-12,5 (s très large, 1H); 10,4 (d, 1H); 9,6 (s, 1H); 8,9 (s, 1 H); 7,4 (d, 2H); 7,1 (d, 1 H); 6,9 (d, 2H), 5,0 (m, 1 H); 3,7 (m, 4H); 3,0 (m, 4H); 2,2-2,5 (m, 2H); 1,3-1,9 (m, 6H)

### Exemple 4 : Chlorhydrate de 7-amino-8-éthyl-2-(2-méthoxy-4-pipéridin-4-yl-phénylamino)-5-oxo-5,8-dihydro-pyrido[2,3-d]pyrimidine-6-carboxamide

### 4.1 : 4-Bromo-2-méthoxy-1-nitro-benzène

On dissout presque totalement 18,4 g (83,64 mmoles) de 2-fluoro-4-bromo-nitrobenzène (Aldrich) dans 300 mL de méthanol anhydre. On additionne goutte à goutte 19,9 mL (106,22 mmoles) d'une solution de méthoxyde de sodium à 30% dans le methanol et on laisse agiter la nuit à températue ambiante. Le méthanol est évaporé sous pression réduite et on reprend le milieu avec de l'acétaté d'éthyle et de l'eau puis on acidifie la phase aqueuse par addition de HClaq 1 N. Après décantation, la phase organique est lavée avec NaClaq saturée, séchée sur Na₂SO₄, filtrée et concentrée sous vide. On obtient 17,4 g de produit attendu sous forme d'un solide jaune. Rendement = 89,7%

### 4.2 : 4-(3-Méthoxy-4-nitro-phényl)-3,6-dihydro-2H-pyridine-1-carboxylate de tert-butyle

Dans un mélange de 4,64 g (20,0 mmoles) de produit préparé en 4.1, de 6,25 g (20,2 mmoles) d'ester pinacolique de l'acide 3,6-dihydro-2*H*-pyridine-1-*N*-Boc-4-boronique (Frontier Scientifique) et de 8,29 g (60,0 mmoles) de carbonate de potassium dans 125 mL de DMF anhydre, on fait barboter de l'argon pendant 10 minutes. On ajoute 0,98 g (1,2 mmole) de PdCl₂dppf.CH₂Cl₂ et on chauffe sous argon à 90°C pendant 3 heures. On dilue avec de l'acétate d'éthyle et on lave deux fois à l'eau et une fois avec NaClaq saturée. La phase organique est séchée sur Na₂SO₄, filtrée et concentrée sous vide. Le solide brut obtenu est purifié par chromatographie sur colonne de silice en éluant avec un gradient cyclohexane : acétate d'éthyle (75 : 25 à 70 : 30). On obtient 6,02 g d'un solide jaune pâle que l'on triture dans le cyclohexane avant essorage et séchage à l'étuve. On récupère finalement 5,89 g de produit attendu sous forme d'un solide blanc. Rendement = 88%.

### 4.3 : 4-(4-Amino-3-méthoxy-phényl)-pipéridine-1-carboxylate de tert-butyle

Dans un autoclave d'hydrogénation, on place 4,27 g (12,77 mmoles) de produit préparé en 4.2 dans 130 mL d'un mélange acétate d'éthyle : éthanol (V/V = 1/1) et on ajoute 0,54 g de Palladium sur charbon à 10% sous atmosphère inerte. Le mélange est agité sous une pression de 3 bars d'hydrogène à température ambiante. Après filtration sur papier fin en fibre de verre et évaporation sous pression réduite, on obtient 3,87 g de produit attendu sous la forme d'un solide rose que l'on utilise tel quel dans l'étape suivante. Rendement = 99%.

### 4.4 : 4-[4-(7-Amino-6-carbamoyl-8-éthyl-5-oxo-5,8-dihydro-pyrido[2,3-d]pyrimidin-2-ylamino)-3-méthoxy-phényl]-pipéridine-1-carboxylate de tert-butyle

Dans un ballon, on place un mélange de 0,45 g (1,63 mmoles) de produit préparé en 2.6 et 1,00 g (3,25 mmoles) de produit préparé en 4.3 dans 3 mL de NMP. La suspension est chauffée à 110°C pendant 3 heures. On ajoute 30 mL d'eau puis 5 mL de NaHCO₃aq saturée et le précipité formé est essoré puis séché à l'étuve. Le solide brut est purifié par chromatographie sur colonne de silice en éluant avec un gradient dichlorométhane : méthanol (100 : 0 à 93 : 7). On obtient 0,52 g du produit attendu sous forme d'une poudre beige. Rendement = 59,5%.

### 4.5 : Chlorhydrate de 7-amino-8-éthyl-2-(2-méthoxy-4-pipéridin-4-yl-phénylamino)-5-oxo-5,8-dihydro-pyrido[2,3-d]pyrimidine-6-carboxamide

0,51g (0,95 mmole) du produit préparé en 4.4 est mis en suspension dans 10 mL d'un mélange dichlorométhane : méthanol (V/V = 8/2) et on ajoute 3,55 mL (14,20 mmoles) d'une solution d'acide chlorhydrique 4N dans le dioxane. On agite à température ambiante pendant la nuit et on ajoute de l'éther. Le solide est essoré, rincé au pentane et séché à l'étuve. On obtient 0,48 g du produit attendu sous forme d'un solide beige que l'on engage tel quel dans l'étape suivante. Rendement (dichlorhydrate) = 99,1%. PF = 117°C. M+H⁺ = 408.
RMN ¹H (DMSO-d6 ; 400 MHz) : δ 11,8 (s large, 1 H); 10, 4 (s très large, <1H); 10,2 (s, 1 H); 9,0 (s, 1 H); 8,9 (s large, 1 H); 8,1 (s large, 1 H); 7,8 (d, 1 H); 7,2 (d, 1 H); 4,3 (q, 2H); 3,4 (m, 2H), 3,0 (m, 2H); 2,8 (m, 1 H); 1,9 (m, 4H); 1,3 (t, 3H).

### Exemple 5 : Chlorhydrate de 7-amino-8-éthyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-N-méthyl-carboxamide

*5.1 : 7-Amino-8-éthyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-N-méthyl-carboxamide* 0,47 g (0,92 mmole) du chlorhydrate préparé en 4.5 est mis en suspension dans 7,8 mL d'un mélange dichlorométhane : acide acétique glacial (V/V = 5/1) et on ajoute le 3,3,3-trifluoropropionaldéhyde (Alfa Aesar) puis le triacétoxyborohydrure de sodium par portions. On agite 2,5 heures et on verse le mélange réactionnel sur 50 mL de NaHCO₃aq. On isole un solide par filtration que l'on purifie par chromatographie sur colonne de silice en éluant avec un gradient dichlorométhane : méthanol (100 : 0 à 90 : 10). Après trituration dans l'éther, on obtient finalement 0,25 g du produit attendu sous forme d'un solide beige. Rendement = 50,9%.

### 5.2 : Chlorhydrate de 7-amino-8-éthyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-N-méthyl-carboxamide

0,19 g (0,36 mmole) du produit préparé en 4.6 est mis en solution dans 4 mL d'un mélange dichlorométhane : méthanol (V/V = 4/1) et on ajoute 1,09 mL (1,09 mmole) d'acide chlorhydrique 1,0 M dans l'éther. On agite la suspension pendant 5 minutes puis on dilue à l'éther et le précipité est essoré, rincé à l'éther, au pentane et séché sous vide. On obtient 0,22 g du produit attendu sous forme d'une poudre ocre. Rendement (dichlorhydrate) = 96,5%. PF = 199°C. M+H⁺ = 534.
RMN ¹H (DMSO-d6 ; 400 MHz) : δ 11,8 (s large, 1 H); 11,2 (s très large, <1H); 10,3 (s large, 1 H); 9,0 (s, 1 H); 8,8 (s , 1 H); 8,1 (s large, 1 H); 7,9 (d, 1 H); 7,2 (s très large, <1H); 7,0 (s, 1 H); 6,9 (d, 1 H); 4,3 (q, 2H); 3,9 (s, 3H); 3,6 (m, 2H), 3,4 (m, 2H); 3,0-3,2 (m, 2H); 2,9 (m, 1 H); 2,0 (m, 4H); 1,3 (t, 3H).

### Exemple 6 : Chlorhydrate de 7-amino-2-[4-(1-cyclopropyl-pipéridin-4-yl)-2-méthoxy-phénylamino]-8-éthyl-5-oxo-5,8-dihydro-pyrido[2,3-d]pyrimidine-6-carboxamide

### 6.1 : 7-Amino-2-[4-(1-cyclopropyl-pipéridin-4-yl)-2-méthoxy-phénylamino]-8-éthyl-5-oxo-5,8-dihydro-pyrido[2,3-d]pyrimidine-6-carboxamide

Le chlorhydrate préparé en 4.5 est passé sous forme base par traitement avec NaOHaq 1 N suivi d'une filtration et séchage à l'étuve. 0,44 g (1,0 mmole) de ce produit est mis en suspension dans 10 mL de méthanol anhydre. On ajoute 0,57 mL (10,0 mmoles) d'acide acétique glacial, du tamis moléculaire 3 Å préalablement séché sous vide, 0,9 mL (4,5 mmoles) de (1-éthoxycyclopropoxy)triméthylsilane et 0,188 g (3,0 mmoles) de cyanoborohydrure de sodium. Le mélange est chauffé 1 heure à 80°C et on ajoute 2 mL de tétrahydrofurane et à nouveau 0,45 mL (2,25 mmoles) de (1-éthoxycyclopropoxy)triméthylsilane. Le mélange réactionnel est chauffé 20 heures à 80°C et on laisse revenir à température ambiante et on évapore à sec. Le résidu obtenu est purifié par chromatographie sur colonne de silice en éluant avec un gradient dichlorométhane : méthanol (96 : 4 à 90 : 10). Après trituration dans un mélange éther/isopropanol (V/V = 3/1) puis acétate d'éthyle/isopropanol, on obtient finalement 0,126 g du produit attendu sous forme d'un solide blanc. Rendement = 26%.

### 6.2 : Chlorhydrate de 7-amino-2-[4-(1-cyclopropyl-pipéridin-4-yl)-2-méthoxy-phénylamino]-8-éthyl-5-oxo-5,8-dihydro-pyrido[2,3-d]pyrimidine-6-carboxamide

A une suspension de 0,126 g (0,26 mmole) de produit préparé en 7.1 dans 2,6 mL de méthanol, on ajoute 0,79 mL (0,79 mmole) d'éther chlorhydrique 1,0 M. On agite 5 minutes à température ambiante puis on dilue à l'éther. Le précipité est essoré, rincé à l'éther, au pentane et séché sous vide. On obtient 0,133 g du produit attendu sous forme d'un solide beige. Rendement (dichlorhydrate) = 92%. PF = 226-228°C. M+H⁺ = 478.
RMN ¹H (DMSO-d₆, 400 MHz) : δ 11,8 (s large, 1H); 10,6 (s large, ∼0,5H); 10,4 (s large, ∼1,5H); 9,0 (s, 1 H); 8,8 (s, 1 H); 8,1 (s large, 1 H); 7,9 (d, 1 H); 7,2 (s très large, <1H); 7,0 (s, 1 H); 6,9 (d, 1 H); 4,3 (q, 2H); 3,9 (s, 3H); 3,6 (m, 2H); 2,8-3,4 (m, 4H); 2,0-2,2 (m, 4H); 1,30 (t, 3H); 1,20 (m, 2H); 0,80 (m; 2H)

### Exemple 7 : 7-Amino-8-éthyl-2-(3-morpholin-4-yl-phénylamino)-5-oxo-5,8-dihydro-pyrido[2,3-d]pyrimidine-6-carboxamide

Dans un tube pour micro-ondes de 10 mL, on place un mélange de 0,25 g (0,93 mmole) de produit préparé en 2.6 et 0,33 g (1,87 mmoles) de 3-morpholin-4-yl-phénylamine dans 4 mL de NMP. Le tube scellé est placé dans le four à micro-ondes (appareil CEM, modèle Discover) et le mélange est chauffé sous pression à 90°C pendant 0,5 heure à 120°C pendant 1 heure à une puissance de 100 W puis refroidi à température ambiante. On ajoute 20 mL d'eau puis 5 mL de NaHCO₃aq saturée et le précipité formé est essoré puis séché à l'étuve. Le solide brut est trituré dans le méthanol, essoré, rincé à l'éther et au pentane et séché sous vide. On obtient finalement 0,16 g du produit attendu sous forme d'une poudre beige. Rendement = 41,8%. PF = 195°C. M+H⁺ = 410.
RMN ¹H (DMSO-d₆, 400 MHz) : δ 11,8 (s large, 1H); 10,3 (d, 1H); 10,0 (s large, 1H); 8,9 (s, 1 H); 8,0 (s très large, < 1 H); 7,3 (m, 2H); 7,1-7,3 (m, 3H); 6,6 (d, 1 H), 4,4 (q, 2H); 3,7 (m, 4H); 3,1 (m, 4H); 1,2 (t, 3H)

### Exemple 8 : 2-[4-(4-Acétyl-pipérazin-1-yl)-phénylamino]-7-amino-8-éthyl-5-oxo-5,8-dihydro-pyrido[2,3-d]pyrimidine-6-carboxamide

### 8.1 : 1-[4-(4-Nitro-phényl)-pipérazin-1-yl]-éthanone

Une solution de 5,0 g (35,44 mmoles) de 1-fluoro-4-nitrobenzène et 4,99 g (38,98 mmoles) de N-acétylpipérazine dans 50 mL d'acétonitrile est chauffée à 60°C pendant 15 heures. On dilue à l'eau et à l'acétate d'éthyle. Après décantation et extraction de la phase aqueuse à l'acétate d'éthyle, les phases organiques sont regroupées, séchées sur Na₂SO₄, filtrées et concentrées sous vide. On obtient 7,7 g du produit attendu sous forme d'un solide jaune utilisé tel quel dans l'étape suivante. Rendement = 88,8%.

### 8.2 : 1-[4-(4-Amino-phényl)-pipérazin-1-yl]-éthanone

Dans un autoclave d'hydrogénation, on place 3,0 g (12,04 mmoles) de produit préparé en 8.1 dans 40 mL d' acétate d'éthyle et on ajoute 0,15 g de palladium sur charbon à 10% sous atmosphère inerte. Le mélange est agité sous une pression de 5 bars d'hydrogène à température ambiante. Après filtration sur célite et évaporation sous pression réduite, on obtient 1,6 g de produit attendu sous forme d'un solide beige que l'on utilise tel quel dans l'étape suivante. Rendement = 61%.

### 8.3 : 2-[4-(4-Acétyl-pipérazin-1-yl)-phénylamino]-7-amino-8-éthyl-5-oxo-5,8-dihydro-pyrido[2,3-d]pyrimidine-6-carboxamide

Dans un tube pour micro-ondes de 10 mL, on place un mélange de 0,30 g (1,12 mmole) de produit préparé en 2.6 et 0,49 g (2,24 mmoles) de produit préparé en 8.2 dans 5 mL de NMP. Le tube scellé est placé dans le four à micro-ondes (appareil CEM, modèle Discover) et le mélange est chauffé sous pression à 90°C pendant 30 minutes à une puissance de 100 W puis refroidi à température ambiante. On ajoute 20 mL d'eau puis 5 mL de NaHCO₃aq saturée et le précipité formé est essoré puis séché à l'étuve. Le solide brut est trituré dans le méthanol, essoré, rincé à l'éther et au pentane et séché sous vide. On obtient finalement 0,30 g du produit attendu sous forme d'une poudre beige. Rendement = 60%. PF = 179°C. M+H⁺ = 548.
RMN ¹H (DMSO-d₆, 400 MHz) : δ 11,7 (s très large, <1 H); 10,4 (d, 1 H), 9,9 (s, 1 H); 8,9 (s, 1 H); 8,0 (s très large, <1 H); 7,6 (d, 2H); 7,1 (d, 1 H); 6,9 (d, 2H); 4,4 (q, 2H); 3,6 (m, 4H); 3,1 (m, 4H); 2,0 (s, 3H); 1,3 (t, 3H)

### Exemple 9 : 7-Amino-2-[4-(cyclopropanecarbonyl-méthyl-amino)-phénylamino]-8-éthyl-5-oxo-5,8-dihydro-pyrido[2,3-d]pyrimidine-6-carboxamide

### 9.1 : Méthyl-(4-nitro-phényl)-cyclopropanecarboxamide

A une solution de 2,0 g (13,14 mmoles) de *N*-méthyl-4-nitroaniline (Aldrich) et de 1,59 mL (19,72 mmoles) de pyridine dans 15 mL de tétrahydrofurane, on ajoute lentement 1,43 mL (15,77 mmoles) de chlorure de cyclopropanoyle. On porte au reflux pendant 2 heures et on évapore à sec. On reprend dans un mélange acétate d'éthyle/eau puis la phase organique est séchée sur Na₂SO₄, filtrée et concentrée sous vide. On obtient le produit attendu sous forme d'une huile jaune qui est engagée telle quelle dans l'étape suivante.

### 9.2 : Méthyl-(4-amino-phényl)-cyclopropanecarboxamide

A une suspension de 3,56 g (54,49 mmoles) de poudre de zinc dans 15 mL d'une solution de NH₄OHaq concentrée à 33%, on ajoute 1,2 g (5,45 mmoles) de produit préparé en 9.1 en solution dans 15 mL de tétrahydrofurane. On agite 1 heure à température ambiante puis le zinc est séparé par filtration et le mélange est extrait à l'éther. Les phases organiques sont regroupées, séchées sur Na₂SO₄, filtrées et concentrées sous vide. Le résidu huileux est purifié par chromatographie sur colonne de silice en éluant avec un gradient cyclohexane : acétate d'éthyle (70 : 30 à 60 : 40). On obtient 3 g du produit attendu sous forme d'une huile jaune. Rendement quantitatif.

### 9.3 : 7-Amino-2-[4-(cyclopropanecarbonyl-méthyl-amino)-phénylamino]-8-éthyl-5-oxo-5,8-dihydro-pyrido[2,3-d]pyrimidine-6-carboxamide

Un mélange de 0,30 g (1,12 mmole) de produit préparé en 2.6 et 0,43 g (2,24 mmoles) de produit préparé en 9.2 dans 4,5 mL de NMP est chauffé à 90°C pendant 3 heures. Après addition d'eau, aucun précipité ne se forme. On laisse 48 heures à température ambiante et on essore le précipité formé. Après trituration dans le dichlorométhane, on obtient 0,17 g de produit attendu sous forme solide. PF = 248°C. M+H⁺ = 422. Rendement = 36%
RMN ¹H (DMSO-d₆, 400 MHz) : δ 11,8 (s très large, <1H); 10,2-10,4 (m, 2H), 9,0 (s, 1 H); 8,0 (s très large, <1 H); 7,8 (d, 2H); 7,4 (d, 1 H); 7,2 (d, 1 H); 4,3 (q, 2H); 3,1 (s, 3H); 1,4 (m, 1 H); 1,2 (t, 3H); 0,8 (m, 2H); 0,6 (m, 2H)

### Exemple 10 : Chlorhydrate du 7-amino-8-éthyl-2-[4-(4-éthyl-pipérazin-1-yl)-2-fluoro-6-méthoxy-phénylamino]-5-oxo-5,8-dihydro-pyrido[2,3-d]pyrimidine-6-carboxamide

### 10.1 : 1,5-Difluoro-3-méthoxy-2-nitro-benzène

A une solution de 2,45 g (14,0 mmoles) de 1,3,5-trifluoro-2nitrobenzène dans l'acétone, on ajoute successivement 2,9 g (21,0 mmoles) de K₂CO₃ en poudre et 3,5 mL (56,0 mmoles) d'iodure de méthyle. Le mélange réactionnel est chauffé à 50°C pendant 2 heures puis filtré et évaporé à sec. Le résidu est repris avec de l'acétate d'éthyle, lavé à l'eau et avec une solution de NaClaq saturée. La phase organique est séchée sur Na₂SO₄, filtrée et concentrée sous vide. On récupère 2,52 g de produit attendu sous forme d'un solide jaune que l'on utilise tel quel dans l'étape suivante. Rendement = 95%.

### 10.2 : 4-(3-Fluoro-5-méthoxy-4-nitro-phényl)-pipérazine-1-carboxylate de tert-butyle

A une solution de 2,48 g (13,1 mmoles) du produit préparé en 10.1 dans 26 mL d'acétonitrile, on ajoute 2,43 g (13,1 mmoles) de pipérazinecarboxylate de tert-butyle et 3,36 mL (19,65 mmoles) de diisopropyléthylamine. Le mélange réactionnel est chauffé à 90°C pendant 20 heures. On dilue à l'acétate d'éthyle et on lave à l'eau puis avec une solution de NaCl_{aq} saturée. Le solide brut est purifié par chromatographie sur colonne de silice en éluant avec un gradient cyclohexane : acétate d'éthyle (85 : 15 à 50 : 50). On isole une fraction de 3,25 g contenant majoritairement le régioisomère 4-(5-fluoro-3-méthoxy-2-nitro-phényl)-pipérazine-1-carboxylicate de tert-butyle. Une fraction secondaire impure est purifiée à nouveau sur colonne de silice en éluant avec un gradient dichlorométhane : acétate d'éthyle (96 : 4 à 94 : 6). On obtient finalement 0,985 g de produit attendu sous forme d'un solide jaune. Rendement = 21 %.

### 10.3 : 4-(4-Amino-3-fluoro-5-méthoxy-phényl)-pipérazine-1-carboxylate de tert-butyle

Dans un autoclave d'hydrogénation, on place 0,975 g (2,74 mmoles) de produit préparé en 9.2 dans 55 mL d'un mélange acétate d'éthyle : éthanol (V/V = 1/1) et on ajoute 0,14 g de palladium sur charbon à 10% sous atmosphère inerte. Le mélange est agité sous une pression de 3 bars d'hydrogène à température ambiante pendant 4h. Après filtration sur papier fin en fibre de verre et évaporation sous pression réduite, on obtient 0,88 g de produit attendu sous forme d'un solide violet que l'on utilise tel quel dans l'étape suivante. Rendement = 99%.

### 10.4 : 4-[4-(7-Amino-6-carbamoyl-8-éthyl-5-oxo-5,8-dihydro-pyrido[2,3-d]pyrimidin-2-ylamino)-3-fluoro-5-méthoxy-phenyl]-pipérazine-1-carboxylate de tert-butyle

Dans un ballon, on place un mélange de 0,36 g (1,35 mmole) de produit préparé en 2.6 et 0,88 g (2,70 mmoles) de produit préparé en 10.3 dans 3 mL de NMP. La suspension est chauffée à 100°C pendant 5,5 heures. On ajoute 50 mL d'eau puis 5 mL de NaHCO₃aq saturée et le précipité formé est essoré puis séché à l'étuve. Le solide brut est purifié par chromatographie sur colonne de silice en éluant avec un gradient dichlorométhane : méthanol (100 : 0 à 96 : 4). On obtient 0,149 g du produit attendu sous forme d'un solide violet. Rendement = 20%.

### 10.5 : Chlorhydrate de 7-amino-8-éthyl-2-(2-fluoro-6-méthoxy-4-pipérazin-1-yl-phénylamino)-5-oxo-5,8-dihydro-pyrido[2,3-d]pyrimidine-6-carboxamide

0,14g (0,23 mmole) du produit préparé en 10.4 est mis en suspension dans 4 mL de méthanol et on ajoute 1,15 mL (4,61 mmoles) d'une solution d'acide chlorhydrique 4N dans le dioxane. On agite à température ambiante pendant la nuit et on ajoute de l'éther. Le solide est essoré, rincé au pentane et séché à l'étuve. On obtient 0,10 g du produit attendu sous forme d'une poudre grise que l'on engage telle quelle dans l'étape suivante. Rendement (dichlorhydrate) = 95%.

### 10. 6 : 7-Amino-8-éthyl-2-[4-(4-éthyl-pipérazin-1-yl)-2-fluoro-6-méthoxy-phénylamino]-5-oxo-5,8-dihydro-pyrido[2,3-d]pyrimidine-6-carboxamide

0,1 g (0,23 mmole) du chlorhydrate préparé en 10.5 est mis en suspension dans 3 mL de 1,2-dichloroéthane et on ajoute 0,13 mL (0,72 mmole) de diisopropyléthylamine, 0,04 mL (1,2 mmoles) d'acétaldéhyde puis 0,10 g (0,48 mmole) de triacétoxyborohydrure de sodium par portions. On agite 2 heures et on verse sur une solution de soude 0,25 N et on ajoute du dichlorométhane. Après décantation, la phase aqueuse est extraite au dichlorométhane et les phases organiques sont regroupées, séchées sur Na₂SO₄, filtrées et concentrées sous vide. On récupère un solide marron clair que l'on purifie par chromatographie sur colonne de silice en éluant avec un gradient dichlorométhane : méthanol (95 : 5 à 90 : 10). On obtient 0,085 g du produit attendu sous forme d'un solide vitreux jaune. Rendement = 73%.

### 10.7 : Chlorhydrate du 7-amino-8-éthyl-2-[4-(4-éthyl-pipérazin-1-yl)-2-fluoro-6-méthoxy-phénylamino]-5-oxo-5,8-dihydro-pyrido[2,3-d]pyrimidine-6-carboxamide

A une solution de 0,08 g (0,18 mmole) de produit préparé en 10.6 dans 5 mL de méthanol, on ajoute 0,53 mL (0,53 mmole) d'éther chlorhydrique 1.0 M. On agite 30 minutes à température ambiante puis on dilue à l'éther. Le précipité est essoré, rincé à l'éther, au pentane et séché sous vide. On obtient 0,133 g du produit attendu sous forme d'un solide beige. Rendement (dichlorhydrate) = 77%. PF > 260°C. M+H⁺ = 485. RMN ¹H (DMSO-d₆, 400 MHz) : δ 11,8 (s large, 1H); 11,0 (s large, 1H), 9,1 (s large, 1 H); 8,9 (s large, 1 H); 8,0 (s large, 1 H), 6,8-7,4 (s très large, < 1 H); 6,45 (d, 1 H); 6,4 (s, 1 H); 3,5-4,4 (s + m, 9H); 3,0-3,3 (m, 6H); 1,3 (t, 3H); 1,0-1,3 (m, 3H)

### Exemple 11 : Chlorhydrate du 7-amino-8-(3-amino-propyl)-2-(4-morpholin-4-yl-phénylamino)-5-oxo-5,8-dihydro-pyrido[2,3-d]pyrimidine-6-carboxamide

### 11.1 : Acide 4-(3-tert-butoxycarbonylamino-propylamino)-2-chloro-pyrimidine-5-carboxylique

Une solution de 2,21 g (11,45 mmoles) d'acide 2,4-dichloro-5-pyrimidinecarboxylique préparé en 2.3 (méthode B) dans 20 mL de THF anhydre est refroidie à 2-5°C avec un bain de glace. On ajoute ensuite goutte à goutte une solution de 4,79 mL (34,35 mmoles) de triéthylamine et 2 g (11,48 mmoles) d'ester tert-butylique de l'acide (3-amino-propyl)-carbamique dans 15 mL de THF anhydre. Le mélange réactionnel est agité à température ambiante pendant 30 minutes. On ajoute de l'acétate d'éthyle et la phase organique est lavée avec HClaq 1 N et à l'eau. Les phases organiques sont regroupées, séchées sur sulfate de sodium, filtrées et concentrées sous vide. On obtient 3,35 g du produit attendu sous forme d'un solide jaune. Rendement = 88,6%.

### 11.2 : Ester tert-butylique de l'acide [3-(2-chloro-5-fluorocarbonyl-pyrimidin-4-ylamino)-propyl]-carbamique

A une suspension de 4,91 g (14,85 mmoles) de produit préparé en 11.1 dans 75 mL de dichlorométhane, on ajoute 2,07 mL (14,85 mmoles) de triéthylamine puis 1,88 mL (22,28 mmoles) de fluorure de cyanuryle. Le mélange est agité à température ambiante pendant 2 heures et la solution est diluée avec 100 mL de dichlorométhane. La phase organique est lavée trois fois avec 100 mL de NaHCO₃aq glacée, séchée sur MgSO₄, filtrée et concentrée sous vide. On obtient 4,56 g du produit attendu que l'on engage directement dans l'étape suivante. Rendement = 98%.

### 11.3 : Ester tert-butylique de l'acide [3-(7-amino-6-carbamoyl-2-chloro-5-oxo-5H-pyrido[2,3-d]pyrimidin-8-yl)-propyl]-carbamique

A une solution refroidie à 2-5°C avec un bain de glace de 1,21 g (14,39 mmoles) de cyano-acétamide dans 20 mL de DMF anhydre, on ajoute 1,73 g (43,17 mmoles) d'hydrure de sodium à 60 % par portions. On agite 15 minutes à 2-5°C puis on additionne rapidement cette suspension à la solution de 4,56 g (13,70 mmoles) du fluorure d'acide préparé en 11.2 dans 30 mL de DMF anhydre et préalablement refroidie à 2-5°C avec un bain de glace. Le mélange est agité une nuit à température ambiante puis on refroidit à 2-5°C et on ajoute encore 0,22 g d'hydrure de sodium à 60%. On agite le milieu pendant une nuit à température ambiante puis on ajoute lentement de la glace pour détruire l'excès d'hydrure et on verse le mélange réactionnel sur un mélange eau-glace. On acidifie en versant une solution aqueuse d'acide chlorhydrique HCl 1 N. Le précipité formé est isolé par filtration, rincé à l'eau puis séché. On obtient le produit attendu sous forme d'un solide orange que l'on utilise tel quel dans l'étape suivante. Rendement = 64%.

### 11.4 : {3-[7-Amino-6-carbamoyl-2-(4-morpholin-4-yl-phenylamino)-5-oxo-5H-pyrido[2,3-d]pyrimidin-8-yl]-propyl}-carbamic acid tert-butyl ester

Dans un tube pour micro-ondes de 10 mL, on place un mélange de 0,60 g (1,51 mmole) de produit préparé en 11.3 et 0,53 g (3,02 mmoles) de 4-morpholin-4-yl-phenylamine dans 6,5 mL de NMP. On place le tube scellé dans le four à micro-ondes (appareil CEM, modèle Discover) et le mélange est chauffé sous pression à 120°C pendant 60 minutes à une puissance de 75 W puis refroidi à température ambiante. On ajoute 15 mL d'eau puis 5 mL de NaHCO₃aq saturée et le précipité formé est essoré puis séché à l'étuve. Le solide brut est trituré dans le méthanol, essoré et séché à l'étuve sous vide. On obtient 0,159 g du produit attendu sous forme solide. Rendement = 20%.

### 11.5 : Chlorhydrate du 7-amino-8-(3-amino-propyl)-2-(4-morpholin-4-yl-phenylamino)-5-oxo-5,8-dihydro-pyrido[2,3-d]pyrimidine-6-carboxamide

0,16 g (0,30 mmole) du produit préparé en 11.4 est mis en suspension dans 4 mL de dioxane anhydre et on ajoute 0,74 mL (2,95 mmoles) d'une solution d'acide chlorhydrique 4N dans le dioxane. On agite à température ambiante pendant 5 heures et on ajoute de l'éther. Le solide est essoré, rincé au pentane et séché à l'étuve. On obtient 0,14 g du produit attendu sous forme d'une poudre blanche. Rendement (dichlorhydrate) = quantitatif. PF = 250°C. M+H⁺ = 439.
RMN ¹H (DMSO-d₆, 400 MHz) : δ 11,9 (s large, 1H); 10,2-10,4 (s très large, <1H); 10,2 (s, 1 H); 9,0 (s, 1 H); 8,4 (s large, 1 H); 8,2 (d, 2H); 7,7 (d, 2H); 7,4 (s large, 1 H); 7,1-7,5 (s très large, <1 H); 4,4 (m, 2H); 3,9 (m, 4H); 3,3 (m, 4H); 2,9 (m, 2H); 2,1 (m, 2H)

### Exemple 12 : 7-Amino-8-éthyl-2-[4-(2-hydroxy-éthyl)-phénylamino]-5-oxo-5,8-dihydro-pyrido[2,3-d]pyrimidine-6-carboxamide

Un mélange de 2,1 g (7,89 mmoles) de produit préparé en 2.6 et 2,17 g (15,79 mmoles) de 2-(4-amino-phenyl)-éthanol dans 10 mL de NMP est chauffé à 100°C pendant 3 heures dans un tube scellé. Le mélange réactionnel chaud est versé sur un mélange de 125 mL d'eau et 25 mL de NaHCO₃aq saturée. Le précipité formé est essoré, rincé à l'eau puis séché à l'étuve sous vide. Après trituration dans l'éther, on obtient 2,5 g de produit attendu sous forme d'un solide marron clair. Rendement = 89,2%.

Un échantillon analytiquement pur est obtenu par trituration de 0,2 g du solide précédent dans le minimum de méthanol, essorage, rinçage à l'éther et séchage à la pompe. On récupère ainsi 0,15 g de produit attendu pur sous forme d'une poudre beige.
PF = 289°C. M+H⁺ = 369.
RMN ¹H (DMSO-d6 ; 400 MHz) : δ 11,8 (s large, 1H); 10,4 (d, 1H); 10,1 (s large, 1H); 9,0 (s, 1 H); 8,0 (s large, 1 H); 7,7 (d, 2H); 7,2 (d, 2H); 4,6 (t, 1 H); 4,4 (m, 2H); 3,6 (m, 2H), 2,7 (m, 2H); 1,3 (t, 3H).

### Exemple 13 : Chlorhydrate du 7-amino-8-éthyl-5-oxo-2-[4-(2-pipéridin-1-yl-éthyl)-phénylamino]-5,8-dihydro-pyrido[2,3-d]pyrimidine-6-carboxamide

### 13.1 : Ester éthylique de l'acide 2-[4-(7-amino-6-carbamoyl-8-éthyl-5-oxo-5,8-dihydro-pyrido[2,3-d]pyrimidin-2-ylamino)-phényl]-méthanesulfonique

A une suspension de 1,0 g (2,71 mmoles) du produit de l'exemple 12 dans 50 mL de tétrahydrofurane refroidie à 2-5°C avec un bain eau-glace, on ajoute 1,51 mL (10,86 mmoles) de triéthylamine puis goutte à goutte 0,84 mL (10,86 mmoles) de chlorure de méthanesulfonyle. Le mélange réactionnel est chauffé à 50°C pendant 2 heures. On verse sur de l'eau et on ajoute de l'acétate d'éthyle. La phase organique est lavée avec HCl_{aq} 0,5N et NaCl_{aq} saturée puis séchée sur Na₂SO₄, filtrée et concentrée sous vide. On obtient 0,94 g du produit attendu sous forme d'un solide marron avec une pureté suffisante pour effectuer l'étape suivante.

### 13.2 : Chlorhydrate du 7-amino-8-éthyl-5-oxo-2-[4-(2-pipéridin-1-yl-éthyl)-phénylamino]-5,8-dihydro-pyrido[2,3-d]pyrimidine-6-carboxamide

Dans un ballon on place 1,1 g (2,46 mmoles) de produit préparé en 13.1 et 1,17 mL (11,83 mmoles) de pipéridine. Le mélange est chauffé à 60°C pendant 2 heures puis concentré. Le résidu est purifié par chromatographie sur colonne de silice en éluant avec un gradient dichlorométhane : méthanol (100 : 0 à 90 : 10). On obtient 0,12 g (0,28 mmole) du produit attendu sous forme base que l'on salifie en traitant avec 0,55 mL (0,55 mmole) d'éther chlorhydrique 1 M dans le méthanol. PF = 216°C. M+H⁺ = 436.
RMN ¹H (DMSO-d₆, 400 MHz) : δ 11,8 (s large, 1 H); 10,4 (s large, ∼1H); 10,2 (s, 1 H); 9,0 (s, 1 H); 8,1 (s large, 1 H); 7,7 (d, 2H); 7,1-7,3 (s très large, < 1 H); 4,4 (q, 2H); 3,5 (m, 2H); 3,2 (m, 2H); 3,1 (m, 2H); 2,9 (m, 2H); 1,7-1,9 (m, 5H); 1,4 (m, 1 H), 1,3 (t, 3H)

### Exemple 14 : Chlorhydrate du 7-amino-2-[4-(4-cyclopropyl-pipérazin-1-yl)-2-difluorométhoxy-phénylamino]-8-éthyl-5-oxo-5,8-dihydro-pyrido[2,3-d]pyrimidine-6-carboxamide

### 14.1 : 2-Difluorométhoxy-4-fluoro-1-nitro-benzène

A une solution de 3,14 g (20,0 mmoles) 5-fluoro-2-nitrophénol dans 36 mL de DMF, on ajoute 6,1 g (40,0 mmoles) de chlorodifluoroacétate de sodium et 3,31 g (24,0 mmoles) de carbonate de sodium en poudre. On chauffe le mélange réactionnel à 100°C pendant 4h30 et on laisse revenir à température ambiante. On ajoute une solution de HClaq 4N et le mélange est agité à l'ambiante pendant 2 h. On dilue avec 100 mL d'eau et 100 mL d'éther. La phase aqueuse est extraite à l'éther puis les phases organiques sont regroupées, lavées avec une solution de NaOHaq 1 N puis NaClaq saturée, séchées sur Na₂SO₄, filtrées et concentrées sous vide. On obtient 3,68 g de produit attendu sous forme d'une huile jaune que l'on engage tel quel dans l'étape suivante. Rendement = 89%.

### 14.2 : 1-Cyclopropyl-4-(3-difluorométhoxy-4-nitro-phényl)-pipérazine

A 2,07 g (10,0 mmoles) du produit préparé en 14.1 dans 30 mL d'acétonitrile on ajoute 5,99 mL (35,0 mmoles) de diispropyléthylamine puis 2, 09 g (10,5 mmoles) de N-cyclopropylpipérazine (fournisseur) finement broyée. Le mélange réactionnel est chauffé à 100°C pendant 1 h30, refroidi et concentré sous vide. Le résidu est repris dans 75 mL d'acétate d'éthyle et lavé avec une solution de NaHCO₃aq saturée et de NaClaq saturée. La phase organique est séchée sur Na₂SO₄, filtrée et concentrée sous vide. Après trituration dans le cyclohexane, le solide est essoré et séché à l'étuve sous vide. On obtient 2,33 g de produit attendu sous forme d'un solide jaune que l'on engage tel quel dans l'étape suivante. Rendement = 74,5%.

### 14.3 4-(4-Cyclopropyl-pipérazin-1-yl)-2-difluorométhoxy-phénylamine

Dans un autoclave d'hydrogénation, on place 2,32 g (7,4 mmoles) de produit préparé en 14.2 dans 50 mL d'un mélange acétate d'éthyle/éthanol (V/V = 1/1) et on ajoute 0,196 g de palladium sur charbon à 10% sous atmosphère inerte. Le mélange est agité sous une pression de 2,5 bars d'hydrogène à température ambiante pendant 1 h45. Après filtration sur papier fin en fibre de verre et évaporation sous pression réduite, on obtient 2,09 g de produit attendu sous forme d'un solide beige que l'on utilise tel quel dans l'étape suivante. Rendement quantitatif.

### 14.4 : 7-Amino-2-[4-(4-cyclopropyl-pipérazin-1-yl)-2-difluorométhoxy-phénylamino]-8-éthyl-5-oxo-5,8-dihydro-pyrido[2,3-d]pyrimidine-6-carboxamide

Dans un ballon, on place un mélange de 0,44 g (1,65 mmole) de produit préparé en 2.6 et 0,93 g (3,3 mmoles) de produit préparé en 14.3 dans 3 mL de NMP. La solution est chauffée à 100°C pendant 18 heures. On ajoute 50 mL d'eau puis 5 mL de NaHCO₃aq saturée et le précipité formé est essoré puis séché à l'étuve. Le solide brut est purifié par chromatographie sur colonne de silice en éluant avec un gradient dichlorométhane : méthanol (100 : 0 à 90 : 10). On obtient finalement 0, 117 g du produit attendu sous forme d'un solide jaune. Rendement = 12%.

### 14.5 : Chlorhydrate du 7-amino-2-[4-(4-cyclopropyl-pipérazin-1-yl)-2-difluorométhoxy-phénylamino]-8-éthyl-5-oxo-5,8-dihydro-pyrido[2,3-d]pyrimidine-6-carboxamide

0,11 g (0,21 mmole) du produit préparé en 14.4 est mis en suspension dans 3 mL de dioxane anhydre et on ajoute 0,63 mL (0,63 mmoles) d'une solution d'acide chlorhydrique 1 M dans l'éther. On dilue à l'éther puis le solide est essoré, rincé au pentane et séché à l'étuve. On obtient 0,11 g du produit attendu sous forme d'une poudre jaune. Rendement (dichlorhydrate) = 87%. PF = 204-206°C. M+H⁺ = 515.
RMN ¹H (DMSO-d₆, 400 MHz) : δ 11,8 (s large, 1H); 11,0 (s large, 1H); 10,1-10,6 (s très large, <1 H); 9,2 (s, 1 H); 8,9 (s, 1 H); 8,0(s large, 1 H); 7,6 (d, 2H); 7,1 (t, 1 H); 7,0 (d, 1 H); 6,9 (s, 1 H); 4,3 (m, 2H); 3,9 (m, 2H); 3,6 (m, 2H); 3,4 (m, 2H); 3,2 (m, 2H); 3,0 (m, 1H); 1,1-1,3 (m, 5H); 0,8 (m, 2H)

### Exemple 15 : Chlorhydrate du (±)-7-amino-2-[4-(trans-2-diméthylamino-cyclopropyl)-phénylamino]-8-éthyl-5-oxo-5,8-dihydro-pyrido[2,3-d]pyrimidine-6-carboxamide

### 15.1 : Acide (±)-trans-2-(4-nitro-phényl)-cyclopropanecarboxylique

On ajoute 20,3 g (125,0 mmoles) d'acide (±)-trans-2-phényl-cyclopropanecarboxylique (Aldrich) par portions à 150 mL d'une solution d'acide nitrique concentrée à 70%. On agite 2h à température ambiante et on observe la formation d'une fine poudre blanche dans le milieu réactionnel. On refroidit à 10°C, on essore le solide et on le rince 4 fois avec 40 mL d'eau puis on le sèche à l'étuve. On solubilise partiellement le solide dans 700 mL de xylène à 160°C et on laisse revenir à température ambiante. Le solide est essoré, rincé au xylène puis au pentane, et on le sèche à l'étuve sous vide. On obtient 12,6 g d'un solide blanc cassé contenant un mélange 90/10 du produit attendu et du régioisomère nitré en ortho. Le mélange est engagé tel quel dans l'étape suivante. Rendement = 48%.

### 15.2 : Ester tert-butylique de l'acide (±)-[trans-2-(4-nitro-phényl)-cyclopropyl]-carbamique

A une suspension de 12,4 g (60,0 mmoles) du mélange préparé en 15.1 dans 150 mL de tert-butanol, on ajoute 9,2 mL (66,0 mmoles) de Et₃N puis goutte à goutte 14,2 mL (66,0 mmoles) d'azoture de diphénylphosphoryle. Le mélange réactionnel est chauffé à 95°C pendant 4h30 puis on revient à la température ambiante. On dilue avec 300 mL d'acétate d'éthyle et la phase organique est lavée avec 150 mL de NaHCO₃aq saturée puis avec NaClaq saturée. Après avaporation, on reprend le résidu solide dans 50 mL d'acétate d'éthyle et 100 mL de cyclohexane et on chauffe jusqu'à dissolution complète. On laisse revenir à l'ambiante, on essore le précipité et on le rince au cyclohéxane et au pentane. Le solide rosé est solubilisé presque totalement dans 150 mL de CH₂Cl₂ puis filtré sur silice en éluant avec du CH₂Cl₂ suivi d'un mélange dichlorométane/acétate d'éthyle (1/4). On évapore le filtrat et on obtient 7,12 g du produit attendu sous forme d'un solide marron clair. Rendement = 42,5%.

### 15.3 : Ester tert-butylique de l'acide (±)-[trans-2-(4-amino-phényl)-cyclopropyl]-carbamique

On dissout à chaud 1,39 g (5,0 mmoles) de produit préparé en 15.2 dans 25 mL d'acétate d'éthyle. Après retour à température ambiante, on ajoute successivement 25 mL d'éthanol absolu et 0,057 g (0,25 mmole) d'oxyde (IV) de platine. On place le mélange réactionnel sous une pression d'hydrogène de 2,75 bars pendant 2 h et on le filtre sur papier fin en fibre de verre. On concentre le filtrat sous vide et on le purifie par chromatographie sur colonne de silice en éluant avec un gradient cyclohexane : acétate d'éthyle (de 70 : 30 à 55 : 45). On obtient 1,05 g du produit attendu. Rendement = 85%.

### 15.4 : Ester tert-butylique de l'acide (±)-{trans-2-[4-(7-amino-6-carbamoyl-8-éthyl-5-oxo-5,8-dihydro-pyrido[2,3-d]pyrimidin-2-ylamino)-phényl]-cyclopropyl}-carbamique

Dans un ballon, on place un mélange de 0,87 g (3,5 mmoles) de produit préparé en 2.6 et 0,47 g (1,75 mmoles) de produit préparé en 15.3 dans 3 mL de NMP. La solution est chauffée à 100°C pendant 18 heures. On ajoute 50 mL d'eau puis 5 mL de NaHCO₃aq saturée et le précipité formé est essoré puis séché à l'étuve. Le solide brut est purifié par chromatographie sur colonne de silice en éluant avec un gradient dichlorométhane : méthanol (98 : 2 à 92,5 : 7,5). On obtient finalement 0,46 g du produit attendu sous forme d'un solide orangé. Rendement = 55%.

### 15.5 : Chlorhydrate (±)-7-Amino-2-[4-(trans-2-amino-cyclopropyl)-phénylamino]-8-éthyl-5-oxo-5,8-dihydro-pyrido[2,3-d]pyrimidine-6-carboxamide

A une suspension de 0,46 g (0,96 mmole) du produit préparé en 15.4 dans 20 mL d'un mélange dichlorométhane/méthanol (1/1), on ajoute 4,8 mL (19,2 mmoles) d'acide chlorhydrique 4N dans le dioxane. On agite 5h à température ambiante puis on dilue à l'éther et le solide est essoré, rincé à l'éther et séché à l'étuve sous vide. On obtient 0,48 g d'un solide violet foncé que l'on engage tel quel dans l'étape suivante. Rendement (dichlorhydrate) quantitatif.

### 15.6: (±)-7-Amino-2-[4-(trans-2-diméthylamino-cyclopropyl)-phénylamino]-8-éthyl-5-oxo-5,8-dihydro-pyrido[2,3-d]pyrimidine-6-carboxamide

A une suspension de 0,19 g (0,43 mmole) du produit préparé en 15.5 dans 5,4 mL d'acétonitrile, on ajoute 0,22 mL (1,29 mmoles) de diisopropyléthylamine, 0,64 mL (8,58 mmoles) de formaline à 70%. Après 3 mn d'agitation, on ajoute 0,13 g (2,14 mmoles) de cyanoborohydrure de sodium et on agite 3 h à température ambiante. Le mélange réactionnel est concentré et repris dans 6 mL d'eau et 3 mL de NaOHaq à 35%. On essore le solide et on le sèche à l'étuve sous vide. On le purifie par chromatographie sur colonne de silice en éluant avec un mélange dichlorométhane : méthanol (98 : 2 à 85 : 15) contenant des traces de NH₄OHaq concentrée. On obtient 0,054 g de produit attendu. Rendement = 31%.

### 15.7 : Chlorhydrate du (±)-7-Amino-2-[4-(trans-2-diméthylamino-cyclopropyl)-phénylamino]-8-éthyl-5-oxo-5,8-dihydro-pyrido[2,3-d]pyrimidine-6-carboxamide

A une solution de 0,05 g (0,13 mmole) de produit préparé en 15.6 dans 2,5 mL de méthanol, on ajoute 0,20 mL (0,4 mmole) d'éther chlorhydrique 2,0 M. On agite 5 mn à température ambiante puis on évapore à sec. Le résidu est purifié par CLHP préparative. La fraction correspond au produit pur attendu est évaporée à sec puis reprise dans du méthanol. On ajoute de l'éther et on essore le solide que l'on sèche à l'étuve sous vide. On obtient 0,031 g du produit attendu. Rendement (dichlorhydrate) = 49%. PF = 208-210°C (decomposition). M+H⁺ = 408.
RMN ¹H (DMSO-d₆, 400 MHz) : δ 11,8 (s large, 1H); 10,7 (s large, 1H); 10,1-10,4 (s très large, <1H); 10,2 (s, 1 H); 9,0 (s, 1 H); 8,1 (s large, 1 H), 7,7 (d, 1 H); 6,45 (d, 1 H); 7,2 (d, 1 H); 4,4 (m, 2H); 3,1 (m, 1 H); 2,9 (d, 3H); 2,85 (d, 3H); 2,7 (m, 1 H); 1,7 (m, 1 H); 1,2-1,4 (m + t, 4H)

### Exemple 16 : Chlorhydrate du 8-éthyl-2-{2-méthoxy-4-[1-(3,3,3-trifluoro-propyl)-pipéridin-4-yl]-phénylamino}-7-méthylamino-5-oxo-5,8-dihydro-pyrido[2,3-d]pyrimidine-6-carboxamide

*16.1* : *Chlorhydrate du 4-(3-méthoxy-4-nitro-phényl)-1,2,3,6-tétrahydro-pyridine* On refroidit à 2-5°C une solution de 10,0 g (29,91 mmoles) de produit préparé en 4.2 dans 120 mL de dichlorométhane avec un bain eau-glace puis on ajoute lentement en 1 h, 60 mL d'une solution de HCl 4N dans le dioxane (Aldrich). Le mélange réactionnel est agité à température ambiante pendant la nuit. Le solide formé est essoré, rincé à l'éther puis séché à l'étuve. On récupère 7,6 g du produit attendu sous forme d'un solide beige. Rendement = 94%.

### 16. 2 : 4-(3-Méthoxy-4-nitro-phényl)-1-(3,3,3-trifluoro-propyl)-1,2,3,6-tétrahydropyridine

On refroidit une solution de 6,6 g (24,38 mmoles) du chlorhydrate préparé en 16.1 dans 110 mL de dichlorométhane avec un bain eau-glace. On ajoute successivement 14 mL (243,8 mmoles) d'acide acétique glacial, 8,19 g (73,14 mmoles) de 3,3,3,-trifluoropropionaldéhyde et 12,9 g (60,95 mmoles) de triacétoxyborohydrure de sodium par portions. On agite 3 h à température ambiante puis on dilue avec du dichlorométhane, de l'eau et une solution de NaOHaq 1 N. On extrait la phase aqueuse au dichlorométhane et les phases organiques sont regroupées, séchées sur Na₂SO₄, filtrées et concentrées sous vide. On récupère 8,4 g d'une huile jaune contenant très majoritairement le produit attendu et engagée telle quelle dans l'étape suivante. Rendement brut quantitatif.

### 16.3 : 2-Méthoxy-4-[1-(3,3,3-trifluoro-propyl)-pipéridin-4-yl]-phénylamine

Dans un autoclave d'hydrogénation, on place 1,65 g (5,0 mmoles) de produit préparé en 16.2 dans 120 mL d'un mélange éthanol/acide acétique (V/V = 1/1) et on ajoute 0,150 g d'oxyde (+IV) de platine sous atmosphère inerte. Le mélange est agité sous une pression de 4 bars d'hydrogène à température ambiante pendant 4 h. Après filtration sur papier fin en fibre de verre et évaporation à sec sous pression réduite. Le résidu huileux est repris dans le chloroforme et lavé avec une solution de NaOHaq 1 N. La phase organique est séchée sur Na₂SO₄, filtrée et concentrée sous vide. On obtient 1,5 g de produit attendu sous forme d'une huile marron que l'on utilise telle quelle dans l'étape suivante. Rendement brut = quantitatif.

### 16.4: 2-Chloro-8-éthyl-7-méthylamino-5-oxo-5,8-dihydro-pyrido[2,3-d]pyrimidine-6-carboxamide

Une suspension de 2 g (7,47 mmoles) de produit préparé en 2.6 dans 40 mL de NMP anhydre est refroidie avec un bain eau-glace et on ajoute 0,45 g (11,21 mmoles) d'hydrure de sodium à 60% en une portion. On agite à froid jusqu'à l'arrêt du dégagement gazeux puis 10 mn à température ambiante. On refrodit la solution avec un bain eau-glace et on ajoute goutte à goutte une solution de 0,56 mL (8,97 mmoles) d'iodure de méthyle dans 5 mL de NMP anhydre. On laisse revenir lentement à température ambiante en agitant pendant 24 h. On ajoute de la glace au mélange réactionnel puis on le verse sur de l'eau. On acidifie jusqu'à pH 1 en ajoutant une solution de HClaq 1 N. On laisse 48 h à température ambiante et on ajoute de l'acétate d'éthyle. On agite vigoureusement pendant 1 h. Après décantation, la phase organique est séchée sur Na₂SO₄, filtrée et concentrée sous vide. Le solide jaune obtenu est trituré dans l'éther, essoré, rincé au pentane et séché à l'étuve sous vide. On obtient 0,7 g d'un mélange 90/10 (CLMS) du produit attendu et du produit de départ sous forme d'un solide jaune que l'on engage tel quel dans l'étape suivante.

### 16.5 : 8-Ethyl-2-{2-méthoxy-4-[1-(3, 3,3-trifluoro-propyl)-pipéridin-4-yl]-phénylamino}-7-méthylamino-5-oxo-5,8-dihydro-pyrido[2,3-d]pyrimidine-6-carboxamide

A une suspension de 0,5 g (1,77 mmole) de produit préparé en 16.4 dans 5 mL de DMF anhydre, on ajoute 1,07 g (3,55 mmoles) de produit préparé en 16.3 et le mélange réactionnel est chauffé à 100°C pendant 4 h. On évapore à sec et le résidu est purifié par chromatographie sur colonne de silice en éluant avec un gradient dichlorométhane : méthanol (100 : 0 à 95 : 5). Après trituration dans le méthanol, on obtient 0,08 g de produit attendu sous forme d'un solide blanc.

### 16.6 : Chlorhydrate du 8-éthyl-2-{2-méthoxy-4-[1-(3,3,3-trifluoro-propyl)-pipéridin-4-yl]-phénylamino}-7-méthylamino-5-oxo-5,8-dihydro-pyrido[2,3-d]pyrimidine-6-carboxamide

A une solution de 0,08 g (0,15 mmole) du produit préparé en 16.5 dans 6 mL d'un mélange dichlorométhane/méthanol (V/V = 1/1), on ajoute 0,44 mL (0,44 mmole) d'une solution d'éther chlorhydrique 1 M. On agite 1 h à température ambiante puis on verse de l'éther. Le solide est essoré, rincé au pentane et séché à l'étuve sous vide. On obtient finalement 0,09 g du produit attendu sous forme d'un solide jaune. Rendement (dichlohydrate) = 99%. M+H⁺ = 548.
RMN ¹H (DMSO-d6 ; 400 MHz) : δ 11,75 (s large, 1 H); 11,1 (s large, 1 H); 10,9 (s large, 1 H); 8,9 (s, 1 H); 8,8 (s , 1 H); 8,05 (s large, 1 H); 7,9 (d, 1 H); 7,0 (s, 1 H); 6,85(d, 1 H); 4,3 (q, 2H); 3,85 (s, 3H); 3,65 (m, 2H), 3,35 (m, 2H); 2,9-3,2 (m, 4H); 2,8 (m, 1 H); 2,75 (s, 3H), 2,0 (m, 4H); 1,2 (t, 3H).

### Exemple 17 : Chlorhydrate du 7-amino-8-(2-hydroxy-2-méthyl-propyl)-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide

### 17.1: Acide 2-chloro-4-(2-hydroxy-2-méthyl-propylamino)-pyrimidine-5-carboxylique

Une suspension de 19,9 g (86,73 mmoles) d'acide 2,4-dichloro-5-pyrimidinecarboxylique dans 155 mL de THF est refroidie avec un bain de glace à 2-5°C et on ajoute goutte à goutte une solution de 8,5 g (95,40 mmoles) de 1-amino-2-methyl-2-propanol et de 36,3 mL (260,19 mmoles) de triéthylamine dans 60 mL de THF. On agite à température ambiante pendant la nuit. On ajoute de l'acétate d'éthyle et de l'eau. Après décantation, on acidifie la phase aqueuse avec une solution aqueuse de HCl 1 N jusqu'à pH 2. Le précipité est isolé par filtration et on obtient 12,6 g du produit attendu sous forme d'un solide jaune pâle que l'on utilise tel quel dans l'étape suivante. Rendement = 59%.

### 17.2 : Fluorure d'acide 2-chloro-4-(2-hydroxy-2-méthyl-propylamino)-pyrimidine-5-carboxylique

A une solution contenant 12,64 g (51,45 mmoles) d'acide préparé en 17.1 et 7,89 mL (56,60 mmoles) de triéthylamine dans 250 mL de dichlorométhane, on ajoute 6,51 mL (77,18 mmoles) de fluorure de cyanuryle. Le mélange est agité à température ambiante pendant la nuit et la solution est diluée avec 590 mL de CH₂Cl₂ et 190 mL de NaHCO₃aq glacée. La phase organique est lavée deux fois avec 250 mL de NaHCO₃aq glacée et séchée sur MgSO₄. Le solvant est ensuite évaporé sous pression réduite. On obtient 12,3 g de produit attendu sous forme d'une huile jaune qui solidifie lentement et que l'on engage telle quelle dans l'étape suivante. Rendement = 96%.

### 17. 3 : 7-Amino-2-chloro-8-(2-hydroxy-2-méthyl-propyl)-5-oxo-5,8-dihydro-pyrido[2,3-d]pyrimidine-6-carboxamide

A une solution refroidie à 2-5°C avec un bain de glace de 4,4 g (52,36 mmoles) de cyanoacétamide dans 70 mL de DMF anhydre, on ajoute 4,19 g (104,72 mmoles) d'hydrure de sodium à 60 % par portions. On agite 15 minutes à 2-5°C puis on ajoute rapidement cette suspension à une solution de 12,35 g (49,87 mmoles) du fluorure d'acide préparé en 17.2 dans 70 mL de DMF anhydre et préalablement refroidie à 2-5°C. Le mélange est agité une nuit à température ambiante puis on refroidit à 2-5°C avec un bain de glace et on ajoute 2,09 g (52,36 mmoles) d'hydrure de sodium à 60% par portions. On agite le mélange réactionnel pendant 3 heures à température ambiante puis on verse sur un mélange de glace, de 100 mL d'eau et de 150 mL de HClaq 1 N. Le précipité jaune formé est isolé par filtration, rincé à l'eau puis séché à l'étuve. Après trituration dans le méthanol et séchage à l'étuve, on obtient finalement 8,84 g du produit attendu sous forme d'un solide jaune. Rendement = 57%.

### 17.4 : 7-Amino-8-(2-hydroxy-2-méthyl-propyl)-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide

A une suspension de 0,82 g (2,62 mmole) de produit préparé en 17.3 dans 6,4 mL de NMP anhydre, on ajoute 1,59 g (5,25 mmoles) de produit préparé en 16.3 et le mélange réactionnel est chauffé à 110°C pendant 1,5 h. On évapore à sec et le résidu est purifié par chromatographie sur colonne de silice en éluant avec un gradient dichlorométhane : méthanol (100 : 0 à 95 : 5) contenant des traces de NH₄OH_{aq} concentrée. On obtient 0,70 g de produit attendu sous forme d'un solide blanc. Rendement = 46%.

### 17.5 : Chlorhydrate du 7-amino-8-(2-hydroxy-2-méthyl-propyl)-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide

On refroidit avec un bain de glace une solution de 0,70 g (1,22 mmoles) du produit préparé en 17.4 dans 17 mL de méthanol et on ajoute 0,91 mL (3,65 mmoles) d'une solution d'acide chlorhydrique 4N dans le dioxane. On agite 0,5 h à température ambiante puis on verse de l'éther. Le solide est essoré, rincé au pentane et séché à l'étuve sous vide. On obtient finalement 0,64 g du produit attendu sous forme d'un solide blanc. Rendement (dichlohydrate) = 91%. PF > 260°C. M+H⁺ = 578
RMN ¹H (DMSO-d₆ + D₂O; 400 MHz): 8,9 (s, 1 H); 7,6 (d, 1 H); 6,95 (s, 1 H); 6,85 (d, 1 H); 4,8 (s très large, 2H); 3,8 (s, 3H); 3,6 (m, 2H), 3,4 (m, 2H); 3,1 (m, 2H); 2,8-2,95 (m, 3H); 2,05 (m, 2H); 1,95 (m, 2H);1,1 (s très large, 6H).

### Exemple 18 : Chlorhydrate du 7-amino-2-[4-(4-éthyl-pipérazin-1-yl)-2-méthoxy-phénylaminol-8-(2-hydroxy-2-méthyl-propyl)-5-oxo-5,8-dihydro-pyrido[2,3-d]pyrimidine-6-carboxamide

### 18.1: 1-Ethyl-4-(3-méthoxy-4-nitro-phényl)-pipérazine

Un mélange de 3,42 g (20 mmoles) de 4-fluoro-2-méthoxy-1-nitrobenzène, de 2,67 mL (21 mmoles) de 1-éthylpipérazine et 5,14 mL (30 mmoles) de diisopropyléthylamine dans 25 mL d'acétonitrile est chauffé 2,5 h à 85°C puis 1 h à 95°C et ensuite agité la nuit en laissant remonter à température ambiante. On évapore l'acétonitrile puis on reprend avec 50 mL d'éther et 25 mL d'eau. La phase aqueuse est extraite à l'éther. Les phases organiques sont réunies, séchées sur Na₂SO₄, filtrées et concentrées sous vide. L'huile obtenue est triturée dans un mélange éther cyclohexane (1/1) et le précipité est isolé par filration, rincé au pentane et séché à l'étuve. On obtient finalement 4,1 g du produit attendu. Rendement = 77%.

### 18.2 : 4-(4-Ethyl-pipérazin-1-yl)-2-méthoxy-phénylamine

Dans un autoclave d'hydrogénation, on place 4,05 g (15,27 mmoles) de produit préparé en 18.1 dans 25 mL d'un mélange acétate d'éthyle : éthanol (V/V = 1/1) et on ajoute 0,32 g de Palladium sur charbon à 10% sous atmosphère inerte. Le mélange est agité sous une pression de 2,5 bars d'hydrogène à température ambiante pendant 2 h. Après filtration sur papier fin en fibre de verre et évaporation sous pression réduite, on obtient 3,51 g de produit attendu sous la forme d'un solide violet que l'on utilise tel quel dans l'étape suivante. Rendement = 98%.

### 18.3 : 7-Amino-2-[4-(4-éthyl-pipérazin-1-yl)-2-méthoxy-phénylamino]-8-(2-hydroxy-2-méthyl-propyl)-5-oxo-5,8-dihydro-pyrido[2,3-d]pyrimidine-6-carboxamide

A une suspension de 1,0 g (3,21 mmoles) de produit préparé en 17.3 dans 5 mL de NMP anhydre, on ajoute 1,51 g (6,42 mmoles) de produit préparé en 18.2 et le mélange réactionnel est chauffé à 100°C pendant 2 h. On laisse revenir à température ambiante puis on dilue avec une solution de NHCO₃aq. Le mélange est agité pendant la nuit et le précipité est isolé par filtration puis séché à l'étuve. Le résidu est purifié par chromatographie sur colonne de silice en éluant avec un gradient dichlorométhane : méthanol (100: 0 à 95: 5) contenant des traces de NH₄OH_{aq} concentrée. Après trituration finale dans le méthanol et séchage à l'étuve, on obtient 0,46 g de produit attendu. Rendement = 28%.

### 18.4 : Chlorhydrate du 7-amino-2-[4-(4-éthyl-pipérazin-1-yl)-2-méthoxy-phénylamino]-8-(2-hydroxy-2-méthyl-propyl)-5-oxo-5,8-dihydro-pyrido[2,3-d]pyrimidine-6-carboxamide

On refroidit avec un bain de glace une solution de 0,46 g (0,9 mmole) du produit préparé en 18.3 dans 13 mL de méthanol et on ajoute 0,67 mL (2,69 mmoles) d'une solution d'acide chlorhydrique 4N dans le dioxane. On agite 0,5 h à température ambiante puis on verse de l'éther. Le solide est essoré, rincé au pentane et séché à l'étuve sous vide. On obtient finalement 0,47 g du produit attendu sous forme d'un solide jaune. Rendement (dichlohydrate) = 90%. PF = 222°C (décomposition). M+H⁺ = 511.
RMN ¹H (DMSO-d₆, 400 MHz) : δ 11,5 (s large, 1H); 10,9 (s large, 1H); 9,1 (s large, 1 H); 8,9 (s large, 1 H); 8,4 (s large, 1 H); 7,45 (d, 1 H); 6,75 (d, 1 H); 6,6 (dd, 1 H); 4,8 (s très large, 1 H); 3,70-4,20 (s + m, 9H); 3,0-3,3 (m, 6H); 1,3 (t, 3H); 1,05 (s très large, 6H)

### Exemple 19 : Chlorhydrate du 7-amino-2-[4-(4-éthyl-pipéridin-1-yl)-2-méthoxy-phénylaminol-8-(2-hydroxy-2-méthyl-propyl)-5-oxo-5,8-dihydro-pyrido[2,3-d]pyrimidine-6-carboxamide

### 19.1: 4-[4-(7-Amino-6-carbamoyl-8-(2-hydroxy-2-méthyl-propyl)-5-oxo-5,8-dihydro-pyrido[2,3-d]pyrimidin-2-ylamino)-3-méthoxy-phényl]-pipéridine-1-carboxylate de tert-butyle

Dans un ballon, on place un mélange de 2,0 g (6,42 mmoles) de produit préparé en 17.3 et 2,95 g (9,62 mmoles) de produit préparé en 4.3 dans 18 mL de NMP. La suspension est chauffée à 110°C pendant 2 heures. On ajoute 30 mL d'eau puis 5 mL de NaHCO₃aq saturée et le précipité formé est essoré puis séché à l'étuve. Le solide brut est trituré dans le méthanol pui séché à l'étuve. On obtient 2,38 g du produit attendu sous forme d'un solide rose que l'on engage tel quel dans l'étape suivante. Rendement = 64%.

### 19.2 : Chlorhydrate de 7-amino-8-(2-hydroxy-2-méthyl-propyl)-2-(2-méthoxy-4-pipéridin-4-yl-phénylamino)-5-oxo-5,8-dihydro-pyrido[2,3-d]pyrimidine-6-carboxamide

On refroidit avec un bain eau-glace une suspension de 2,28 g (3,92 mmoles) du produit préparé en 19.1 dans 70 mL de dichlorométhane et on ajoute lentement 9,8 mL (39,20 mmoles) d'une solution d'acide chlorhydrique 4N dans le dioxane. On agite à température ambiante pendant 1 h et on ajoute de l'éther. Le solide est essoré, rincé au pentane et séché à l'étuve. On obtient 2,2 g du produit attendu sous forme d'un solide beige que l'on engage tel quel dans l'étape suivante. Rendement (dichlorhydrate) quantitatif.

### 19. 3 : 7-Amino-8-(2-hydroxy-2-méthyl-propyl)-2-[4-(4-éthyl-pipéridin-1-yl)-2-méthoxy-phénylamino]-5-oxo-5,8-dihydro-pyrido[2,3-d]pyrimidine-6-carboxamide

0,6 g (1,08 mmoles) du chlorhydrate préparé en 19.2 est mis en suspension dans 14 mL de 1,2-dichloroéthane et on ajoute 0,56 mL (3,25 mmoles) de diisopropyléthylamine, 0,30 mL (5,41 mmoles) d'acétaldéhyde puis 0,46 g (2,16 mmoles) de triacétoxyborohydrure de sodium par portions. On agite 2 heures et on verse sur une solution de soude 0,25 N et on ajoute du dichlorométhane. Après décantation, la phase aqueuse est extraite au dichlorométhane et les phases organiques sont regroupées, séchées sur Na₂SO₄, filtrées et concentrées sous vide. On récupère un solide marron clair de 0,58 g que l'on purifie par chromatographie sur colonne de silice en éluant avec un gradient dichlorométhane : méthanol (95 : 5 à 90 : 10). On obtient 0,26 g du produit attendu sous forme d'un solide jaune. Rendement = 47%.

### 19.4 : Chlorhydrate du 7-amino-8-(2-hydroxy-2-méthyl-propyl)-2-[4-(4-éthyl-pipéridin-1-yl)-2-méthoxy-phénylamino]-5-oxo-5,8-dihydro-pyrido[2,3-d]pyrimidine-6-carboxamide

A une solution de 0,25 g (0,50 mmole) de produit préparé en 19.3 dans 10 mL d'un mélange dichlorométnane-méthanol (4/1), on ajoute 2 mL (2 mmoles) d'éther chlorhydrique 1.0 M. On agite 10 minutes à température ambiante puis on dilue à l'éther. Le précipité est essoré, rincé à l'éther, au pentane et séché sous vide. On obtient 0,28 g du produit attendu sous forme d'un solide jaune. Rendement (dichlorhydrate) = 96%. PF = 200°C (décomposition). M+H⁺ = 510.
RMN ¹H (DMSO-d₆, 400 MHz) : δ 11,5 (s large, 1H); 10,4 (s large, 1H); 9,1 (s, 1H); 8,95 (s, 1 H); 8,45 (s large, 1 H); 7,6 (d, 1 H); 7,0 (s, 1 H); 6,85 (d, 1 H); 4,8 (s large, 2H); 3,85 (s, 3H); 3,6 (m, 2H); 3,1 (m, 2H); 3,05 (m, 2H); 2,85 (m, 1 H); 2,1-2,2 (m, 4H); 1,3 (t, 3H); 1,1 (s très large, 6H)

### Exemple 20: Chlorhydrate du 7-amino-2-(2-méthoxy-4-pipéridin-4-yl-phénylamino)-5-oxo-8-phényl-5,8-dihydro-pyrido[2,3-d]pyrimidine-6-carboxamide

### 20.1: 2-Méthylsulfanyl-4-phénylamino-5-pyrimidine carboxylate d'éthyle

A une solution de 5,0 g (21,49 mmoles) de 4-chloro 2-méthylsulfanyl-5-pyrimidine carboxylate d'éthyle et 4,9 mL (53,72 mmoles) d'aniline dans 75 mL de THF, on ajoute 7,49 mL (53,72 mmoles) de triéthylamine. Le mélange est agité à température ambiante pendant la nuit. Après évaporation du THF, on ajoute une solution de HClaq 1 N et on extrait à l'acétate d'éthyle. La phase organique est lavée avec une solution aqueuse saturée en NaHCO₃ puis en NaCl. On sèche la phase organique sur MgSO₄. Après filtration, on concentre le filtrat et on obtient 3,99 g du produit attendu sous forme d'un solide beige que l'on engage tel quel dans l'étape suivante. Rendement = 64 %.

### 20.2 : Acide 2-méthylsulfanyl-4-phénylamino-5-pyrimidine carboxylique

Un mélange contenant 3,98 g (13,75 mmoles) de produit préparé en 20.1, 34,4 mL (34,4 mmoles) de NaOH 1 N et 35 mL d'éthanol est agité à température ambiante pendant une nuit. On évapore l'éthanol sous pression réduite et on dilue le résidu dans 100 mL d'eau. On additionne 65 mL d'une solution de HCl aqueux 1 N et on essore le précipité formé. On rince le solide avec de l'eau et on le sèche sous vide. On obtient 2,88 g de produit attendu sous forme d'un solide beige. Rendement = 92%.

### 20.3 : Fluorure d'acide 2-méthylsulfanyl-4-phénylamino-5-pyrimidine carboxylique

A une solution contenant 2,88 g (11,02 mmoles) de produit préparé en 20.2 et 2,1 mL (15,0 mmoles) de triéthylamine dans 55 mL de CH₂Cl₂, on ajoute 1,40 mL (16,53 mmoles) de fluorure de cyanuryle. Le mélange est agité à température ambiante pendant la nuit et lavée 2 fois avec 50 mL d'une solution aqueuse glacée de NaHCO₃. La phase organique est lavée avec 75 mL d'eau glacée et séchée sur MgSO₄. Le solvant est ensuite évaporé sous pression réduite. On obtient 3,0 g de produit attendu sous forme d'un solide jaune. Le rendement est quantitatif.

### 20.4: 7-Amino-2-méthylsulfanyl-5-oxo-8-phényl-5,8-dihydro-pyrido[2,3-d]pyrimidine-6-carboxamide

A une solution de 1,0 g (11,96 mmoles) de cyanoacétamide dans 17 mL de DMF anhydre refroidie à 0-5°C, on ajoute 0,96 g (23,93 mmoles) de NaH à 60 %. Après l'addition, on agite 10 minutes à température ambiante puis on refroidit à nouveau avec un bain eau-glace et on ajoute une solution de 3,0 g du fluorure d'acide (11,39 mmoles) préparé en 20.3 dans 17 mL de DMF anhydre. Le mélange réactionnel est agité à température ambiante pendant la nuit. On refroidit le mélange réactionnel avec un bain eau-glace et on ajoute 0,48 g (11,96 mmoles) de NaH à 60%. On agite 5 h à température ambiante puis on verse le mélange réactionnel sur une solution aqueuse glacée de HCl 0,5 N. Le précipité formé est isolé par filtration, rincé à l'eau, essoré puis séché à l'étuve. On obtient 3,44 g d'un solide jaune composé majoritairement du produit atendu sous forme non cyclisée.

On reprend le solide obtenu précédemment dans 50 mL de n-butanol et on chauffe au reflux pendant une nuit. Après retour à l'ambiante, on isole le précipité par filtration et on le sèche à l'étuve. On obtient 1,69 g du produit attendu sous forme d'un solide jaune. Rendement = 45%

### 20.5: 7-Amino-2-méthanesulfonyl-5-oxo-8-phényl-5,8-dihydro-pyrido[2,3-d]pyrimidine-6-carboxamide et 7-amino-2-méthanesulfinyl-5-oxo-8-phényl-5,8-dihydro-pyrido[2,3-d]pyrimidine-6-carboxamide

A une suspension de 1,69 g (5,16 mmoles) de composé obtenu en 20.4 dans 100 mL de chloroforme, on ajoute 3,18 g (12,91 mmoles) d'acide méta-chloroperbenzoïque. On agite à température ambiante pendant 24 heures et on ajoute 0,44 g (2,58 mmoles) d'acide *méta*-chloroperbenzoïque. On agite 24 heures puis on sépare l'insoluble par filtration. Le filtrat est concentré sous vide puis trituré dans le méthanol et séché à l'étuve. On obtient 1,12 g d'un solide jaune pâlé composé d'un mélange de sulfone et sulfoxyde attendus (environ 80/20 en RMN ¹H).

### 20.6 Ester tert-butylique de l'acide 4-[4-(7-amino-6-carbamoyl-5-oxo-8-phényl

### 5,8-dihydro-pyrido[2,3-d]pyrimidin-2-ylamino)-3-méthoxy-phényl]-pipéridine-1-carboxylique

0,4 g (1,11 mmoles) du mélange de sulfone et du sulfoxyde obtenu en 20.5 et 0,51 g (1,67 mmoles) de produit préparé en 16.3 sont chauffés 110°C pendant 6 h dans 5 mL de NMP. On ajoute de l'eau et on isole le solide par filtration puis on le sèche à l'étuve. Le résidu solide de 0,42 g est purifié par chromatographie sur colonne de silice en éluant avec un gradient dichlorométhane : méthanol (100 : 0 à 97: 3). On obtient 0,16 g de produit attendu sous forme d'un solide marron clair. Rendement = 46%.

### 20.7 : Chlorhydrate du 7-amino-2-(2-méthoxy-4-pipéridin-4-yl-phénylamino)-5-oxo-8-phényl-5,8-dihydro-pyrido[2,3-d]pyrimidine-6-carboxamide

On refroidit avec un bain de glace une solution de 0,15 g (0,26 mmole) du produit préparé en 20.6 dans 4 mL de CH₂Cl₂ et on ajoute 0,99 mL (3,94 mmoles) d'une solution d'acide chlorhydrique 4N dans le dioxane. On agite 0,5h à température ambiante puis on verse de l'éther. Le solide est essoré, rincé au pentane et séché à l'étuve sous vide. On obtient 0,136 g d'un solide rose pâle que l'on purifie sur phase inverse pour obtenir finalement 0,1 g du produit attendu sous forme d'un solide jaune. Rendement (dichlohydrate) = 68 %. PF = 228°C (decomposition). M+H⁺ = 487.
RMN ¹H (DMSO-d₆, 400 MHz) : δ 11,3 (s large, 1 H); 10,25 (s large, 1 H); 8,9-9,0 (s+m, 2H); 8,8 (m, 1 H); 8,3 (s, 1 H); 7,7 (m, 3H); 7,5 (m, 2H); 7,25 (s large, 1 H); 7,15 (m, 1 H); 6,8 (s, 1 H); 6,7 (s large, 1 H); 6,3 (m, 1 H); 3,95 (s, 3H); 3,4 (m, 2H); 3,0 (m, 2H); 2,75 (m, 1 H); 1,75-1,95 (m, 4H)

### Exemple 21: Chlorhydrate du 7-amino-2-[4-(1-cyclopropyl-pipéridin-4-yl)-2-méthoxy-phénylamino]-8-(2-hydroxy-2-méthyl-propyl)-5-oxo-5,8-dihydro-pyrido[2,3-d]pyrimidine-6-carboxamide

### 21.1: 7-Amino-2-[4-(1-cyclopropyl-pipéridin-4-yl)-2-méthoxy-phénylamino]-8-(2-hydroxy-2-méthyl-propyl)-5-oxo-5,8-dihydro-pyrido[2,3-d]pyrimidine-6-carboxamide

A une solution de 0,46 g (0,83 mmole) de chlorhydrate préparé en 20.7 dans 14 mL de méthanol anhydre, on ajoute successivement 0,42 mL (2,48 mmoles) de diisopropyléthylamine, 0,47 mL (8,26 mmoles) d'acide acétique, du tamis moléculaire 3 Å finement broyé et préalablement séché à l'étuve sous vide à 60°C pendant une nuit, 0,75 mL (3,72 mmoles) de (1-éthoxycyclopropoxy)triméthylsilane et 0,16 g (2,48 mmoles) de cyanoborohydrure de sodium. Le mélange est chauffé à 80°C pendant une nuit et on évapore à sec. Le résidu obtenu est purifié par chromatographie sur colonne de silice en éluant avec un gradient dichlorométhane : méthanol (96 : 4 à 90 : 10). On obtient finalement 0,16g du produit attendu sous forme d'un solide blanc. Rendement = 37%.

### 21.2 : Chlorhydrate du 7-amino-2-[4-(1-cyclopropyl-pipéridin-4-yl)-2-méthoxy-phénylamino]-8-(2-hydroxy-2-méthyl-propyl)-5-oxo-5,8-dihydro-pyrido[2,3-d]pyrimidine-6-carboxamide

A une suspension de 0,137 g (0,26 mmole) de produit préparé en 21.1 dans 5 mL de CH₂Cl₂, on ajoute 1,05 mL (1,05 mmole) d'éther chlorhydrique 1,0 M. On agite 5 minutes à température ambiante puis on dilue à l'éther. Le précipité est essoré, rincé à l'éther, au pentane et séché sous vide. On obtient 0,12 g du produit attendu sous forme d'un solide jaune. Rendement (dichlorhydrate) = 77%. PF = 206°C (décomposition). M+H⁺ = 568
RMN ¹H (DMSO-d₆, 400 MHz): δ 11,5 (s large, 1 H); 10,7 (s large, <1H); 10,6 (m, 1 H); 9,1 (s, 1 H); 8,9 (s, 1 H); 8,4 (s large, 1 H); 7,6 (d, 1 H); 7,0 (s, 1 H); 6,85 (d, 1 H); 4,8 (s large, 2H); 3,8 (s, 3H); 3,6 (m, 2H); 3,2-3,4 (m, 3H); 3,05 (m, 1 H); 2,8 (m, 2H); 2,15 (m, 2H); 1,95 (m, 2H); 1,15 (m, 2H); 1,0 (s très large, 6H); 0,8 (m, 2H)

### Exemple 22 : Chlorhydrate du 7-amino-8-(2-hydroxy-2-méthyl-propyl)-2-{2-méthoxy-4-[1-(2-méthoxy-éthyl)-pipéridin-4-yl]-phénylamino}-5-oxo-5,8-dihydro-pyrido[2,3-d]pyrimidine-6-carboxamide

### 22.1: 7-Amino-2-{4-[1-(2-chloro-éthyl)-pipéridin-4-yl]-2-méthoxy-phénylamino}-8-(2-hydroxy-2-méthyl-propyl)-5-oxo-5,8-dihydro-pyrido[2,3-d]pyrimidine-6-carboxamide

A une suspension de 0,60 g (1,08 mmoles) de chlorhydrate préparé en 20.7 dans 15 mL de dichloroéthane anhydre, on ajoute successivement 0,56 mL (3,25 mmoles) de diisopropyléthylamine, 0,4 mL (5,41 mmoles) de méthoxyacétaldéhyde (lot reçu de la société TCI Fine Chemicals) et 0,46 g de triacétoxyborohydrure de sodium. Le mélange est agité à température ambiante pendant 2 h puis dilué avec 15 mL d'un mélange eau-NaOHaq 1 N (2/1). On agite 10 min puis on dilue avec de l'acétate d'éthyle. La phase organique est séchée sur MgSO₄, filtrée et concentrée sous vide. On obtient 0,41 g d'un solide jaune correspondant au 7-amino-2-{4-[1-(2-chloro-éthyl)-pipéridin-4-yl]-2-méthoxy-phénylamino}-8-(2-hydroxy-2-méthyl-propyl)-5-oxo-5,8-dihydro-pyrido[2,3-d]pyrimidine-6-carboxamide et non au 7-amino-8-(2-hydroxy-2-méthyl-propyl)-2-{2-méthoxy-4-[1-(2-méthoxy-éthyl)-pipéridin-4-yl]-phénylamino}-5-oxo-5,8-dihydro-pyrido[2,3-d]pyrimidine-6-carboxamide initialement attendu. Rendement = 69%.

### 22.2 : 7-Amino-8-(2-hydroxy-2-méthyl-propyl)-2-{2-méthoxy-4-[1-(2-méthoxy-éthyl)-pipéridin-4-yl]-phénylamino}-5-oxo-5,8-dihydro-pyrido[2,3-d]pyrimidine-6-carboxamide

A une suspension de 0,29 g (0,54 mmole) du produit préparé en 22.1 dans 9 mL de méthanol anhydre, on ajoute 0,37 g (2,68 mmoles) de K₂CO₃ et on chauffe au reflux pendant 1 h. On évapore à sec et on reprend avec le résidu avec de l'eau et du CH₂Cl₂. La phase organique est séchée sur MgSO₄, filtrée et concentrée sous vide. Le produit brut est purifié par chromatographie sur colonne de silice en éluant avec un gradient dichlorométhane : méthanol (90 : 10). Après trituration dans le méthanol, on obtient finalement 0,22 g du produit attendu sous forme d'un solide jaune. Rendement = 78%.

### 22.3 : 7-Amino-8-(2-hydroxy-2-méthyl-propyl)-2-{2-méthoxy-4-[1-(2-méthoxy-éthyl)-pipéridin-4-yl]-phénylamino}-5-oxo-5,8-dihydro-pyrido[2,3-d]pyrimidine-6-carboxamide

A une suspension de 0,22 g (0,41 mmole) de produit préparé en 22.2 dans 8 mL d'un mélange CH₂Cl₂-méthanol (4/1), on ajoute 1,63 mL (1,63 mmoles) d'éther chlorhydrique 1,0 M. On agite 5 minutes à température ambiante puis on dilue à l'éther. Le précipité est essoré, rincé à l'éther, au pentane et séché sous vide. On obtient 0.23 du produit attendu sous forme d'un solide jaune pâle. Rendement (dichlorhydrate) = 92%. PF = 170°C (décomposition). M+H⁺ = 540
RMN ¹H (DMSO-d₆, 400 MHz) : δ 11,5 (s large, 1 H); 10,35 (s large, 1 H); 10,6 (m, 1 H); 9,05 (s, 1 H); 8,9 (s, 1 H); 8,4 (s large, 1 H); 7,6 (d, 1 H); 7,0 (s, 1 H); 6,8 (d, 1 H); 4,75(s large, 2H); 3,7-3,85 (m+s, 5H); 3,6 (m, 2H); 3,2-3,4 (m+s, 5H); 3,1 (m, 2H); 2,85 (m, 1 H); 2,15 (m, 2H); 2,0 (m, 2H); 1,0 (s très large, 6H);

### Exemple 23 : Chlorhydrate du 7-amino-2-[2-méthoxy-4-(1-méthyl-pipéridin-4-yl)-phénylamino]-5-oxo-8-phényl-5,8-dihydro-pyrido[2,3-d]pyrimidine-6-carboxamide

### 23.1: 7-Amino-2-[2-méthoxy-4-(1-méthyl-pipéridin-4-yl)-phénylamino]-5-oxo-8-phényl-5,8-dihydro-pyrido[2,3-d]pyrimidine-6-carboxamide

0,2 g (0,55 mmole) du mélange de sulfone et du sulfoxyde obtenu en 20.5 et 0,24 g (1,11 mmoles) de 2-méthoxy-4-(1-méthyl-pipéridin-4-yl)-phenylamine ( préparée selon la méthode décrite dans le brevet WO09024824) sont chauffés 110°C pendant 6 h dans 5 mL de NMP. On évapore à sec sous vide et le résidu est purifié par chromatographie sur colonne de silice en éluant avec un gradient dichlorométhane : méthanol (100 : 0 à 95: 5) contenant des traces de NH₄OH_{aq} concentrée. On obtient 0,096 g de produit attendu sous forme d'un solide beige. Rendement = 35%.

### 23.2 : Chlorhydrate du 7-amino-2-[2-méthoxy-4-(1-méthyl-pipéridin-4-yl)-phénylamino]-5-oxo-8-phényl-5,8-dihydro-pyrido[2,3-d]pyrimidine-6-carboxamide

On refroidit avec un bain de glace une solution de 0,065 g (0,13 mmole) du produit préparé en 23.1 dans 3 mL de CH₂Cl₂ et on ajoute 0,39 mL (0,39 mmole) d'une solution d'acide chlorhydrique 1 M dans l'éther. On agite 10 mn à température ambiante puis on verse de l'éther. Le solide est essoré, rincé au pentane et séché à l'étuve sous vide. On obtient 0,059 g du composé attendu. Rendement (dichlohydrate) = 80%.
PF = 256°C (décomposition). M+H⁺ = 500
RMN ¹H (DMSO-d₆, 400 MHz) : δ 11,3 (s large, 1H); 10,25 (s large, 1H); 9,0 (s, 1H); 8,35 (s, 1 H); 7,7 (m, 3H); 7,5 (m, 2H); 7,25 (s large, 1 H); 7,15 (m, 1 H); 6,8 (s, 1 H); 6,7 (s large, 1 H); 6,3 (m, 1 H); 3,8 (s, 3H); 3,45 (m, 2H); 3,0 (m, 2H); 2,8 (d, 3H); 2,7 (m, 1 H); 1,85-2,05 (m, 4H)

### Exemple 24 : Chlorhydrate du 7-amino-2-[2-méthoxy-4-(2-pyrrolidin-1-yl-éthyl)-phénylamino]-5-oxo-8-phényl-5,8-dihydro-pyrido[2,3-d]pyrimidine-6-carboxamide

### 24.1: 2-Méthoxy-1-nitro-4-vinyl-benzène

Dans un mélange de 5,19 g (22,37 mmoles) de produit préparé en 4.1 dans 56 mL de n-propanol sous argon, on ajoute 4,32 g (31,31 mmoles) de trifluorovinylborate de potassium, 3,12 mL (22,37 mmoles) de triéthylamine et on fait barboter de l'argon pendant 10 minutes. On ajoute 0,36 g (0,45 mmole) de PdCl₂dppf.CH₂Cl₂ et on chauffe sous argon à 100°C pendant 3 heures. Après retour à température ambiante, le mélange réactionnel est concentré à sec puis repris avec un mélange dichorométhane-eau. Après décantation, on lave deux fois à l'eau et une fois avec NaClaq saturée. La phase organique est séchée sur Na₂SO₄, filtrée et concentrée sous vide. Le résidu obtenu est purifié par chromatographie sur colonne de silice en éluant avec un gradient cyclohexane : acétate d'éthyle (95 : 5 à 70 : 30). On obtient 3,1 g du produit attendu sous forme d'une huile brune que l'on utilise tel quel dans l'étape suivante. Rendement = 77%.

### 24.2 : 1-[2-(3-Méthoxy-4-nitro-phényl)-éthyl]-pyrrolidine

Dans un tube scellé, on place 3,07 g (17,17 mmoles) du produit préparé en 24.1 et 7,13 mL (85,84 mL) de pyrrolidine dans 60 mL de méthanol anhydre. On chauffe sous pression à 100°C pendant 4 h. Après retour à température ambiante, on évapore à sec. Le résidu obtenu est purifié par chromatographie sur colonne de silice en éluant avec un gradient dichlorométhane : méthanol (100 : 0 à 90 : 10). On obtient 3,22 g du produit attendu sous forme d'un solide jaune. Rendement = 75%.

### 24.3 : 2-Méthoxy-4-(2-pyrrolidin-1-yl-éthyl)-phénylamine

Dans une solution de 3,2 g (12,86 mmoles) de produit préparé en 24.2 dans 120 mL de méthanol sous argon, on ajoute 0,41 g de palladium sur charbon, 4,1 g (64,32 mmoles) de formiate d'ammonium et on chauffe au reflux pendant 2 h. Après filttration, on concentre le filtrat à sec et on obtient 2,6 g de produit attendu sous forme d'un solide marron clair que l'on engage tel quel dans l'étape suivante. Rendement = 92%.

### 24.4 : 7-Amino-2-[2-méthoxy-4-(2-pyrrolidin-1-yl-éthyl)-phénylamino]-5-oxo-8-phényl-5,8-dihydro-pyrido[2,3-d]pyrimidine-6-carboxamide

0,2 g (0,55 mmole) du mélange de sulfone et du sulfoxyde obtenu en 20.5 et 0,24 g (1,11 mmoles) d'aniline préparée en 24.3 sont chauffés à 110°C pendant 6 h dans 5 mL de NMP. On évapore à sec sous vide et le résidu est purifié par chromatographie sur colonne de silice en éluant avec un gradient dichlorométhane : méthanol (100 : 0 à 90: 10) contenant des traces de NH₄OH_{aq} concentrée. On obtient 0,07 g de produit attendu sous forme d'un solide vitreux jaune. Rendement = 25%.

### 24.5 : Chlorhydrate du 7-amino-2-[2-méthoxy-4-(2-pyrrolidin-1-yl-éthyl)-phénylamino]-5-oxo-8-phényl-5,8-dihydro-pyrido[2,3-d]pyrimidine-6-carboxamide

On refroidit avec un bain de glace une solution de 0,07 g (0,14 mmole) du produit préparé en 24.4 dans 3 mL de méthanol et on ajoute 0,42 mL (0,42 mmole) d'une solution d'acide chlorhydrique 1 M dans l'éther. On agite 10 mn à température ambiante puis on verse de l'éther. Le solide est essoré, rincé au pentane et séché à l'étuve sous vide. On obtient 0,075 g du composé attendu. Rendement (dichlohydrate) = 93%.
M+H⁺ = 500
RMN ¹H (DMSO-d₆ + D2O, 400 MHz, T = 130°C) : 9,0 (s, 1 H); 7,7 (m, 3H); 7,4 (m, 2H); 7,25 (d, 1 H); 6,85 (s, 1 H); 6,4 (d, 1 H); 3,8 (s, 3H); 3,1-3,5 (m, 6H); 2,9 (m, 2H); 2,0 (m, 4H)

Le **tableau 1** qui suit illustre les structures chimiques et les propriétés physiques de quelques composés de formule (I) selon l'invention. Dans ce tableau :
➢ Me et Et représentent respectivement des groupes méthyle et éthyle,
➢ dans la colonne « LC/UV/MS », sont indiqués successivement, la méthode analytique de chromatographie liquide haute performance utilisée (A, B, C ou D) et détaillée ci-dessous, le temps de rétention (abrégé tr) du composé exprimé en minute, et le pic MH⁺ identifié par spectrométrie de masse.
   **Méthode A :**
      Colonne : Kromasil C18, 50x 2,1 mm, 3,5 µm
      Solvant A : H₂O/ACN/TFA (1000/30/0.5); solvant B : ACN/TFA (1000/0.5); débit = 0,5 mL/mn
      Gradient : 100/0 (0 min) to 0/100 (12 min) to 0/100 (15 min)
      Détection : 220 nM
      Ionisation : ESI+
   **Méthode B :**
      Colonne : Gemini, 50x 3 mm, 3µm
      Solvant A : H₂O + 0.1% HCO₂H; solvant B : ACN + 0.1% HCO₂H; débit = 1 mL/mn
      Gradient : 95/5 (0 min) to 0/100 (5,5 min) to 0/100 (7,5 min)
      Détection : 220 nM
      Ionisation : ESI+
   **Méthode C** :
      Colonne : Kromasil C18, 50x 2,1 mm, 3,5 µm
      Solvant A: CH3CO2NH4 5 mM; solvant B : ACN; débit = 0,5 mL/mn
      Gradient : 100/0 (0 min) to 0/100 (13 min) to 0/100 (16 min)
      Détection : 220 nM
      Ionisation : ESI+
   **Méthode D** :
      Colonne : Acquity BEH C18, 50x 2,1 mm ; 1,7 µm
      Solvant A : H2O +0.05% TFA; solvant B : ACN +0.035TFA; débit = 1 mL/mn
      Gradient : T0 :98%A ; T1,6 à T2,1 min :100%B ; T2,5 à T3min: 98%A
      Détection : 220 nM
      Ionisation : ESI+
➢ dans la colonne « Forme », « - » indique que le composé est sous forme de base libre, alors que « HCl » indique que le composé est sous forme de chlorhydrate,
➢ dans la colonne « Composés », « RAC » indique que le composé est sous forme de mélange racémique,
➢ « ¤ » signifie que la donnée n'est pas disponible.

**Tableau 1**

| **N°** | **Composés** | **LC/UV/MS** | | | **Forme** |
|---|---|---|---|---|---|
| | | Méthode | tr | MH⁺ | |
| 1 | 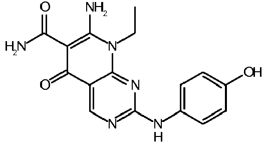 | A | ¤ | 341 | - |
| 2 | 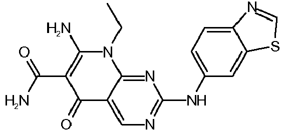 | A | 5,5 | 382 | - |
| 3 | 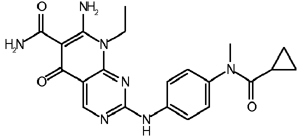 | A | 5,7 | 422 | - |
| 4 | 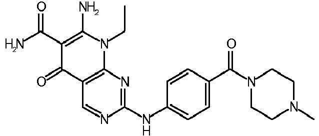 | A | 4,1 | 451 | - |
| 5 | 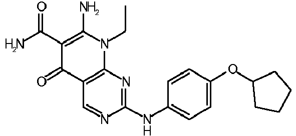 | A | 9,8 | 409 | - |
| 6 | 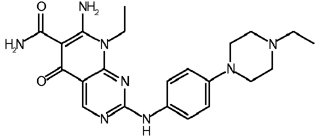 | A | 4,2 | 437 | - |
| 7 | 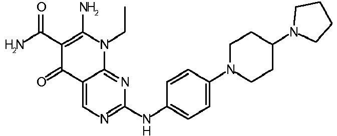 | A | 4,4 | 477 | - |
| 8 | 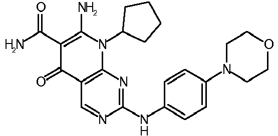 | B | 11,4 | 450 | - |
| 9 | 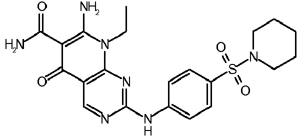 | A | 5,9 | 472 | - |
| 10 | 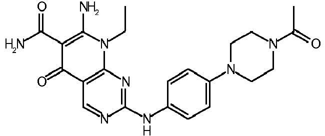 | B | 9,3 | 451 | - |
| 12 | 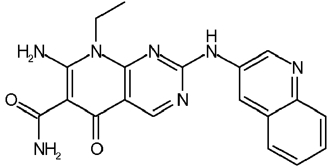 | A | 5,8 | 376 | - |
| 13 | 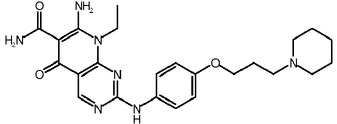 | A | 5,5 | 466 | - |
| 14 | 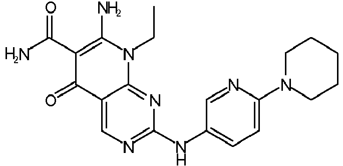 | A | 6,7 | 409 | - |
| 15 | 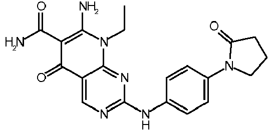 | A | 5,5 | 408 | - |
| 16 | 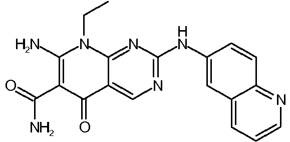 | A | 4,5 | 376 | - |
| 17 | 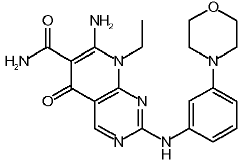 | A | 5,5 | 410 | - |
| 18 | 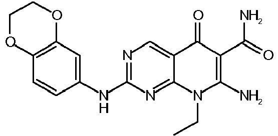 | A | 5,8 | 383 | - |
| 19 | 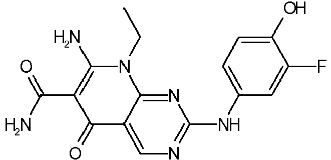 | A | 5,1 | 359 | - |
| 20 | 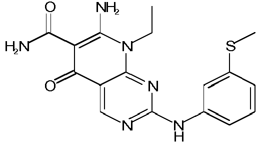 | A | 6,6 | 371 | - |
| 21 | 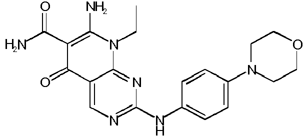 | B | 4,3 | 410 | - |
| 22 | 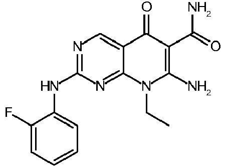 | B | 6,1 | 343 | - |
| 25 | 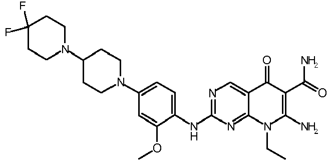 | A | 3,9 | 557 | HCl |
| 26 | 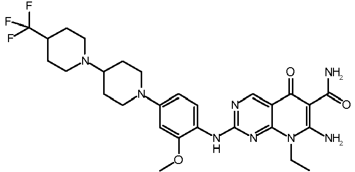 | A | 3,9 | 589 | HCl |
| 27 | 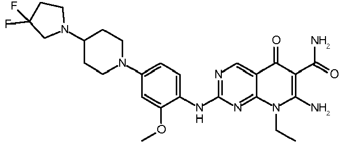 | C | 0,8 | 543 | HCl |
| 28 | 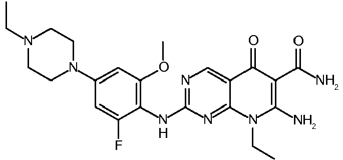 | C | 0,7 | 485 | HCl |
| 29 | 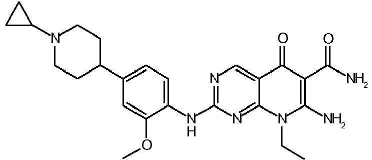 | C | 0,76 | 478 | HCl |
| 30 | 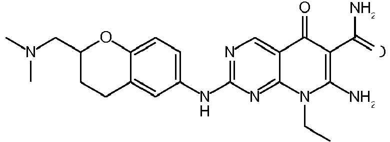 | C | 0,6 | 438 | HCl |
| 31 | 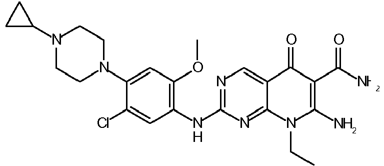 | C | 0,78 | 513 | HCl |
| 32 | 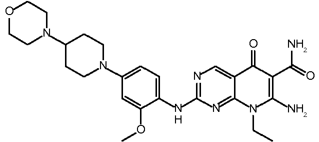 | C | 0,7 | 523 | HCl |
| 33 | 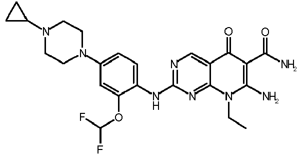 | D | 0,75 | 515 | HCl |
| 34 | 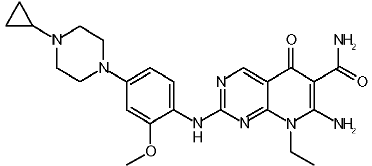 | C | 0,7 | 479 | HCl |
| 35 | 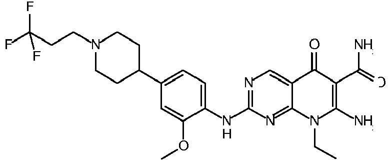 | C | 0,8 | 534 | HCl |
| 36 | 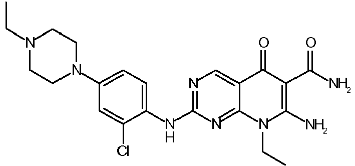 | C | 0,71 | 471 | HCl |
| 37 | 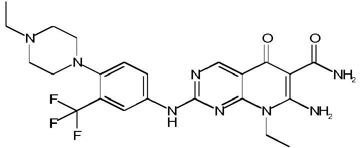 | D | 0,80 | 505 | HCl |
| 38 | 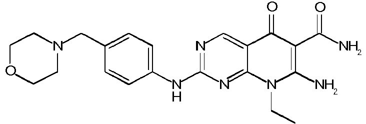 | C | 2,98 | 424 | - |
| 39 | 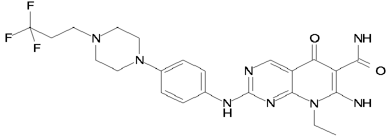 | D | 0,74 | 505 | HCl |
| 40 | 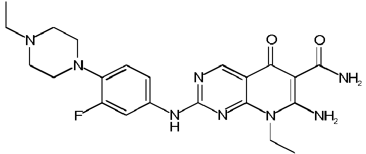 | D | 0,69 | 455 | HCl |
| 42 | 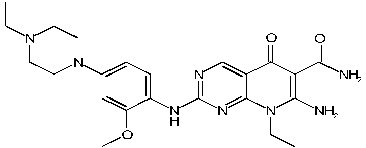 | D | 0,69 | 467 | HCl |
| 43 | 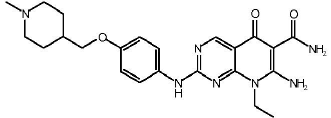 | D | 0,70 | 452 | HCl |
| 44 | 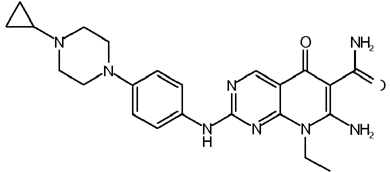 | D | 0,67 | 449 | HCl |
| 45 | 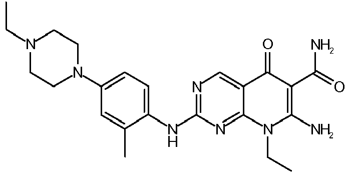 | D | 0,67 | 451 | HCl |
| 46 | 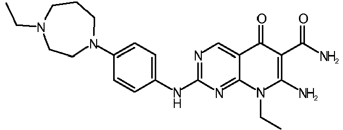 | D | 0,68 | 451 | HCl |
| 48 | 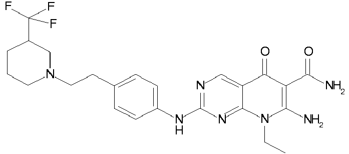 | D | 0,87 | 504 | HCl |
| 49 | 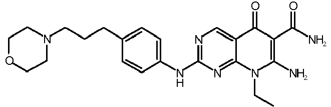 | D | 0,71 | 452 | HCl |
| 50 | 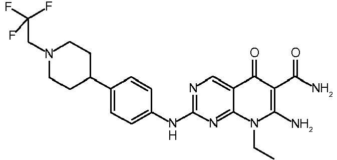 | D | 0,75 | 490 | HCl |
| 51 | 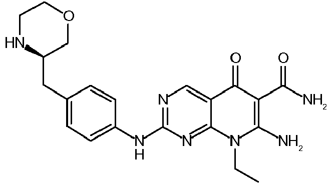 | D | 0,58 | 424 | HCl |
| 52 | 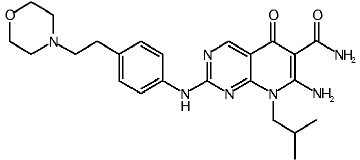 | D | 0,66 | 466 | HCl |
| 53 | 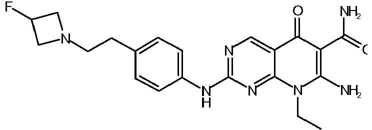 | D | 0,59 | 426 | - |
| 54 | 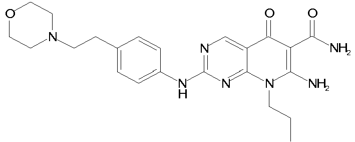 | D | 0,62 | 452 | HCl |
| 55 | 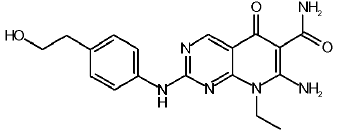 | D | 0,69 | 369 | - |
| 56 | 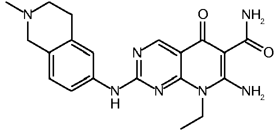 | D | 0,55 | 394 | - |
| 57 | 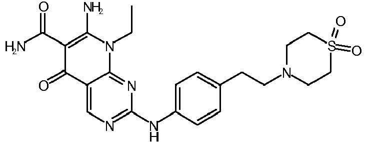 | D | 0,60 | 486 | HCl |
| 58 | 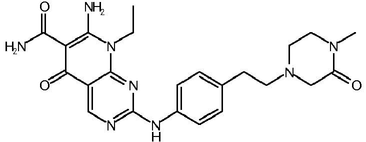 | D | 0,57 | 465 | HCl |
| 59 | 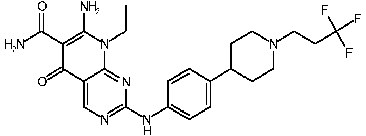 | D | 0,71 | 504 | HCl |
| 60 | 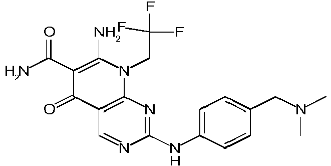 | D | 0,63 | 436 | HCl |
| 61 | 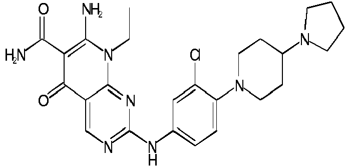 | D | 0,70 | 511 | HCl |
| 62 | 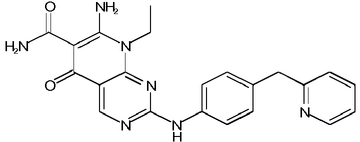 | D | 0,60 | 416 | HCl |
| 63 | 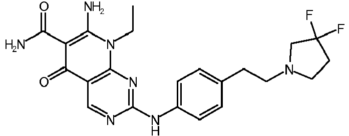 | D | 0,64 | 458 | HCl |
| 64 | 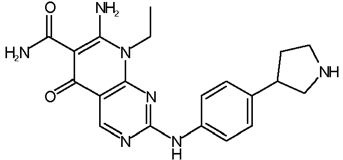 | C | 0,59 | 394 | HCl |
| 65 | 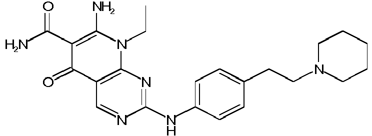 | D | 0,64 | 436 | HCl |
| 66 | 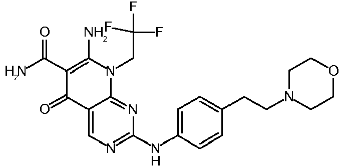 | D | 0,65 | 492 | HCl |
| 67 | 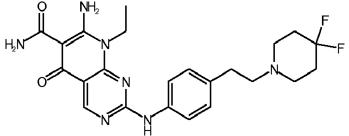 | D | 0,65 | 472 | HCl |
| 68 | 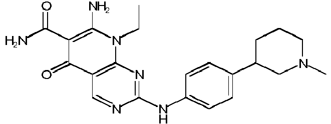 | D | 0,59 | 408 | HCl |
| 69 | 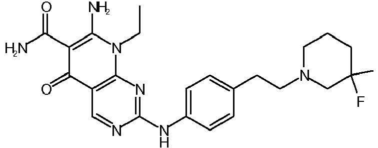 | D | 0,64 | 472 | HCl |
| 70 | 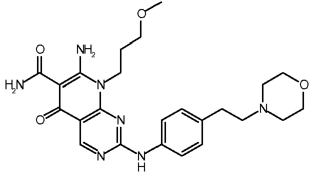 | D | 0,61 | 482 | HCl |
| 71 | 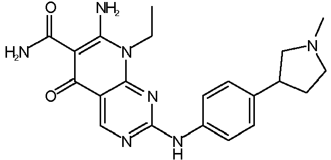 | D | 0,62 | 422 | HCl |
| 72 | 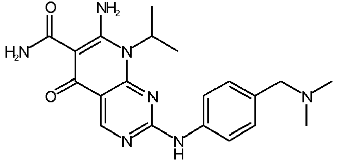 | D | 0,59 | 396 | - |
| 73 | 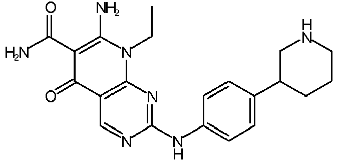 | D | 0,62 | 408 | HCl |
| 74 | 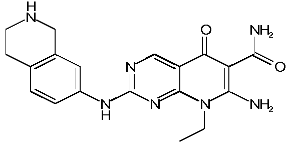 | D | 0,56 | 380 | HCl |
| 75 | 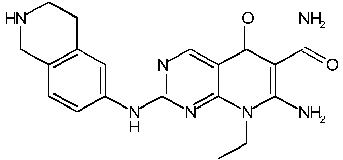 | D | 2,52 | 380 | HCl |
| 76 | 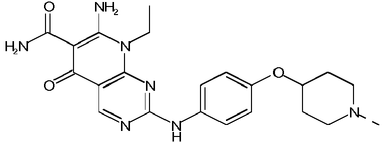 | D | 0,62 | 438 | HCl |
| 77 | 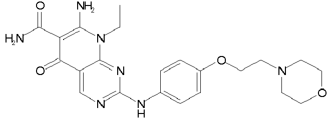 | D | 0,59 | 454 | HCl |
| 78 | 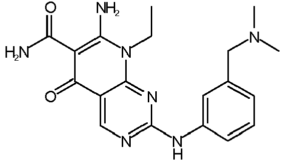 | D | 0,59 | 382 | HCl |
| 79 | 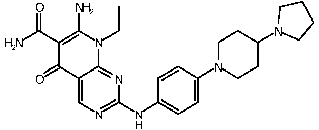 | D | 0,57 | 477 | HCl |
| 80 | 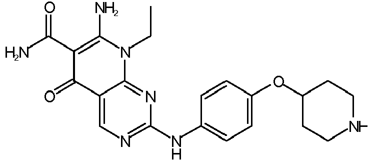 | D | 0,61 | 424 | HCl |
| 81 | 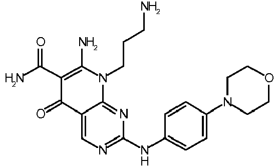 | D | 0,55 | 439 | HCl |
| 82 | 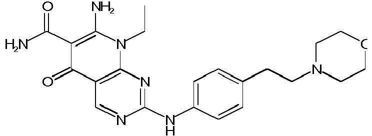 | D | 0,59 | 438 | HCl |
| 83 | 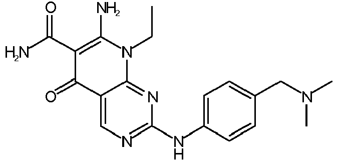 | D | 0,56 | 382 | HCl |
| 84 | 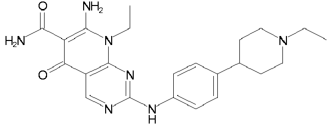 | D | 0,63 | 436 | HCl |
| 85 | 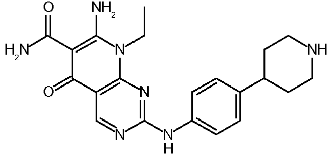 | D | 0,60 | 408 | HCl |
| 86 | 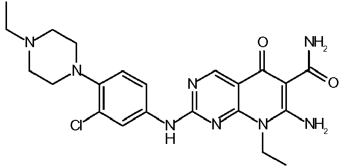 | D | 0,66 | 471 | HCl |
| 87 | 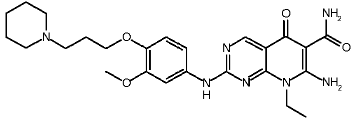 | D | 0,67 | 496 | HCl |
| 88 | 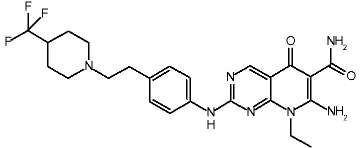 | D | 0,72 | 504 | HCl |
| 89 | 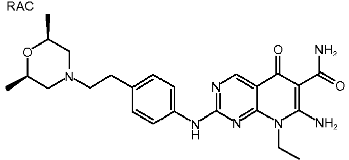 | D | 0,66 | 466 | HCl |
| 90 | 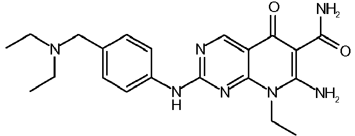 | D | 0,61 | 410 | HCl |
| 91 | 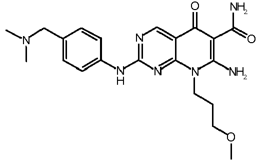 | D | 0,60 | 426 | HCl |
| 92 | 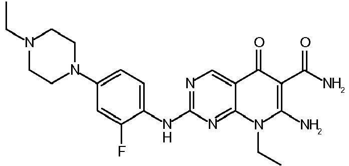 | D | 0,67 | 455 | HCl |
| 93 | 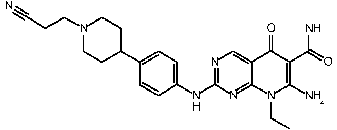 | D | 0,68 | 461 | HCl |
| 94 | 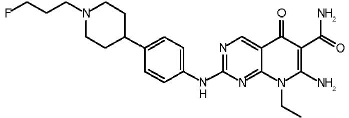 | D | 0,7 | 468 | HCl |
| 95 | 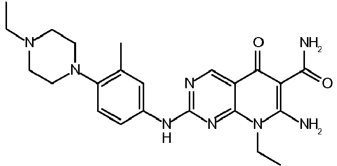 | D | 0,7 | 451 | HCl |
| 96 | 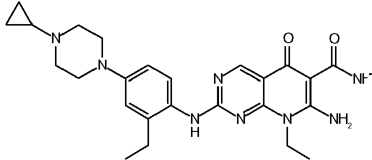 | D | 0,72 | 477 | HCl |
| 97 | 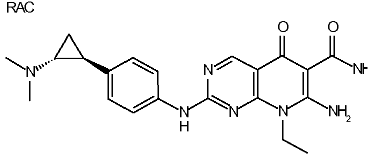 | D | 0,67 | 408 | HCl |
| 98 | 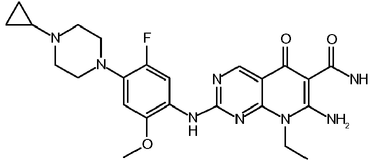 | D | 0,75 | 497 | HCl |
| 99 | 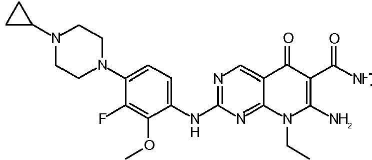 | D | 0,74 | 497 | HCl |
| 100 | 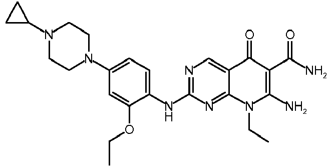 | D | 0,76 | 493 | HCl |
| 101 | 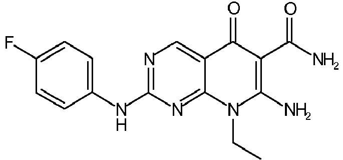 | B | 6,3 | 343 | - |
| 102 | 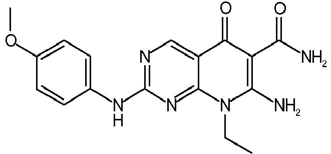 | B | 6,2 | 355 | HCl |
| 103 | 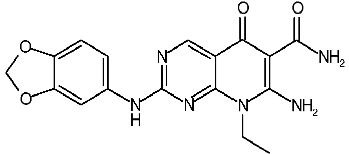 | B | 6,2 | 369 | HCl |
| 104 | 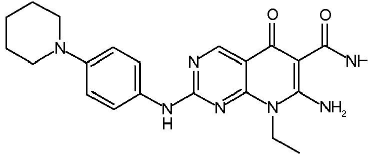 | B | 5,2 | 408 | HCl |
| 105 | 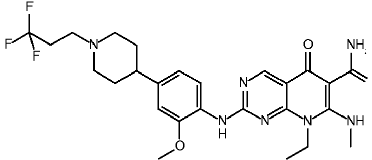 | C | 4,4 | 548 | HCl |
| 106 | 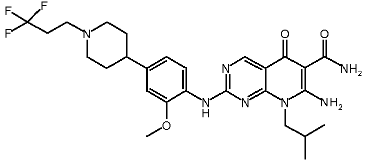 | D | 0,83 | 562 | HCl |
| 107 | 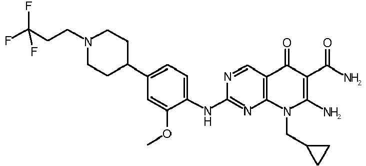 | D | 0,8 | 560 | HCl |
| 108 | 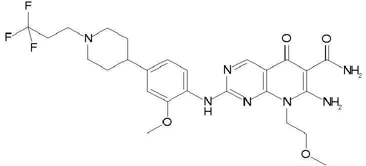 | D | 0,77 | 564 | HCl |
| 109 | 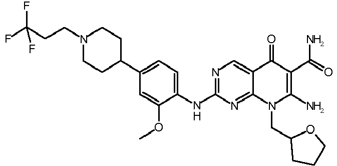 | D | 0,9 | 590 | HCl |
| 110 | 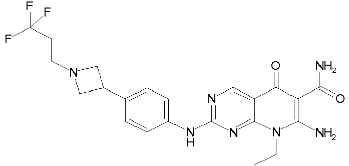 | D | 0,8 | 476 | HCl |
| 111 | 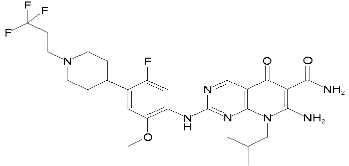 | D | 0,9 | 580 | HCl |
| 112 | 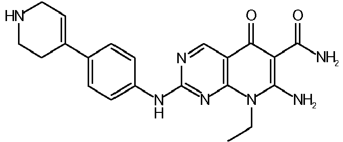 | D | 0,7 | 406 | HCl |
| 113 | 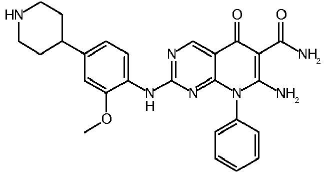 | D | 0,7 | 487 | HCl |
| 114 | 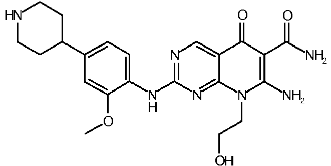 | D | 0,6 | 455 | HCl |
| 115 | 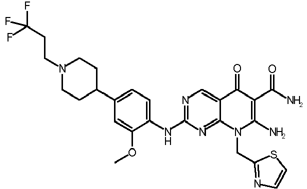 | D | 604 | 0,78 | HCl |
| 116 | 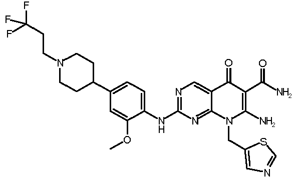 | D | 604 | 0,73 | HCl |
| 117 | 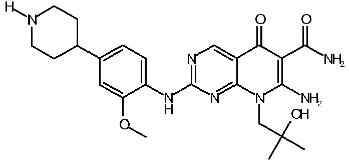 | D | 482 | 0,66 | - |
| 118 | 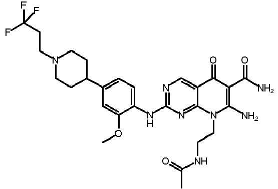 | D | 592 | 0,71 | HCl |
| 119 | 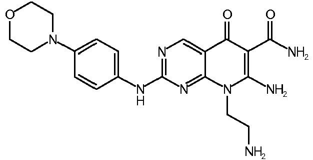 | D | 426 | 0,55 | HCl |
| 120 | 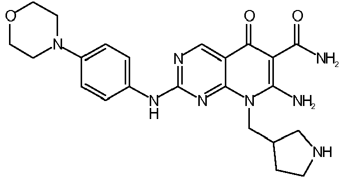 | D | 466 | 0,63 | HCl |
| 121 | 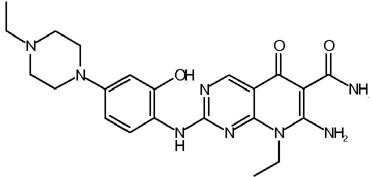 | D | 454 | 0,64 | HCl |
| 122 | 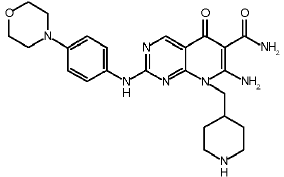 | D | 480 | 0,67 | HCl |
| 123 | 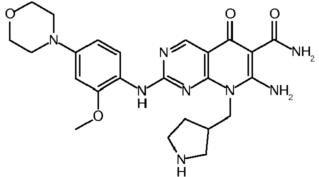 | D | 496 | 0,67 | HCl |
| 124 | 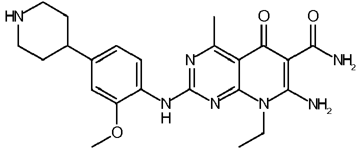 | D | 458 | 0,77 | HCl |
| 125 | 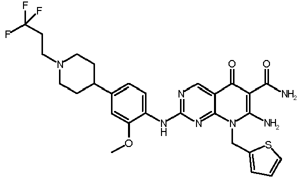 | D | 603 | 0,88 | HCl |
| 126 | 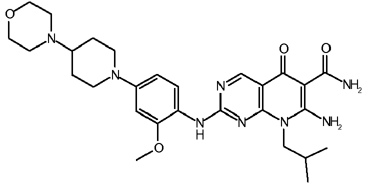 | D | 552 | 0,73 | HCl |
| 127 | 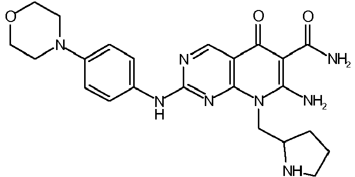 | D | 466 | 0,92 | HCl |
| 128 | 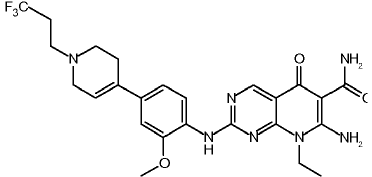 | B | 533 | 0,88 | HCl |
| 129 | 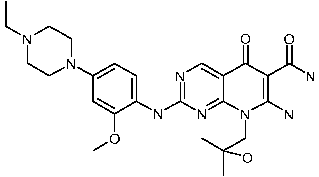 | D | 511 | 0,63 | HCl |
| 130 | 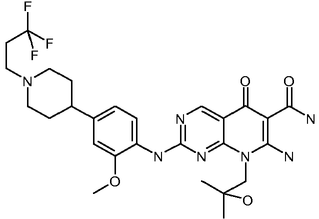 | D | 578 | 0,74 | HCl |
| 131 | 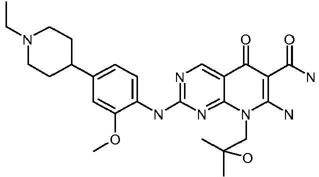 | D | 510 | 0,67 | HCl |
| 132 | 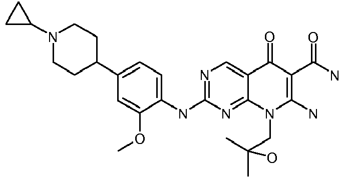 | D | 522 | 1,89 | HCl |
| 133 | 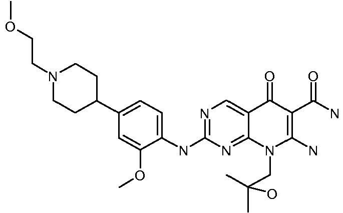 | D | 540 | 0,68 | HCl |
| 134 | 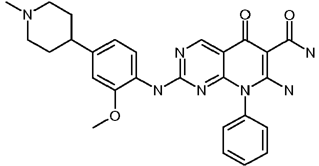 | D | 500 | 0,70 | HCl |
| 135 | 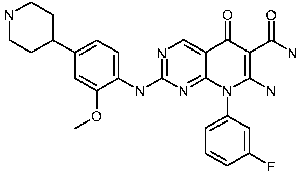 | D | 504 | 0,70 | HCl |
| 136 | 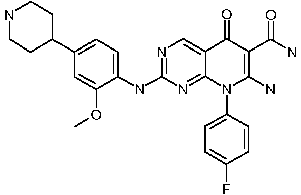 | D | 504 | 0,70 | HCl |
| 137 | 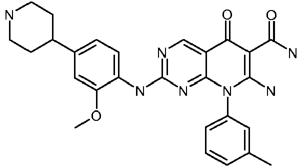 | C | 500 | 0,93 | HCl |
| 138 | 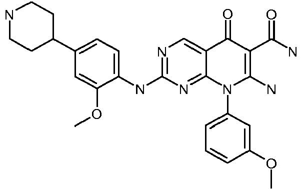 | C | 516 | 0,87 | HCl |
| 139 | 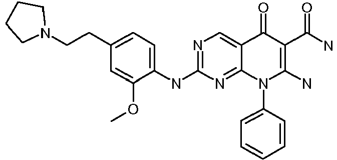 | D | 500 | 0,72 | HCl |
| 140 | 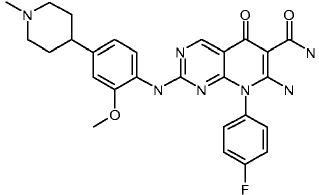 | D | 518 | 1,29 | HCl |
| 141 | 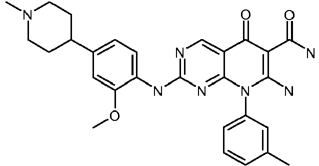 | C | 514 | 0,87 | HCl |
| 142 | 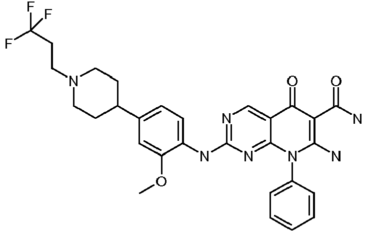 | C | 582 | 1,03 | HCl |
| 143 | 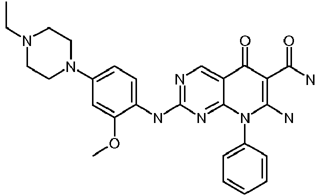 | C | 515 | 0,86 | HCl |

Les composés selon l'invention ont fait l'objet d'essais pharmacologiques permettant de déterminer leur effet inhibiteur de CaMKII sur l'isoforme δ.

Des essais ont consisté à mesurer l'activité *in vitro* des composés de l'invention sur CaMKIIδ.

Les composés selon l'invention ont été testés *in vitro* pour leur capacité à inhiber la fonction kinase de la Calcium/Calmodulin-dependent protein Kinase II deta (CaMKIIδ). CaMKIIδ est une sérine/thréonine kinase intra cellulaire. Elle possède un domaine enzymatique capable d'utiliser l'ATP pour s'autophosphoryler. Sa fonction kinase lui permet d'utiliser l'ATP pour phosphoryler ses substrats au niveau des sérines et/ou des thréonines. Plusieurs tests enzymatiques et cellulaires sont utilisés pour évaluer l'activité des produits vis à vis de la fonction kinase de CaMKIIδ.

L'activité kinase de CaMKIIδ est évaluée par un test radioactif sur l'enzyme CaMKIIδ recombinante. On mesure la quantité d'ATP-γ33P incorporée dans le substrat spécifique Autocamtide-2 lors de sa phosphorylation par CaMKIIδ. L'effet des produits est quantifié par la concentration de produit inhibant l'activité totale de CaMKIIδ de 50% (Concentration d'inhibition de 50% = CI₅₀). Pour la détermination des CI₅₀, le produit est dilué dans 100% de DMSO pour obtenir une solution mère de 10 mM. La gamme de concentration testée lors du test radioactif s'étale de 3 à 10000 nM avec une concentration finale dans le test de 1% DMSO. Cette gamme de concentration peut, pour les composés les plus puissants, être étendue à 0.1 nM. Le jour de la manipulation, 5µl des composés sont déposés dans chaque puits d'une plaque 96 puits à 10 fois la concentration de celle à tester. Chaque concentration est testée en duplicate sur la même plaque. Les contrôles négatifs (0% d'activité) et positifs (100% d'activité) recevront 5 µL de solution DMSO 10%. Un pré-mix réactionnel contenant le tampon kinase 1X et un mélange ATP-MgCl₂, est préparé. Extemporanément, on ajoute au pré-mix, un mélange contenant le substrat Autocamtide-2 (100 µM) avec la calmoduline et le calcium, et la solution d'enzyme CaMKIIδ. On dépose immédiatement 45 µL de ce mélange par puits. Les concentrations finales dans le volume de 50 µL final sont les suivantes : composé 1X, 0.37 nM de CaMKIIδ (Invitrogen référence PV3373), 10 µM d'ATP (1 µCi par puits), 100 µM d'Autocamtide-2, 8 µg/mL de Calmoduline, 15 mM MgCl2, 400 µM CaCl2, 10 mM de β-glycérolphosphate, et 1% de DMSO. Deux contrôles négatifs sont réalisés sur chaque plaque, un premier sans enzyme CaMKIIδ, et un second sans substrat Autocamtide-2 ; ces deux éléments y sont remplacés par de l'eau. La plaque est ensuite incubée deux heures à 37°C sous agitation douce. La réaction est stoppée par ajout de 20 µL par puits d'une solution d'H₃PO₄. On transfère 50 µL de chaque puits sur une plaque à filtre P81 Whatman. Après 2 rinçages avec de l'H₃PO₄ (150 µl / puits par lavage), suivis de 2 rinçages par 150 µL d'H₂O, 50 µL de scintillant sont ajoutés par puits. La quantité de substrat phosphorylé est détectée par un compteur à scintillation liquide.

L'activité inhibitrice vis à vis de CaMKIIδ est donnée par la concentration qui inhibe 50% de l'activité de CaMKIIδ. Les CI₅₀ sont généralement comprises entre 10 µM et 10⁻⁵ µM. Dans le tableau qui suit les CI₅₀ mesurés de plusieurs composés (I) de l'invention sont présentées à titre d'exemples.

| n° du composé | IC₅₀ (nM) |
|---|---|
| composé n° 32 | 2 |
| composé n° 61 | 8 |
| composé n° 29 | 17 |
| composé n° 35 | 22 |
| composé n° 75 | 37 |
| composé n° 113 | 39 |
| composé n° 114 | 57 |
| composé n°76 | 118 |
| composé n° 52 | 139 |
| composé n° 4 | 260 |
| composé n° 78 | 268 |
| composé n° 96 | 396 |
| composé n° 60 | 663 |

Il apparaît donc que les composés selon l'invention ont une activité inhibitrice de CaMKIIδ.

Les composés selon l'invention peuvent donc être utilisés pour la préparation de médicaments inhibiteurs de CaMKII et en particulier de médicaments inhibiteurs de CaMKIIδ.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, notamment dans le traitement et/ou la prévention des pathologies dans lesquelles CaMKII est impliqué et en particulier dans lesquelles CaMKIIδ est impliqué.

La présente invention, selon un autre de ses aspects, concerne également l'utilisation d'un composé de formule (I) pour la préparation d'un médicament destiné à la prévention et/ou au traitement des pathologies cardiovasculaires incluant l'infarctus du myocarde, l'hypertrophie ventriculaire, la fibrose myocardique, l'insuffisance cardiaque, les arythmies cardiaques et la resténose, ainsi que des pathologies associées au développement de fibrose incluant la fibrose hépatique, pancréatique, rénale, pulmonaire, cutanée, intestinale et oculaire. Un composé de formule (I) peut également être utilisé dans le traitement et/ou la prévention d'autres pathologies rénales comme l'insuffisance rénale aiguë ainsi que dans le traitement de l'athérosclérose, de l'arthrite rhumatoïde, de la maladie de Parkinson et d'un accident vasculaire cérébral.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement et/ou de prévention des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule générale (I) 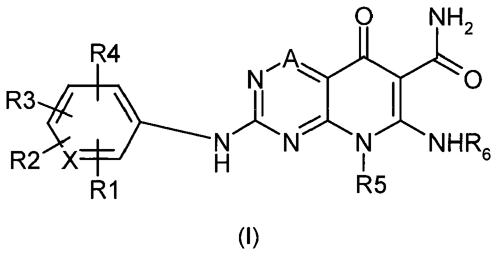 dans laquelle :
➢ A représente CH ou C(alkyle) ;
➢ X représente CH, C(alkyle) ou N ;
➢ R₁, R₂, R₃ et R₄, identiques ou différents, représentent chacun indépendamment les uns des autres :
• un atome d'hydrogène ;
• un alkyle linéaire, ramifié ou cyclique, éventuellement substitué par un ou plusieurs :
o atomes d'halogène ;
○ -OR₉ ;
○ -NR₉R'₉ ;
o -CN ;
o -C(O)OR₉ ;
o -C(O)NR₉R_{9'} ;
o -S(O)ₚR₁₀;
○ -S(O)₂NR₉R'₉ ;
où **R₉, R'₉**, **R₁₀ et p** sont tels que définis ci-dessous
• un groupe -S(O)ₚR₁₀ où p et R₁₀ sont tels que définis ci-dessous;
• un groupe -OR₁₀ où R₁₀ est tel que defini ci-dessous;
• un atome d'halogène ;
• un groupe -N(R₁₁)C(O)R₁₂, où
(i) **R₁₁ et R₁₂** représentent, indépendamment l'un de l'autre, un atome d'hydrogène ou un alkyle, linéaire, ramifié ou cyclique, éventuellement substitué par un ou plusieurs substituants choisis parmi les atomes d'halogène, les groupes -OR₉ et les groupes -NR₉R'₉ , ou
(ii) **R₁₁ et R₁₂** forment ensemble avec les atomes auxquels ils sont rattachés un hétérocycloalkyle, de manière à former un lactame;
• un groupe -N(R₁₄)-CH₂-C(O)NR₁₅R₉, où **R₁₄ et R₁₅** forment ensemble avec les atomes auxquels ils sont rattachés un hétérocycloalkyle, de manière à former une pipérazinone et où **R₉** est tel que défini ci-dessous ;
• un groupe -C(O)NR₁₆R₁₇ avec **R₁₆ et R₁₇** forment ensemble avec l'atome d'azote auquel ils sont rattachés un hétérocycloalkyle,
• un groupement **-T-U,** où :
■ T représente :
○ une liaison simple,
○ un groupe alkylène, linéaire ou ramifié ;
○ un groupe -C(O)-,
○ un groupe -S(O)ₚ- où p est tel que défini ci-dessous, ou
○ un groupe -O-(CH₂)ₙ - où n est tel que defini ci-dessous,
avec U représentant un hétérocycle comprenant un ou plusieurs hétéroatomes choisi parmi N, O et S(O)p, où p est tel que défini ci-dessous, ledit hétérocycle étant saturé, insaturé ou aromatique, éventuellement mono- ou di- ou poly-substitué par un, deux ou plusieurs substituant(s) choisi(s) parmi :
❖ les groupes -OR₇, où R₇ est tel que défini ci-dessous,
❖ les atomes d'halogène,
❖ les groupes -C(O)R₇ où R₇ est tel que défini ci-dessous,
❖ les alkyles linéaires, ramifiés ou cycliques, éventuellement substitués par un ou plusieurs substituants choisis parmi les atomes d'halogène, les groupes -OR₁₀, les groupes -NR₉R'₉ et le groupe -CN, où R₉, R'₉ et R₁₀ sont tels que définis ci-dessous; et
❖ les hétérocycles saturés, insaturés ou aromatiques, éventuellement substitué par un ou plusieurs substituants choisis parmi les atomes d'halogène, les groupes -OR₉ , les groupes -NR₉R'₉ et les groupes alkyle, lesdits groupes alkyle étant éventuellement substitués par un ou plusieurs atomes d'halogène ; ou
**■ T** représente :
○ un groupe -C(O)- ;
○ un groupe -S(O)₂- ; ou
○ un groupe -O-(C2-C3)alkylène- ;
avec **U** représentant un groupe -NR₉R'₉ où R₉ et R'₉ sont tels que définis ci-dessous ; ou
■ **T** représente :
○ un groupe -C(O)- ; ou
○ un groupe -O-(C2-C3)alkylène-;
avec **U** représentant un groupe -OR₉, où R₉ est tel que défini ci-dessous ; ou
■ **T** représente :
○ un groupe alkylène, linéaire ou ramifié; ou
○ un groupe -O-(C2-C3)alkylène-;
avec **U** représentant un groupe -NR₈R₉ où R₉ et R₈ sont tels que définis ci-dessous;
ou bien deux groupements adjacents choisis parmi **R₁**, **R₂**, **R₃ et R₄** sont liés et forment avec les deux carbones qui les portent un hétérocycle saturé, insaturé ou aromatique, éventuellement substitué par un ou plusieurs groupes alkyle, linéaire, ramifié ou cyclique, lesdits groupes alkyle étant éventuellement substitués par un ou plusieurs substituants choisis parmi les atomes d'halogène, les groupes -OR₁₀, et les groupes -NR₉R'₉ , où R₉, R'₉ et R₁₀ sont tels que définis ci-dessous, ledit hétérocycle étant fusionné avec le cycle aromatique,
➢ **R₅** représente :
• un alkyle, linéaire ou ramifié, éventuellement substitué par un ou plusieurs substituants choisis parmi les atomes d'halogène, les groupes -OR₉, les groupes -NR₉R'₉ , le groupe -CN , les groupes -C(O)NR₉R_{9'} les groupes - S(O)ₚR₁₀ et les groupes cycloalkyle éventuellement substitués par un groupe - NR₉R'₉, où R₉, R'₉, R₁₀ et p sont tels que définis ci-dessous,
• un groupe cycloalkyle, éventuellement substitué par un groupe -NR₉R'₉, où R₉ et R'₉ sont tels que définis ci-dessous,
• un groupe alcoxy -OR₉, où R₉ est tel que défini ci-dessous,
• un aryle éventuellement substitué par un ou plusieurs substituants choisis parmi les groupes alkyle (C1-C3), les atomes d'halogène et les groupes -O-(C1-C3) alkyle, ou
• un groupe -(CH₂)ₜ-R₁₃, où **R₁₃** et **t** sont tels que définis ci-dessous,
➢ **R₆** représente un atome d'hydrogène ou un alkyle linéaire, ramifié ou cyclique, et où :
➢ **R₇** représente un atome d'hydrogène ou un alkyle linéaire, ramifié ou cyclique, éventuellement substitué par un ou plusieurs substituants choisis parmi les atomes d'halogène, les groupes -OR₉ et les groupes -NR₉R'₉ avec R₉ et R'₉ tels que définis ci-dessus;
➢ **R₈** représente un groupe hétéroaryle;
➢ **R₉ et R'₉** représentent, indépendamment l'un de l'autre, un atome d'hydrogène ou un alkyle linéaire, ramifié ou cyclique ;
➢ **R₁₀** représente un atome d'hydrogène ou un alkyle linéaire, ramifié ou cyclique éventuellement substitué par un ou plusieurs atomes d'halogène,
➢ **R₁₃** représente un hétéroaryle ou un hétérocycloalkyle éventuellement substitué par un ou plusieurs substituants choisis parmi les alkyles linéaires, ramifiés ou cycliques, étant entendu que lorsque ledit hétérocycloalkyle comprend au moins un atome d'azote, ce dernier peut éventuellement porter ledit substituant,
➢ **t** représente 1 ou 2,
➢ **n** représente 0, 1, 2 ou 3, et
➢ **p** représente 0, 1 ou 2,
sous forme d'acide, de base ou de sel d'addition à un acide ou à une base, ainsi que sous forme d'hydrate ou de solvat.

2. Composé selon la revendication 1, **caractérisé en ce que** :
**A** représente CH ou C(CH₃);
et/ou
X représente CH ou N.

3. Composé selon la revendication 1 ou 2, **caractérisé en ce que** :
➢ **A** représente CH,
➢ **X** représente CH, C(alkyle) ou N,
➢ **R₁**, **R₂**, **R₃ et R₄,** identiques ou différents, représentent chacun indépendamment les uns des autres :
○ un atome d'hydrogène,
○ un alkyle linéaire, ramifié ou cyclique, éventuellement substitué par un ou plusieurs atomes d'halogène, un groupe -OR₉ ou -NR₉R'₉, où R₉ et R'₉ représentent indépendamment l'un de l'autre un atome d'hydrogène ou un alkyle linéaire, ramifié ou cyclique,
○ un groupe -S(O)ₚR₁₀ ou un groupe -OR₁₀, où R₁₀ représente un atome d'hydrogène ou un alkyle linéaire, ramifié ou cyclique éventuellement substitué par un ou plusieurs atomes d'halogène, et p représente 0, 1 ou 2,
○ un atome d'halogène,
○ un groupe -N(R₁₁)C(O)R₁₂, où R₁₁ et R₁₂ représentent, indépendamment l'un de l'autre, un atome d'hydrogène ou un alkyle, linéaire, ramifié ou cyclique ou R₁₁ et R₁₂ forment ensemble avec les atomes auxquels ils sont rattachés un hétérocycloalkyle, de façon à former un lactame ;
○ un groupement -T-U, où
■ T représente :
• une liaison simple,
• un groupe alkylène, linéaire ou ramifié,
• un groupe -C(O)-,
• un groupe -S(O)ₚ, où p représente 0, 1 ou 2,
• Un groupe -O-(CH₂)ₙ- où n représente 0, 1, 2 ou 3,
et U représente un hétérocycle comprenant un ou plusieurs hétéroatomes choisi parmi N, O et S(O)p où p représente 0, 1 ou 2, ledit hétérocycle étant de formule : 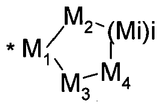 Où
• * représente la position d'attachement de U à T ;
• **M1** représente un atome de C ou N ;
• **M2** et **M3** identiques ou différents représentent un atome de C, N, O ou S(O)p où p=0, 1 ou 2 ;
• **M4** représente un atome de C, C(=O), N, O ou S(O)p où p=0, 1 ou 2 ;
Chacun des **Mi,** identiques ou différents, représentent un atome de C, C(=O), N, O ou S(O)p où p=0, 1 ou 2 ;
• i=0, 1, 2 ou 3 ;
étant entendu que chacun des M1, M2, M3, M4 ou Mi peut être éventuellement substitué si une valence est disponible et/ou les M1, M2, M3, M4 ou Mi adjacents peuvent être liés par une double liaison le cas échéant ;
ledit hétérocycle U étant saturé, insaturé ou aromatique éventuellement mono- ou di- ou poly-substitué par un, deux ou plusieurs substituant(s) choisi(s) parmi :
○ les groupes -OR₇, où R₇ représente un atome d'hydrogène ou un alkyle linéaire, ramifié ou cyclique,
○ les atomes d'halogène,
○ les groupes -COR₇ où R₇ représente un atome d'hydrogène ou un alkyle linéaire, ramifié ou cyclique,
○ les alkyles linéaires, ramifiés ou cycliques, éventuellement substitués par un ou plusieurs atome(s) d'halogène,
○ les hétérocycles saturés, insaturés ou aromatiques, notamment un hétérocycle comprenant un N, éventuellement substitués par un ou plusieurs groupements choisis parmi les atomes d'halogène et les groupes alkyle éventuellement substitués par un ou plusieurs atomes d'halogène;
ou bien deux groupements adjacents parmi R₁, R₂, R₃ et R₄ sont liés et forment avec les deux carbones qui les portent un hétérocycle saturé, insaturé ou aromatique, éventuellement substitué par un ou plusieurs groupe(s) alkyle(s), linéaire(s), ramifié(s) ou cyclique(s), le(s)dits groupe(s) alkyle(s) étant éventuellement substitué(s) par au moins un groupe choisi parmi les groupes - NR₉R'₉, où R₉ et R'₉ représentent indépendamment l'un de l'autre un atome d'hydrogène ou un groupe alkyle, ledit hétérocycle étant fusionné avec le cycle aromatique.

4. Composé selon l'une quelconque des revendications 1 à 3, **caractérisé en ce qu'**il répond à la formule (I') suivante : 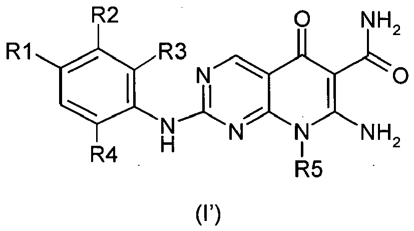 dans laquelle :
➢ **R₁** représente:
• un atome d'hydrogène,
• un alkyle linéaire, ramifié ou cyclique, éventuellement substitué par un ou plusieurs substituants choisis parmi les atomes d'halogène, les groupes -NR₉R'₉, où R₉ et R'₉ représentent indépendamment l'un de l'autre un atome d'hydrogène ou un groupe alkyle,
• -OR₁₀, où R₁₀ représente un atome d'hydrogène ou un alkyle linéaire, ramifié ou cyclique éventuellement substitué par un ou plusieurs atomes d'halogène,
• un atome d'halogène,
• un groupement -T-U, où
T représente :
○une liaison simple,
oun groupe groupe alkylène,
oun groupe- C(O)-,
o un groupe -O-(CH₂)ₙ où n représente 0, 1, 2 ou 3,
o et **U** représente un hétérocycle saturé, insaturé ou aromatique, éventuellement mono- ou di-substitué par un substituant,
**➢R₂**, **R₃ et R₄** identiques ou différents représentent indépendamment :
o Un atome d'hydrogène,
o un alkyle linéaire, ramifié ou cyclique, éventuellement substitué par un ou plusieurs substituants choisis parmi les atomes d'halogène et les groupes -NR₉R'₉, où R₉ et R'₉ représentent indépendamment l'un de l'autre un atome d'hydrogène ou un alkyle,
o un groupe -OR₁₀, où R₁₀ représente un atome d'hydrogène ou un alkyle éventuellement substitué par un ou plusieurs atomes d'halogène,
o un halogène,
o un groupe -T-U avec T représentant une liaison et U un morpholinyle, ou
**➢R₁ et R₂** sont liés et forment avec les deux carbones qui les portent un hétérocycle choisi parmi une pipéridine, un thiazole, un tétrahydrofurane et un dioxane, ledit hétérocycle étant éventuellement substitué par un alkyle linéaire, ramifié ou cyclique éventuellement substitué par -NR₉R'₉, où R₉ et R'₉ représentent indépendamment l'un de l'autre un atome d'hydrogène ou un méthyle,
ledit hétérocycle étant fusionné avec le cycle aromatique,
➢ **R₅** représente (i) un alkyle linéaire, ramifié ou cyclique comprenant de 1 à 5 atomes de carbone, éventuellement substitué par un ou plusieurs substituant(s) choisis parmi les atomes d'halogène ou un groupe -OH, (ii) un aryle éventuellement sustitué par un ou plusieurs substituants choisis parmi les groupes alkyle (C1-C3), les atomes d'halogène et les groupes -O-(C1-C3)alkyle;
sous forme d'acide, de base ou de sel d'addition à un acide ou à une base, ainsi que sous forme d'hydrate ou de solvat.

5. Composé selon l'une quelconque des revendications 1 à 4, caractérisé en ce
➢ **A** représente CH ; et/ou
➢ **X** représente CH ; et/ou
➢ **R₁** est différent d'un atome d'hydrogène ; et/ou
➢ **R₅** représente un alkyle linéaire, ramifié ou cyclique; et/ou
➢ **U** représente un hétérocycle saturé, insaturé ou aromatique, éventuellement mono- ou di-substitué comprenant au moins un atome d'azote et/ou un atome d'oxygène, et/ou
➢ **U** représente un hétérocycle saturé comprenant au moins un atome d'azote ; et/ou
➢ **U** représente un hétérocycle choisi parmi :
o un azétidinyle;
o un pyrrolidinyle;
o un oxopyrrolidinyle;
o un pipéridinyle;
o un 1,2,3,6-tétrahydro-pyridinyle;
o un pyridinyle;
o un pipérazinyle;
o un diazépanyle;
o un morpholinyle;
o un 1,1-dioxo-1lambda6-thiomorpholin-4-yle.

6. Composé selon l'une quelconque des revendications précédentes, **caractérisé en ce que R₁** est choisi parmi :

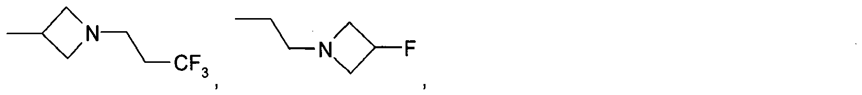

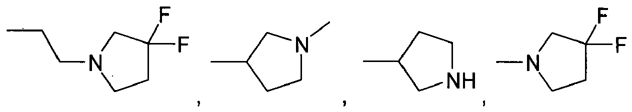

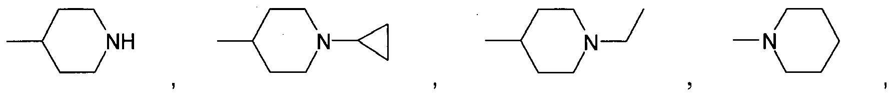

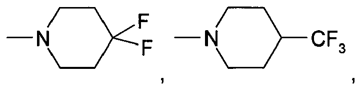

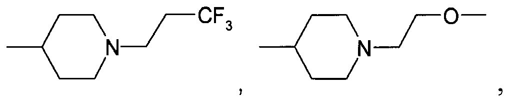

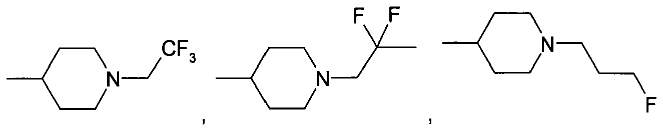

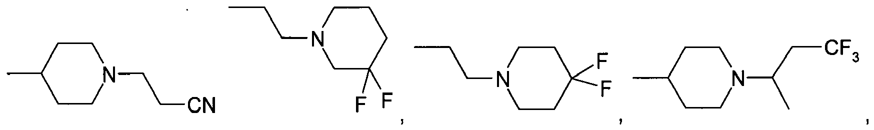

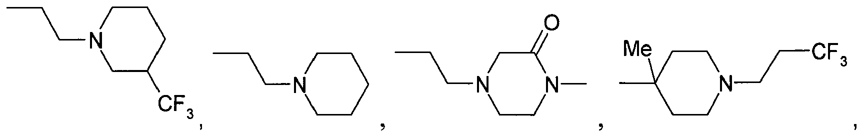

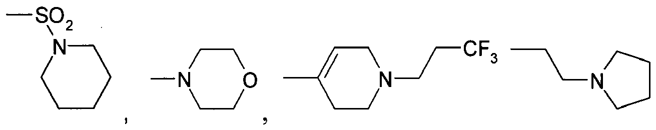

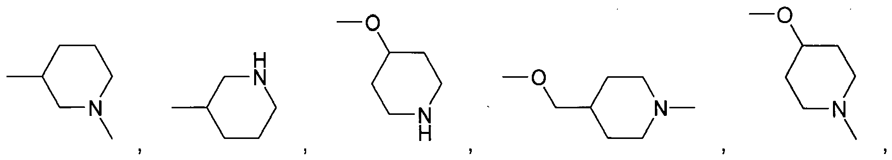

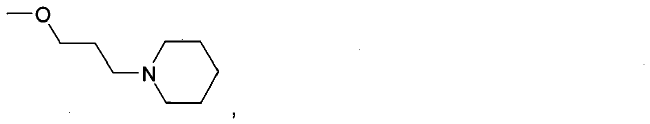

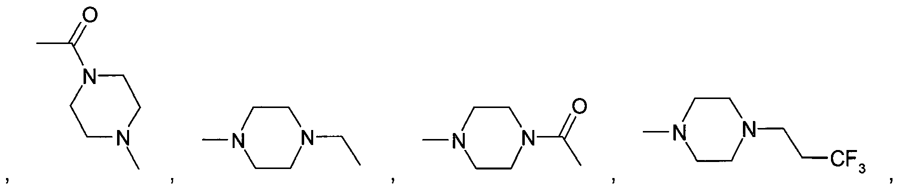

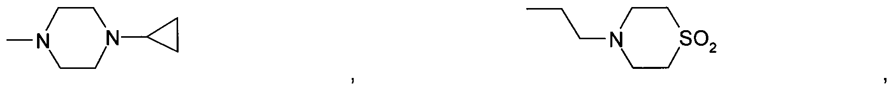

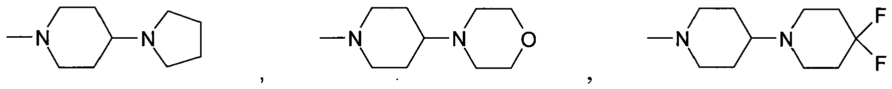

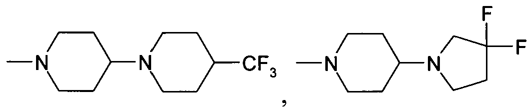

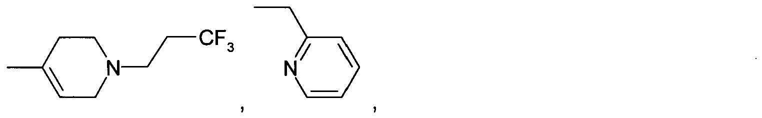

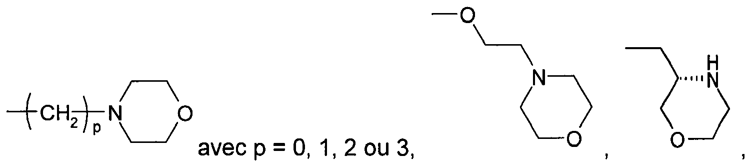

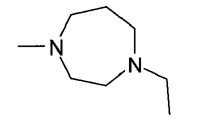

7. Composé selon l'une quelconque des revendications précédentes, **caractérisé en ce que A** et **X** représentent CH.

8. Composé selon l'une quelconque des revendications précédentes, **caractérisé en ce que U** représente un hétérocycle saturé comprenant au moins un atome d'azote.

9. Composé selon l'une quelconque des revendications précédentes, **caractérisé en ce que** il est choisi parmi :
7-Amino-8-éthyl-2-(4-hydroxy-phénylamino)-5-oxo-5,8-dihydro-pyrido[2,3-d]pyrimidine-6-carboxamide;
7-Amino-2-(benzothiazol-6-ylamino)-8-éthyl-5-oxo-5,8-dihydro-pyrido[2,3-d]pyrimidine-6-carboxamide ;
7-Amino-2-[4-(cyclopropanecarbonyl-méthyl-amino)-phénylamino]-8-éthyl-5-oxo-5,8-dihydro-pyrido[2,3-d]pyrimidine-6-carboxamide ;
7-Amino-8-éthyl-2-[4-(4-méthyl-pipérazine-1-carbonyl)-phénylamino]-5-oxo-5,8-dihydro-pyrido[2,3-d]pyrimidine-6-carboxamide ;
7-Amino-2-(4-cyclopentyloxy-phénylamino)-8-éthyl-5-oxo-5,8-dihydro-pyrido[2,3-d]pyrimidine-6-carboxamide ;
7-Amino-8-éthyl-2-[4-(4-éthyl-pipérazine-1-yl)-phénylamino]-5-oxo-5,8-dihydro-pyrido[2,3-d]pyrimidine-6-carboxamide ;
7-Amino-8-éthyl-5-oxo-2-[4-(4-pyrrolidin-1-yl-pipéridin-1-yl)-phénylamino]-5,8-dihydro-pyrido[2,3-d]pyrimidine-6-carboxamide ;
7-Amino-8-cyclopentyl-2-(4-morpholin-4-yl-phénylamino)-5-oxo-5,8-dihydro-pyrido[2,3-d]pyrimidine-6-carboxamide ;
7-Amino-8-éthyl-5-oxo-2-[4-(pipéridine-1-sulfonyl)-phénylamino]-5,8-dihydro-pyrido[2,3-d]pyrimidine-6-carboxamide ;
2-[4-(4-Acétyl-pipérazin-1-yl)-phénylamino]-7-amino-8-éthyl-5-oxo-5,8-dihydro-pyrido[2,3-d]pyrimidine-6-carboxamide ;
7-Amino-8-éthyl-2-(4-morpholin-4-yl-benzylamino)-5-oxo-5,8-dihydro-pyrido[2,3-d]pyrimidine-6-carboxamide ;
7-Amino-8-éthyl-5-oxo-2-(quinolin-3-ylamino)-5,8-dihydro-pyrido[2,3-d]pyrimidine-6-carboxylamide ;
7-Amino-8-éthyl-5-oxo-2-[4-(3-pipéridin-1-yl-propoxy)-phénylamino]-5,8-dihydro-pyrido[2,3-d]pyrimidine-6-carboxamide ;
7-Amino-8-éthyl-5-oxo-2-(3,4,5,6-tétrahydro-2H-[1,2']bipyridinyl-5'-ylamino)-5,8-dihydro-pyrido[2,3-d]pyrimidine-6-carboxamide ;
7-Amino-8-éthyl-5-oxo-2-[4-(2-oxo-pyrrolidin-1-yl)-phénylamino]-5,8-dihydro-pyrido[2,3-d]pyrimidine-6-carboxamide ;
7-Amino-8-éthyl-5-oxo-2-(quinolin-6-ylamino)-5,8-dihydro-pyrido[2,3-d]pyrimidine-6-carboxamide ;
7-Amino-8-éthyl-2-(3-morpholin-4-yl-phénylamino)-5-oxo-5,8-dihydro-pyrido[2,3-d]pyrimidine-6-carboxamide ;
7-Amino-2-(2,3-dihydro-benzo[1,4]dioxin-6-ylamino)-8-éthyl-5-oxo-5,8-dihydro-pyrido[2,3-d]pyrimidine-6-carboxamide ;
7-Amino-8-éthyl-2-(3-fluoro-4-hydroxy-phénylamino)-5-oxo-5,8-dihydro-pyrido[2,3-d]pyrimidine-6-carboxamide ;
7-Amino-8-éthyl-2-(3-méthylsulfanyl-phénylamino)-5-oxo-5,8-dihydro-pyrido[2,3-d]pyrimidine-6-carboxamide ;
7-Amino-8-éthyl-2-(4-morpholin-4-yl-phénylamino)-5-oxo-5,8-dihydro-pyrido[2,3-d]pyrimidine-6-carboxamide ;
7-Amino-8-éthyl-2-(2-fluoro-phénylamino)-5-oxo-5,8-dihydro-pyrido[2,3-d]pyrimidine-6-carboxamide ;
7-Amino-2-[4-(4,4-difluoro-[1,4']bipipéridinyl-1'-yl)-2-méthoxy-phénylamino]-8-éthyl-5-oxo-5,8-dihydro-pyrido[2,3-d]pyrimidine-6-carboxamide
7-Amino-8-éthyl-2-[2-méthoxy-4-(4-trifluorométhyl-[1,4']bipipéridinyl-1'-yl)-phénylamino]-5-oxo-5,8-dihydro-pyrido[2,3-d]pyrimidine-6-carboxamide
7-Amino-2-{4-[4-(3,3-difluoro-pyrrolidin-1-yl)-pipéridin-1-yl]-2-méthoxy-phénylamino}-8-éthyl-5-oxo-5,8-dihydro-pyrido[2,3-d]pyrimidine-6-carboxamide
7-Amino-8-éthyl-2-[4-(4-éthyl-pipérazin-1-yl)-2-fluoro-6-méthoxy-phénylamino]-5-oxo-5,8-dihydro-pyrido[2,3-d]pyrimidine-6-carboxamide ;
7-Amino-2-[4-(1-cyclopropyl-pipéridin-4-yl)-2-méthoxy-phénylamino]-8-éthyl-5-oxo-5,8-dihydro-pyrido[2,3-d]pyrimidine-6-carboxamide ;
7-Amino-2-(2-diméthylaminométhyl-chroman-6-ylamino)-8-éthyl-5-oxo-5,8-dihydro-pyrido[2,3-d]pyrimidine-6-carboxamide ;
7-Am i no-2-[5-chloro-4-(4-cyclopropyl-pipérazi n-1-yl)-2-méthoxy-phénylam ino]-8-éthyl-5-oxo-5,8-dihydro-pyrido[2,3-d]pyrimidine-6-carboxamide ;
7-Amino-8-éthyl-2-[2-méthoxy-4-(4-morpholin-4-yl-pipéridin-1-yl)-phénylamino]-5-oxo-5,8-dihydro-pyrido[2,3-d]pyrimidine-6-carboxyamide ;
7-Amino-2-[4-(4-cyclopropyl-pipérazin-1-yl)-2-difluorométhoxy-phénylamino]-8-éthyl-5-oxo-5,8-dihydro-pyrido[2,3-d]pyrimidine-6-carboxamide ;
7-Amino-2-[4-(4-cyclopropyl-pipérazin-1-yl)-2-méthoxy-phénylamino]-8-éthyl-5-oxo-5,8-dihydro-pyrido[2,3-d]pyrimidine-6-carboxamide ;
7-Amino-8-éthyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide ;
7-Amino-2-[2-chloro-4-(4-éthyl-pipérazin-1-yl)-phénylamino]-8-éthyl-5-oxo-5,8-dihydro-pyrido[2,3-d]pyrimidine-6-carboxamide ;
7-Amino-8-éthyl-2-[4-(4-éthyl-pipérazin-1-yl)-3-trifluorométhyl-phénylamino]-5-oxo-5,8-dihydro-pyrido[2,3-d]pyrimidine-6-carboxamide ;
7-Amino-8-éthyl-2-(4-morpholin-4-ylméthyl-phénylamino)-5-oxo-5,8-dihydro-pyrido[2,3-d]pyrimidine-6-carboxamide ;
7-Amino-8-éthyl-5-oxo-2-{4-[4-(3,3,3-trifluoro-propyl)-pipérazin-1-yl]-phénylamino}-5,8-dihydro-pyrido[2,3-d]pyrimidine-6-carboxamide ;
7-Amino-8-éthyl-2-[4-(4-éthyl-pipérazin-1-yl)-3-fluoro-phénylamino]-5-oxo-5,8-dihydro-pyrido[2,3-d]pyrimidine-6-carboxamide ;
6-(4-Morpholin-4-yl-phénylamino)-9-oxo-1,3,4,9-tétrahydro-2H-1,4a,5,7-tétraazaphénanthrene-10-carboxamide ;
7-Amino-8-éthyl-2-[4-(4-éthyl-pipérazin-1-yl)-2-méthoxy-phénylamino]-5-oxo-5,8-dihydro-pyrido[2,3-d]pyrimidine-6-carboxamide ;
7-Amino-8-éthyl-2-[4-(1-méthyl-pipéridin-4-ylméthoxy)-phénylamino]-5-oxo-5,8-dihydro-pyrido[2,3-d]pyrimidine-6-carboxamide ;
7-Amino-2-[4-(4-cyclopropyl-pipérazin-1-yl)-phénylamino]-8-éthyl-5-oxo-5,8-dihydro-pyrido[2,3-d]pyrimidine-6-carboxamide ;
7-Amino-8-éthyl-2-[4-(4-éthyl-pipérazin-1-yl)-2-méthyl-phénylamino]-5-oxo-5,8-dihydro-pyrido[2,3-d]pyrimidine-6-carboxamide ;
7-Amino-8-éthyl-2-[4-(4-éthyl-[1,4]diazépan-1-yl)-phénylamino]-5-oxo-5,8-dihydro-pyrido[2,3-d]pyrimidine-6-carboxamide ;
8-(4-Morpholin-4-yl-phénylamino)-5-oxo-1,2,3,5-tétrahydro-3,7,9,9b-tétraaza-cyclopenta[a]naphthalène-4-carboxamide;
7-Amino-8-éthyl-5-oxo-2-{4-[2-(3-trifluorométhyl-pipéridin-1-yl)-éthyl]-phénylamino}-5,8-dihydro-pyrido[2,3-d]pyrimidine-6-carboxamide;
7-Amino-8-éthyl-2-[4-(3-morpholin-4-yl-propyl)-phénylamino]-5-oxo-5,8-dihydro-pyrido[2,3-d]pyrimidine-6-carboxamide;
7-Amino-8-éthyl-5-oxo-2-{4-[1-(2,2,2-trifluoro-éthyl)-pipéridin-4-yl]-phénylamino}-5,8-dihydro-pyrido[2,3-d]pyrimidine-6-carboxamide;
7-Amino-8-éthyl-2-(4-(S)-1-morpholin-3-ylméthyl-phénylamino)-5-oxo-5,8-dihydro-pyrido[2,3-d]pyrimidine-6-carboxamide;
7-Amino-8-isobutyl-2-[4-(2-morpholin-4-yl-éthyl)-phénylamino]-5-oxo-5,8-dihydro-pyrido[2,3-d]pyrimidine-6-carboxamide;
7-Amino-8-éthyl-2-{4-[2-(3-fluoro-azétidin-1-yl)-éthyl]-phénylamino}-5-oxo-5,8-dihydro-pyrido[2,3-d]pyrimidine-6-carboxamide;
7-Amino-2-[4-(2-morpholin-4-yl-éthyl)-phénylamino]-5-oxo-8-propyl-5,8-dihydro-pyrido[2,3-d]pyrimidine-6-carboxamide;
7-Amino-8-éthyl-2-[4-(2-hydroxy-éthyl)-phénylamino]-5-oxo-5,8-dihydro-pyrido[2,3-d]pyrimidine-6-carboxamide;
7-amino-8-éthyl-2-(4-hydroéthyl-phénylamino)-5-oxo-5,8-dihydropyrido[2,3-d]pyrimidine-6-carboxamide ;
7-Amino-8-éthyl-2-(2-méthyl-1,2,3,4-tétrahydro-isoquinolin-6-ylamino)-5-oxo-5,8-dihydro-pyrido[2,3-d]pyrimidine-6-carboxamide;
7-Amino-2-{4-[2-(1,1-dioxo-1lambda6-thiomorpholin-4-yl)-éthyl]-phénylamino}-8-éthyl-5-oxo-5,8-dihydro-pyrido[2,3-d]pyrimidine-6-carboxamide;
7-Amino-8-éthyl-2-{4-[2-(4-méthyl-3-oxo-pipérazin-1-yl)-éthyl]-phénylamino}-5-oxo-5,8-dihydro-pyrido[2,3-d]pyrimidine-6-carboxamide;
7-Amino-8-éthyl-5-oxo-2-{4-[1-(3,3,3-trifluoro-propyl)-pipéridin-4-yl]-phénylamino}-5,8-dihydro-pyrido[2,3-d]pyrimidine-6-carboxamide;
7-Amino-2-(4-diméthylaminométhyl-phénylamino)-5-oxo-8-(2,2,2-trifluoro-éthyl)-5,8-dihydro-pyrido[2,3-d]pyrimidine-6-carboxamide;
7-Amino-2-[3-chloro-4-(4-pyrrolidin-1-yl-pipéridin-1-yl)-phénylamino]-8-éthyl-5-oxo-5,8-dihydro-pyrido[2,3-d]pyrimidine-6-carboxamide;
7-Amino-8-éthyl-5-oxo-2-(4-pyridin-2-ylméthyl-phénylamino)-5,8-dihydro-pyrido[2,3-d]pyrimidine-6-carboxamide;
7-Amino-2-{4-[2-(3,3-difluoro-pyrrolidin-1-yl)-éthyl]-phénylamino}-8-éthyl-5-oxo-5,8-dihydro-pyrido[2,3-d]pyrimidine-6-carboxamide;
7-Amino-8-éthyl-5-oxo-2-(4-pyrrolidin-3-yl-phénylamino)-5,8-dihydro-pyrido[2,3-d]pyrimidine-6-carboxamide;
7-Amino-8-éthyl-5-oxo-2-[4-(2-pipéridin-1-yl-éthyl)-phénylamino]-5,8-dihydro-pyrido[2,3-d]pyrimidine-6-carboxamide;
7-Amino-2-[4-(2-morpholin-4-yl-éthyl)-phénylamino]-5-oxo-8-(2,2,2-trifluoro-ethyl)-5,8-dihydro-pyrido[2,3-d]pyrimidine-6-carboxamide
7-Amino-2-{4-[2-(4,4-difluoro-pipéridin-1-yl)-éthyl]-phénylamino}-8-éthyl-5-oxo-5,8-dihydro-pyrido[2,3-d]pyrimidine-6-carboxamide;
7-Amino-8-éthyl-2-[4-(1-méthyl-pyrrolidin-3-yl)-phénylamino]-5-oxo-5,8-dihydro-pyrido[2,3-d]pyrimidine-6-carboxamide;
7-Amino-2-{4-[2-(3,3-difluoro-pipéridin-1-yl)-éthyl]-phénylamino}-8-éthyl-5-oxo-5,8-dihydro-pyrido[2,3-d]pyrimidine-6-carboxamide;
7-Amino-8-(3-méthoxy-propyl)-2-[4-(2-morpholin-4-yl-éthyl)-phénylamino]-5-oxo-5,8-dihydro-pyrido[2,3-d]pyrimidine-6-carboxamide;
7-Amino-8-éthyl-2-[4-(1-méthyl-pipéridin-3-yl)-phénylamino]-5-oxo-5,8-dihydro-pyrido[2,3-d]pyrimidine-6-carboxamide;
7-Amino-2-(4-diméthylaminométhyl-phénylamino)-8-isopropyl-5-oxo-5,8-dihydro-pyrido[2,3-d]pyrimidine-6-carboxamide;
7-Amino-8-éthyl-5-oxo-2-(4-pipéridin-3-yl-phénylamino)-5,8-dihydro-pyrido[2,3-d]pyrimidine-6-carboxamide;
7-Amino-8-éthyl-5-oxo-2-(1,2,3,4-tétrahydro-isoquinolin-7-ylamino)-5,8-dihydro-pyrido[2,3-d]pyrimidine-6-carboxamide;
7-Amino-8-éthyl-5-oxo-2-(1,2,3,4-tétrahydro-isoquinolin-7-ylamino)-5,8-dihydro-pyrido[2,3-d]pyrimidine-6-carboxamide;
7-Amino-8-éthyl-2-[4-(1-méthyl-pipéridin-4-yloxy)-phénylamino]-5-oxo-5,8-dihydro-pyrido[2,3-d]pyrimidine-6-carboxamide;
7-Amino-8-éthyl-2-[4-(2-morpholin-4-yl-éthoxy)-phénylamino]-5-oxo-5,8-dihydro-pyrido[2,3-d]pyrimidine-6-carboxamide;
7-Amino-2-(3-diméthylaminométhyl-phénylamino)-8-éthyl-5-oxo-5,8-dihydro-pyrido[2,3-d]pyrimidine-6-carboxamide;
7-Amino-8-éthyl-5-oxo-2-[4-(4-pyrrolidin-1-yl-pipéridin-1-yl)-phénylamino]-5,8-dihydro-pyrido[2,3-d]pyrimidine-6-carboxamide;
7-Amino-8-éthyl-5-oxo-2-[4-(pipéridin-4-yloxy)-phénylamino]-5,8-dihydro-pyrido[2,3-d]pyrimidine-6-carboxamide;
7-Amino-8-(3-amino-propyl)-2-(4-morpholin-4-yl-phénylamino)-5-oxo-5,8-dihydro-pyrido[2,3-d]pyrimidine-6-carboxamide;
7-Amino-8-éthyl-2-[4-(2-morpholin-4-yl-éthyl)-phénylamino]-5-oxo-5,8-dihydro-pyrido[2,3-d]pyrimidine-6-carboxamide;
7-Amino-2-(4-diméthylaminométhyl-phénylamino)-8-éthyl-5-oxo-5,8-dihydro-pyrido[2,3-d]pyrimidine-6-carboxamide;
7-Amino-8-éthyl-2-[4-(1-éthyl-pipéridin-4-yl)-phénylamino]-5-oxo-5,8-dihydro-pyrido[2,3-d]pyrimidine-6-carboxamide;
7-Amino-8-éthyl-5-oxo-2-(4-pipéridin-4-yl-phénylamino)-5,8-dihydro-pyrido[2,3-d]pyrimidine-6-carboxamide;
7-Amino-2-[3-chloro-4-(4-éthyl-pipérazin-1-yl)-phénylamino]-8-éthyl-5-oxo-5,8-dihydro-pyrido[2,3-d]pyrimidine-6-carboxamide
7-Amino-8-éthyl-2-[3-méthoxy-4-(3-pipéridin-1-yl-propoxy)-phénylamino]-5-oxo-5,8-dihydro-pyrido[2,3-d]pyrimidine-6-carboxamide
7-Amino-8-éthyl-5-oxo-2-{4-[2-(4-trifluorométhyl-pipéridin-1-yl)-éthyl]-phénylamino}-5,8-dihydro-pyrido[2,3-d]pyrimidine-6-carboxamide
7-Amino-2-{4-[2-(cis-2,6-diméthyl-morpholin-4-yl)-éthyl]-phénylamino}-8-éthyl-5-oxo-5,8-dihydro-pyrido[2,3-d]pyrimidine-6-carboxamide
7-Amino-2-(4-diéthylaminométhyl-phénylamino)-8-éthyl-5-oxo-5,8-dihydro-pyrido[2,3-d]pyrimidine-6-carboxamide
7-Amino-2-(4-diméthylaminométhyl-phénylamino)-8-(3-méthoxy-propyl)-5-oxo-5,8-dihydro-pyrido[2,3-d]pyrimidine-6-carboxamide
7-Am ino-8-éthyl-2-[4-(4-éthyl-pipérazin-1-yl)-2-fluoro-phénylamino]-5-oxo-5,8-dihydro-pyrido[2,3-d]pyrimidine-6-carboxamide
7-Amino-2-{4-[1-(2-cyano-éthyl)-pipéridin-4-yl]-phénylamino}-8-éthyl-5-oxo-5,8-dihydro-pyrido[2,3-d]pyrimidine-6-carboxamide
7-Amino-8-éthyl-2-{4-[1-(3-fluoro-propyl)-pipéridin-4-yl]-phénylamino}-5-oxo-5,8-dihydro-pyrido[2,3-d]pyrimidine-6-carboxylic acid amide
7-Amino-8-éthyl-2-[4-(4-éthyl-pipérazin-1-yl)-3-méthyl-phénylamino]-5-oxo-5,8-dihydro-pyrido[2,3-d]pyrimidine-6-carboxamide
7-Amino-2-[4-(4-cyclopropyl-pipérazin-1-yl)-2-éthyl-phénylamino]-8-éthyl-5-oxo-5,8-dihydro-pyrido[2,3-d]pyrimidine-6-carboxamide
(±)-7-Amino-2-trans-[4-(2-diméthylamino-cyclopropyl)-phénylamino]-8-éthyl-5-oxo-5,8-dihydro-pyrido[2,3-d]pyrimidine-6-carboxamide
7-Am ino-2-[4-(4-cyclopropyl-pipérazi n-1-yl)-5-fluoro-2-méthoxy-phénylam ino]-8-éthyl-5-oxo-5,8-dihydro-pyrido[2,3-d]pyrimidine-6-carboxamide
7-Amino-2-[4-(4-cyclopropyl-pipérazin-1-yl)-3-fluoro-2-méthoxy-phénylamino]-8-éthyl-5-oxo-5,8-dihydro-pyrido[2,3-d]pyrimidine-6-carboxamide
7-Am ino-2-[4-(4-cyclopropyl-pi pérazi n-1-yl)-2-ethoxy-phénylam i no]-8-éthyl-5-oxo-5, 8-dihydro-pyrido[2,3-d]pyrimidine-6-carboxamide
7-Amino-8-éthyl-5-oxo-2-(4-propyl-phénylamino)-5,8-dihydro-pyrido[2,3-d]pyrimidine-6-carboxamide
7-Amino-8-éthyl-5-oxo-2-(4-propoxy-phénylamino)-5,8-dihydro-pyrido[2,3-d]pyrimidine-6-carboxamide
7-Amino-8-éthyl-2-(6-méthoxy-pyridin-3-ylamino)-5-oxo-5,8-dihydro-pyrido[2,3-d]pyrimidine-6-carboxamide
7-Amino-8-éthyl-2-(4-fluoro-phénylamino)-5-oxo-5,8-dihydro-pyrido[2,3-d]pyrimidine-6-carboxamide
7-Amino-8-éthyl-2-(4-méthoxy-phénylamino)-5-oxo-5,8-dihydro-pyrido[2,3-d]pyrimidine-6-carboxamide
7-Amino-2-(benzo[1,3]dioxol-5-ylamino)-8-éthyl-5-oxo-5,8-dihydro-pyrido[2,3-d]pyrimidine-6-carboxamide
7-Amino-8-éthyl-5-oxo-2-(4-pipéridin-1-yl-phénylamino)-5,8-dihydro-pyrido[2,3-d]pyrimidine-6-carboxamide
8-Éthyl-2-{2-méthoxy-4-[1-(3,3,3-trifluoro-propyl)-pipéridin-4-yl]-phénylamino}-7-méthylamino-5-oxo-5,8-dihydro-pyrido[2,3-d]pyrimidine-6-carboxamide
7-Amino-8-isobutyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-cyclopropylméthyl-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-(2-méthoxy-éthyl)-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-(tétrahydro-furan-2-ylméthyl)-5,8-dihydro-pyrido[2,3-d]pyrimidine-6-carboxamide
7-Amino-8-éthyl-5-oxo-2-{4-[1-(3,3,3-trifluoro-propyl)-azetidin-3-yl]-phénylamino}-5,8-dihydro-pyrido[2,3-d]pyrimidine-6-carboxamide
7-Amino-2-{5-fluoro-2-méthoxy-4-[1-(3,3,3-trifluoro-propyl)-pipéridin-4-yl]-phénylamino}-8-isobutyl-5-oxo-5,8-dihydro-pyrido[2,3-d]pyrimidine-6-carboxamide
7-Amino-8-éthyl-5-oxo-2-[4-(1,2,3,6-tétrahydro-pyridin-4-yl)-phénylamino]-5,8-dihydro-pyrido[2,3-d]pyrimidine-6-carboxamide
7-Amino-2-(2-méthoxy-4-pipéridin-4-yl-phénylamino)-5-oxo-8-phényl-5,8-dihydro-pyrido[2,3-d]pyrimidine-6-carboxamide
7-Amino-8-(2-hydroxy-éthyl)-2-(2-méthoxy-4-pipéridin-4-yl-phénylamino)-5-oxo-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-thiazol-2-ylméthyl-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-thiazol-5-ylméthyl-5,8-dihydro-pyrido[2,3-d]pyrimidine-6-carboxamide
7-Amino-8-(2-hydroxy-2-méthyl-propyl)-2-(2-méthoxy-4-pipéridin-4-yl-phénylamino)-5-oxo-5,8-dihydro-pyrido[2,3-d]pyrimidine-6-carboxamide
8-(2-Acétylamino-éthyl)-7-amino-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-(2-amino-éthyl)-2-(4-morpholin-4-yl-phénylamino)-5-oxo-5,8-dihydro-pyrido[2,3-d]pyrimidine-6-carboxamide
7-Amino-2-(4-morpholin-4-yl-phénylamino)-5-oxo-8-pyrrolidin-3-ylméthyl-5,8-dihydro-pyrido[2,3-d]pyrimidine-6-carboxamide
7-Amino-8-éthyl-2-[4-(4-éthyl-pipérazin-1-yl)-2-hydroxy-phénylamino]-5-oxo-5,8-dihydro-pyrido[2,3-d]pyrimidine-6-carboxamide
7-Amino-2-(4-morpholin-4-yl-phénylamino)-5-oxo-8-pipéridin-4-ylméthyl-5,8-dihydro-pyrido[2,3-d]pyrimidine-6-carboxamide
7-Amino-2-(2-méthoxy-4-morpholin-4-yl-phénylamino)-5-oxo-8-pyrrolidin-3-ylméthyl-5,8-dihydro-pyrido[2,3-d]pyrimidine-6-carboxamide
7-Amino-8-éthyl-2-(2-méthoxy-4-pipéridin-4-yl-phénylamino)-4-méthyl-5-oxo-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-thiophèn-2-ylméthyl-5,8-dihydro-pyrido[2,3-d]pyrimidine-6-carboxamide
7-Amino-8-isobutyl-2-[2-méthoxy-4-(4-morpholin-4-yl-pipéridin-1-yl)-phénylamino]-5-oxo-5,8-dihydro-pyrido[2,3-d]pyrimidine-6-carboxamide
7-Amino-2-(4-morpholin-4-yl-phénylamino)-5-oxo-8-pyrrolidin-2-ylméthyl-5,8-dihydro-pyrido[2,3-d]pyrimidine-6-carboxamide
7-Amino-8-éthyl-2-{2-méthoxy-4-[1-(3,3,3-trifluoro-propyl)-1,2,3,6-tétrahydro-pyridin-4-yl]-phénylamino}-5-oxo-5,8-dihydro-pyrido[2,3-d]pyrimidine-6-carboxamide
7-Amino-2-[4-(4-éthyl-pipérazin-1-yl)-2-méthoxy-phénylamino]-8-(2-hydroxy-2-méthyl-propyl)-5-oxo-5,8-dihydro-pyrido[2,3-d]pyrimidine-6-carboxamide
7-Amino-8-(2-hydroxy-2-méthyl-propyl)-2-{2-méthoxy-4-[1-(3,3,3-trifluoro-propyl)-pipéridin-4-yl]-phénylamino}-5-oxo-5,8-dihydro-pyrido[2,3-d]pyrimidine-6-carboxamide
7-Amino-8-(2-hydroxy-2-méthyl-propyl)-2-[4-(4-éthyl-pi péridin-1-yl)-2-méthoxy-phénylamino]-5-oxo-5,8-dihydro-pyrido[2,3-d]pyrimidine-6-carboxamide
7-Amino-2-[4-(1-cyclopropyl-pipéridin-4-yl)-2-méthoxy-phénylamino]-8-(2-hydroxy-2-méthyl-propyl)-5-oxo-5,8-dihydro-pyrido[2,3-d]pyrimidine-6-carboxamide
7-Amino-8-(2-hydroxy-2-méthyl-propyl)-2-{2-méthoxy-4-[1-(2-méthoxy-éthyl)-pipéridin-4-yl]-phénylamino}-5-oxo-5,8-dihydro-pyrido[2,3-d]pyrimidine-6-carboxamide
7-Amino-2-[2-méthoxy-4-(1-méthyl-pipéridin-4-yl)-phénylamino]-5-oxo-8-phényl-5,8-dihydro-pyrido[2,3-d]pyrimidine-6-carboxamide
7-Amino-8-(3-fluoro-phényl)-2-[2-méthoxy-4-pipéridin-4-yl-phénylamino]-5-oxo-5,8-dihydro-pyrido[2,3-d]pyrimidine-6-carboxamide
7-Amino-8-(4-fluoro-phényl)-2-[2-méthoxy-4-pipéridin-4-yl-phénylamino]-5-oxo-5,8-dihydro-pyrido[2,3-d]pyrimidine-6-carboxamide
7-Amino-2-[2-méthoxy-4-pipéridin-4-yl-phénylamino]-5-oxo-8-m-tolyl-5,8-dihydro-pyrido[2,3-d]pyrimidine-6-carboxamide
7-Amino-2-[2-méthoxy-4-pipéridin-4-yl-phénylamino]-5-oxo-8-p-tolyl-5,8-dihydro-pyrido[2,3-d]pyrimidine-6-carboxamide
7-Amino-8-(3-méthoxy-phényl)-2-(2-méthoxy-4-pipéridin-4-yl-phénylamino)-5-oxo-5,8-dihydro-pyrido[2,3-d]pyrimidine-6-carboxamide
7-Amino-2-[2-méthoxy-4-(2-pyrrolidin-1-yl-éthyl)-phénylamino]-5-oxo-8-phényl-5,8-dihydro-pyrido[2,3-d]pyrimidine-6-carboxamide
7-Amino-8-(4-fluoro-phényl)-2-[2-méthoxy-4-(1-méthyl-pipéridin-4-yl)-phénylamino]-5-oxo-5,8-dihydro-pyrido[2,3-d]pyrimidine-6-carboxamide
7-Amino-2-[2-méthoxy-4-(1-méthyl-pipéridin-4-yl)-phénylamino]-5-oxo-8-m-tolyl-5,8-dihydro-pyrido[2,3-d]pyrimidine-6-carboxamide
7-Amino-2-{2-méthoxy-4-[1-(3,3,3-trifluoro-propyl)-pipéridin-4-yl]-phénylamino}-5-oxo-8-phényl-5,8-dihydro-pyrido[2,3-d]pyrimidine-6-carboxamide
7-Amino-2-[4-(4-éthyl-pipérazin-1-yl)-2-méthoxy-phénylamino]-5-oxo-8-phényl-5,8-dihydro-pyrido[2,3-d]pyrimidine-6-carboxamide
à l'état de base ou de sel d'addition à un acide, ainsi qu'à l'état d'hydrate ou de solvat.

10. Procédé de préparation d'un composé de formule (I) selon l'une quelconque des revendications 1 à 9, **caractérisé en ce qu'**il comprend la réaction d'un composé de formule (IX) 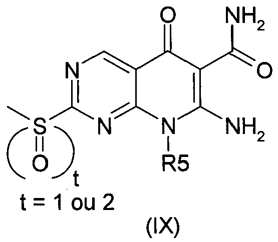 avec une amine primaire de formule (A) : 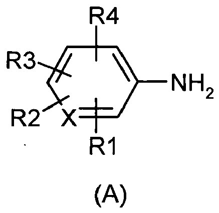 dans lesquelles R₁, R₂, R₃, R₄ et R₅ sont définis selon l'une quelconque des revendications 1 à 9 et t représente 1 ou 2.

11. Procédé de préparation d'un composé de formule (I) selon l'une quelconque des revendications 1 à 9, **caractérisé en ce qu'**il comprend la réaction d'un composé de formule générale (XIV) ou (XV) : 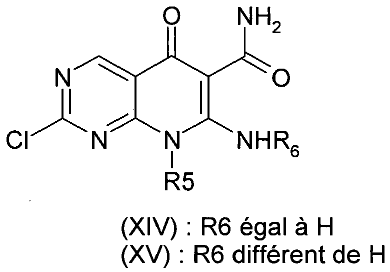 Et d'un composé de formule générale (A) : 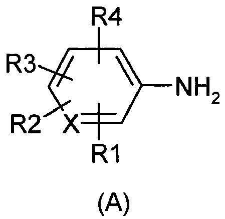 dans lesquelles R₁, R₂, R₃, R₄, R₅ et R₆ sont définis selon l'une quelconque des revendications 1 à 9.

12. Composé de formule (VII) :

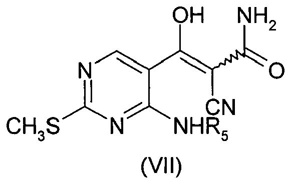

13. Composé de formule (VIII) : 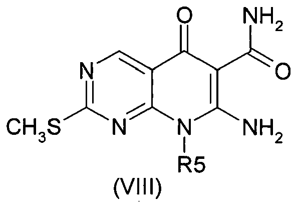 dans laquelle R₅ est défini selon l'une quelconque des revendications 1 à 8.

14. Composé de formule (IX) : 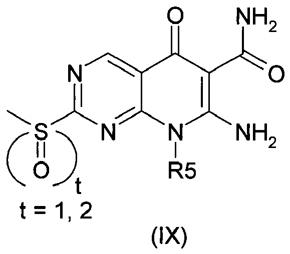 dans laquelle **R₅** est défini selon l'une quelconque des revendications 1 à 8 et t représente 1 ou 2.

15. Composé de formule (XV) : 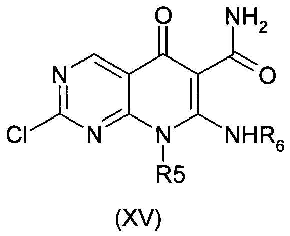 dans laquelle **R₅** et **R**₆ sont définis selon l'une quelconque des revendications 1 à 8 et **R₆** différent de H.

16. Médicament, **caractérisé en ce qu'**il comprend un composé de formule (I) selon l'une quelconque des revendications 1 à 9, ou un sel d'addition de ce composé à un acide pharmaceutiquement acceptable, ou encore un hydrate ou un solvat du composé de formule (I).

17. Composition pharmaceutique, **caractérisée en ce qu'**elle comprend un composé de formule (I) selon l'une quelconque des revendications 1 à 9, ou un sel pharmaceutiquement acceptable, un hydrate ou un solvat de ce composé, ainsi qu'au moins un excipient pharmaceutiquement acceptable.

18. Composé de formule (I) selon l'une quelconque des revendications 1 à 9 pour son utilisation dans le traitement et/ou la prévention de toute pathologie dans laquelle la kinase CaMKII est impliquée.

19. Composé selon la revendication 18 pour son utilisation dans le traitement et/ou la prévention des pathologies cardiovasculaires incluant l'infarctus du myocarde, l'hypertrophie ventriculaire, la fibrose myocardique, l'insuffisance cardiaque, les arythmies cardiaques et la resténose, ainsi que des pathologies vasculaires liées à une dysfonction endothéliale incluant l'athérosclérose, ainsi que des pathologies associées au développement de fibrose incluant la fibrose hépatique, pancréatique, rénale, pulmonaire, cutanée, intestinale et oculaire, la transplantation rénale, la transplantation hépatique ainsi que des pathologies aiguës incluant l'insuffisance rénale aiguë, l'insuffisance pulmonaire aiguë et l'insuffisance hépatique aiguë, ainsi que des pathologies associées à une mort neuronale ou une neurodégénérescence incluant l'accident vasculaire cérébral et la maladie de Parkinson, ainsi que des pathologies d'origine inflammatoire telles que la douleur chronique et l'arthrite rhumatoïde.

## Patentansprüche

1. Verbindung, die der allgemeinen Formel (I) 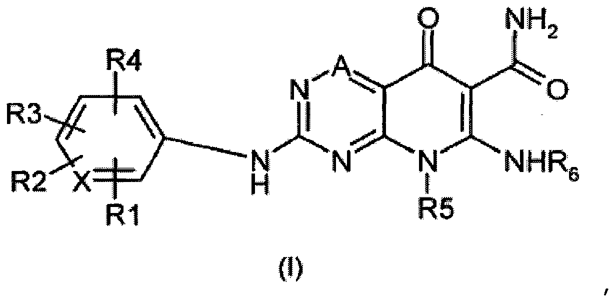 mit den folgenden Bedeutungen entspricht:
➢ **A** bedeutet CH oder C-(Alkyl);
➢ **X** bedeutet CH, C-(Alkyl) oder N;
➢ **R₁, R₂**, **R₃ und R₄,** die gleich oder verschieden sind, bedeuten jeweils unabhängig voneinander:
• ein Wasserstoffatom;
• ein geradkettiges, verzweigtes oder cyclisches Alkyl, das gegebenenfalls durch einen oder mehrere der folgenden Substituenten substituiert ist:
o Halogenatome;
o -OR₉;
○ -NR₉R^{'}₉;
o -CN;
o -C(O)OR₉;
○ -C(O)NR₉R_{9'};
o -S(O)ₚR₁₀;
○ -S(O)₂NR₉R^{'}₉;
worin **R₉, R^{'}₉, R₁₀ und p** wie unten definiert sind,
• eine -S(O)ₚR₁₀-Gruppe, in der p und R₁₀ wie unten definiert sind;
• eine -OR₁₀-Gruppe, in der R₁₀ wie unten definiert ist;
• ein Wasserstoffatom;
• eine -N(R₁₁)C(O)R₁₂-Gruppe, in der
(i) **R₁₁ und R₁₂** unabhängig voneinander ein Wasserstoffatom oder ein geradkettiges, verzweigtes oder cyclisches Alkyl, das gegebenenfalls durch einen oder mehrere Substituenten, ausgewählt aus Halogenatomen, den -OR₉-Gruppen und den -NR₉R^{'}₉-Gruppen, substituiert ist, sind, oder
(ii) **R₁₁ und R₁₂** gemeinsam mit den Atomen, an die sie gebunden sind, ein Heterocycloalkyl bilden, so dass ein Lactam entsteht;
• eine -N(R₁₄)-CH₂-C(O)NR₁₅R₉-Gruppe, in der **R₁₄ und R₁₅** gemeinsam mit den Atomen, an die sie gebunden sind, ein Heterocycloalkyl bilden, so dass ein Piperazinon entsteht, und in der **R₉** wie unten definiert ist;
• eine -C(O)NR₁₆R₁₇-Gruppe, wobei **R₁₆ und R₁₇** gemeinsam mit dem Stickstoffatom, an das sie gebunden sind ein Heterocycloalkyl bilden,
• eine **-T-U-**Gruppierung, in der
■ **T** Folgendes bedeutet:
o eine einfache Bindung,
o eine geradkettige oder verzweigte Alkylengruppe;
o eine -C(O)-Gruppe,
o eine -S(O)ₚ-Gruppe, in der p wie unten definiert ist, oder
o eine -O-(CH₂)ₙ-Gruppe, in der n wie unten definiert ist,
wobei **U** einen Heterocyclus, der ein oder mehrere Heteroatome, ausgewählt aus N, O und S(O)ₚ, umfasst, wobei p wie unten definiert ist, wobei der Heterocyclus gesättigt, ungesättigt oder aromatisch und gegebenenfalls einfach oder zweifach oder mehrfach durch einen, zwei oder mehrere Substituenten aus der folgenden Reihe substituiert ist:
❖ den -OR₇-Gruppen, wobei R₇ wie unten definiert ist,
❖ den Halogenatomen,
❖ den -C(O)R₇-Gruppen, wobei R₇ wie unten definiert ist,
❖ den geradkettigen, verzweigten oder cyclischen Alkylresten, die gegebenenfalls durch einen oder mehrere Substituenten aus der Reihe Halogenatome, -OR₁₀-Gruppen, -NR₉R^{'}₉-Gruppen und -CN-Gruppe substituiert sind, wobei R₉, R^{'}₉ und R₁₀ wie unten definiert sind; und
❖ den gesättigten, ungesättigten oder aromatischen Heterocyclen, die gegebenenfalls durch einen oder mehrere Substituenten aus der Reihe Halogenatome, -OR₉-Gruppen, -NR₉R^{'}₉-Gruppen und Alkylgruppen, wobei diese Alkylgruppen gegebenenfalls durch ein oder mehrere Halogenatome substituiert sind, substituiert sind; oder
■ **T** Folgendes bedeutet:
o eine -C(O)-Gruppe;
o eine -S(O)₂-Gruppe; oder
o eine -O-(C2-C3)-Alkylen-Gruppe; wobei **U** eine -NR₉R'₉-Gruppe, in der R₉ und R^{'}₉ wie unten definiert sind, bedeutet;
oder
■ **T** Folgendes bedeutet:
o eine -C(O)-Gruppe;
o eine -O-(C2-C3)-Alkylen-Gruppe;
wobei **U** eine -OR₉-Gruppe, in der R₉ und wie unten definiert ist, bedeutet; oder
■ **T** Folgendes bedeutet:
o eine geradkettige oder verzweigte Alkylengruppe; oder
o eine -O-(C2-C3)-Alkylen-Gruppe;
wobei **U** eine -NR₈R₉-Gruppe, in der R₈ und R₉ wie unten definiert sind, bedeutet;
oder es sind zwei benachbarte Gruppierungen, die aus **R₁, R₂**, **R₃ und R₄** ausgewählt sind, miteinander verbunden und bilden mit den beiden Kohlenstoffen, von denen sie getragen werden, einen gesättigten, ungesättigten oder aromatischen Heterocyclus, der gegebenenfalls durch eine oder mehrere geradkettige, verzweigte oder cyclische Alkylgruppen substituiert ist, wobei diese Alkylgruppen gegebenenfalls durch einen oder mehrere Substituenten aus der Reihe Halogenatome, -OR₁₀-Gruppen und -NR₉R^{'}₉-Gruppen substituiert sind, wobei R₉, R^{'}₉ und R₁₀ wie unten definiert sind, wobei der Heterocyclus mit dem aromatischen Cyclus anelliert ist,
➢ **R₅** bedeutet:
• ein geradkettiges oder verzweigtes Alkyl, das gegebenenfalls durch einen oder mehrere Substituenten aus der Reihe Halogenatome, den -OR₉-Gruppen, den -NR₉R^{'}₉-Gruppen, der -CN-Gruppe, den -C(O)NR₉R_{9'}-Gruppen, den -S(O)ₚR₁₀-Gruppen und den gegebenenfalls durch eine -NR₉R^{'}₉-Gruppe substituierten Cycloalkylgruppen substituiert ist, wobei R₉, R^{'}₉, R₁₀ und p wie unten definiert sind,
• eine Cycloalkylgruppe, die gegebenenfalls durch eine -NR₉R'₉-Gruppe, in der R₉ und R^{'}₉ wie unten definiert sind, substituiert ist,
• eine Alkoxy-OR₉-Gruppe, in der R₉ wie unten definiert ist,
• ein Aryl, das gegebenenfalls durch einen oder mehrere Substituenten aus der Reihe (C1-C3)-Alkylgruppen, Halogenatome und den -O-(C1-C3)-Alkylgruppen substituiert ist, oder
• eine -(CH₂)ₜ-R₁₃-Gruppe, in der **R₁₃ und t** wie unten definiert sind,
• **R₆** bedeutet ein Wasserstoffatom oder ein geradkettiges, verzweigtes oder cyclisches Alkyl, und wobei:
➢ **R₇** ein Wasserstoffatom oder ein geradkettiges, verzweigtes oder cyclisches Alkyl, das gegebenenfalls durch einen oder mehrere Substituenten aus der Reihe Halogenatome, -OR₉-Gruppen und den -NR₉R^{'}₉-Gruppen, wobei R₉ und R^{'}₉ wie oben definiert sind, substituiert ist;
➢ **R₈** bedeutet eine Heteroarylgruppe;
➢ **R₉ und R^{'}₉** bedeuten unabhängig voneinander ein Wasserstoffatom oder ein geradkettiges, verzweigtes oder cyclisches Alkyl;
➢ **R₁₀** bedeutet ein Wasserstoffatom oder ein geradkettiges, verzweigtes oder cyclisches Alkyl, das gegebenenfalls durch ein oder mehrere Halogenatome substituiert ist,
➢ **R₁₃** bedeutet ein Heteroaryl oder ein Heterocycloalkyl, das gegebenenfalls durch einen oder mehrere Substituenten aus der Reihe der geradkettigen, verzweigten oder cyclischen Alkyle substituiert ist, mit der Maßgabe, dass, wenn das Heterocycloalkyl mindestens ein Stickstoffatom umfasst, letzteres gegebenenfalls den Substituenten tragen kann,
➢ **t** bedeutet 1 oder 2,
➢ **n** bedeutet 0, 1, 2 oder 3, und
➢ **p** bedeutet 0, 1 oder 2,
in Form einer Säure, einer Base oder eines Säure- oder Basenadditionssalzes sowie in Form eines Hydrats oder Solvats.

2. Verbindung nach Anspruch 1, **dadurch gekennzeichnet, dass**:
**A** CH oder C(CH₃) bedeutet;
und/oder
**X** CH oder N bedeutet.

3. Verbindung nach Anspruch 1 oder 2, **dadurch gekennzeichnet, dass**:
➢ **A** CH bedeutet;
➢ **X** CH, C-(Alkyl) oder N bedeutet,
➢ **R₁, R₂**, **R₃ und R₄,** die gleich oder verschieden sind, jeweils unabhängig voneinander Folgendes bedeuten:
o ein Wasserstoffatom,
o ein geradkettiges, verzweigtes oder cyclisches Alkyl, das gegebenenfalls durch ein oder mehrere Halogenatome, eine -OR₉-Gruppe substituiert ist, in der R₉ und R^{'}₉ unabhängig voneinander ein Wasserstoffatom oder ein geradkettiges, verzweigtes oder cyclisches Alkyl bedeuten,
o eine -S(O)ₚR₁₀-Gruppe oder eine -OR₁₀-Gruppe, in der R₁₀ ein Wasserstoffatom oder ein geradkettiges, verzweigtes oder cyclisches Alkyl, das gegebenenfalls durch ein oder mehrere Halogenatome substituiert ist, bedeutet und p 0, 1 oder 2 bedeutet,
o ein Halogenatom,
o eine -N(R₁₁)C(O)R₁₂-Gruppe, in der R₁₁ und R₁₂ unabhängig voneinander ein Wasserstoffatom oder ein geradkettiges, verzweigtes oder cyclisches Alkyl bedeutet, oder R₁₁ und R₁₂ gemeinsam mit den Atomen, an die sie gebunden sind, ein Heterocycloalkyl bilden, so dass ein Lactam entsteht;
o eine-T-U-Gruppierung, in der
■ T Folgendes bedeutet:
• eine einfache Bindung,
• eine geradkettige oder verzweigte Alkylengruppe,
• eine -C(O)-Gruppe,
• eine -S(O)p-Gruppe, in der p 0, 1 oder 2 bedeutet,
• eine -O-(CH₂)ₙ-Gruppe, in der n 0, 1, 2 oder 3 bedeutet,
und **U** bedeutet einen Heterocyclus, der ein oder mehrere Heteroatome aus der Reihe N, O und S(O)ₚ umfasst, wobei p 0, 1 oder 2 bedeutet, wobei der Heterocyclus der folgenden Formel entspricht: 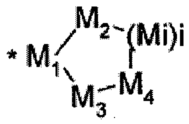 in der
• * die Position der Bindung von U an T bedeutet;
• **M1** ein C- oder N-Atom bedeutet;
• **M2** und **M3** gleich oder verschieden sind und ein C-, N-, O-Atom oder S(O)ₚ, worin p=0, 1 oder 2, bedeuten;
• **M4** ein C-Atom, C(=O), N, O oder S(O)ₚ, worin p=0, 1 oder 2, bedeutet;
wobei jedes **Mi,** die gleich oder verschieden sind, ein C-Atom, C(=O), N, O oder S(O)ₚ, worin p=0, 1 oder 2, bedeutet;
• **i**=0, 1, 2 oder 3;
mit der Maßgabe, dass jedes von M1, M2, M3, M4 oder Mi gegebenenfalls substituiert sein kann, wenn eine Valenz verfügbar ist und/oder benachbarte M1, M2, M3, M4 oder Mi gegebenenfalls über eine Doppelbindung verbunden sein können;
wobei der Heterocyclus **U** gesättigt, ungesättigt oder aromatisch ist und gegebenenfalls einfach oder zweifach oder mehrfach durch einen, zwei oder
mehrere Substituent(en) aus der folgenden Reihe substituiert ist:
o den -OR₇-Gruppen, in denen R₇ ein Wasserstoffatom oder ein geradkettiges, verzweigtes oder cyclisches Alkyl bedeutet,
o den Halogenatomen,
o den -COR₇-Gruppen, in denen R₇ ein Wasserstoffatom oder geradkettiges, verzweigtes oder cyclisches Alkyl bedeutet,
o den geradkettigen, verzweigten oder cyclischen Alkylgruppen, die gegebenenfalls durch ein oder mehrere Halogenatom(e) substituiert sind,
o den gesättigten, ungesättigten oder aromatischen Heterocyclen, insbesondere einem Heterocyclus, der einen N umfasst, die gegebenenfalls durch eine oder mehrere Gruppierungen aus der Reihe der Halogenatome und der Alkylgruppen, die gegebenenfalls durch ein oder mehrere Halogenatome substituiert sind, substituiert sind;
oder zwei benachbarte Gruppierungen aus der Reihe R₁, R₂, R₃ und R₄ sind miteinander verbunden und bilden mit den zwei Kohlenstoffen, von denen sie getragen werden, einen gesättigten, ungesättigten oder aromatischen Heterocyclus, der gegebenenfalls durch eine oder mehrere geradkettige, verzweigte oder cyclische Alkylgruppe(n) substituiert ist, wobei diese Alkylgruppe(n) gegebenenfalls durch mindestens eine Gruppe aus der Reihe der -NR₉R^{'}₉-Gruppen substituiert ist/sind, wobei R₉ und R^{'}₉ unabhängig voneinander ein Wasserstoffatom oder eine Alkylgruppe bedeuten,
wobei der Heterocyclus mit dem aromatischen Cyclus anelliert ist.

4. Verbindung nach einem der Ansprüche 1 bis 3, **dadurch gekennzeichnet, dass** sie der folgenden Formel (I'): 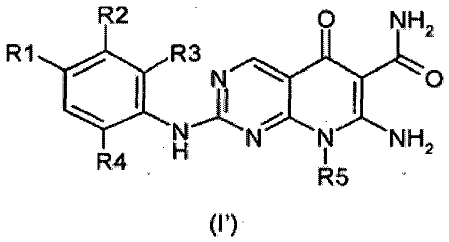 mit den folgenden Bedeutungen entspricht:
➢ **R₁** bedeutet:
• ein Wasserstoffatom;
• ein geradkettiges, verzweigtes oder cyclisches Alkyl, das gegebenenfalls durch einen oder mehrere Substituenten aus der Reihe der Halogenatome, der -NR₉R^{'}₉-Gruppen substituiert ist, wobei R₉ und R^{'}₉ unabhängig voneinander ein Wasserstoffatom oder eine Alkylgruppe bedeuten,
• -OR₁₀, worin R₁₀ ein Wasserstoffatom oder ein geradkettiges, verzweigtes oder cyclisches Alkyl, das gegebenenfalls durch ein oder mehrere Halogenatome substituiert ist, bedeutet,
• ein Halogenatom,
• eine -T-U-Gruppierung, in der
T Folgendes bedeutet:
o eine einfache Bindung,
o eine Alkylengruppe,
o eine -C(O)--Gruppe,
o eine -O-(CH₂)ₙ-Gruppe, in der n 0, 1, 2 oder 3 bedeutet,
o und **U** bedeutet einen gesättigten, ungesättigten oder aromatischen Heterocyclus, der gegebenenfalls einfach oder zweifach durch einen Substituenten substituiert ist,
➢ **R₂**, **R₃ und R₄,** die gleich oder verschieden sind, unabhängig Folgendes bedeuten:
○ ein Wasserstoffatom,
○ ein geradkettiges, verzweigtes oder cyclisches Alkyl, das gegebenenfalls durch einen oder mehrere Substituenten aus der Reihe der Halogenatome und der -NR₉R^{'}₉-Gruppen, in denen R₉ und R^{'}₉ unabhängig voneinander ein Wasserstoffatom oder eine Alkylgruppe bedeuten, substituiert ist,
○ eine -OR₁₀-Gruppe, in der R₁₀ ein Wasserstoffatom oder ein Alkyl, das gegebenenfalls durch ein oder mehrere Halogenatome substituiert ist, bedeutet,
○ ein Halogen,
○ eine -T-U-Gruppe, wobei T eine Bindung und U ein Morpholinyl bedeutet, oder
➢ **R₂ und R₂** sind miteinander verbunden und bilden mit den beiden Kohlenstoffen, von denen sie getragen werden, einen Heterocyclus aus der Reihe Piperidin, Thiazol, Tetrahydrofuran und Dioxan, wobei der Heterocyclus gegebenenfalls durch ein geradkettiges, verzweigtes oder cyclisches Alkyl, das gegebenenfalls durch -NR₉R^{'}₉ substituiert ist, wobei R₉ und R^{'}₉ unabhängig voneinander ein Wasserstoffatom oder ein Methyl bedeuten, substituiert ist,
wobei der Heterocyclus mit dem aromatischen Cyclus anelliert ist,
➢ **R₅** bedeutet (i) ein geradkettiges, verzweigtes oder cyclisches Alkyl, das 1 bis 5 Kohlenstoffatome umfasst und das gegebenenfalls durch einen oder mehrere Substituent(en) aus der Reihe der Halogenatome oder einer -OH-Gruppe substituiert ist, (ii) ein Aryl, das gegebenenfalls durch einen oder mehrere Substituenten aus der Reihe der (C1-C3)-Alkyl-Gruppen, der Halogenatome und der -O-(C1-C3)-Alkyl-Gruppen substituiert ist;
in Form einer Säure, einer Base oder eines Säure- oder Basenadditionssalzes sowie in Form eines Hydrats oder Solvats.

5. Verbindung nach einem der Ansprüche 1 bis 4, **dadurch gekennzeichnet, dass**
➢ **A** CH bedeutet; und/oder
➢ **X** CH bedeutet; und/oder
➢ **R₁** von einem Wasserstoffatom verschieden ist; und/oder
➢ **R₅** ein geradkettiges, verzweigtes oder cyclisches Alkyl bedeutet; und/oder
➢ **U** einen gesättigten, ungesättigten oder aromatischen Heterocyclus bedeutet, der gegebenenfalls einfach oder zweifach substituiert ist und mindestens ein Stickstoffatom und/oder ein Sauerstoffatom umfasst, und/oder
➢ **U** einen gesättigten Heterocyclus bedeutet, der mindestens ein Stickstoffatom umfasst; und/oder
➢ **U** einen Heterocyclus aus der folgenden Reihe bedeutet:
○ ein Azetidinyl;
○ ein Pyrrolidinyl;
○ ein Oxopyrrolidinyl;
○ ein Piperidinyl;
○ ein 1,2,3,6-Tetrahydropyridinyl;
○ ein Pyridinyl;
○ ein Piperazinyl,
○ ein Diazepanyl;
○ ein Morpholinyl;
○ ein 1,1-Dioxo-llambda6-thiomorpholin-4-yl.

6. Verbindung nach einem der vorhergehenden Ansprüche, **dadurch gekennzeichnet, dass R₁** aus den Folgenden ausgewählt ist:

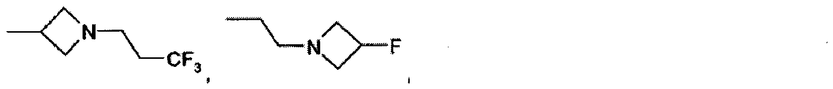

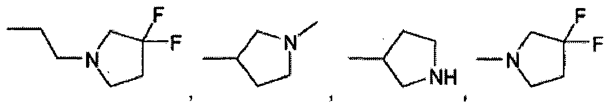

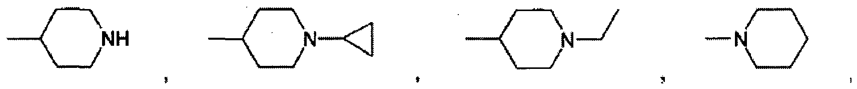

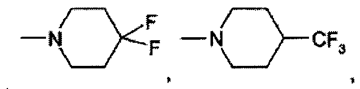

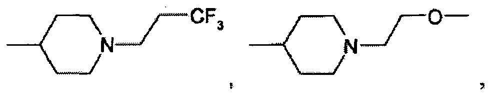

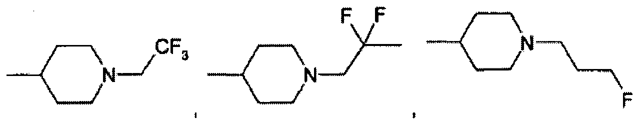

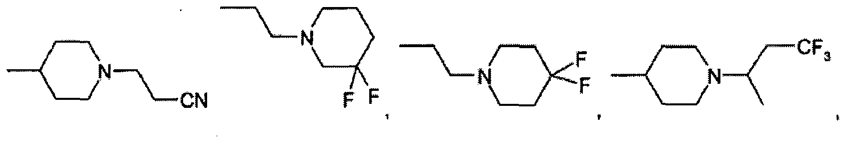

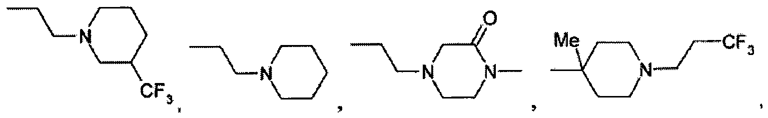

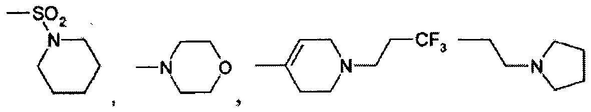

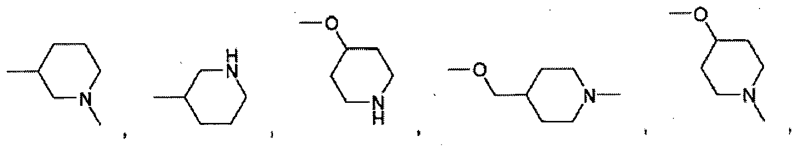

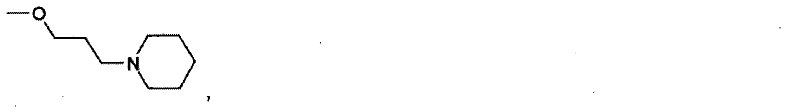

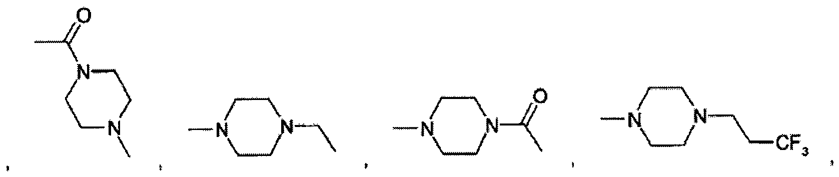

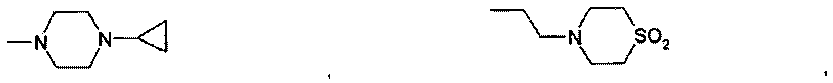

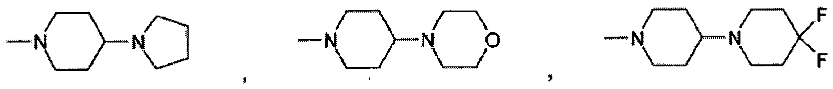

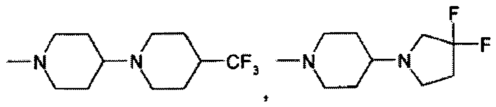

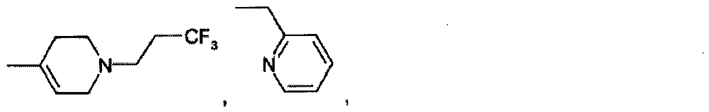

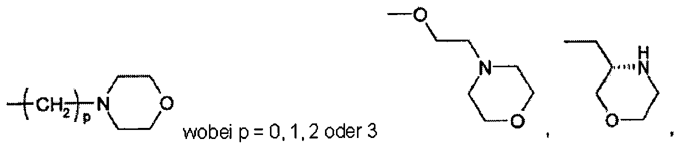

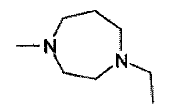

7. Verbindung nach einem der vorhergehenden Ansprüche, **dadurch gekennzeichnet, dass A** und **X** CH bedeuten.

8. Verbindung nach einem der vorhergehenden Ansprüche, **dadurch gekennzeichnet, dass U** einen gesättigten Heterocyclus, der mindestens ein Stickstoffatom umfasst, bedeutet.

9. Verbindung nach einem der vorhergehenden Ansprüche, **dadurch gekennzeichnet, dass** sie aus den Folgenden ausgewählt ist:
7-Amino-8-ethyl-2-(4-hydroxyphenylamino)-5-oxo-5,8-dihydropyrido[2,3-d]pyrimidin-6-carboxamid;
7-Amino-2-(benzothiazol-6-ylamino)-8-ethyl-5-oxo-5,8-dihydropyrido[2,3-d]pyrimidin-6-carboxamid;
7-Amino-2-[4-(cyclopropancarbonylmethylamino)phenyl-amino]-8-ethyl-5-oxo-5,8-dihydropyrido[2,3-d]pyrimidin-6-carboxamid;
7-Amino-8-ethyl-2-[4-(4-methylpiperazin-1-carbonyl)phenylamino]-5-oxo-5,8-dihydropyrido[2,3-d]pyrimidin-6-carboxamid;
7-Amino-2-(4-cyclopentyloxyphenylamino)-8-ethyl-5-oxo-5,8-dihydropyrido[2,3-d]pyrimidin-6-carboxamid;
7-Amino-8-ethyl-2-[4-(4-ethylpiperazin-1-yl)phenyl-amino]-5-oxo-5,8-dihydropyrido[2,3-d]pyrimidin-6-carboxamid;
7-Amino-8-ethyl-5-oxo-2-[4-(4-pyrrolidin-1-ylpiperidin-1-yl)phenylamino]-5,8-dihydropyrido[2,3-d]pyrimidin-6-carboxamid;
7-Amino-8-cyclopentyl-2-(4-morpholin-4-ylphenylamino)-5-oxo-5,8-dihydropyrido[2,3-d]pyrimidin-6-carboxamid;
7-Amino-8-ethyl-5-oxo-2-[4-(piperidine-1-sulfonyl)phenylamino]-5,8-dihydropyrido[2,3-d]pyrimidin-6-carboxamid;
2-[4-(4-Acetylpiperazin-1-yl)phenylamino]-7-amino-8-ethyl-5-oxo-5,8-dihydropyrido[2,3-d]pyrimidin-6-carboxamid;
7-Amino-8-ethyl-2-(4-morpholin-4-ylbenzylamino)-5-oxo-5,8-dihydropyrido[2,3-d]pyrimidin-6-carboxamid;
7-Amino-8-ethyl-5-oxo-2-(chinolin-3-ylamino)-5,8-dihydropyrido[2,3-d]pyrimidin-6-carboxylamid;
7-Amino-8-ethyl-5-oxo-2-[4-(3-piperidin-1-yl-propoxy)phenylamino]-5,8-dihydropyrido[2,3-d]pyrimidin-6-carboxamid;
7-Amino-8-ethyl-5-oxo-2-(3,4,5,6-tetrahydro-2H-[1,2']bipyridinyl-5'-ylamino)-5,8-dihydropyrido[2,3-d]pyrimidin-6-carboxamid;
7-Amino-8-ethyl-5-oxo-2-[4-(2-oxopyrrolidin-1-yl)phenylamino]-5,8-dihydropyrido[2,3-d]pyrimidin-6-carboxamid;
7-Amino-8-ethyl-5-oxo-2-(chinolin-6-ylamino)-5,8-dihydropyrido[2,3-d]pyrimidin-6-carboxamid;
7-Amino-8-ethyl-2-(3-morpholin-4-ylphenylamino)-5-oxo-5,8-dihydropyrido[2,3-d]pyrimidin-6-carboxamid;
7-Amino-2-(2,3-dihydrobenzo[1,4]dioxin-6-ylamino)-8-ethyl-5-oxo-5,8-dihydropyrido[2,3-d]pyrimidin-6-carboxamid;
7-Amino-8-ethyl-2-(3-fluor-4-hydroxyphenylamino)-5-oxo-5,8-dihydropyrido[2,3-d]pyrimidin-6-carboxamid;
7-Amino-8-ethyl-2-(3-methylsulfanylphenylamino)-5-oxo-5,8-dihydropyrido[2,3-d]pyrimidin-6-carboxamid;
7-Amino-8-ethyl-2-(4-morpholin-4-ylphenylamino)-5-oxo-5,8-dihydropyrido[2,3-d]pyrimidin-6-carboxamid;
7-Amino-8-ethyl-2-(2-fluorphenylamino)-5-oxo-5,8-dihydropyrido[2,3-d]pyrimidin-6-carboxamid;
7-Amino-2-[4-(4,4-difluor[1,4']bipiperidinyl-1'-yl)-2-methoxyphenylamino]-8-ethyl-5-oxo-5,8-dihydro-pyrido[2,3-d]pyrimidin-6-carboxamid
7-Amino-8-ethyl-2-[2-methoxy-4-(4-trifluormethyl[1,4']bipiperidinyl-1'-yl)phenylamino]-5-oxo-5,8-dihydropyrido[2,3-d]pyrimidin-6-carboxamid
7-Amino-2-{4-[4-(3,3-difluorpyrrolidin-1-yl)piperidin-1-yl]-2-methoxyphenylamino}-8-ethyl-5-oxo-5,8-dihydropyrido[2,3-d]pyrimidin-6-carboxamid
7-Amino-8-ethyl-2-[4-(4-ethylpiperazin-1-yl)-2-fluor-6-methoxyphenylamino]-5-oxo-5,8-dihydropyrido[2,3-d]pyrimidin-6-carboxamid;
7-Amino-2-[4-(1-cyclopropylpiperidin-4-yl)-2-methoxyphenylamino]-8-ethyl-5-oxo-5,8-dihydro-pyrido[2,3-d]pyrimidin-6-carboxamid;
7-Amino-2-(2-dimethylaminomethylchroman-6-ylamino)-8-ethyl-5-oxo-5,8-dihydropyrido[2,3-d]pyrimidin-6-carboxamid;
7-Amino-2-[5-chlor-4-(4-cyclopropylpiperazin-1-yl)-2-methoxyphenylamino]-8-ethyl-5-oxo-5,8-dihydro-pyrido[2,3-d]pyrimidin-6-carboxamid;
7-Amino-8-ethyl-2-[2-methoxy-4-(4-morpholin-4-ylpiperidin-1-yl)phenylamino]-5-oxo-5,8-dihydropyrido[2,3-d]pyrimidin-6-carboxyamid;
7-Amino-2-[4-(4-cyclopropylpiperazin-1-yl)-2-difluormethoxyphenylamino]-8-ethyl-5-oxo-5,8-dihydro-pyrido[2,3-d]pyrimidin-6-carboxamid;
7-Amino-2-[4-(4-cyclopropylpiperazin-1-yl)-2-methoxyphenylamino]-8-ethyl-5-oxo-5,8-dihydro-pyrido[2,3-d]pyrimidin-6-carboxamid;
7-Amino-8-ethyl-2-{2-methoxy-4-[1-(3,3,3-trifluorpropyl)piperidin-4-yl]phenylamino}-5-oxo-5,8-dihydro-pyrido[2,3-d]pyrimidin-6-carboxamid;
7-Amino-2-[2-chlor-4-(4-ethylpiperazin-1-yl)phenyl-amino]-8-ethyl-5-oxo-5,8-dihydropyrido[2,3-d]pyrimidin-6-carboxamid;
7-Amino-8-ethyl-2-[4-(4-ethylpiperazin-1-yl)-3-trifluormethylphenylamino]-5-oxo-5,8-dihydropyrido[2,3-d]pyrimidin-6-carboxamid;
7-Amino-8-ethyl-2-(4-morpholin-4-ylmethylphenylamino)-5-oxo-5,8-dihydropyrido[2,3-d]pyrimidin-6-carboxamid;
7-Amino-8-ethyl-5-oxo-2-{4-[4-(3,3,3-trifluorpropyl)piperazin-1-yl]phenylamino}-5,8-dihydropyrido[2,3-d]pyrimidin-6-carboxamid;
7-Amino-8-ethyl-2-[4-(4-ethylpiperazin-1-yl)-3-fluorphenylamino]-5-oxo-5,8-dihydropyrido[2,3-d]pyrimidin-6-carboxamid;
6-(4-Morpholin-4-ylphenylamino)-9-oxo-1,3,4,9-tetrahydro-2H-1,4a,5,7-tetraazaphenanthren-10-carboxamid;
7-Amino-8-ethyl-2-[4-(4-ethylpiperazin-1-yl)-2-methoxyphenylamino]-5-oxo-5,8-dihydropyrido[2,3-d]pyrimidin-6-carboxamid;
7-Amino-8-ethyl-2-[4-(1-methylpiperidin-4-ylmethoxy)phenylamino]-5-oxo-5,8-dihydropyrido[2,3-d]pyrimidin-6-carboxamid;
7-Amino-2-[4-(4-cyclopropylpiperazin-1-yl)phenylamino]-8-ethyl-5-oxo-5,8-dihydropyrido[2,3-d]pyrimidin-6-carboxamid;
7-Amino-8-ethyl-2-[4-(4-ethylpiperazin-1-yl)-2-methyl-phenylamino]-5-oxo-5,8-dihydropyrido[2,3-d]pyrimidin-6-carboxamid;
7-Amino-8-ethyl-2-[4-(4-ethyl[1,4]diazepan-1-yl)phenyl-amino]-5-oxo-5,8-dihydropyrido[2,3-d]pyrimidin-6-carboxamid;
8-(4-Morpholin-4-ylphenylamino)-5-oxo-1,2,3,5-tetrahydro-3,7,9,9b-tetraazacyclopenta[a]naphthalin-4-carboxamid;
7-Amino-8-ethyl-5-oxo-2-{4-[2-(3-trifluormethylpiperidin-1-yl)ethyl]phenylamino}-5,8-dihydropyrido[2,3-d]pyrimidin-6-carboxamid;
7-Amino-8-ethyl-2-[4-(3-morpholin-4-ylpropyl)phenylamino]-5-oxo-5,8-dihydropyrido[2,3-d]pyrimidin-6-carboxamid;
7-Amino-8-ethyl-5-oxo-2-{4-[1-(2,2,2-trifluorethyl)piperidin-4-yl]phenylamino}-5,8-dihydropyrido[2,3-d]pyrimidin-6-carboxamid;
7-Amino-8-ethyl-2-(4-(S)-1-morpholin-3-ylmethylphenylamino)-5-oxo-5,8-dihydropyrido[2,3-d]pyrimidin-6-carboxamid;
7-Amino-8-isobutyl-2-[4-(2-morpholin-4-ylethyl)phenylamino]-5-oxo-5,8-dihydropyrido[2,3-d]pyrimidin-6-carboxamid;
7-Amino-8-ethyl-2-{4-[2-(3-fluorazetidin-1-yl)ethyl]phenylamino}-5-oxo-5,8-dihydropyrido[2,3-d]pyrimidin-6-carboxamid;
7-Amino-2-[4-(2-morpholin-4-ylethyl)phenylamino]-5-oxo-8-propyl-5,8-dihydropyrido[2,3-d]pyrimidin-6-carboxamid;
7-Amino-8-ethyl-2-[4-(2-hydroxyethyl)phenylamino]-5-oxo-5,8-dihydropyrido[2,3-d]pyrimidin-6-carboxamid;
7-amino-8-ethyl-2-(4-hydroethylphenylamino)-5-oxo-5,8-dihydropyrido[2,3-d]pyrimidin-6-carboxamid;
7-Amino-8-ethyl-2-(2-methyl-1,2,3,4-tetrahydroisochinolin-6-ylamino)-5-oxo-5,8-dihydropyrido[2,3-d]pyrimidin-6-carboxamid;
7-Amino-2-f4-[2-(1,1-dioxo-1lambda6-thiomorpholin-4-yl)ethyl]phenylamino}-8-ethyl-5-oxo-5,8-dihydro-pyrido[2,3-d]pyrimidin-6-carboxamid;
7-Amino-8-ethyl-2-{4-[2-(4-methyl-3-oxopiperazin-1-yl)ethyl]phenylamino}-5-oxo-5,8-dihydropyrido[2,3-d]pyrimidin-6-carboxamid;
7-Amino-8-ethyl-5-oxo-2-{4-[1-(3,3,3-trifluorpropyl)piperidin-4-yl]phenylamino}-5,8-dihydropyrido[2,3-d]pyrimidin-6-carboxamid;
7-Amino-2-(4-dimethylaminomethylphenylamino)-5-oxo-8-(2,2,2-trifluorethyl)-5,8-dihydropyrido[2,3-d]pyrimidin-6-carboxamid;
7-Amino-2-[3-chlor-4-(4-pyrrolidin-1-ylpiperidin-1-yl)phenylamino]-8-ethyl-5-oxo-5,8-dihydropyrido[2,3-d]pyrimidin-6-carboxamid;
7-Amino-8-ethyl-5-oxo-2-(4-pyridin-2-ylmethylphenyl-amino)-5,8-dihydropyrido[2,3-d]pyrimidin-6-carboxamid;
7-Amino-2-{4-[2-(3,3-difluorpyrrolidin-l-yl)ethyl]phenylamino}-8-ethyl-5-oxo-5,8-dihydropyrido[2,3-d]pyrimidin-6-carboxamid;
7-Amino-8-ethyl-5-oxo-2-(4-pyrrolidin-3-ylphenylamino)-5,8-dihydropyrido[2,3-d]pyrimidin-6-carboxamid;
7-Amino-8-ethyl-5-oxo-2-[4-(2-piperidin-1-ylethyl)phenylamino]-5,8-dihydropyrido[2,3-d]pyrimidin-6-carboxamid;
7-Amino-2-[4-(2-morpholin-4-ylethyl)phenylamino]-5-oxo-8-(2,2,2-trifluorethyl)-5,8-dihydropyrido[2,3-d]pyrimidin-6-carboxamid
7-Amino-2-{4-[2-(4,4-difluorpiperidin-1-yl)ethyl]phenylamino}-8-ethyl-5-oxo-5,8-dihydropyrido[2,3-d]pyrimidin-6-carboxamid;
7-Amino-8-ethyl-2-[4-(1-methylpyrrolidin-3-yl)phenyl-amino]-5-oxo-5,8-dihydropyrido[2,3-d]pyrimidin-6-carboxamid;
7-Amino-2-{4-[2-(3,3-difluorpiperidin-1-yl)ethyl]phenylamino}-8-ethyl-5-oxo-5,8-dihydropyrido[2,3-d]pyrimidin-6-carboxamid;
7-Amino-8-(3-methoxypropyl)-2-[4-(2-morpholin-4-ylethyl)phenylamino]-5-oxo-5,8-dihydropyrido[2,3-d]pyrimidin-6-carboxamid;
7-Amino-8-ethyl-2-[4-(1-methylpiperidin-3-yl)phenyl-amino]-5-oxo-5,8-dihydropyrido[2,3-d]pyrimidin-6-carboxamid;
7-Amino-2-(4-dimethylaminomethylphenylamino)-8-isopropyl-5-oxo-5,8-dihydropyrido[2,3-d]pyrimidin-6-carboxamid;
7-Amino-8-ethyl-5-oxo-2-(4-piperidin-3-ylphenylamino)-5,8-dihydropyrido[2,3-d]pyrimidin-6-carboxamid;
7-Amino-8-ethyl-5-oxo-2-(1,2,3,4-tetrahydroisochinolin-7-ylamino)-5,8-dihydropyrido[2,3-d]pyrimidin-6-carboxamid;
7-Amino-8-ethyl-5-oxo-2-(1,2,3,4-tetrahydroisochinolin-7-ylamino)-5,8-dihydropyrido[2,3-d]pyrimidin-6-carboxamid;
7-Amino-8-ethyl-2-[4-(1-methylpiperidin-4-yloxy)phenylamino]-5-oxo-5,8-dihydropyrido[2,3-d]pyrimidin-6-carboxamid;
7-Amino-8-ethyl-2-[4-(2-morpholin-4-ylethoxy)phenyl-amino]-5-oxo-5,8-dihydropyrido[2,3-d]pyrimidin-6-carboxamid;
7-Amino-2-(3-dimethylaminomethylphenylamino)-8-ethyl-5-oxo-5,8-dihydropyrido[2,3-d]pyrimidin-6-carboxamid;
7-Amino-8-ethyl-5-oxo-2-[4-(4-pyrrolidin-1-ylpiperidin-1-yl)phenylamino]-5,8-dihydropyrido[2,3-d]pyrimidin-6-carboxamid;
7-Amino-8-ethyl-5-oxo-2-[4-(piperidin-4-yloxy)phenylamino]-5,8-dihydropyrido[2,3-d]pyrimidin-6-carboxamid;
7-Amino-8-(3-aminopropyl)-2-(4-morpholin-4-ylphenylamino)-5-oxo-5,8-dihydropyrido[2,3-d]pyrimidin-6-carboxamid;
7-Amino-8-ethyl-2-[4-(2-morpholin-4-ylethyl)phenylamino]-5-oxo-5,8-dihydropyrido[2,3-d]pyrimidin-6-carboxamid;
7-Amino-2-(4-dimethylaminomethylphenylamino)-8-ethyl-5-oxo-5,8-dihydropyrido[2,3-d]pyrimidin-6-carboxamid;
7-Amino-8-ethyl-2-[4-(1-ethylpiperidin-4-yl)phenylamino]-5-oxo-5,8-dihydropyrido[2,3-d]pyrimidin-6-carboxamid;
7-Amino-8-ethyl-5-oxo-2-(4-piperidin-4-ylphenylamino)-5,8-dihydropyrido[2,3-d]pyrimidin-6-carboxamid;
7-Amino-2-[3-chlor-4-(4-ethylpiperazin-1-yl)phenylamino]-8-ethyl-5-oxo-5,8-dihydropyrido[2,3-d]pyrimidin-6-carboxamid
7-Amino-8-ethyl-2-[3-methoxy-4-(3-piperidin-1-ylpropoxy)phenylamino]-5-oxo-5,8-dihydropyrido[2,3-d]pyrimidin-6-carboxamid
7-Amino-8-ethyl-5-oxo-2-{4-[2-(4-trifluormethylpiperidin-1-yl)ethyl]phenylamino}-5,8-dihydropyrido[2,3-d]pyrimidin-6-carboxamid
7-Amino-2-{4-[2-(cis-2,6-dimethylmorpholin-4-yl)ethyl]phenylamino}-8-ethyl-5-oxo-5,8-dihydropyrido[2,3-d]pyrimidin-6-carboxamid
7-Amino-2-(4-diethylaminomethylphenylamino)-8-ethyl-5-oxo-5,8-dihydropyrido[2,3-d]pyrimidin-6-carboxamid
7-Amino-2-(4-dimethylaminomethylphenylamino)-8-(3-methoxypropyl)-5-oxo-5,8-dihydropyrido[2,3-d]pyrimidin-6-carboxamid
7-Amino-8-ethyl-2-[4-(4-ethylpiperazin-1-yl)-2-fluor-phenylamino]-5-oxo-5,8-dihydropyrido[2,3-d]pyrimidin-6-carboxamid
7-Amino-2-{4-[1-(2-cyanoethyl)piperidin-4-yl]phenyl-amino}-8-ethyl-5-oxo-5,8-dihydropyrido[2,3-d]pyrimidin-6-carboxamid
7-Amino-8-ethyl-2-{4-[1-(3-fluorpropyl)piperidin-4-yl]phenylamino}-5-oxo-5,8-dihydropyrido[2,3-d]pyrimidin-6-carbonsäureamid
7-Amino-8-ethyl-2-[4-(4-ethylpiperazin-1-yl)-3-methylphenylamino]-5-oxo-5,8-dihydropyrido[2,3-d]pyrimidin-6-carboxamid
7-Amino-2-[4-(4-cyclopropylpiperazin-1-yl)-2-ethylphe-nylamino]-8-ethyl-5-oxo-5,8-dihydropyrido[2,3-d]pyrimidin-6-carboxamid
(±)-7-Amino-2-trans-[4-(2-dimethylaminocyclopropyl)phenylamino]-8-ethyl-5-oxo-5,8-dihydropyrido[2,3-d]pyrimidin-6-carboxamid
7-Amino-2-[4-(4-cyclopropylpiperazin-1-yl)-5-fluor-2-methoxyphenylamino]-8-ethyl-5-oxo-5,8-dihydro-pyrido[2,3-d]pyrimidin-6-carboxamid
7-Amino-2-[4-(4-cyclopropylpiperazin-1-yl)-3-fluor-2-methoxyphenylamino]-8-ethyl-5-oxo-5,8-dihydro-pyrido[2,3-d]pyrimidin-6-carboxamid
7-Amino-2-[4-(4-cyclopropylpiperazin-1-yl)-2-ethoxy-phenylamino]-8-ethyl-5-oxo-5,8-dihydropyrido[2,3-d]pyrimidin-6-carboxamid
7-Amino-8-ethyl-5-oxo-2-(4-propylphenylamino)-5,8-dihydropyrido[2,3-d]pyrimidin-6-carboxamid
7-Amino-8-ethyl-5-oxo-2-(4-propoxyphenylamino)-5,8-dihydropyrido[2,3-d]pyrimidin-6-carboxamid
7-Amino-8-ethyl-2-(6-methoxypyrid-3-ylamino)-5-oxo-5,8-dihydropyrido[2,3-d]pyrimidin-6-carboxamid
7-Amino-8-ethyl-2-(4-fluorphenylamino)-5-oxo-5,8-dihydropyrido[2,3-d]pyrimidin-6-carboxamid
7-Amino-8-ethyl-2-(4-methoxyphenylamino)-5-oxo-5,8-dihydropyrido[2,3-d]pyrimidin-6-carboxamid
7-Amino-2-(benzo[1,3]dioxol-5-ylamino)-8-ethyl-5-oxo-5,8-dihydropyrido[2,3-d]pyrimidin-6-carboxamid
7-Amino-8-ethyl-5-oxo-2-(4-piperidin-1-ylphenylamino)-5,8-dihydropyrido[2,3-d]pyrimidin-6-carboxamid
8-Ethyl-2-{2-methoxy-4-[1-(3,3,3-trifluorpropyl)piperidin-4-yl]phenylamino}-7-methylamino-5-oxo-5,8-dihydropyrido[2,3-d]pyrimidin-6-carboxamid
7-Amino-8-isobutyl-2-{2-methoxy-4-[1-(3,3,3-trifluorpropyl)piperidin-4-yl]phenylamino}-5-oxo-5,8-dihydropyrido[2,3-d]pyrimidin-6-carboxamid
7-Amino-8-cyclopropylmethyl-2-{2-methoxy-4-[1-(3,3,3-trifluorpropyl)piperidin-4-yl]phenylamino}-5-oxo-5,8-dihydropyrido[2,3-d]pyrimidin-6-carboxamid
7-Amino-8-(2-methoxyethyl)-2-{2-methoxy-4-[1-(3,3,3-trifluorpropyl)piperidin-4-yl]phenylamino}-5-oxo-5,8-dihydropyrido[2,3-d]pyrimidin-6-carboxamid
7-Amino-2-{2-methoxy-4-[1-(3,3,3-trifluorpropyl)piperidin-4-yl]phenylamino}-5-oxo-8-(tetrahydrofuran-2-ylmethyl)-5,8-dihydropyrido[2,3-d]pyrimidin-6-carboxamid
7-Amino-8-ethyl-5-oxo-2-{4-[1-(3,3,3-trifluorpropyl)azetidin-3-yl]phenylamino}-5,8-dihydropyrido[2,3-d]pyrimidin-6-carboxamid
7-Amino-2-{5-fluor-2-methoxy-4-[1-(3,3,3-trifluorpropyl)piperidin-4-yl]phenylamino}-8-isobutyl-5-oxo-5,8-dihydropyrido[2,3-d]pyrimidin-6-carboxamid
7-Amino-8-ethyl-5-oxo-2-[4-(1,2,3,6-tetrahydropyridin-4-yl)phenylamino]-5,8-dihydropyrido[2,3-d]pyrimidin-6-carboxamid
7-Amino-2-(2-methoxy-4-piperidin-4-ylphenylamino)-5-oxo-8-phenyl-5,8-dihydropyrido[2,3-d]pyrimidin-6-carboxamid
7-Amino-8-(2-hydroxyethyl)-2-(2-methoxy-4-piperidin-4-ylphenylamino)-5-oxo-5,8-dihydropyrido[2,3-d]pyrimidin-6-carboxamid
7-Amino-2-{2-methoxy-4-[1-(3,3,3-trifluorpro-pyl)piperidin-4-yl]phenylamino}-5-oxo-8-thiazol-2-ylmethyl-5,8-dihydropyrido[2,3-d]pyrimidin-6-carboxamid
7-Amino-2-{2-methoxy-4-[1-(3,3,3-trifluorpropyl)piperidin-4-yl]phenylamino}-5-oxo-8-thiazol-5-ylmethyl-5,8-dihydropyrido[2,3-d]pyrimidin-6-carboxamid
7-Amino-8-(2-hydroxy-2-methylpropyl)-2-(2-methoxy-4-piperidin-4-ylphenylamino)-5-oxo-5,8-dihydropyrido[2,3-d]pyrimidin-6-carboxamid
8-(2-Acetylaminoethyl)-7-amino-2-{2-methoxy-4-[1-(3,3,3-trifluorpropyl)piperidin-4-yl]phenylamino}-5-oxo-5,8-dihydropyrido[2,3-d]pyrimidin-6-carboxamid
7-Amino-8-(2-aminoethyl)-2-(4-morpholin-4-ylphenylamino)-5-oxo-5,8-dihydropyrido[2,3-d]pyrimidin-6-carboxamid
7-Amino-2-(4-morpholin-4-ylphenylamino)-5-oxo-8-pyrrolidin-3-ylmethyl-5,8-dihydropyrido[2,3-d]pyrimidin-6-carboxamid
7-Amino-8-ethyl-2-[4-(4-ethylpiperazin-1-yl)-2-hydroxyphenylamino]-5-oxo-5,8-dihydropyrido[2,3-d]pyrimidin-6-carboxamid
7-Amino-2-(4-morpholin-4-ylphenylamino)-5-oxo-8-piperidin-4-ylmethyl-5,8-dihydropyrido[2,3-d]pyrimidin-6-carboxamid
7-Amino-2-(2-methoxy-4-morpholin-4-ylphenylamino)-5-oxo-8-pyrrolidin-3-ylmethyl-5,8-dihydropyrido[2,3-d]pyrimidin-6-carboxamid
7-Amino-8-ethyl-2-(2-methoxy-4-piperidin-4-ylphenyl-amino)-4-methyl-5-oxo-5,8-dihydropyrido[2,3-d]pyrimi-din-6-carboxamid
7-Amino-2-{2-methoxy-4-[1-(3,3,3-trifluorpro-pyl)piperidin-4-yl]phenylamino}-5-oxo-8-thiophen-2-ylmethyl-5,8-dihydropyrido[2,3-d]pyrimidin-6-carboxamid
7-Amino-8-isobutyl-2-[2-methoxy-4-(4-morpholin-4-ylpiperidin-1-yl)phenylamino]-5-oxo-5,8-dihydro-pyrido[2,3-d]pyrimidin-6-carboxamid
7-Amino-2-(4-morpholin-4-ylphenylamino)-5-oxo-8-pyrrolidin-2-ylmethyl-5,8-dihydropyrido[2,3-d]pyrimidin-6-carboxamid
7-Amino-8-ethyl-2-{2-methoxy-4-[1-(3,3,3-trifluor-propyl)-1,2,3,6-tetrahydropyridin-4-yl]phenylamino}-5-oxo-5,8-dihydropyrido[2,3-d]pyrimidin-6-carboxamid
7-Amino-2-[4-(4-ethylpiperazin-1-yl)-2-methoxyphe-nylamino]-8-(2-hydroxy-2-methylpropyl)-5-oxo-5,8-dihydropyrido[2,3-d]pyrimidin-6-carboxamid
7-Amino-8-(2-hydroxy-2-methylpropyl)-2-{2-methoxy-4-[1-(3,3,3-trifluorpropyl)piperidin-4-yl]phenylamino}-5-oxo-5,8-dihydropyrido[2,3-d]pyrimidin-6-carboxamid
7-Amino-8-(2-hydroxy-2-methylpropyl)-2-[4-(4-ethyl-piperidin-1-yl)-2-methoxyphenylamino]-5-oxo-5,8-dihydropyrido[2,3-d]pyrimidin-6-carboxamid
7-Amino-2-[4-(1-cyclopropylpiperidin-4-yl)-2-methoxyphe-nylamino]-8-(2-hydroxy-2-methylpropyl)-5-oxo-5,8-dihydropyrido[2,3-d]pyrimidin-6-carboxamid
7-Amino-8-(2-hydroxy-2-methylpropyl)-2-{2-methoxy-4-[1-(2-methoxyethyl)piperidin-4-yl]phenylamino}-5-oxo-5,8-dihydropyrido[2,3-d]pyrimidin-6-carboxamid
7-Amino-2-[2-methoxy-4-(1-methylpiperidin-4-yl)phenylamino]-5-oxo-8-phenyl-5,8-dihydropyrido[2,3-d]pyrimidin-6-carboxamid
7-Amino-8-(3-fluorphenyl)-2-[2-methoxy-4-piperidin-4-ylphenylamino]-5-oxo-5,8-dihydropyrido[2,3-d]pyrimidin-6-carboxamid
7-Amino-8-(4-fluorphenyl)-2-[2-methoxy-4-piperidin-4-ylphenylamino]-5-oxo-5,8-dihydropyrido[2,3-d]pyrimidin-6-carboxamid
7-Amino-2-[2-methoxy-4-piperidin-4-ylphenylamino]-5-oxo-8-m-tolyl-5,8-dihydropyrido[2,3-d]pyrimidin-6-carboxamid
7-Amino-2-[2-methoxy-4-piperidin-4-ylphenylamino]-5-oxo-8-p-tolyl-5,8-dihydropyrido[2,3-d]pyrimidin-6-carboxamid
7-Amino-8-(3-methoxyphenyl)-2-(2-methoxy-4-piperidin-4-ylphenylamino)-5-oxo-5,8-dihydropyrido[2,3-d]pyrimidin-6-carboxamid
7-Amino-2-[2-methoxy-4-(2-pyrrolidin-1-ylethyl)phenyl-amino]-5-oxo-8-phenyl-5,8-dihydropyrido[2,3-d]pyrimidin-6-carboxamid
7-Amino-8-(4-fluorphenyl)-2-[2-methoxy-4-(1-methyl-piperidin-4-yl)phenylamino]-5-oxo-5,8-dihydropyrido[2,3-d]pyrimidin-6-carboxamid
7-Amino-2-[2-methoxy-4-(1-methylpiperidin-4-yl)phenyl-amino]-5-oxo-8-m-tolyl-5,8-dihydropyrido[2,3-d]pyrimidin-6-carboxamid
7-Amino-2-{2-methoxy-4-[1-(3,3,3-trifluorpropyl)piperidin-4-yl]phenylamino}-5-oxo-8-phenyl-5,8-dihydropyrido[2,3-d]pyrimidin-6-carboxamid
7-Amino-2-[4-(4-ethylpiperazin-1-yl)-2-methoxyphenyl-amino]-5-oxo-8-phenyl-5,8-dihydropyrido[2,3-d]pyrimidin-6-carboxamid
als Base oder als Säureadditionssalz, sowie als Hydrat oder Solvat.

10. Verfahren zur Herstellung einer Verbindung der Formel (I) nach einem der Ansprüche 1 bis 9, **dadurch gekennzeichnet, dass** es das Umsetzen einer Verbindung der Formel (IX) 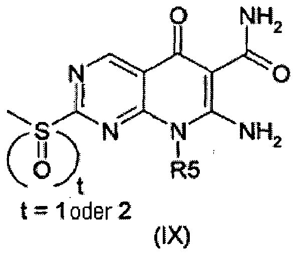 mit einem primären Amin der Formel (A): 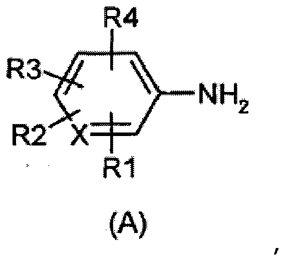 in denen R₁, R₂, R₃, R₄ und R₅ wie in einem der Ansprüche 1 bis 9 definiert sind und t 1 oder 2 bedeutet, umfasst.

11. Verfahren zur Herstellung einer Verbindung der Formel (I) nach einem der Ansprüche 1 bis 9, **dadurch gekennzeichnet, dass** es das Umsetzen einer Verbindung der allgemeinen Formel (XIV) oder (XV): 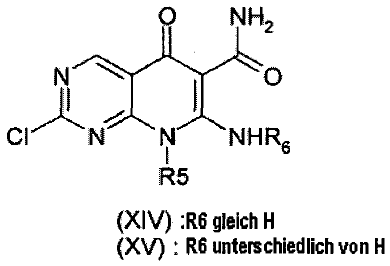 und eine Verbindung der allgemeinen Formel (A): 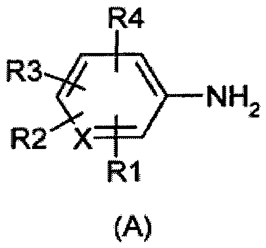 in denen R₁, R₂, R₃, R₄, R₅ und R₆ wie in einem der Ansprüche 1 bis 9 definiert sind,
umfasst.

12. Verbindung der Formel (VII):

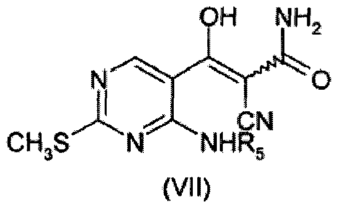

13. Verbindung der Formel (VIII): 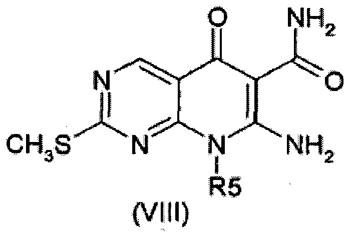 in der R₅ wie in einem der Ansprüche 1 bis 8 definiert ist.

14. Verbindung der Formel (IX) : 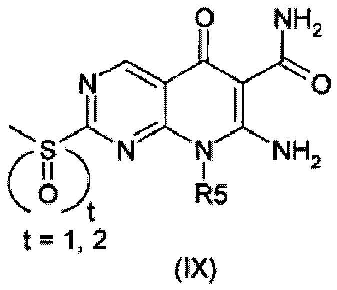 in der **R₅** wie in einem der Ansprüche 1 bis 8 definiert ist und t 1 oder 2 bedeutet.

15. Verbindung der Formel (XV): 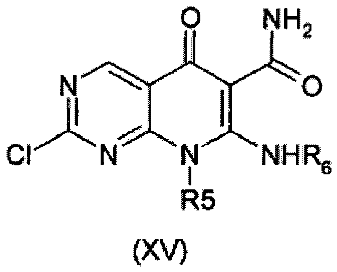 in der **R₅** und **R₆** wie in einem der Ansprüche 1 bis 8 definiert sind und **R₆** von H verschieden ist.

16. Arzneimittel, **dadurch gekennzeichnet, dass** es eine Verbindung der Formel (I) nach einem der Ansprüche 1 bis 9 oder ein Additionssalz dieser Verbindung mit einer pharmazeutisch annehmbaren Säure oder auch einem Hydrat oder ein Solvat der Verbindung der Formel (I) umfasst.

17. Pharmazeutische Zusammensetzung, **dadurch gekennzeichnet, dass** sie eine Verbindung der Formel (I) nach einem der Ansprüche 1 bis 9 oder ein pharmazeutisch unbedenkliches Salz, ein Hydrat oder ein Solvat dieser Verbindung, sowie mindestens einen pharmazeutisch annehmbaren Grundstoff umfasst.

18. Verbindung der Formel (I) nach einem der Ansprüche 1 bis 9 zur Verwendung in der Behandlung und/oder Vorbeugung von jeglicher Pathologie, an der die CaMKII-Kinase impliziert ist.

19. Verbindung nach Anspruch 18 zur Verwendung in der Behandlung und/oder Vorbeugung von Herz-Kreislauf-Pathologien, einschließlich Myokardinfarkt, Ventrikel-Hypertrophie, Myokardfibrose, Herzinsuffizienz, Herzrhythmusstörungen und Restenose, sowie Gefäßpathologien, die mit einer Enotheldysfunktion verbunden sind, einschließlich Arterosklerose, sowie Pathologien, die mit dem Entstehen von Fibrose assoziiert sind, einschließlich Leberfibrose, Pankreasfibrose, Nierenfibrose, Lungenfibrose, Hautfibrose, Darmfibrose und Augenfibrose, Nierentransplantation, Lebertransplantation sowie akuten Pathologien, einschließlich akuter Niereninsuffizienz, akuter Lungeninsuffizienz und akuter Leberinsuffizienz, sowie Pathologien, die mit dem Neuronentod oder einer Nervendegeneration assoziiert sind, einschließlich Schlaganfall und Morbus Parkinson, sowie Pathologien entzündlichen Ursprungs, wie chronischen Schmerzen und rheumatoider Arthritis.

## Claims

1. Compound corresponding to the general formula (I) 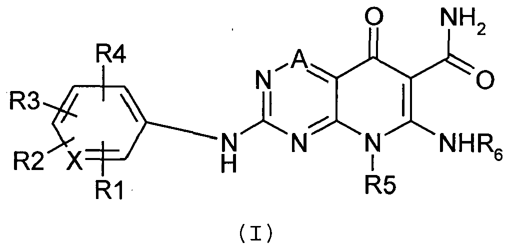 in which:
➢ **A** represents CH or C(alkyl);
➢ **X** represents CH, C(alkyl) or N;
**➢ R₁**, **R₂, R₃** and **R₄,** which may be identical or different, represent, independently of each other:
• a hydrogen atom;
• a linear, branched or cyclic alkyl, optionally substituted with one or more of the following:
○ halogen atoms;
○ -OR₉;
○ -NR₉R'₉;
○ -CN;
○ -C(O)OR₉;
○ -C(O)NR₉R_{9';}
○ -S(O)ₚR_{10;}
○ -S(O)₂NR₉R'₉;
in which **R₉, R'₉, R₁₀** and **p** are as defined below
• a group -S(O)ₚR₁₀ in which p and R₁₀ are as defined below;
• a group -OR₁₀ in which R₁₀ is as defined below;
• a halogen atom;
• a group -N(R₁₁)C(O)R₁₂, in which
(i) **R₁₁** and **R₁₂** represent, independently of each other, a hydrogen atom or a linear, branched or cyclic alkyl, optionally substituted with one or more substituents chosen from halogen atoms, groups -OR₉ and groups -NR₉R'₉, or
(ii) **R₁₁** and **R₁₂** form, together with the atoms to which they are attached, a heterocycloalkyl, so as to form a lactam;
• a group -N(R₁₄)-CH₂-C(O)NR₁₅R₉, in which **R₁₄** and **R₁₅** form, together with the atoms to which they are attached, a heterocycloalkyl, so as to form a piperazinone and in which **R₉** is as defined below;
• a group -C(O)NR₁₆R₁₇ with **R₁₆** and **R₁₇** form, together with the nitrogen atom to which they are attached, a heterocycloalkyl,
• a group **-T-U,** in which:
**■ T** represents:
○ a single bond,
○ a linear or branched alkylene group;
○ a group -C(O)-,
○ a group -S(O)ₚ- in which p is as defined below, or
○ a group -O-(CH₂)ₙ- in which n is as
defined below,
with **U** representing a heterocycle comprising one or more heteroatoms chosen from N, O and S(O)p, in which p is as defined below, the said heterocycle being saturated, unsaturated or aromatic, optionally mono- or di- or polysubstituted with one, two or several substituents chosen from:
❖ groups -OR₇, in which R₇ is as defined below,
❖ halogen atoms,
❖ groups -C(O)R₇ in which R₇ is as defined below,
❖ linear, branched or cyclic alkyls, optionally substituted with one or more substituents chosen from halogen atoms, groups -OR₁₀, groups -NR₉R'₉ and the group -CN, in which R₉, R'₉ and R₁₀ are as defined below; and
❖ saturated, unsaturated or aromatic heterocycles, optionally substituted with one or more substituents chosen from halogen atoms, groups -OR₉, groups -NR₉R'₉ and groups alkyl, the said alkyl groups being optionally substituted with one or more halogen atoms; or
**■ T** represents:
○ a group -C(O)-;
○ a group -S(O)₂-; or
○ a group -O-(C2-C3)alkylene-;
with **U** representing a group -NR₉R'₉ in which R₉ and R'₉ are as defined below; or
**■ T** represents:
○ a group -C(O)-; or
○ a group -O-(C2-C3)alkylene-;
with **U** representing a group -OR₉, in which R₉ is as defined below; or
**■ T** represents:
○ a linear or branched alkylene group; or
○ a group -O-(C2-C3)alkylene-;
with **U** representing a group -NR₈R₉ in which R₉ and R₈ are as defined below;
or alternatively two adjacent groups chosen from **R₁**, **R₂, R₃** and **R₄** are linked and form, with the two carbons that bear them, a saturated, unsaturated or aromatic heterocycle, optionally substituted with one or more linear, branched or cyclic alkyl groups, the said alkyl groups being optionally substituted with one or more substituents chosen from halogen atoms, groups -OR₁₀, and groups -NR₉R'₉, in which R₉, R'₉ and R₁₀ are as defined below,
the said heterocycle being fused with the aromatic ring,
➢ **R₅** represents:
• a linear or branched alkyl, optionally substituted with one or more substituents chosen from halogen atoms, groups -OR₉, groups -NR₉R'₉, the group -CN, groups -C(O)NR₉R_{9'}, groups -S(O)ₚR₁₀ and cycloalkyl groups optionally substituted with a group -NR₉R'₉, in which R₉, R'₉, R₁₀ and p are as defined below,
• a cycloalkyl group, optionally substituted with a group -NR₉R'₉, in which R₉ and R'₉ are as defined below,
• an alkoxy group -OR₉, in which R₉ is as defined below,
• an aryl optionally substituted with one or more substituents chosen from groups (C1-C3)alkyl, halogen atoms and groups -O-(C1-C3)alkyl, or
• a group -(CH₂)ₜ-R₁₃, in which **R₁₃** and **t** are as defined below,
➢ **R₆** represents a hydrogen atom or a linear, branched or cyclic alkyl,
and in which:
➢ **R₇** represents a hydrogen atom or a linear, branched or cyclic alkyl, optionally substituted with one or more of the following substituents chosen from halogen atoms, groups -OR₉ and groups -NR₉R'₉ with R₉ and R'₉ as defined above;
➢ **R₈** represents a heteroaryl group;
**➢ R₉** and **R'₉** represent, independently of each other, a hydrogen atom or a linear, branched or cyclic alkyl;
**➢ R₁₀** represents a hydrogen atom or a linear, branched or cyclic alkyl optionally substituted with one or more halogen atoms,
➢ **R₁₃** represents a heteroaryl or a heterocycloalkyl optionally substituted with one or more substituents chosen from linear, branched or cyclic alkyls, it being understood that when the said heterocycloalkyl comprises at least one nitrogen atom, this atom may optionally bear the said substituent,
➢ t represents 1 or 2,
➢ n represents 0, 1, 2 or 3, and
➢ **p** represents 0, 1 or 2,
in the form of acid, base or addition salt with an acid or a base, and also in the form of hydrate or solvate.

2. Compound according to Claim 1, **characterized in that**:
**A** represents CH or C(CH₃);
and/or
**X** represents CH or N.

3. Compound according to Claim 1 or 2, **characterized in that**:
**➢ A** represents CH,
**➢ X** represents CH, C(alkyl) or N,
➢ **R₁**, **R₂, R₃** and **R₄,** which may be identical or different, represent, independently of each other:
○ a hydrogen atom,
○ a linear, branched or cyclic alkyl, optionally substituted with one or more of the following halogen atoms, a group -OR₉ or -NR₉R'₉, in which R₉ and R'₉ represent, independently of each other, a hydrogen atom or a linear, branched or cyclic alkyl,
○ a group -S(O)ₚR₁₀ or a group -OR₁₀, in which R₁₀ represents a hydrogen atom or a linear, branched or cyclic alkyl optionally substituted with one or more halogen atoms, and p represents 0, 1 or 2,
○ a halogen atom,
○ a group -N(R₁₁)C(O)R₁₂, in which R₁₁ and R₁₂ represent, independently of each other, a hydrogen atom or a linear, branched or cyclic alkyl or R₁₁ and R₁₂ form, together with the atoms to which they are attached, a heterocycloalkyl, so as to form a lactam;
○ a group -T-U, in which
■ T represents:
• a single bond,
• a linear or branched alkylene group,
• a group -C(O)-,
• a group -S(O)ₚ, in which p represents 0, 1 or 2,
• A group -O-(CH₂)ₙ- in which n represents 0, 1, 2 or 3,
and U represents a heterocycle comprising one or more heteroatoms chosen from N, O and S(O)p in which p represents 0, 1 or 2, the said heterocycle being of the formula: 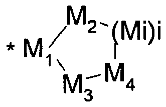 in which
• * represents the position of attachment of U to T;
• **M1** represents a C or N atom;
• **M2** and **M3,** which may be identical or different, represent a C, N or O atom or S(O)p in which p = 0, 1 or 2;
• **M4** represents a C, C(=O), N, O or S(O)p atom in which p = 0, 1 or 2;
each of the **Mi,** which may be identical or different, represent a C, C(=O), N, O or S(O)p atom in which p = 0, 1 or 2;
• **i**=0, 1, 2 or 3;
it being understood that each of the M1, M2, M3, M4 or Mi may be optionally substituted if a valency is available and/or the adjacent M1, M2, M3, M4 or Mi may be attached via a double bond, where appropriate;
the said heterocycle **U** being saturated, unsaturated or aromatic, optionally mono- or di- or
polysubstituted with one, two or several substituents chosen from:
○ groups -OR₇, in which R₇ represents a hydrogen atom or a linear, branched or cyclic alkyl,
○ halogen atoms,
○ groups -COR₇ in which R₇ represents a hydrogen atom or a linear, branched or cyclic alkyl,
○ linear, branched or cyclic alkyls, optionally substituted with one or more halogen atoms,
○ saturated, unsaturated or aromatic heterocycles, especially a heterocycle comprising an N, optionally substituted with one or more groups chosen from halogen atoms and groups alkyl optionally substituted with one or more halogen atoms;
or alternatively two adjacent groups from among R₁, R₂, R₃ and R₄ are linked and form, with the two carbons that bear them, a saturated, unsaturated or aromatic heterocycle, optionally substituted with one or more linear, branched or cyclic alkyl groups, the said alkyl groups being optionally substituted with at least one group chosen from groups -NR₉R'₉, in which R₉ and R'₉ represent, independently of each other, a hydrogen atom or an alkyl group, the said heterocycle being fused with the aromatic ring.

4. Compound according to any one of Claims 1 to 3,
**characterized in that** it
corresponds to formula (I') below: 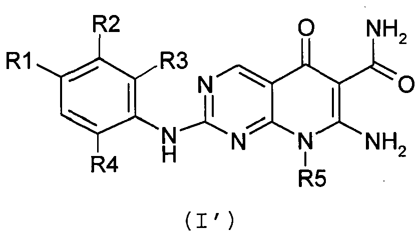 in which:
**➢ R₁** represents:
• a hydrogen atom,
• a linear, branched or cyclic alkyl, optionally substituted with one or more of the following substituents chosen from halogen atoms, groups -NR₉R'₉, in which R₉ and R'₉ represent, independently of each other, a hydrogen atom or an alkyl group,
• -OR₁₀, in which R₁₀ represents a hydrogen atom or a linear, branched or cyclic alkyl optionally substituted with one or more halogen atoms,
• a halogen atom,
• a group -T-U, in which
T represents:
○ a single bond,
○ an alkylene group,
○ a group -C(O)-,
○ a group -O-(CH₂)ₙ in which n represents 0, 1, 2 or 3,
○ and **U** represents a saturated, unsaturated or aromatic heterocycle, optionally mono- or disubstituted with a substituent,
➢ **R₂, R₃** and **R₄,** which may be identical or different, independently represent:
○ a hydrogen atom,
○ a linear, branched or cyclic alkyl, optionally substituted with one or more of the following substituents chosen from halogen atoms and groups -NR₉R'₉, in which R₉ and R'₉ represent, independently of each other, a hydrogen atom or an alkyl,
○ a group -OR₁₀, in which R₁₀ represents a hydrogen atom or an alkyl optionally substituted with one or more halogen atoms,
○ a halogen,
○ a group -T-U with T representing a bond and U a morpholinyl, or
➢ **R₁** and **R₂** are linked and form, with the two carbon atoms that bear them, a heterocycle chosen from a piperidine, a thiazole, a tetrahydrofuran and a dioxane, the said heterocycle being optionally substituted with a linear, branched or cyclic alkyl optionally substituted with -NR₉R'₉, in which R₉ and R'₉ represent, independently of each other, a hydrogen atom or a methyl,
the said heterocycle being fused with the aromatic ring,
➢ **R₅** represents (i) a linear, branched or cyclic alkyl comprising from 1 to 5 carbon atoms, optionally substituted with one or more substituents chosen from halogen atoms or a group -OH, (ii) an aryl optionally substituted with one or more substituents chosen from groups (C1-C3)alkyl, halogen atoms and groups -O-(C1-C3)alkyl;
in the form of acid, base or addition salt with an acid or a base, and also in the form of hydrate or solvate.

5. Compound according to any one of Claims 1 to 4, **characterized in that**
**➢ A** represents CH; and/or
**➢ X** represents CH; and/or
**➢ R₁** is other than a hydrogen atom; and/or
➢ **R₅** represents a linear, branched or cyclic alkyl; and/or
➢ **U** represents a saturated, unsaturated or aromatic heterocycle, optionally mono- or disubstituted, comprising at least one nitrogen atom and/or one oxygen atom, and/or
**➢ U** represents a saturated heterocycle comprising at least one nitrogen atom; and/or
**➢ U** represents a heterocycle chosen from:
o an azetidinyl;
o a pyrrolidinyl;
o an oxopyrrolidinyl;
○ a piperidyl;
○ a 1,2,3,6-tetrahydropyridyl;
○ a pyridyl;
○ a piperazinyl;
○ a diazepanyl;
○ a morpholinyl;
○ a 1,1-dioxo-1lambda6-thiomorpholin-4-yl.

6. Compound according to any one of the preceding claims, **characterized in that R₁** is chosen from:

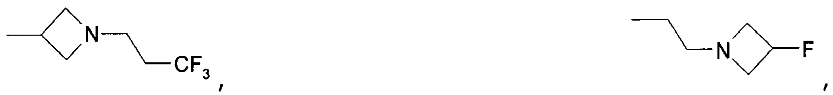

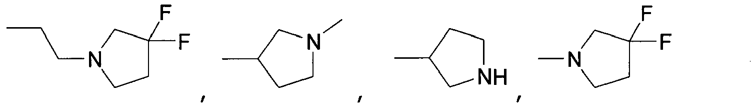

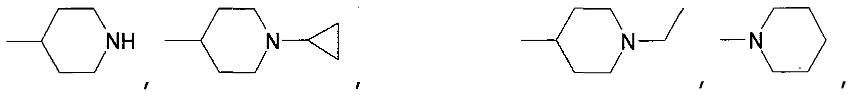

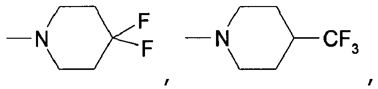

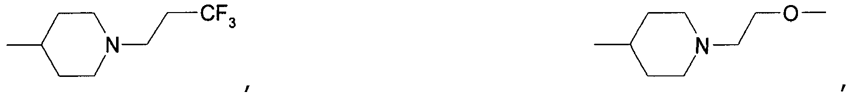

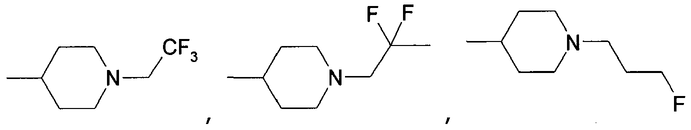

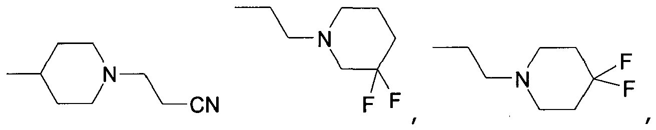

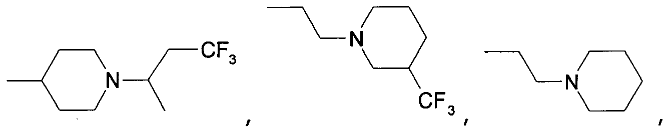

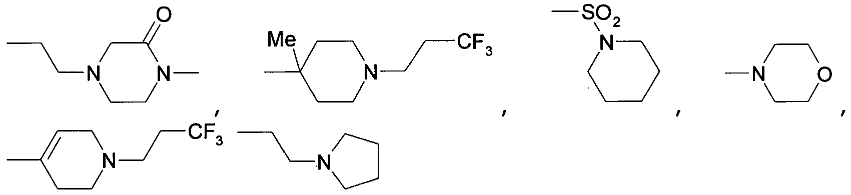

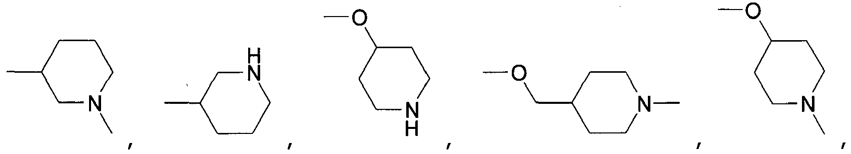

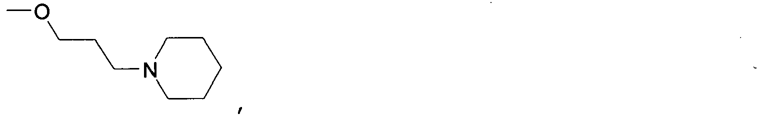

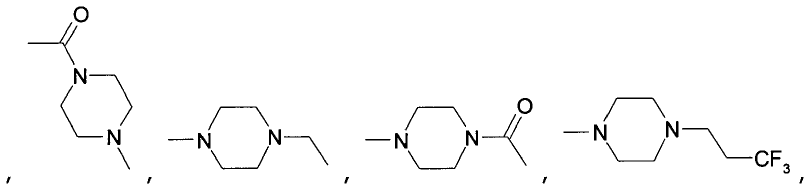

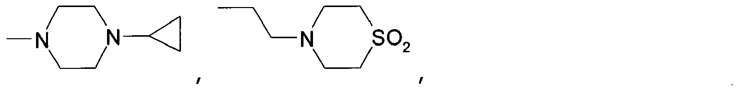

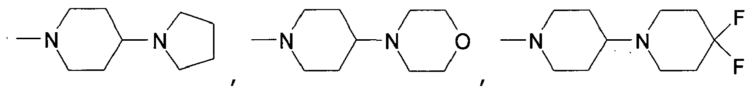

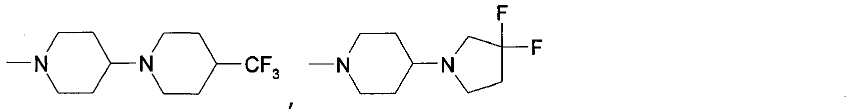

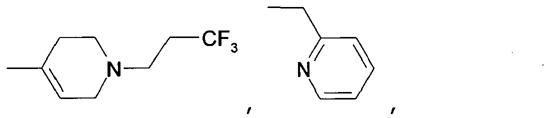

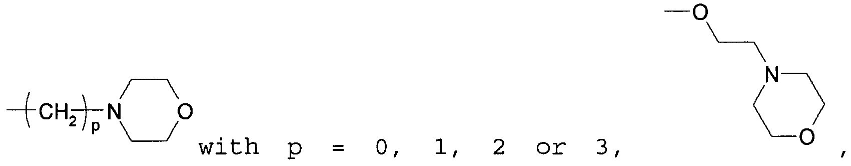

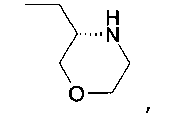

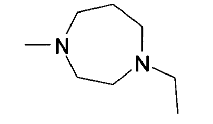

7. Compound according to any one of the preceding claims, **characterized in that A** and **X** represent CH.

8. Compound according to any one of the preceding claims, **characterized in that U** represents a saturated heterocycle comprising at least one nitrogen atom.

9. Compound according to any one of the preceding claims, **characterized in that** it is chosen from:
7-Amino-8-ethyl-2-(4-hydroxyphenylamino)-5-oxo-5,8-dihydropyrido[2,3-d]pyrimidine-6-carboxamide;
7-Amino-2-(benzothiazol-6-ylamino)-8-ethyl-5-oxo-5,8-dihydropyrido[2,3-d]pyrimidine-6-carboxamide;
7-Amino-2-[4-(cyclopropanecarbonylmethylamino)phenylamino]-8-ethyl-5-oxo-5,8-dihydropyrido[2,3-d]pyrimidine-6-carboxamide;
7-Amino-8-ethyl-2-[4-(4-methylpiperazine-1-carbonyl)phenylamino]-5-oxo-5,8-dihydropyrido[2,3-d]pyrimidine-6-carboxamide;
7-Amino-2-(4-cyclopentyloxyphenylamino)-8-ethyl-5-oxo-5,8-dihydropyrido[2,3-d]pyrimidine-6-carboxamide;
7-Amino-8-ethyl-2-[4-(4-ethylpiperazin-1-yl)phenylamino]-5-oxo-5,8-dihydropyrido[2,3-d]pyrimidine-6-carboxamide;
7-Amino-8-ethyl-5-oxo-2-[4-(4-pyrrolidin-1-ylpiperid-1-yl)phenylamino]-5,8-dihydropyrido[2,3-d]pyrimidine-6-carboxamide;
7-Amino-8-cyclopentyl-2-(4-morpholin-4-ylphenylamino)-5-oxo-5,8-dihydropyrido[2,3-d]pyrimidine-6-carboxamide;
7-Amino-8-ethyl-5-oxo-2-[4-(piperidine-1-sulfonyl)phenylamino]-5,8-dihydropyrido[2,3-d]pyrimidine-6-carboxamide;
2-[4-(4-Acetylpiperazin-1-yl)phenylamino]-7-amino-8-ethyl-5-oxo-5,8-dihydropyrido[2,3-d]pyrimidine-6-carboxamide;
7-Amino-8-ethyl-2-(4-morpholin-4-ylbenzylamino)-5-oxo-5,8-dihydropyrido[2,3-d]pyrimidine-6-carboxamide;
7-Amino-8-ethyl-5-oxo-2-(quinolin-3-ylamino)-5,8-dihydropyrido[2,3-d]pyrimidine-6-carboxylamide;
7-Amino-8-ethyl-5-oxo-2-[4-(3-piperid-1-ylpropoxy)phenylamino]-5,8-dihydropyrido[2,3-d]pyrimidine-6-carboxamide;
7-Amino-8-ethyl-5-oxo-2-(3,4,5,6-tetrahydro-2H-[1,2']bipyridyl-5'-ylamino)-5,8-dihydropyrido[2,3-d]pyrimidine-6-carboxamide;
7-Amino-8-ethyl-5-oxo-2-[4-(2-oxopyrrolidin-1-yl)phenylamino]-5,8-dihydropyrido[2,3-d]pyrimidine-6-carboxamide;
7-Amino-8-ethyl-5-oxo-2-(quinolin-6-ylamino)-5,8-dihydropyrido[2,3-d]pyrimidine-6-carboxamide;
7-Amino-8-ethyl-2-(3-morpholin-4-ylphenylamino)-5-oxo-5,8-dihydropyrido[2,3-d]pyrimidine-6-carboxamide;
7-Amino-2-(2,3-dihydrobenzo[1,4]dioxin-6-ylamino)-8-ethyl-5-oxo-5,8-dihydropyrido[2,3-d]pyrimidine-6-carboxamide;
7-Amino-8-ethyl-2-(3-fluoro-4-hydroxyphenylamino)-5-oxo-5,8-dihydropyrido[2,3-d]pyrimidine-6-carboxamide;
7-Amino-8-ethyl-2-(3-methylsulfanylphenylamino)-5-oxo-5,8-dihydropyrido[2,3-d]pyrimidine-6-carboxamide;
7-Amino-8-ethyl-2-(4-morpholin-4-ylphenylamino)-5-oxo-5,8-dihydropyrido[2,3-d]pyrimidine-6-carboxamide;
7-Amino-8-ethyl-2-(2-fluorophenylamino)-5-oxo-5,8-dihydropyrido[2,3-d]pyrimidine-6-carboxamide;
7-Amino-2-[4-(4,4-difluoro[1,4']bipiperidyl-1'-yl)-2-methoxyphenylamino]-8-ethyl-5-oxo-5,8-dihydropyrido[2,3-d]pyrimidine-6-carboxamide
7-Amino-8-ethyl-2-[2-methoxy-4-(4-trifluoromethyl[1,4']bipiperidyl-1'-yl)phenylamino]-5-oxo-5,8-dihydropyrido[2,3-d]pyrimidine-6-carboxamide
7-Amino-2-{4-[4-(3,3-difluoropyrrolidin-1-yl)piperid-1-yl]-2-methoxyphenylamino}-8-ethyl-5-oxo-5,8-dihydropyrido[2,3-d]pyrimidine-6-carboxamide
7-Amino-8-ethyl-2-[4-(4-ethylpiperazin-1-yl)-2-fluoro-6-methoxyphenylamino]-5-oxo-5,8-dihydropyrido[2,3-d]pyrimidine-6-carboxamide;
7-Amino-2-[4-(1-cyclopropylpiperid-4-yl)-2-methoxyphenylamino]-8-ethyl-5-oxo-5,8-dihydropyrido[2,3-d]pyrimidine-6-carboxamide;
7-Amino-2-(2-dimethylaminomethylchroman-6-ylamino)-8-ethyl-5-oxo-5,8-dihydropyrido[2,3-d]pyrimidine-6-carboxamide;
7-Amino-2-[5-chloro-4-(4-cyclopropylpiperazin-1-yl)-2-methoxyphenylamino]-8-ethyl-5-oxo-5,8-dihydropyrido[2,3-d]pyrimidine-6-carboxamide;
7-Amino-8-ethyl-2-[2-methoxy-4-(4-morpholin-4-ylpiperid-1-yl)phenylamino]-5-oxo-5,8-dihydropyrido[2,3-d]pyrimidine-6-carboxyamide;
7-Amino-2-[4-(4-cyclopropylpiperazin-1-yl)-2-difluoromethoxyphenylamino]-8-ethyl-5-oxo-5,8-dihydropyrido[2,3-d]pyrimidine-6-carboxamide;
7-Amino-2-[4-(4-cyclopropylpiperazin-1-yl)-2-methoxyphenylamino]-8-ethyl-5-oxo-5,8-dihydropyrido[2,3-d]pyrimidine-6-carboxamide;
7-Amino-8-ethyl-2-{2-methoxy-4-[1-(3,3,3-trifluoropropyl)piperid-4-yl]phenylamino}-5-oxo-5,8-dihydropyrido[2,3-d]pyrimidine-6-carboxamide;
7-Amino-2-[2-chloro-4-(4-ethylpiperazin-1-yl)phenylamino]-8-ethyl-5-oxo-5,8-dihydropyrido[2,3-d]pyrimidine-6-carboxamide;
7-Amino-8-ethyl-2-[4-(4-ethylpiperazin-1-yl)-3-trifluoromethylphenylamino]-5-oxo-5,8-dihydropyrido[2,3-d]pyrimidine-6-carboxamide;
7-Amino-8-ethyl-2-(4-morpholin-4-ylmethylphenylamino)-5-oxo-5,8-dihydropyrido[2,3-d]pyrimidine-6-carboxamide;
7-Amino-8-ethyl-5-oxo-2-{4-[4-(3,3,3-trifluoropropyl)piperazin-1-yl]phenylamino}-5,8-dihydropyrido[2,3-d]pyrimidine-6-carboxamide;
7-Amino-8-ethyl-2-[4-(4-ethylpiperazin-1-yl)-3-fluorophenylamino]-5-oxo-5,8-dihydropyrido[2,3-d]pyrimidine-6-carboxamide;
6-(4-Morpholin-4-ylphenylamino)-9-oxo-1,3,4,9-tetrahydro-2H-1,4a,5,7-tetraaza-phenanthrene-10-carboxamide;
7-Amino-8-ethyl-2-[4-(4-ethylpiperazin-1-yl)-2-methoxyphenylamino]-5-oxo-5,8-dihydropyrido[2,3-d]pyrimidine-6-carboxamide;
7-Amino-8-ethyl-2-[4-(1-methylpiperid-4-ylmethoxy)phenylamino]-5-oxo-5,8-dihydropyrido[2,3-d]pyrimidine-6-carboxamide;
7-Amino-2-[4-(4-cyclopropylpiperazin-1-yl)phenylamino]-8-ethyl-5-oxo-5,8-dihydropyrido[2,3-d]pyrimidine-6-carboxamide;
7-Amino-8-ethyl-2-[4-(4-ethylpiperazin-1-yl)-2-methylphenylamino]-5-oxo-5,8-dihydropyrido[2,3-d]pyrimidine-6-carboxamide;
7-Amino-8-ethyl-2-[4-(4-ethyl[1,4]diazepan-1-yl)phenylamino]-5-oxo-5,8-dihydropyrido[2,3-d]pyrimidine-6-carboxamide;
8-(4-Morpholin-4-ylphenylamino)-5-oxo-1,2,3,5-tetrahydro-3,7,9,9b-tetraaza-cyclopenta[a]naphthalene-4-carboxamide;
7-Amino-8-ethyl-5-oxo-2-{4-[2-(3-trifluoromethylpiperid-1-yl)ethyl]phenylamino}-5,8-dihydropyrido[2,3-d]pyrimidine-6-carboxamide;
7-Amino-8-ethyl-2-[4-(3-morpholin-4-ylpropyl)phenylamino]-5-oxo-5,8-dihydropyrido[2,3-d]pyrimidine-6-carboxamide;
7-Amino-8-ethyl-5-oxo-2-{4-[1-(2,2,2-trifluoroethyl)piperid-4-yl]phenylamino}-5,8-dihydropyrido[2,3-d]pyrimidine-6-carboxamide;
7-Amino-8-ethyl-2-(4-(S)-1-morpholin-3-ylmethylphenylamino)-5-oxo-5,8-dihydropyrido[2,3-d]pyrimidine-6-carboxamide;
7-Amino-8-isobutyl-2-[4-(2-morpholin-4-ylethyl)phenylamino]-5-oxo-5,8-dihydropyrido[2,3-d]pyrimidine-6-carboxamide;
7-Amino-8-ethyl-2-{4-[2-(3-fluoroazetidin-1-yl)ethyl]phenylamino}-5-oxo-5,8-dihydropyrido[2,3-d]pyrimidine-6-carboxamide;
7-Amino-2-[4-(2-morpholin-4-ylethyl)phenylamino]-5-oxo-8-propyl-5,8-dihydropyrido[2,3-d]pyrimidine-6-carboxamide;
7-Amino-8-ethyl-2-[4-(2-hydroxyethyl)phenylamino]-5-oxo-5,8-dihydropyrido[2,3-d]pyrimidine-6-carboxamide;
7-amino-8-ethyl-2-(4-hydroethylphenylamino)-5-oxo-5,8-dihydropyrido[2,3-d]pyrimidine-6-carboxamide;
7-Amino-8-ethyl-2-(2-methyl-1,2,3,4-tetrahydroisoquinolin-6-ylamino)-5-oxo-5,8-dihydropyrido[2,3-d]pyrimidine-6-carboxamide;
7-Amino-2-{4-[2-(1,1-dioxo-1lambda6-thiomorpholin-4-yl)ethyl]phenylamino}-8-ethyl-5-oxo-5,8-dihydropyrido[2,3-d]pyrimidine-6-carboxamide;
7-Amino-8-ethyl-2-{4-[2-(4-methyl-3-oxopiperazin-1-yl)ethyl]phenylamino}-5-oxo-5,8-dihydropyrido[2,3-d]pyrimidine-6-carboxamide;
7-Amino-8-ethyl-5-oxo-2-{4-[1-(3,3,3-trifluoropropyl)piperid-4-yl]phenylamino}-5,8-dihydropyrido[2,3-d]pyrimidine-6-carboxamide;
7-Amino-2-(4-dimethylaminomethylphenylamino)-5-oxo-8-(2,2,2-trifluoroethyl)-5,8-dihydropyrido[2,3-d]pyrimidine-6-carboxamide;
7-Amino-2-[3-chloro-4-(4-pyrrolidin-1-ylpiperid-1-yl)phenylamino]-8-ethyl-5-oxo-5,8-dihydropyrido[2,3-d]pyrimidine-6-carboxamide;
7-Amino-8-ethyl-5-oxo-2-(4-pyrid-2-ylmethylphenylamino)-5,8-dihydropyrido[2,3-d]pyrimidine-6-carboxamide;
7-Amino-2-{4-[2-(3,3-difluoropyrrolidin-1-yl)ethyl]phenylamino}-8-ethyl-5-oxo-5,8-dihydropyrido[2,3-d]pyrimidine-6-carboxamide;
7-Amino-8-ethyl-5-oxo-2-(4-pyrrolidin-3-ylphenylamino)-5,8-dihydropyrido[2,3-d]pyrimidine-6-carboxamide;
7-Amino-8-ethyl-5-oxo-2-[4-(2-piperid-1-ylethyl)phenylamino]-5,8-dihydropyrido[2,3-d]pyrimidine-6-carboxamide;
7-Amino-2-[4-(2-morpholin-4-ylethyl)phenylamino]-5-oxo-8-(2,2,2-trifluoroethyl)-5,8-dihydropyrido[2,3-d]pyrimidine-6-carboxamide
7-Amino-2-{4-[2-(4,4-difluoropiperid-1-yl)ethyl]phenylamino}-8-ethyl-5-oxo-5,8-dihydropyrido[2,3-d]pyrimidine-6-carboxamide;
7-Amino-8-ethyl-2-[4-(1-methylpyrrolidin-3-yl)phenylamino]-5-oxo-5,8-dihydropyrido[2,3-d]pyrimidine-6-carboxamide;
7-Amino-2-{4-[2-(3,3-difluoropiperid-1-yl)ethyl]phenylamino}-8-ethyl-5-oxo-5,8-dihydropyrido[2,3-d]pyrimidine-6-carboxamide;
7-Amino-8-(3-methoxy-propyl)-2-[4-(2-morpholin-4-ylethyl)phenylamino]-5-oxo-5,8-dihydropyrido[2,3-d]pyrimidine-6-carboxamide;
7-Amino-8-ethyl-2-[4-(1-methylpiperid-3-yl)phenylamino]-5-oxo-5,8-dihydropyrido[2,3-d]pyrimidine-6-carboxamide;
7-Amino-2-(4-dimethylaminomethylphenylamino)-8-isopropyl-5-oxo-5,8-dihydropyrido[2,3-d]pyrimidine-6-carboxamide;
7-Amino-8-ethyl-5-oxo-2-(4-piperid-3-ylphenylamino)-5,8-dihydropyrido[2,3-d]pyrimidine-6-carboxamide;
7-Amino-8-ethyl-5-oxo-2-(1,2,3,4-tetrahydroisoquinolin-7-ylamino)-5,8-dihydropyrido[2,3-d]pyrimidine-6-carboxamide;
7-Amino-8-ethyl-5-oxo-2-(1,2,3,4-tetrahydroisoquinolin-7-ylamino)-5,8-dihydropyrido[2,3-d]pyrimidine-6-carboxamide;
7-Amino-8-ethyl-2-[4-(1-methylpiperid-4-yloxy)phenylamino]-5-oxo-5,8-dihydropyrido[2,3-d]pyrimidine-6-carboxamide;
7-Amino-8-ethyl-2-[4-(2-morpholin-4-ylethoxy)phenylamino]-5-oxo-5,8-dihydropyrido[2,3-d]pyrimidine-6-carboxamide;
7-Amino-2-(3-dimethylaminomethylphenylamino)-8-ethyl-5-oxo-5,8-dihydropyrido[2,3-d]pyrimidine-6-carboxamide;
7-Amino-8-ethyl-5-oxo-2-[4-(4-pyrrolidin-1-ylpiperid-1-yl)phenylamino]-5,8-dihydropyrido[2,3-d]pyrimidine-6-carboxamide;
7-Amino-8-ethyl-5-oxo-2-[4-(piperid-4-yloxy)phenylamino]-5,8-dihydropyrido[2,3-d]pyrimidine-6-carboxamide;
7-Amino-8-(3-aminopropyl)-2-(4-morpholin-4-ylphenylamino)-5-oxo-5,8-dihydropyrido[2,3-d]pyrimidine-6-carboxamide;
7-Amino-8-ethyl-2-[4-(2-morpholin-4-ylethyl)phenylamino]-5-oxo-5,8-dihydropyrido[2,3-d]pyrimidine-6-carboxamide;
7-Amino-2-(4-dimethylaminomethylphenylamino)-8-ethyl-5-oxo-5,8-dihydropyrido[2,3-d]pyrimidine-6-carboxamide;
7-Amino-8-ethyl-2-[4-(1-ethylpiperid-4-yl)phenylamino]-5-oxo-5,8-dihydropyrido[2,3-d]pyrimidine-6-carboxamide;
7-Amino-8-ethyl-5-oxo-2-(4-piperid-4-ylphenylamino)-5,8-dihydropyrido[2,3-d]pyrimidine-6-carboxamide;
7-Amino-2-[3-chloro-4-(4-ethylpiperazin-1-yl)phenylamino]-8-ethyl-5-oxo-5,8-dihydropyrido[2,3-d]pyrimidine-6-carboxamide
7-Amino-8-ethyl-2-[3-methoxy-4-(3-piperid-1-ylpropoxy)phenylamino]-5-oxo-5,8-dihydropyrido[2,3-d]pyrimidine-6-carboxamide
7-Amino-8-ethyl-5-oxo-2-{4-[2-(4-trifluoromethylpiperid-1-yl)ethyl]phenylamino}-5,8-dihydropyrido[2,3-d]pyrimidine-6-carboxamide
7-Amino-2-{4-[2-(cis-2,6-dimethylmorpholin-4-yl)ethyl]phenylamino}-8-ethyl-5-oxo-5,8-dihydropyrido[2,3-d]pyrimidine-6-carboxamide
7-Amino-2-(4-diethylaminomethylphenylamino)-8-ethyl-5-oxo-5,8-dihydropyrido[2,3-d]pyrimidine-6-carboxamide
7-Amino-2-(4-dimethylaminomethylphenylamino)-8-(3-methoxy-propyl)-5-oxo-5,8-dihydropyrido[2,3-d]pyrimidine-6-carboxamide
7-Amino-8-ethyl-2-[4-(4-ethylpiperazin-1-yl)-2-fluorophenylamino]-5-oxo-5,8-dihydropyrido[2,3-d]pyrimidine-6-carboxamide
7-Amino-2-{4-[1-(2-cyanoethyl)piperid-4-yl]phenylamino}-8-ethyl-5-oxo-5,8-dihydropyrido[2,3-d]pyrimidine-6-carboxamide
7-Amino-8-ethyl-2-{4-[1-(3-fluoropropyl)piperid-4-yl]phenylamino}-5-oxo-5,8-dihydropyrido[2,3-d]pyrimidine-6-carboxylic acid amide
7-Amino-8-ethyl-2-[4-(4-ethylpiperazin-1-yl)-3-methylphenylamino]-5-oxo-5,8-dihydropyrido[2,3-d]pyrimidine-6-carboxamide
7-Amino-2-[4-(4-cyclopropylpiperazin-1-yl)-2-ethylphenylamino]-8-ethyl-5-oxo-5,8-dihydropyrido[2,3-d]pyrimidine-6-carboxamide
(±)-7-Amino-2-trans-[4-(2-dimethylaminocyclopropyl)phenylamino]-8-ethyl-5-oxo-5,8-dihydropyrido[2,3-d]pyrimidine-6-carboxamide
7-Amino-2-[4-(4-cyclopropylpiperazin-1-yl)-5-fluoro-2-methoxyphenylamino]-8-ethyl-5-oxo-5,8-dihydropyrido[2,3-d]pyrimidine-6-carboxamide
7-Amino-2-[4-(4-cyclopropylpiperazin-1-yl)-3-fluoro-2-methoxyphenylamino]-8-ethyl-5-oxo-5,8-dihydropyrido[2,3-d]pyrimidine-6-carboxamide
7-Amino-2-[4-(4-cyclopropylpiperazin-1-yl)-2-ethoxyphenylamino]-8-ethyl-5-oxo-5,8-dihydropyrido[2,3-d]pyrimidine-6-carboxamide
7-Amino-8-ethyl-5-oxo-2-(4-propylphenylamino)-5,8-dihydropyrido[2,3-d]pyrimidine-6-carboxamide
7-Amino-8-ethyl-5-oxo-2-(4-propoxyphenylamino)-5,8-dihydropyrido[2,3-d]pyrimidine-6-carboxamide
7-Amino-8-ethyl-2-(6-methoxypyrid-3-ylamino)-5-oxo-5,8-dihydropyrido[2,3-d]pyrimidine-6-carboxamide
7-Amino-8-ethyl-2-(4-fluorophenylamino)-5-oxo-5,8-dihydropyrido[2,3-d]pyrimidine-6-carboxamide
7-Amino-8-ethyl-2-(4-methoxyphenylamino)-5-oxo-5,8-dihydropyrido[2,3-d]pyrimidine-6-carboxamide
7-Amino-2-(benzo[1,3]dioxol-5-ylamino)-8-ethyl-5-oxo-5,8-dihydropyrido[2,3-d]pyrimidine-6-carboxamide
7-Amino-8-ethyl-5-oxo-2-(4-piperid-1-ylphenylamino)-5,8-dihydropyrido[2,3-d]pyrimidine-6-carboxamide
8-Ethyl-2-{2-methoxy-4-[1-(3,3,3-trifluoropropyl)piperid-4-yl]phenylamino}-7-methylamino-5-oxo-5,8-dihydropyrido[2,3-d]pyrimidine-6-carboxamide
7-Amino-8-isobutyl-2-{2-methoxy-4-[1-(3,3,3-trifluoropropyl)piperid-4-yl]phenylamino}-5-oxo-5,8-dihydropyrido[2,3-d]pyrimidine-6-carboxamide
7-Amino-8-cyclopropylmethyl-2-{2-methoxy-4-[1-(3,3,3-trifluoropropyl)piperid-4-yl]phenylamino}-5-oxo-5,8-dihydropyrido[2,3-d]pyrimidine-6-carboxamide
7-Amino-8-(2-methoxyethyl)-2-{2-methoxy-4-[1-(3,3,3-trifluoropropyl)piperid-4-yl]phenylamino}-5-oxo-5,8-dihydropyrido[2,3-d]pyrimidine-6-carboxamide
7-Amino-2-{2-methoxy-4-[1-(3,3,3-trifluoropropyl)piperid-4-yl]phenylamino}-5-oxo-8-(tetrahydrofuran-2-ylmethyl)-5,8-dihydropyrido[2,3-d]pyrimidine-6-carboxamide
7-Amino-8-ethyl-5-oxo-2-{4-[1-(3,3,3-trifluoropropyl)azetidin-3-yl]phenylamino}-5,8-dihydropyrido[2,3-d]pyrimidine-6-carboxamide
7-Amino-2-{5-fluoro-2-methoxy-4-[1-(3,3,3-trifluoropropyl)piperid-4-yl]phenylamino}-8-isobutyl-5-oxo-5,8-dihydropyrido[2,3-d]pyrimidine-6-carboxamide
7-Amino-8-ethyl-5-oxo-2-[4-(1,2,3,6-tetrahydropyrid-4-yl)phenylamino]-5,8-dihydropyrido[2,3-d]pyrimidine-6-carboxamide
7-Amino-2-(2-methoxy-4-piperid-4-ylphenylamino)-5-oxo-8-phenyl-5,8-dihydropyrido[2,3-d]pyrimidine-6-carboxamide
7-Amino-8-(2-hydroxyethyl)-2-(2-methoxy-4-piperid-4-ylphenylamino)-5-oxo-5,8-dihydropyrido[2,3-d]pyrimidine-6-carboxamide
7-Amino-2-{2-methoxy-4-[1-(3,3,3-trifluoropropyl)piperid-4-yl]phenylamino}-5-oxo-8-thiazol-2-ylmethyl-5,8-dihydropyrido[2,3-d]pyrimidine-6-carboxamide
7-Amino-2-{2-methoxy-4-[1-(3,3,3-trifluoropropyl)piperid-4-yl]phenylamino}-5-oxo-8-thiazol-5-ylmethyl-5,8-dihydropyrido[2,3-d]pyrimidine-6-carboxamide
7-Amino-8-(2-hydroxy-2-methylpropyl)-2-(2-methoxy-4-piperid-4-ylphenylamino)-5-oxo-5,8-dihydropyrido[2,3-d]pyrimidine-6-carboxamide
8-(2-Acetylaminoethyl)-7-amino-2-{2-methoxy-4-[1-(3,3,3-trifluoropropyl)piperid-4-yl]phenylamino}-5-oxo-5,8-dihydropyrido[2,3-d]pyrimidine-6-carboxamide
7-Amino-8-(2-aminoethyl)-2-(4-morpholin-4-ylphenylamino)-5-oxo-5,8-dihydropyrido[2,3-d]pyrimidine-6-carboxamide
7-Amino-2-(4-morpholin-4-ylphenylamino)-5-oxo-8-pyrrolidin-3-ylmethyl-5,8-dihydropyrido[2,3-d]pyrimidine-6-carboxamide
7-Amino-8-ethyl-2-[4-(4-ethylpiperazin-1-yl)-2-hydroxyphenylamino]-5-oxo-5,8-dihydropyrido[2,3-d]pyrimidine-6-carboxamide
7-Amino-2-(4-morpholin-4-ylphenylamino)-5-oxo-8-piperid-4-ylmethyl-5,8-dihydropyrido[2,3-d]pyrimidine-6-carboxamide
7-Amino-2-(2-methoxy-4-morpholin-4-ylphenylamino)-5-oxo-8-pyrrolidin-3-ylmethyl-5,8-dihydropyrido[2,3-d]pyrimidine-6-carboxamide
7-Amino-8-ethyl-2-(2-methoxy-4-piperid-4-ylphenylamino)-4-methyl-5-oxo-5,8-dihydropyrido[2,3-d]pyrimidine-6-carboxamide
7-Amino-2-{2-methoxy-4-[1-(3,3,3-trifluoropropyl)piperid-4-yl]phenylamino}-5-oxo-8-thiophen-2-ylmethyl-5,8-dihydropyrido[2,3-d]pyrimidine-6-carboxamide
7-Amino-8-isobutyl-2-[2-methoxy-4-(4-morpholin-4-ylpiperid-1-yl)phenylamino]-5-oxo-5,8-dihydropyrido[2,3-d]pyrimidine-6-carboxamide
7-Amino-2-(4-morpholin-4-ylphenylamino)-5-oxo-8-pyrrolidin-2-ylmethyl-5,8-dihydropyrido[2,3-d]pyrimidine-6-carboxamide
7-Amino-8-ethyl-2-{2-methoxy-4-[1-(3,3,3-trifluoropropyl)-1,2,3,6-tetrahydropyrid-4-yl]phenylamino}-5-oxo-5,8-dihydropyrido[2,3-d]pyrimidine-6-carboxamide
7-Amino-2-[4-(4-ethylpiperazin-1-yl)-2-methoxyphenylamino]-8-(2-hydroxy-2-methylpropyl)-5-oxo-, 5,8-dihydropyrido[2,3-d]pyrimidine-6-carboxamide
7-Amino-8-(2-hydroxy-2-methylpropyl)-2-{2-methoxy-4-[1-(3,3,3-trifluoropropyl)piperid-4-yl]phenylamino}-5-oxo-5,8-dihydropyrido[2,3-d]pyrimidine-6-carboxamide
7-Amino-8-(2-hydroxy-2-methylpropyl)-2-[4-(4-ethylpiperid-1-yl)-2-methoxyphenylamino]-5-oxo-5,8-dihydropyrido[2,3-d]pyrimidine-6-carboxamide
7-Amino-2-[4-(1-cyclopropylpiperid-4-yl)-2-methoxyphenylamino]-8-(2-hydroxy-2-methylpropyl)-5-oxo-5,8-dihydropyrido[2,3-d]pyrimidine-6-carboxamide
7-Amino-8-(2-hydroxy-2-methylpropyl)-2-{2-methoxy-4-[1-(2-methoxyethyl)piperid-4-yl]phenylamino}-5-oxo-5,8-dihydropyrido[2,3-d]pyrimidine-6-carboxamide
7-Amino-2-[2-methoxy-4-(1-methylpiperid-4-yl)phenylamino]-5-oxo-8-phenyl-5,8-dihydropyrido[2,3-d]pyrimidine-6-carboxamide
7-Amino-8-(3-fluorophenyl)-2-[2-methoxy-4-piperid-4-ylphenylamino]-5-oxo-5,8-dihydropyrido[2,3-d]pyrimidine-6-carboxamide
7-Amino-8-(4-fluorophenyl)-2-[2-methoxy-4-piperid-4-ylphenylamino]-5-oxo-5,8-dihydropyrido[2,3-d]pyrimidine-6-carboxamide
7-Amino-2-[2-methoxy-4-piperid-4-ylphenylamino]-5-oxo-8-m-tolyl-5,8-dihydropyrido[2,3-d]pyrimidine-6-carboxamide
7-Amino-2-[2-methoxy-4-piperid-4-ylphenylamino]-5-oxo-8-p-tolyl-5,8-dihydropyrido[2,3-d]pyrimidine-6-carboxamide
7-Amino-8-(3-methoxyphenyl)-2-(2-methoxy-4-piperid-4-ylphenylamino)-5-oxo-5,8-dihydropyrido[2,3-d]pyrimidine-6-carboxamide
7-Amino-2-[2-methoxy-4-(2-pyrrolidin-1-ylethyl)phenylamino]-5-oxo-8-phenyl-5,8-dihydropyrido[2,3-d]pyrimidine-6-carboxamide
7-Amino-8-(4-fluorophenyl)-2-[2-methoxy-4-(1-methylpiperid-4-yl)phenylamino]-5-oxo-5,8-dihydropyrido[2,3-d]pyrimidine-6-carboxamide
7-Amino-2-[2-methoxy-4-(1-methylpiperid-4-yl)phenylamino]-8-(3-methoxyphenyl)-5-oxo-5,8-dihydropyrido[2,3-d]pyrimidine-6-carboxamide
7-Amino-2-{2-methoxy-4-[1-(3,3,3-trifluoropropyl)piperid-4-yl]phenylamino}-5-oxo-8-phenyl-5,8-dihydropyrido[2,3-d]pyrimidine-6-carboxamide
7-Amino-2-[4-(4-ethylpiperazin-1-yl)-2-methoxyphenylamino]-5-oxo-8-phenyl-5,8-dihydropyrido[2,3-d]pyrimidine-6-carboxamide
in the form of base or of acid-addition salt, and also in the form of hydrate or solvate.

10. Process for preparing a compound of formula (I) according to any one of Claims 1 to 9, **characterized in that** it comprises the reaction of a compound of formula (IX) 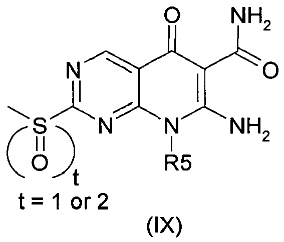 with a primary amine of formula (A): 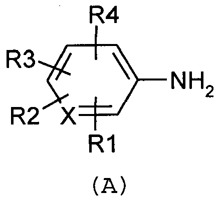 in which R₁, R₂, R₃, R₄ and R₅ are defined according to any one of Claims 1 to 9 and t represents 1 or 2.

11. Process for preparing a compound of formula (I) according to any one of Claims 1 to 9, **characterized in that** it comprises the reaction of a compound of general formula (XIV) or (XV): 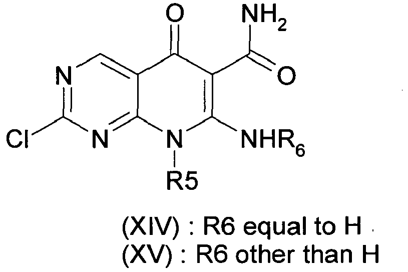 with a compound of general formula (A): 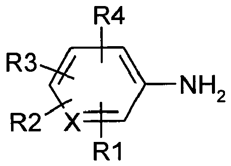 in which R₁, R₂, R₃, R₄, R₅ and R₆ are defined according to any one of Claims 1 to 9.

12. Compound of formula (VII) :

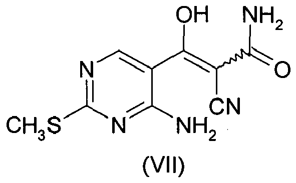

13. Compound of formula (VIII): 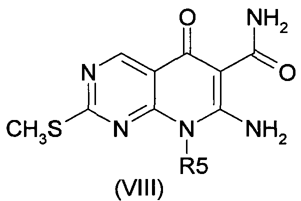 in which R₅ is defined according to any one of Claims 1 to 8.

14. Compound of formula (IX) : 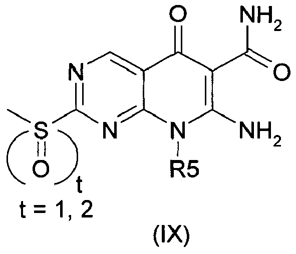 in which **R₅** is defined according to any one of Claims 1 to 8 and t represents 1 or 2.

15. Compound of formula (XV): 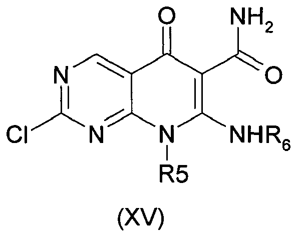 in which **R₅** and **R₆** are defined according to any one of Claims 1 to 8.

16. Medicament, **characterized in that** it comprises a compound of formula (I) according to any one of Claims 1 to 9, or an addition salt of this compound with a pharmaceutically acceptable acid, or alternatively a hydrate or a solvate of the compound of formula (I).

17. Pharmaceutical composition, **characterized in that** it comprises a compound of formula (I) according to any one of Claims 1 to 9, or a pharmaceutically acceptable salt, a hydrate or a solvate of this compound, and also at least one pharmaceutically acceptable excipient.

18. Compound of formula (I) according to any one of Claims 1 to 9, for its use in treating and/or preventing any pathology in which the kinase CaMKII is involved.

19. Compound according to Claim 18, for its use in the treatment and/or prevention of cardiovascular pathologies including myocardial infarction, ventricular hypertrophy, myocardial fibrosis, cardiac insufficiency, cardiac arrhythmia and restenosis, and also vascular pathologies associated with an endothelial dysfunction, including atherosclerosis, and also pathologies associated with the development of fibrosis, including hepatic, pancreatic, renal, pulmonary, cutaneous, intestinal and ocular fibrosis, kidney transplantation, liver transplantation, and also acute pathologies including acute renal insufficiency, acute pulmonary insufficiency and acute hepatic insufficiency, and also pathologies associated with neuronal death or degeneration, including strokes and Parkinson's disease, and also pathologies of inflammatory origin, such as chronic pain and rheumatoid arthritis.
